# EUROPEAN PATENT APPLICATION

(11) **EP 4 491 193 A2**
(43) Date of publication of application: **15.01.2025**
(21) Application number: 24195755.4
(22) Date of filing: 01.07.2020
(51) Int. Cl.: A61K 39/00

(54) **ANTI-CD154 ANTIBODIES AND USES THEREOF**

(30) Priority: 01.07.2019 US 201962869489 P; 30.04.2020 US 202063018123 P
(62) Divisional of application: 20764933.6
(71) Applicant: Tonix Pharma Limited, Dublin A96 HW25 (IE)
(72) Inventor: LEDERMAN, Seth, South Dartmouth, 02748 (US)
(74) Representative: Haley Guiliano International LLP

(57) **Abstract**

This disclosure relates to anti-human CD154 antibodies with modified effector function. This disclosure also relates to the use of these anti-human CD154 antibodies in treating conditions associated with CD154 activation, such as transplant rejection, inflammatory conditions and disease, dysfunctional immune responses associated with viral infections and diseases, autoimmune conditions and disease, allergic conditions, atherosclerotic conditions, or neurodegenerative conditions and diseases. It also relates to the use of these anti-human CD154 antibodies in inducing central tolerance and hematopoietic chimerism in transplant patients.

## Description

### Cross-Reference to Related Applications

This application claims priority from United States Provisional Application No. 62/869,489, filed July 1, 2019 and United States Provisional Application No. 63/018,123, filed April 30, 2020, the contents of which are hereby incorporated by reference in their entirety.

### Sequence Listing

The instant application contains a Sequence Listing which has been submitted electronically in ASCII format and is hereby incorporated by reference in its entirety. Said ASCII copy, created on July 1, 2020, is named 104545-0037-WO1_SL.txt and is 753,090 bytes in size.

### Field of the Disclosure

The present disclosure relates to isolated antibodies with modified (*e.g*., selectively reduced) effector functions that bind to CD154. The disclosure also relates to nucleic acid molecules encoding such antibodies, compositions comprising such antibodies and methods of using such antibodies and compositions for inhibiting an immune response, such as in the treatment of transplant rejection, inflammatory, autoimmune, dysfunctional immune responses associated with viral infections and diseases, allergic, atherosclerotic, or neurodegenerative conditions and diseases.

### Background Of The Disclosure

CD154 (also known as CD40 ligand, CD40L, gp39, TNF-related activation protein (TRAP), 5c8 antigen, T-BAM) is a protein primarily expressed on activated CD4+ T cells and is recognized as the molecular basis for T cell helper function (Lederman, S., et al. J. Exp. Med. 175: 1091-1101 (1992). CD 154 is a member of the TNF superfamily of molecules and is functionally expressed as a homotrimer. Some of the CD154 units, however, have shortened peptide chains, such that CD154 trimers can be considered heterotrimers of elements all encoded by the CD154 gene (Karpusas M, et al. Structure. 3(10):1031-9 (1995); Hsu YM, et al. J Biol Chem. 272(2):911-5 (1997)). CD154 binds to CD40 on antigen-presenting cells (APC), which leads to many effects depending on the target cell type. The primary binding partner for CD154 is CD40, although other binding partners αMβ2 (Mac-1), α5β1 integrin and αIIbβ3 have been described (El Fakhry Y, et al. J Biol Chem. 287:18055 (2012); Wolf D, et al. Circ Res. 109:1269 (2011); Michel NA, et al Front Cardiovasc Med. 4:40 (2017)). CD154 acts as a costimulatory molecule for B cells and affects the function of CD4+ T follicular helper cells (TFH cells). On TFH cells, CD154 promotes B cell maturation and function by engaging CD40 on the B cell surface and thereby facilitating cell-cell communication in a humoral immune response. CD40 triggering by CD154 stimulates adaptive immune system processes in B cells including immunoglobulin class switch recombination and somatic hypermutation (Lederman S, et al. Curr Opin Hematol. 3(1):77-86 (1996)). Absence of CD154, for example, in the X-linked Hyper IgM syndrome, leads to deficiencies in the formation of germinal centers, class switch recombination and antibody affinity maturation. (Webster EA, et al. Arthritis Rheum. 42(6):1291-6 (1999)). The CD40-CD154 interaction is involved in normal T-B cell interactions, including increased co-stimulation, T-cell priming, cytokine production, antibody-class switching and affinity maturation, and antibody production (Lederman, S., et al. J. Exp. Med. 175:1091-1101 (1992); Lederman, S., et al., Journal of Immunol. 149:3817-3826 (1992); Lederman, S., et al., Journal of Immunol. 152:2163 (1994); Cleary, A.M., et al., Journal of Immunol., 155:3329-3337 (1995); Muramatsu, MK et al. Cell 102: 553 (2000); Xu Y and Song G, J. Biomed Sci. 11(4):426-38 (2004); Quezada SA et al., Annu Rev Immunol. 22:307-28 (2004); and U.S. Patent Nos. 5,474,771; 5,933,816; 6,331,615; 6,340,459; 6,403,091; 6,451,310; 6,455,044; 6,592,868; 6,610,294; 6,793,924; 7,070,777; and 9,765,150).

CD154 also interacts with CD40 on activated endothelial cells (Yellin MJ et al, J. Exp. Med 182:1857-1864 (1995)), activated fibroblasts (Yellin, MJ et al. J Leukoc Biol. 58:209-216 (1995)), in other cell types and in many cancers (Paulie, S, et al. Cancer Immunol Immunother, 20, 23-8 (1985)). Supernatants of severe acute respiratory syndrome-associated coronavirus (SARS-CoV) infected human lung epithelial Calu-3 cells and induce CD40 on dendritic cells (Yoshikawa T, et al. J. Virol. 83(7): 3039-3048 (2009)). In the retina, during inflammation, CD40 is expressed on endothelial cells, Müller glia (macroglia in the retina), microglia, ganglion cells, and retinal pigment epithelial cells (Subauste, CS, Front Immunol 10:2958 (2019); Portillo J-AC, et al. J Immunol. 181:8719-26 (2008); Portillo J-AC, et al. Diabetologia. 57:2222-31(2014); Portillo J-AC, et al., Mol Vis. 15:1383-9 (2009)). The roles of CD154 in an immune response are normally tightly regulated in tissues over time. Dysfunctional immune responses can occur with abnormal CD154 expression and function in certain tissues and at certain times, contributing to syndromes such as acute respiratory distress syndrome (ARDS), autoimmune diseases, vasculopathies and the promotion of cancers.

The soluble form of CD154 (sCD154), which results from the shedding of membrane-bound CD154, plays a role in the production of proinflammatory cytokines and has been linked to various autoimmune and vascular disorders (Yellin, MJ, et al., J. Immunol. 152:598 (1994); Yacoub D et al., J Biol Chem. 288(50):36083-93 (2013)). Activated platelets produce CD154 and platelet derived CD154, particularly soluble CD154, has been linked to pathology. (Henn V, et al. Nature 391:591-594 (1998); Xu H, et al. Transplantation. 72(11):1858-61. (2001); Danese S, et al. Gut. 52(10):1435-41. (2003); and Charafeddine AH, et al. Am J Transplant. 12(11):3143-51 (2012)).

The monoclonal antibody 5c8 is a murine anti-human CD154 that potently blocks CD154 function. (Lederman, S., et al. J. Exp. Med. 175: 1091-1101 (1992); Lederman, S., et al., Journal of Immunol. 149:3817-3826 (1992); Lederman, S., et al., Journal of Immunol. 152:2163 (1994); and Cleary, A.M., et al., Journal of Immunol., 155:3329-3337 (1995)). A humanized anti-human CD154 IgG1 antibody (hu5c8, ruplizumab or ANTOVA^{®}) was generated and tested in non-human primates and humans. A crystal structure of hu5c8 showed the unique binding of hu5c8 to the CD154 trimer and antibody contacts with CD154 monomer (Karpusas M, et al. Structure. 9(4):321-9. (2001)). CD154 blockade has demonstrated efficacy in models of autoimmunity, humoral immunity and allotransplantation (Pierson RN 3rd, et al. Transplantation. 68(11): 1800-5 (1999); Chang AC, et al. Transplant Proc. 31(1-2):95 (1999); Kenyon NS, et al. Proc Natl Acad Sci U S A. 96(14):8132-7 (1999); Kenyon NS, et al. Diabetes. 48(7):1473-81. (1999); Elster EA, et al. Transplantation. 72(9): 1473-8. (2001); Elster EA, et al. Transplant Proc. 33(1-2):675-6 (2001); Cho CS, et al. Transplantation. 72(4):587-97 (2001); Pierson RN 3rd, et al. Immunol Res. 23(2-3):253-62 (2001); Pfeiffer S, et al. J Heart Lung Transplant. 20(2):250 (2001); Xu H, et al. Transplant Proc. 33(1-2):223-4 (2001); Xu H, Transplantation. 74(7):940-3 (2002); Crowe JE Jr, et al. Am J Transplant. 3(6):680-8 (2003); Ferrant JL, et al., International Immunol October 5;11:1583 (2004); Kawai T, et al., Am J Transplant. 4(9): 1391-8 (2004); Preston EH, et al. Am J Transplant. 5(5): 1032-41 (2005); Xu H, et al. J Immunol. 170(5):2776-82 (2003); Wu G, et al. Xenotransplantation. 12(3):197-208 (2005); Smith RN, Am J Transplant. 6(8): 1790-8 (2006); Zhang T, et al. Transplantation. 102(3):e90-e100 (2018)). However, clinical trials of hu5c8, in systemic lupus erythematosus (SLE) (Huang W, et al. Arthritis Rheum. 46(6): 1554-62 (2002); Boumpas DT, et al, Arthritis Rheum. 48:719-27. (2003); Grammer AC, et al. J Clin Invest. 112: 1506-20. (2003)) and transplantation (Kawai T, et al. Nat Med. 2000;6: 114. (2000); Koyama I, et al., Transplantation. 77(3):460-2. (2004)) were halted due to an increased incidence of platelet activation and thromboembolic events (Law and Grewal Adv Exp Med Biol. 647:8-36 (2009)). One mechanism for platelet activation may relate to CD40 on platelets and activation by soluble CD 154 (Inwald DP, et al, Circ Res. 92(9): 1041-8 (2003)). Several observations suggest that a mechanism for thrombosis in anti-CD154 treated patients is mediated by Fc gamma RIIa receptor (FcγRIIA, FCGR2A, CD32A)-dependent platelet activation by immune complexes, particularly higher ordered complexes, comprised of anti-CD154 antibodies and soluble CD154 (Robles-Carrillo L, et al., J Immunol. 185(3): 1577-83. (2010)). Eliminating Fc binding to Fc receptors by mutating the Fc region to make aglycosyl hu5c8 (IgG1 N297Q) has been shown to strongly decrease or eliminate such thromboembolism (Shock, A, et al., Arthritis Res Ther. 17:234 (2015) and Xie et al., Journal of Immunol. 192(9):4083 (2014)), but also reduced the antibody's efficacy in inhibiting or preventing transplant rejection in rhesus renal and islet allotransplantation models (Ferrant JL et al., International Immunol (11): 1583 (2004)). Entirely eliminating the Fc region, such as in an anti-CD154 pegylated Fab' antibody fragment (Dapirolizumab pegol (DZP), CDP7657, Biogen and UCB) also reduced the risk of thrombotic events but failed to treat systemic lupus erythematosus (SLE) in a Phase IIb clinical trial (Waters J, Biocentury; October 26, (2018)). Kim *et al.* generated an Fc silent anti-human CD154 single domain antibody (dAb; BMS-986004; Letolizumab, BMS2h-572-633-CT-L2) by fusing the variable domain of an anti-human CD154 dAb with the Fc from CTLA4-Ig (abatacept, ORENCIA^{®}) and a linker termed "CT Long Fc" with amino acid sequence EPKSSDK (SEQ ID NO: 325) (Kim SC et al., Am J Transplant 17(5):1182-1192 (2017); and U.S. Patent No. 9,765,150). BMS-986004 effectively prevented kidney rejection in nonhuman primates and was also devoid of platelet activation, thrombosis or thromboembolism (Kim SC et al., Am J Transplant 17(5):1182-1192 (2017).

U.S. Patent No. 9,765,150 also described BMS-986003 (BMS-2h572-633-CT), which shares the same amino acid sequence as BMS-986004, except for a non-native glycine residue at its amino-terminus. BMS-986003, however, induced anti-drug antibodies (ADA) in treated monkeys. The ADA were directed to the dAb (non-Fc) portion of the molecule and these antibodies were shown to block the binding of BMS-986003 to CD154 suggesting the ADA may be neutralizing. In addition, these ADA resulted in increased clearance of BMS- 986003 in several monkeys. In contrast, a chimeric murine 5c8 human IgG1 chimeric antibody had expected long plasma half-life. (U.S. Patent No. 9,765,150).

While BMS-986004 demonstrated efficacy in an immune thrombocytopenic purpura (ITP) clinical trial, the pharmacokinetic profile may be suboptimal (NCT02273960; "Study to Evaluate Safety and Efficacy in Adult Subjects With ITP (ITP)"; results accessed July 1, 2019).

The mechanism of anti-CD154 treatment of transplantation rejection or autoimmunity is not completely understood. Anti-CD154 therapy appears more effective than anti-CD40 therapy, suggesting that blockade of their interaction is not symmetrical. The monoclonal antibody (mAb) hu5c8 appears more effective than a different humanized anti-CD154 mAb, IDEC-131 (O'Neill NA, et al. Transplantation. 101(9):2038-2047 (2017)). Further, an increase in regulatory T cells, specifically Foxp3+ T cells, associated with anti-CD154 therapy may relate to efficacy (Ferrer IR, et al., Proc Natl Acad Sci U S A. 108(51):20701-6 (2011)). Another observation supporting a role for regulatory T cells was that dimeric soluble CD40, but not monovalent CD40 induced T regulatory cells (CD4+CD25+ T cells) and treated transplant rejection (Masunaga T, et al. Transplantation. 80:1614-1622 (2005)). Those experiments also showed that valency in CD154 ligation or blockade may be relevant to efficacy (Masunaga T, et al. Transplantation. 80:1614-1622 (2005)). Anti-CD154 treatment was shown to be particularly effective in facilitating allotransplant with mixed allogeneic chimerism (Kawai T, et al., Am J Transplant. 4(9):1391-8 (2004) and in xenotransplantation (Längin M, et al., Nature. 564(7736):430-433 (2018)).

Antibodies have a variety of effector functions mediated by the Fc including FcyR binding and complement C1q binding (the first component of complement activation), which relate to effector functions and complement dependent cytotoxicity. Engineering antibodies may result in increasing or decreasing one or more effector functions. At the extreme, the Fc can be removed, for example in a F(ab) construct, but for therapeutic effects, such constructs typically require other changes to increase half-life, such as modification with polyethylene glycol or PEGylation. Entirely eliminating the Fc region can affect an anti-CD 154 antibody's efficacy-an anti-CD 154 pegylated Fab' antibody fragment (Dapirolizumab pegol (DZP), CDP7657, Biogen and UCB) failed to treat SLE in a Phase IIb clinical trial (Waters J, Biocentury; October 26, (2018)). A "silent" N297Q IgG1 variant (asialo- or algyco-) of hu5c8 lacked FcyR binding, but was effective in inhibiting the humoral immune response, but not organ rejection in non-human primates. (Tao, M.H. and Morrison, S.L. J. Immunol. 143:2595-2601. (1989), FerrantJ.L. et al., International Immunol (11):1583 (2004)). Therefore, a solution to balancing efficacy and safety of anti-CD154 antibodies is to engineer an anti-CD154 antibody with a modified Fc with selectively decreased effector function. Prior efforts on modifying effector function have focused on substitutions in the IgG hinge/CH2 region, which can affect FcyR and C1q binding. A consideration in designing antibodies is to maintain the CH2/CH3 region of the Fc domain that is required for interactions with FcRn, the neonatal Fc receptor, which confers extended serum half-life.

Among IgG1 antibodies, substitution studies identified several residues that affect Fc binding to C1q and to various FcyRs (FcγRI/CD64, FcyRIIa/CD32a, FcyRIIb/CD32b and FcyRIIIa/CD16a and FcyRIIIb/CD16b). For example, Fc residues at positions 234 and 235 have been shown to have a modulating effect on Fc-CD64 binding (Canfield, SM and Morrison, S L J. Exp. Med. 173:1483-1491 (1991); Chappel, MS, et al. Proc. Natl Acad. Sci. USA, 88, 9036-9040 (1991); Alegre, M-L, et al., J. Immunol. 148: 3461-3468 (1992); Hezareh, M, et al. J. Virol. 75, 12161-12168 (2001), Fc-CD32A binding (Hezareh, M, et al. J. Virol. 75, 12161-12168 (2001); Armour, KL, et al., Mol. Immunol. 40, 585-593. (2003)), Fc-CD16 binding (Hezareh, M, et al. J. Virol. 75, 12161-12168 (2001)) and Fc-C1q binding (Xu, D, et al, Cell. Immunol. 200, 16-26 (2000); Hezareh, M, et al. J. Virol. 75, 12161-12168 (2001)). Also, CH2 position 331 was shown to be involved in Fc-CD64 binding (Canfield, SM and Morrison, S L J. Exp. Med. 173:1483-1491 (1991)) and Fc-C1q binding (Tao, MH, et al., J. Exp. Med. 178, 661- 667 (1993).; Hezareh, M, et al. J. Virol. 75, 12161-12168 (2001); Idusogie, EE, et al. J. Immunol. 164:4178- 4184 (2000)). As an example, affinity for FcyRs (FcγI, FcyRIIa, and FcyRIIIa) and C1q has been reduced by the substitutions in IgG1 of L234F/L235E/P331S (Oganesyan V, et al. Acta Crystallogr D Biol Crystallogr. 64 (Pt 6):700-704 (2008)). As another example, tepliczumab is a humanized OKT3 antibody with IgG1 Fc with reduced effector functions due to substitutions (L234A, L235A) that is in development for the treatment of diabetes (Xu, D, et al, Cell. Immunol. 200, 16-26. (2000); Herold KC, et al. N Engl J Med. 10.105 (2019)). Another modified (termed "silent") IgG1 Fc modification with substitutions C220S, C226S, C229S, and P238S was employed to make a fusion protein expressing CTLA4 in CTL4-Ig (Abatacept, ORENCIA^{®}) The cysteine substitutions remove the disulfide bonds between the heavy chains (HCs) in a dimer (C226S and C229S). Using this modified Fc, Kim *et al.* generated an Fc silent humanized anti-human CD154 antibody, which was a mutated version of hu5c8 ("hu5c8-mod") that contained the same modified, non-Fc binding IgG1 tail as the anti-CD154 dAb BMS-986004 (Kim SC et al., Am J Transplant 17(5):1182-1192 (2017)). U.S. Patent 9,765,150 also describes an anti-human CD154 "5c8" antibody generated by fusing part or all of the variable domain of "5c8" with the Fc from abatacept and a linker of either "CT Long" with amino acid sequence AST-EPKSSDK (SEQ ID NO: 326) or "CT Short" with amino acid sequence AS (SEQ ID NO: 214) (U.S. Patent No. 9,765,150).

Among IgG2 antibodies, affinity for FcyR has been decreased by substitutions H268Q/309L/A330S/P331S in a muted version that has been called "IgG2m4" (An, Z. et al, MAbs 1:572-579. (2009)). Another engineered Fc includes V234A/G237A/P238S/H268A/V309L/A330S/P331S substitutions that eliminate affinities for FcgR and C1q complement protein (Vafa, O. et al., Methods. 1;65(1): 114-26. (2014)).

Among IgG4 antibodies, affinity for FcyR has been decreased by the F234A and L235A substitutions. These substitutions have been combined together with the S228P substitution in the core hinge (IgG1 and IgG2 have a proline in position 228), which further stabilizes the IgG4 molecule to prevent Fab arm exchange (Silva, JP et al., J Biol Chem. 290(9):5462-5469. (2015)), in a "muted" version of IgG4, that has been called "IgG4 Pro-AlaAla" (Tao, M.H. and Morrison, S.L. J. Immunol. 143:2595-2601. (1989) and Alegre, M-L, et al., J. Immunol. 148: 3461-3468. (1992)). Dulaglutide is a fusion protein that includes a glucagon like peptide-1 (GLP-1) agonist fused to an IgG4 Fc of the "IgG4 Pro-AlaAla" type, which is marketed as TRULICITY^{®}. Others have modified IgG4 Fc with substitutions F234A and L235A with the S228P substitution (Xu, D, et al, Cell. Immunol. 200, 16-26. (2000)), or employed the substitutions L235E with the S228P substitution without modifying the F234 (Reddy MP, et al., J Immunol. 164:1925-1933. (2000) and Li, X, et al. Int J Clin Exp Med. 8(3):3607-18. (2015)).

Antibody engineering is a complex and costly technology with unpredictable results *in vivo* (Saeed AF, et al., Frontiers in Microbiology; Article 495; March (2017)). An antibody engineering strategy including structural changes or other modifications that are optimized for one antibody may compromise another antibody *in vivo* (Yan B, et al., The Journal of Biol. Chem. 287(8):5891-97 (2012)). The effect of a mutation on a particular residue (*e.g*., N297Q) in a particular antibody (*e.g*. IgG1) can have a very different clinical effect from another kind of mutation at the same antibody (*e.g*., N297A in IgG1). Moreover, the clinical effects of intramolecular changes within one subtype of antibody (*e.g*., an L235 mutation in IgG1) may be entirely unpredictable when the same intramolecular changes occur within another subtype of antibody (*e.g*. an L235 mutation within IgG4). Intramolecular changes also may result in different clinical effects, depending on how various mutations may pair up within an antibody. Species-specific differences add to the unpredictability of the effect of an Fc receptor mutation on the efficacy vs toxicity of anti-CD154 antibodies. In the past, anti-CD154 antibodies developed for use in transplantation studies did not cause thrombosis in pre-clinical rodent and certain non-human primates, but a number of human patients experienced thromboembolic complications in clinical trials with the same antibodies (i.e., ruplizumab, toralizumab, and ABI793) (Pinelli DF and Ford M.L. Immunotherapy 7(4): 399-410 (2015)).

To date, there has not been a fully human or humanized anti-CD 154 antibody that satisfies the need for a product that can effectively prevent in humans transplant rejections (including graft versus host disease), inflammatory conditions and diseases, autoimmune conditions and diseases, dysfunctional immune responses associated with viral infections and diseases, allergic conditions, atherosclerotic conditions, or neurodegenerative conditions and diseases, with an acceptable level of side effects, such as thromboembolic side effects. This highlights the need for antibodies and variations thereof, that bind to CD154 with high affinity, and inhibit the downstream effects of CD154:CD40 binding, in the absence of toxic side effects such as thrombosis.

### Summary Of The Invention

A first aspect of the present disclosure provides isolated anti-CD154 antibodies, that bind to mammalian CD154. In some embodiments, the anti-CD154 antibody comprises a human or humanized variable domain, wherein the variable domain comprises a heavy chain variable region (VH) and a light chain variable region (VL), and wherein the VH is operably linked to a human Fc domain with modified effector functions. Optionally, one or more effector functions are reduced. Optionally, one or more effector functions are eliminated. In some embodiments the antibody is divalent. In other embodiments, the anti-CD154 antibody is functionally monovalent.

A second aspect of the present disclosure provides nucleic acid molecules encoding the anti-CD154 antibodies disclosed herein. In some embodiments, separate nucleic acid molecules encode a heavy chain and a light chain of an anti-CD154 immunoglobulin. In other embodiments, the same nucleic acid molecule encodes both a heavy chain and a light chain of an anti-CD 154 immunoglobulin.

A third aspect of the present disclosure provides vectors comprising the disclosed nucleic acid molecules. In some embodiments, the vector comprises a nucleotide sequence that encodes the heavy chain of a disclosed anti-CD 154 antibody. Optionally, the vector comprises a nucleotide sequence that encodes the light chain of a disclosed antibody. The vector may comprise a nucleotide sequence encoding the heavy chain and the light chain of a disclosed anti-CD 154 antibody.

A fourth aspect of the present disclosure provides transformed cells comprising the disclosed nucleic acid molecules or the disclosed vectors.

A fifth aspect of the present disclosure provides a pharmaceutical composition comprising an anti-CD154 antibody disclosed herein and a pharmaceutically acceptable carrier. In some embodiments, the pharmaceutical composition comprises a therapeutically effective amount of the anti-CD154 antibody disclosed herein.

A sixth aspect of the present disclosure provides a method for inhibiting CD154 activity. In some embodiments, CD154 activity is inhibited by contacting CD154 with an anti-CD154 antibody disclosed herein. Optionally, CD154 is inhibited by administering an anti-CD154 antibody disclosed herein to a subject in need thereof. In some embodiments, the anti-CD154 antibody is administered in a pharmaceutical composition disclosed herein.

A seventh aspect of the present disclosure provides a method of inhibiting an immune response in a subject. In some embodiments, the immune response is inhibited by administering an anti-CD154 antibody disclosed herein to a subject in need thereof. In some embodiments, the anti-CD154 antibody is administered in a pharmaceutical composition disclosed herein. The immune response may be a humoral response, such as an antibody-mediated response. The immune response may be a cell-mediated response, such as one or more of a cytotoxic T-cell mediated immune response, a macrophage mediated response, a natural killer (NK) cell mediated immune response or a cytokine mediated response. The immune response could be a mixed humoral and cell-mediated response. The immune response may be a complement-mediated response. The immune response may be a primary response or a secondary response. The immune response may be a dysfunctional immune response, for example, an immune response to a virus that may lead to cytokine release syndrome (CRS), cytokine storm or acute respiratory distress syndrome (ARDS). The immune response may be a tissue specific immune response, such as an inflammation of the lungs, heart, or kidney, for example lung inflammation.

An eighth aspect of the present disclosure provides a method of inducing hematopoietic chimerism in a transplant recipient. In some embodiments, the method comprises administering to the recipient an anti-CD154 antibody disclosed herein, and transplanting into the recipient hematopoietic stem cells, thereby inducing hematopoietic chimerism in the recipient. In some embodiments, the anti-CD154 antibody is administered in a pharmaceutical composition disclosed herein.

A ninth aspect of the present disclosure provides a method of inducing central tolerance in a transplant recipient. In some embodiments, the method comprises administering to the recipient an anti-CD154 antibody disclosed herein, transplanting into the recipient hematopoietic stem cells, and transplanting a donor tissue into the recipient, wherein the transplanted hematopoietic stem cells produce immune cells that are tolerant of the donor tissue, thereby inducing central tolerance in the recipient. In some embodiments, the anti-CD154 antibody is administered in a pharmaceutical composition disclosed herein.

A tenth aspect of the present disclosure provides a method of inhibiting or preventing a xenotransplant rejection in a subject. In some embodiments, the method comprises administering to the subject an effective amount of an anti-CD 154 antibody disclosed herein. In some embodiments, the anti-CD154 antibody is administered in a pharmaceutical composition disclosed herein.

Particular embodiments of the disclosure are set forth in the following numbered paragraphs:
1. An isolated antibody that binds to CD 154, comprising a human or humanized variable domain, wherein the variable domain comprises a heavy chain variable region (VH) and a light chain variable region (VL), and wherein the VH is operably linked to a human Fc domain with modified effector functions.
2. The antibody of paragraph 1, wherein one or more effector functions are reduced.
3. The antibody of paragraph 1 or 2, wherein one or more effector functions are eliminated.
4. The antibody of any one of paragraphs 1-3, wherein the VH is operably linked to a human Fc region, wherein the human Fc region comprises a human hinge sequence and the human Fc domain, wherein the human hinge sequence is between the VH and the human Fc domain.
5. The antibody of paragraph 4, wherein the hinge comprises the amino acid sequence of any one of SEQ ID NOs: 76-90.
6. The antibody of any one of paragraphs 1-5, wherein the Fc domain is derived from an IgG4 Fc (or crystallizable fragment) region.
7. The antibody of paragraph 6, wherein the Fc domain comprises one or more amino acid modifications that modifies effector functions.
8. The antibody of paragraph 7, wherein the antibody comprises an amino acid modification at any one of the positions selected from the group consisting of S228, L235, G237, E318, and N297 or a combination thereof, wherein the numbering of amino acid residues is according to the EU index as set forth in Edelman.
9. The antibody of paragraph 8, wherein the antibody comprises an amino acid modification selected from the group consisting of S228P, F234A, L235A, L235E, G237A, E318A, and N297Q or a combination thereof.
10. The antibody of any one of paragraphs 1-5, wherein the Fc domain is derived from an IgG1 Fc (or crystallizable fragment) region and comprises one or more amino acid modifications that modifies effector functions.
11. The antibody of paragraph 10, wherein the antibody comprises an amino acid modification at any one of the positions selected from the group consisting of E216, R217, K218, C219, C220, C226, C229, P230, E233, L234, L235, G236, G237, P238, S239, V240, F241, K246, L251, T260, D265, V266, H268, W277, N297, E318, K322, P329, A330, P331, Q347, N348, T350, L351, K360, T366, N390, K392, T394, D399, S400, F405, Y407, K409, T411, or a combination thereof, wherein the numbering of amino acid residues is according to the EU index as set forth in Edelman.
12. The antibody of paragraph 11, wherein the antibody comprises an amino acid modification selected from the group consisting of C220S, C226S, C229S, P230S, E233P, L234A, L234F, L234V, L235A, L235E, L235V, G236E, G237A, P238S, D265S, D265A, H268Q, W277T, N297G, N297Q, N297D, N297A, E318A, K322A, P329G, P329A, A330S, P331S, Q347R, Q347E, Q347K, T350V, L351Y, K360D, K360E, T366A, T366I, T366L, T366M, T366V, N390R, N390K, N390D, K392V, K392M, K392R, K392L, K392F, K392E, T394W, D399R, D399W, D399K, S400E, S400D, S400R, S400K, F405A, F405I, F405M, F405T, F405S, F405V, F405W, Y407A, Y407I, Y407L, Y407V, K409F, K409I, K409S, K409W, T411N, T411R, T411Q, T411K, T411D, T411E, T411W, ΔE216-E222, K246R/L251E/T260R, InR234/235, InV235/236, InR236/237, InR237/238, InV238/239, InN238/239, InL238/239, InE238/239, InG238/239, InS239/240, InG240/241, InE240/241, InG240/241, InL238/239/P238Q, InE238/239/N348A, InS239/240/V266A, and InR237/238/G236A or a combination thereof.
13. The antibody of any one of paragraphs 1-5, wherein the Fc domain is derived from an IgG2 Fc (or crystallizable fragment) region.
14. The antibody of paragraph 13, wherein the antibody comprises an amino acid modification at any one of the positions selected from the group consisting of V234, G237, P238, H268, V309, A330, and P331 or a combination thereof, wherein the numbering of amino acid residues is according to the EU index as set forth in Edelman.
15. The antibody of paragraph 14, wherein the antibody comprises an amino acid modification selected from the group consisting of V234A, G237A, P238S, H268Q, H268A, V309L, A330S, and P331S, or a combination thereof.
16. The antibody of any one of paragraphs 1-5, wherein the Fc domain comprises the amino acid sequence selected from the group consisting of SEQ ID NOs: 3-9, 12-18 and 238-241.
17. The antibody according to paragraph 4, wherein the Fc region comprises the amino acid sequence selected from the group consisting of SEQ ID NOs: 21-37, 40-56 and 243-251.
18. The antibody of any one of paragraphs 1-17, wherein the VH comprises
   (a) a heavy chain CDR1 having the amino acid sequence of SEQ ID NO: 57,
   (b) a heavy chain CDR2 having the amino acid sequence of SEQ ID NO: 58, and
   (c) a heavy chain CDR3 having the amino acid sequence of SEQ ID NO: 59; and
   wherein the VL comprises
   (a) a light chain CDR1 having the amino acid sequence of SEQ ID NO: 60,
   (b) a light chain CDR2 having the amino acid sequence of SEQ ID NO: 61, and
   (c) a light chain CDR3 having the amino acid sequence of SEQ ID NO: 62.
19. The antibody of any one of paragraphs 1-18, wherein the VH comprises the amino acid sequence selected from the group consisting of SEQ ID NO: 63, 64, 252 and 253.
20. The antibody of any one of paragraphs 1-19, wherein the VL comprises the amino acid sequence of SEQ ID NO: 65 or 66.
21. The antibody of any one of paragraphs 4-20, wherein the antibody further comprises a CH1 domain, wherein the CH1 domain is operably linked to
   (a) the C-terminal end of the VH, and
   (b) the N-terminal end of the hinge.
22. The antibody of paragraph 21, wherein the CH1 domain comprises an amino acid sequence that is at least 80% identical to the amino acid sequence of any one of SEQ ID NOs: 67, 70, and 73.
23. The antibody of any one of paragraphs 1-22, wherein the antibody comprises a linker between the VH and the Fc domain.
24. The antibody of any one of paragraphs 4-22, wherein the antibody comprises a linker between the VH and the hinge.
25. The antibody of paragraph 21 or 22, wherein the antibody comprises a linker between the VH and the CH1 domain.
26. The antibody of any one of paragraphs 23-25, wherein the linker comprises the amino acid sequence of any one of SEQ ID NOs: 199-223 and 327-330.
27. The antibody of any one of paragraphs 1-26, wherein the VH is operably linked to the amino acid sequence of any one of SEQ ID NOs: 3-9, 12-18, 21-37, 40-56 and 238-241 and 243-251.
28. The antibody of any one of paragraphs 1-27, wherein the heavy chain comprises the amino acid sequence of any one of SEQ ID NOs: 121-132, 135-146, 149, 151-160, 163, 165-174, 266-277, and 279-288.
29. The antibody of any one of paragraphs 1-28, wherein the light chain comprises the amino acid sequence of SEQ ID NO: 195 or 196.
30. The antibody of any one of paragraphs 1-29, wherein the antibody is monoclonal.
31. The antibody of any one of paragraphs 1-30, wherein the antibody is chimeric.
32. The antibody of any one of paragraphs 1-31, wherein the antibody is humanized.
33. The antibody of any one of paragraphs 1-17, 21-27 and 30, wherein the antibody is human.
34. The antibody of any one of paragraphs 1-33, wherein the binding of the antibody to human CD154 inhibits the interaction between human CD154 and human CD40.
35. The antibody of any one of paragraphs 1-34, wherein the antibody blocks the activation of one or more of B cells, macrophages, dendritic cells, or endothelial cells by inhibiting binding of CD 154 to CD40.
36. The antibody of any one of paragraphs 1-35, wherein the antibody has one or more of the following effects when administered to a subject:
   (a) decreased risk of thrombosis or thromboembolic events compared to hu5c8 antibody;
   (b) decreased activation of platelets expressing CD154;
   (c) inhibition of CD154 shedding; and
   (d) alteration of the expression or activity of downstream targets of CD154-CD40 signaling.
37. The antibody of any one of paragraphs 1-36, wherein the antibody has a K_{D} of less than 50 pM for CD154, such as 5-25 pM or 9.5-23 pM.
38. The antibody of any one of paragraphs 1-37, wherein the antibody does not comprise the amino acid sequence consisting of any one of SEQ ID NOs: 119, 120, 133, 134, 147, 148, 150, 161, 162, 164, 230, 234 and 278.
39. An isolated nucleic acid molecule encoding a light chain and a heavy chain of an anti-CD154 antibody of any one of paragraphs 1-38.
40. An isolated nucleic acid molecule encoding an anti-CD 154 antibody comprising the amino acid sequence of any one of SEQ ID NOs: 121-132, 135-146, 149, 151-160, 163, 165-174, 195, 196, 266-277, and 279-288.
41. A first isolated nucleic acid molecule and a second isolated nucleic acid molecule, wherein the first isolated nucleic acid molecule encodes a heavy chain comprising the amino acid sequence of any one of SEQ ID NOs: 121-132, 135-146, 149, 151-160, 163, 165-174, 266-277 and 279-288 and the second isolated nucleic acid molecule encodes a light chain comprising the amino acid sequence of SEQ ID NO: 195 or 196.
42. A first isolated nucleic acid molecule and a second isolated nucleic acid molecule, wherein the first isolated and second nucleic acid molecules encode, respectively a heavy chain and a light chain of an anti-CD154 antibody of any one of paragraphs 1-38.
43. A vector comprising the isolated nucleic acid molecule according to paragraph 39 or 40.
44. A first vector and a second vector, wherein the first vector comprises the first isolated nucleic acid molecule according to paragraph 41 or 42, and the second vector comprises the second isolated nucleic acid molecule according to paragraph 41 or 42.
45. A transformed cell comprising the isolated nucleic acid molecule according to paragraph 39 or 40, the first and second isolated nucleic acid molecules according to paragraph 41 or 42, the vector according to paragraph 43, or the first and second vectors according to paragraph 44.
46. A pharmaceutical composition comprising an anti-CD154 antibody of any one of paragraphs 1-38 and a pharmaceutically acceptable carrier.
47. A pharmaceutical composition comprising the isolated nucleic acid molecule according to paragraph 39 or 40, the first and second isolated nucleic acid molecules according to paragraph 41 or 42, the vector according to paragraph 43, or the first and second vectors according to paragraph 44, and a pharmaceutically acceptable excipient.
48. A pharmaceutical composition comprising the transformed cell according to paragraph 45 and a pharmaceutically acceptable excipient.
49. A method of inhibiting an immune response in a subject comprising administering to the subject a therapeutically effective amount of an antibody of any one of paragraphs 1-38 or the pharmaceutical composition of paragraph any one of paragraphs 46-48.
50. The method of paragraph 49, wherein the immune response is a humoral response.
51. The method of paragraph 50, wherein the immune response is an antibody-mediated response.
52. The method of paragraph 49, wherein the immune response is a cell-mediated response.
53. The method of paragraph 52, wherein the cell-mediated response is one or more of a cytotoxic T-cell mediated immune response, a macrophage mediated response, a natural killer (NK) cell mediated immune response or a cytokine mediated response.
54. The method of paragraph 49, wherein the immune response is a mixed humoral and cell-mediated response.
55. The method of paragraph 54, wherein the mixed response is one or more of an antibody-mediated response, a cytotoxic T-cell mediated immune response, a macrophage mediated response, a natural killer (NK) cell mediated immune response or a cytokine mediated response.
56. The method of any one of paragraphs 49-55, wherein the subject is human.
57. The method of any one of paragraphs 49-55, wherein the subject is non-human.
58. The method of paragraph 57, wherein the subject is a monkey.
59. The method of any one of paragraphs 49-58, wherein the subject has received or will receive a cell, tissue or organ transplant.
60. The method of paragraph 59, wherein the transplant is an allogeneic transplant, autologous transplant or a xenogeneic transplant.
61. The method of paragraph 59 or 60, wherein the cell is an engineered cell or an ex-vivo expanded cell.
62. The method of paragraph 61, wherein one or more genes in the cell is modified using one or more techniques selected from a group consisting of transduction to express a cDNA, a CRISPR/Cas9 system, RNAi technology and retroviral technology.
63. The method of paragraphs 61 or 62, wherein the cell is modified to express a chimeric antigen receptor (CAR) on its surface.
64. The method of any one of paragraphs 59-63, wherein the cell is selected from a group consisting of a stem cell, a regulatory T (Treg) cell, a CAR-T cell, a CAR-B cell, a tumor-infiltrating lymphocyte (TIL).
65. The method of any one of paragraphs 59-64, wherein the method comprises treating or preventing a transplant rejection in the subject.
66. The method of paragraph 65, wherein the transplant rejection is an acute or a chronic humoral rejection of a grafted cell, tissue, or organ.
67. The method of paragraph 65 or 66, wherein the transplant rejection is an acute or chronic graft rejection in a graft recipient of an allogeneic transplant or xenotransplant.
68. The method of paragraph 66 or 67, wherein the method promotes a long-term graft survival of the grafted cell, tissue or organ, wherein said long-term graft survival is selected from the group consisting of
   (a) at least 6 months post-transplant;
   (b) at least 1year post-transplant; and
   (c) at least 5 years post-transplant.
69. The method of any one of paragraphs 65-68, wherein the transplant rejection is associated with a hematopoietic cell or bone marrow transplant, an allogeneic transplant of pancreatic islet cells, graft vs host disease, or a solid organ transplant selected from the group consisting of a heart transplant, a kidney transplant, a liver transplant, a lung transplant, a pancreas transplant, a kidney-pancreas transplant, a heart-lung transplant, kidney-heart transplant, a kidney-heart-pancreas transplant, a heart-liver transplant, a heart-liver-kidney transplant, a heart-lung-kidney transplant, a heart-lung-liver transplant, a lung-kidney transplant, a lung-liver transplant, a liver-intestines-pancreas transplant, an intestines-pancreas transplant, a liver-kidney-intestines-pancreas transplant, and a kidney-intestines transplant.
70. The method of any one of paragraphs 56-58, wherein the subject has an immune-related disease, atherosclerotic disorder, or neurodegenerative disorder.
71. The method of any one of paragraphs 56-58 and 70, wherein the subject had or is at risk of having a stroke, a transient ischemic attack (TIA), an aneurysm, or a dissecting aneurysm.
72. The method of paragraph 70, wherein the immune-related disease is selected from a group consisting of type I diabetes, juvenile diabetes, autoimmune diabetes, autoimmune hemolytic anemia, rheumatoid arthritis, systemic lupus erythematosus (SLE), psoriasis, multiple sclerosis, inflammatory bowel disease, Addison's disease, Crohn's disease, Graves' disease, Sjögren's syndrome, Hashimoto's thyroiditis, myasthenia gravis, vasculitis, pernicious anemia, celiac diseases, Guillain-Barre syndrome, ankylosing spondylitis, primary biliary cirrhosis, lupus nephritis, Goodpasture's disease, polymyositis, dermatomyositis, psoriasis, temporal arteritis, Churg-Strauss syndrome, transverse myelitis, thyroiditis, ulcerative colitis, sarcoidosis, hemolytic anemia, idiopathic thrombocytopenic purpura, neuromyelitis optica spectrum disorder, paroxysmal nocturnal hemoglobinuria, atypical hemolytic uremic syndrome, Behcet's disease, diabetic retinopathy (DR), diabetic macular edema (DME), wet age-related macular degeneration (AMD), and macular edema following retinal vein occlusion (MEfRVO).
73. The method of paragraph 70, wherein the immune-related disease is selected from a group consisting of severe acute respiratory syndrome (SARS), Middle East respiratory syndrome (MERS), coronavirus disease 2019 (COVID-19), cytokine release syndrome (CRS), and cytokine storm syndrome.
74. The method of paragraph 70, wherein the immune-related disease is selected from a group consisting of acute respiratory distress syndrome (ARDS), pneumonia, bronchitis, pneumonitis, and chronic obstructive pulmonary disease (COPD).
75. The method of paragraph 70, wherein the neurodegenerative disorder is selected from a group consisting of Alzheimer's disease, traumatic brain injury (TBI), chronic traumatic encephalitis (CTE), amyotrophic lateral sclerosis (ALS) and Parkinson's disease.
76. The method of paragraph 70, wherein the atherosclerotic disorder is selected from a group consisting of angina pectoris, myocardial infarction, carotid stenosis, transient ischemic attacks and cerebral vascular accident (CVA).
77. The method according to paragraph 70, wherein the immune-related disease is an allergic condition.
78. The method according to paragraph 77, wherein the allergic condition is selected from the group consisting of allergic rhinitis, asthma, atopic eczema, anaphylaxis, insect venom allergy, drug allergy, and food allergy.
79. The method of paragraph 49, wherein the immune response is a primary response or a secondary response.
80. The method of any one of paragraphs 49-79, wherein the antibody is administered systemically.
81. The method of paragraph 80, wherein the anti-CD 154 antibody is administered subcutaneously, intravenously, intravitreally, orally, via inhalation, transdermally, or rectally.
82. The method of any one of paragraphs 49-79, wherein the antibody is administered locally.
83. The method of any one of paragraphs 49-82, wherein the anti-CD 154 antibody is administered in combination with one or more additional agents selected from the group consisting of anti-thrombotic drugs, anti-platelet drugs, non-steroidal anti-inflammatory drugs (NSAIDs) and anti-allergy drugs.
84. The method of paragraph 83, wherein the anti-CD154 antibody is administered prior to, subsequently to, or simultaneously with the one or more additional agents.
85. The method of paragraph 83 or 84, wherein the anti-thrombotic drug is selected from a group consisting of a glycoprotein IIb/IIIa receptor antagonist, a direct or indirect factor Xa inhibitor and an anticoagulant.
86. The method of paragraph 85, wherein the anticoagulant is selected from a group consisting of heparin, warfarin, rivaroxaban (XARELTO^{®}), ximelgatran (EXANTA^{®}), dabigatran (PRADAXA^{®}), apixaban (ELIQUIS^{®}), edoxaban (SAVAYSA^{®}), enoxaparin (LOVENOX^{®}), and fondaparinux (ARIXTRA^{®}).
87. The method of paragraph 85, wherein the glycoprotein IIb/IIIa receptor antagonist is selected from a group consisting abciximab (REOPRO^{®}), rivaroxaban (XARELTO^{®}), apixaban (ELIQUIS^{®}), edoxaban (SAVAYSA^{®}), idrabiotaparinux, tirofiban (AGGRASTAT^{®}), and eptifibatide (INTEGRILIN^{®}).
88. The method of paragraph 85, wherein the direct or indirect factor Xa inhibitor is selected from a group consisting of apixaban (ELIQUIS^{®}), idrabiotaparinux, fondaparinux ARIXTRA^{®}), and rivaroxaban (XARELTO^{®}).
89. The method of paragraph 83 or 84, wherein the anti-platelet drug is selected from the group consisting of drugs inhibiting the TXA2 pathway, adenosine diphosphate (ADP) pathway inhibitors, thrombin inhibitors, Protease activated receptor-1 (PAR-1) inhibitors and phosphodiesterase (PDE) inhibitors.
90. The method of paragraph 89, wherein the ADP pathway inhibitor is selected from a group consisting of clopidogrel (PLAVIX^{®}), ticlopidine (TICLID^{®}), prasugrel (EFFIENT^{®}), ticagrelor (BRILINTA^{®}), cangrelor (KENGREAL^{®}) and elinogrel.
91. The method of paragraph 89, wherein the PDE inhibitor is selected from a group consisting of dipyridamole (PERSANTINE^{®}) and cilostazol (PLETAL^{®}).
92. The method of paragraph 83 or 84, wherein the NSAID is selected from the group consisting of acetylsalicylic acid (aspirin), celecoxib (CELEBREX^{®}), diclofenac (VOLTAREN^{®}, PENNSAID^{®}, SOLARAZE^{®}, ZIPSOR^{®}, CATAFLAM^{®}, ZORVOLEX^{®}), diflunisal (DOLOBID^{®}), etodolac (LODINE^{®} SR, ECCOXOLAC^{®}), ibuprofen (BRUFEN^{®}, ADVIL^{®}, MOTRIN^{®}), indomethacin (INDOCIN^{®}), ketoprofen (ORUDIS^{®}), ketorolac (TORADOL^{®}, ACULAR^{®}, SPRIX^{®}), nabumetone (RELAFEN^{®}), naproxen (AFLAXEN^{®}, ALEVE^{®}, ANAPROX^{®}, NAPRELAN^{®}), oxaprozin (DAYPRO^{®}), DAYRUN^{®}, DURAPROX^{®}), piroxicam (FELDENE^{®}), salsalate (MONO-GESIC^{®}, SALFLEX^{®}, DISALCID^{®}, SALSITAB^{®}), sulindac (CLINORIL^{®}), tolmetin (TOLECTIN^{®}) and prasugrel (EFFIENT^{®}).
93. The method of any one of paragraphs 49-92, wherein the anti-CD 154 antibody is administered in combination with one or more supplemental agents selected from the group consisting of immunosuppressive drugs, immunomodulatory drugs, anti-CD2 antibodies, anti-CD3 antibodies, anti-CD4 antibodies, anti-CD28 antibodies, anti-CD52 antibodies, mTOR inhibitors, calcineurin inhibitors, and antiviral drugs.
94. The method of paragraph 93, wherein the anti-CD154 antibody is administered prior to, subsequently to, or simultaneously with the one or more supplemental agents.
95. The method of paragraph 93 or 94, wherein the immunosuppressive drug or immunomodulatory drug is selected from the group consisting of cyclosporine A, tacrolimus (FK-506), doxorubicin (ADRIAMYCIN^{®}), azathioprine (IMURAN^{®}), busulfan (BUSULFEX^{®}), cyclophosphamide (CYTOXAN^{®}), fludarabine, 5- fluorouracil, methotrexate (OTREXUP^{®}, RASUVO^{®}, RHEUMATREX^{®}, TREXALL^{®}), mycophenolate mofetil (CELLCEPT^{®}), mizoribine (BREDININ^{™}), leflunomide, a nonsteroidal anti-inflammatory, adrenocortical steroids, rapamycin (RAPAMUNE^{®}), deoxyspergualin, FTY720, muromonab-CD3 (ORTHOCLONE OKT3^{®}), alemtuzumab (CAMPATH^{®}, MABCAMPATH^{®}, CAMPATH-1H^{®}, LEMTRADA^{®}), basiliximab (SIMULECT^{®}), daclizumab (ZINBRYTA^{®}), eculizumab (SOLIRIS^{®}), rituximab (RITUXAN^{®}, MABTHERA^{®}), bortezomib (VELCADE^{®}, CHEMOBORT^{®}, BORTECAD^{®}), siplizumab, anti-thymocyte globulin (THYMOGLOBULIN^{®}, ATGAM^{®}), leronlimab, siltuximbab (SYLVANT^{®}), sarilumab (KEVZARA^{®}), tocilizumab (ACTEMRA^{®}), bevacizumab (AVASTIN^{®}), ranibizumab (LUCENTIS^{®}), aflibercept (EYLEA^{®}) and inhibitors of Bruton's tyrosine kinase (BTK), including zanubrutinib (BRUKINSA^{®}), acalabrutinib (CALQUENCE^{®}) and ibrutinib (IMBRUVICA^{®}).
96. The method of paragraph 93 or 94, wherein the mTOR inhibitor is selected from the group consisting of rapamycin (RAPAMUNE^{®}), everolimus (AFINITOR^{®}), temsirolimus (TORISEL^{®}), ridaforolimus, and deforolimus.
97. The method of paragraph 93 or 94, wherein the calcineurin inhibitor is selected from the group consisting of cyclosporine (NEORAL^{®}, SANDIMMUNE^{®}, GENGRAF^{®}, RESTASIS^{®}), tacrolimus (FK506, ENVARSUS^{®}, HECORIA^{®}, PROGRAF^{®} PROTOPIC^{®}, ASTRAGRAF^{®}), and pimecrolimus (ELIDEL^{®}).
98. The method of paragraph 93 or 94, wherein the antiviral drug is selected from the group consisting of ribavirin, interferon (alfacon-1), chloroquine, hydroxychloroquine, EIDD-2801, EIDD-1931, GS-5734, GS-441524, ivermectin, favipiravir, indomethacin, chlorpromazine, penciclovir, nafomostat, nitazoxanide and remdesivir.
99. A method of inducing hematopoietic chimerism in a transplant recipient, the method consisting of administering to the recipient one or more doses of an anti-CD154 antibody, and transplanting into the recipient hematopoietic stem cells, thereby inducing hematopoietic chimerism in the recipient,
   wherein the anti-CD154 antibody is an anti-CD154 antibody according to any one of paragraphs 1-38.
100. The method of paragraph 99, wherein the anti-CD154 antibody is administered prior to, subsequently to or simultaneously with the transplantation of the hematopoietic stem cells.
101. The method of paragraph 99 or 100, wherein the anti-CD 154 antibody is administered at a dose of 5-50 mg/kg.
102. The method of any one of paragraphs 99-101, wherein the anti-CD 154 antibody is administered subcutaneously, intravenously, intravitreally, orally, via inhalation, transdermally, or rectally.
103. The method of any one of paragraphs 99-102, wherein the method further comprises a step of conditioning the transplant recipient prior to the transplantation with stem cells.
104. The method of paragraph 103, wherein the anti-CD154 antibody is administered prior to, subsequent to, or simultaneously with the conditioning step.
105. The method of paragraph 103 or 104, wherein the conditioning step is selected from a group consisting of total body irradiation, administration of one or more BCL-2 inhibitors, administration of busulfan, administration of fludarabine phosphate, administration of cyclophosphamide, administration of immunosuppressive one or more T cell-depleting antibodies, administration of cyclosporine A (CsA), administration of tacrolimus (FK-506), administration of one or more interleukin-2 (IL-2) receptor inhibitors, administration of IL-15 receptor inhibitors, administration of rapamycin, administration of one or more anti-αβ T cell receptor antibodies, and administration of one or more CD 122 antagonists, administration of kidney donor-derived CD34+ hematopoietic stem cells and CD3+ T-cells (MDR-101 cellular therapy) or a combination thereof.
106. The method of paragraph 105, wherein the one or more T cell-depleting antibodies are selected from the group consisting of anti-CD4, anti-CD8, anti-CD45, anti-CTLA4, anti-CD20, and anti-CD33 antibodies or a combination thereof.
107. The method of paragraph any one of paragraphs 99-106, wherein the transplant recipient has cancer.
108. The method of paragraph any one of paragraphs 99-107, wherein the transplant is a bone marrow transplant.
109. A method of inducing central tolerance in a transplant recipient, the method comprising administering to the recipient one or more doses of an anti-CD 154 antibody, transplanting into the recipient hematopoietic stem cells, and transplanting a donor tissue into the recipient, wherein the hematopoietic stem cells produce immune cells that are tolerant of the donor tissue, thereby inducing central tolerance in the recipient, and
   wherein the anti-CD154 antibody is an anti-CD154 antibody according to any one of paragraphs 1-38.
110. The method of paragraph 109, wherein the anti-CD154 antibody is administered prior to, subsequently to or simultaneously with the transplantation of the hematopoietic stem cells.
111. The method of paragraph 109 or 110, wherein the anti-CD154 antibody is administered at a dose of 5-50 mg/kg.
112. The method of any one of paragraphs 109-111, wherein the anti-CD154 antibody is administered subcutaneously, intravenously, intravitreally, orally, via inhalation, transdermally, or rectally.
113. The method of any one of paragraphs 109-112, wherein the method further comprises one or more treatments to condition the recipient for hematopoietic stem cell transplantation.
114. The method of paragraph 113, wherein the one or more treatments to condition the recipient for hematopoietic stem cell transplantation is selected from a group consisting of total body irradiation, administration of abatacept, administration of one or more BCL-2 inhibitors, administration of busulfan, administration of fludarabine phosphate, administration of cyclophosphamide, administration of one or more immunosuppressive T cell-depleting antibodies, administration of cyclosporine A (CsA), administration of FK-506, administration of one or more interleukin-2 (IL-2) inhibitors, administration of rapamycin, administration of one or more anti-αβ T cell receptor antibodies, and administration of one or more CD 122 antagonists or a combination thereof.
115. The method of paragraph 114, wherein the one or more T cell-depleting antibodies are selected from the group consisting of anti-CD4, anti-CD8, anti-CD45, anti-CTLA4, anti-CD20, and anti-CD33 antibodies or a combination thereof.
116. A method of inhibiting xenotransplant rejection in a subject, comprising administering to the subject an effective amount of an anti-CD154 antibody, wherein the anti-CD154 antibody is an anti-CD154 antibody according to any one of paragraphs 1-38.
117. The method of paragraph 116, wherein the anti-CD 154 antibody is administered at a dose of 5-50 mg/kg.
118. The method of paragraph 116 or 117, wherein the xenotransplant is from a non-human donor selected from a group consisting of a pig, a mini-swine, and a non-human primate.
119. The method of paragraph 118, wherein the non-human donor is a pig or mini-swine that has been engineered to decrease or eliminate expression of one or more genes selected from the group consisting of porcine endogenous retroviruses (PERV), α-1,3-galactosyltransferase (GGTA1), cytidine monophosphate-N-acetylneuraminic acid hydroxylase (CMAH), β1,4-N-acetylgalactosaminyltransferase (β4GalNT2), and MHC class I.
120. The method of paragraph 119, wherein the PERV is a PERV A, a PERV B, or a PERV C.
121. The method of paragraph 119 or 120, wherein the expression of all PERV genes has been eliminated in the pig or mini-swine.
122. The method of any one of paragraphs 119-121, wherein the expression of the one or more genes is decreased or eliminated using CRISPR/Cas9 gene editing.
123. The method of any one of paragraphs 118-122, wherein the non-human donor has been engineered to express one or more human proteins selected from the group consisting of a complement regulatory protein, human α-galactosidase, a coagulation regulatory protein, a human anti-inflammatory protein, and human CTLA-4-Ig or a combination thereof.
124. The method of paragraph 123, wherein the one or more human proteins are expressed in all tissues of the non-human donor.
125. The method of paragraph 123, wherein the one or more human proteins are expressed in a tissue-specific manner in the non-human donor.
126. The method of any one of paragraphs 123-125, wherein the complement regulatory protein is selected from the group consisting of human decay-accelerating factor (CD55), membrane cofactor protein (CD46) and CD59.
127. The method of any one of paragraphs 123-125, wherein the coagulation regulatory proteins are selected from the group consisting of thrombomodulin, endothelial protein C receptor, tissue factor pathway inhibitor, CD39, and CD73.
128. The method of any one of paragraphs 123-125, wherein the human anti-inflammatory proteins are selected from the group consisting of hemeoxygenase-1 (HO-1) and A20.
129. The method of any one of paragraphs 116-128, wherein the xenotransplant rejection is associated with a solid organ transplant selected from the group consisting of a heart transplant, a kidney transplant, a liver transplant, a lung transplant, a pancreas transplant, a kidney-pancreas transplant, a heart-lung transplant, kidney-heart transplant, a kidney-heart-pancreas transplant, a heart-liver transplant, a heart-liver-kidney transplant, a heart-lung-kidney transplant, a heart-lung-liver transplant, a lung-kidney transplant, a lung-liver transplant, a liver-intestines-pancreas transplant, an intestines-pancreas transplant, a liver-kidney-intestines-pancreas transplant, and a kidney-intestines transplant.
130. The method of paragraph 49, wherein the immune response is complement-mediated.
131. The method of any one of paragraphs 49-98, wherein the anti-CD 154 antibody is administered in combination with one or more fusion peptides that bind to and block the function of CD28, optionally selected from the group consisting of abatacept and belatocept (NULOJIX^{®}).

### Brief Description Of The Drawings

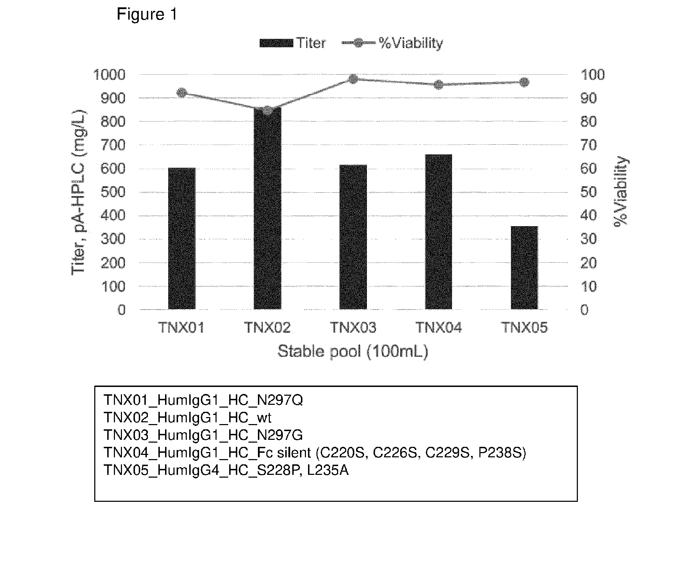

Figure 1 provides the titer and viability (%) of the five stable pools of CHO cells expressing anti-CD154 mAb (TNX01-TNX05) in 100 mL fed-batch production.

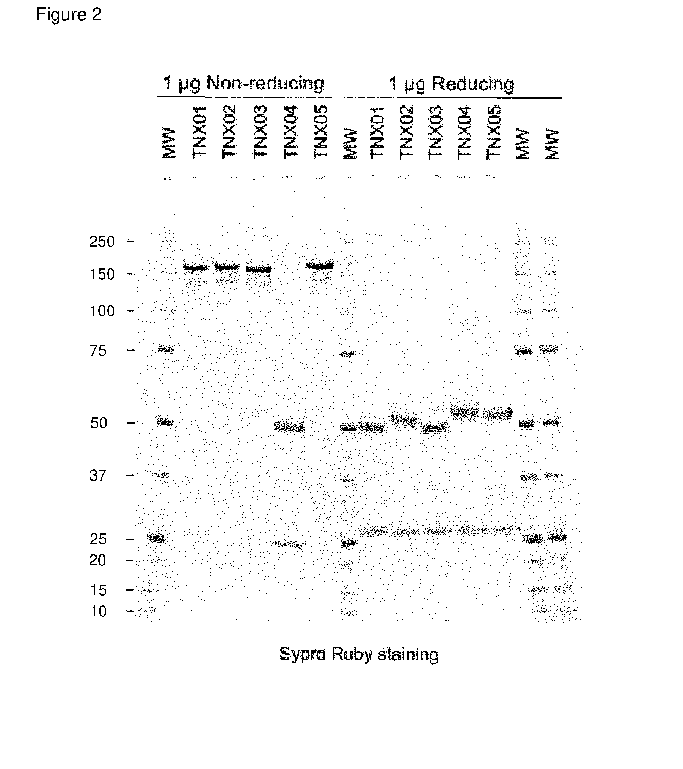

Figure 2 provides SDS-PAGE analysis of each of anti-CD154 mAbs (TNX01-TNX05) obtained during the process of protein generation and purification. MW is the protein standard which indicates the molecular weight of each band obtained on the Sypro Ruby stained gel under non-reducing conditions. The TNX04 antibody appears as two separate bands on the non-reducing gel because it contains Cys-Ser substitutions that disrupt the disulfide bonds that connect the heavy chain and light chain and that connect the two heavy chains.

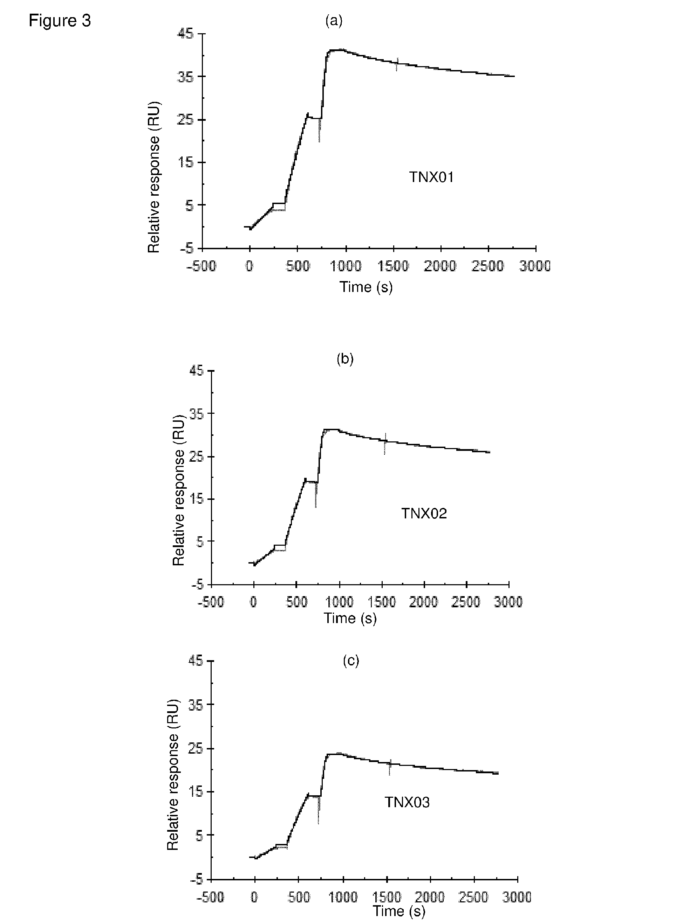

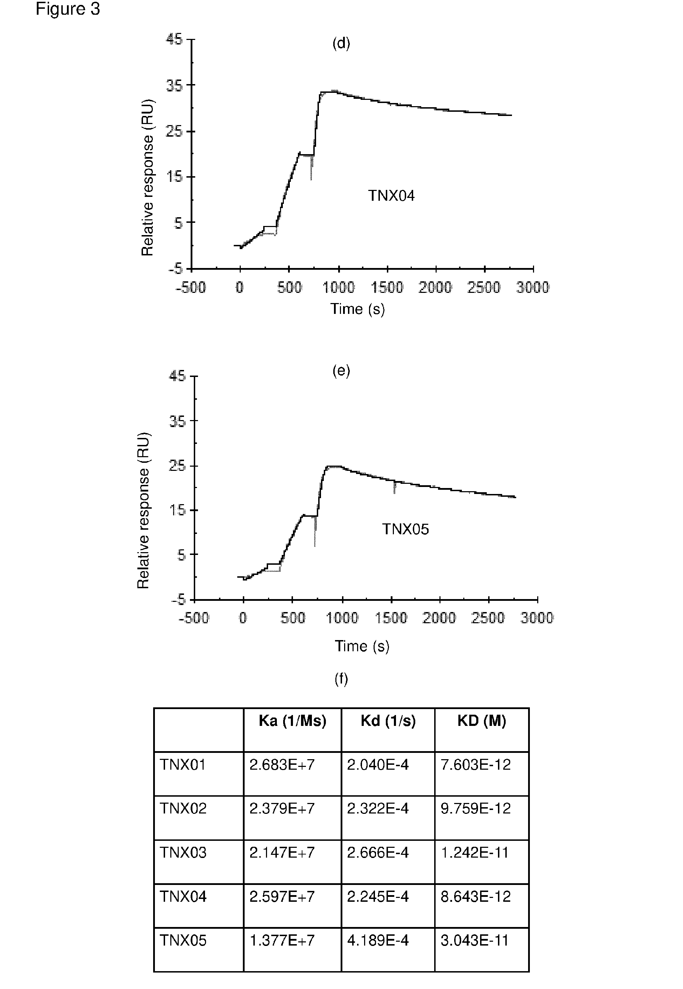

Figures 3(a)-3(e) provide representative CD154 sensorgrams for TNX01-TNX05, respectively, binding to cognate antigen CD154. Avidity measurements of purified anti-CD154 antibodies were performed by surface plasmon resonance (SPR) using the BIACORE^{®} T200 instrument and a sensorchip CM-5. The change in refractive index was plotted as response in resonance units (RUs) versus time in the sensorgram using the Biacore T200 Evaluation software. For each sensorgram, the CD154 binding data was fit using a 1:1 binding model. Figure 3(f) summarizes the resulting Ka (1/Ms), Kd (1/s) and KD (M) values.

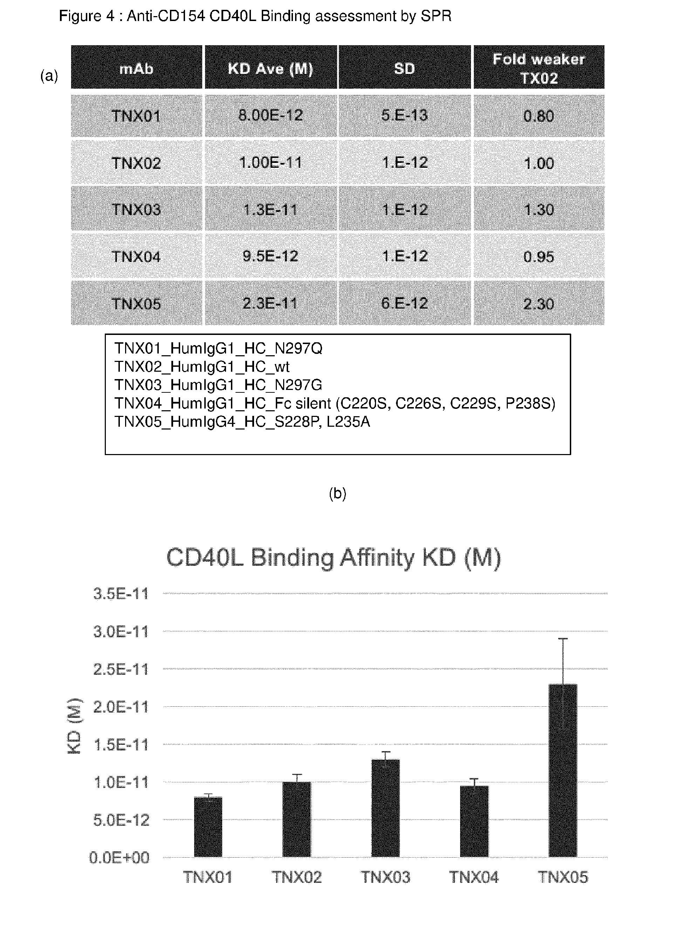

Figures 4(a) and 4(b) provide the kinetic results based on the 1:1 binding model fit to the sensorgrams of Figure 3. Average values for sCD40L (i.e. sCD154) Binding Affinity KD (M) are shown with standard deviations (n=3) for each of the TNX01-TNX05 anti-CD154 antibodies.

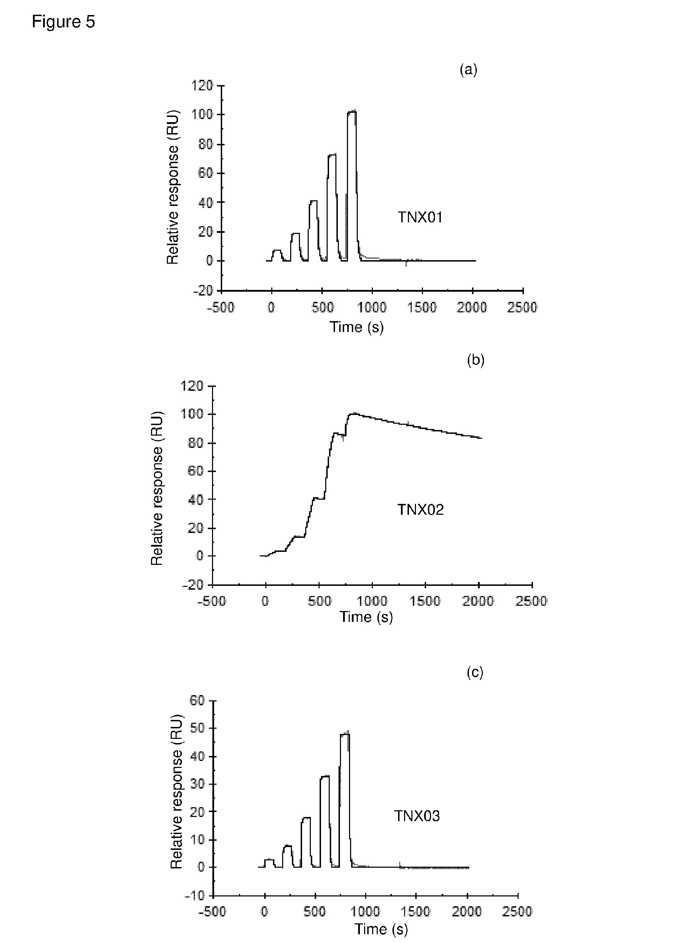

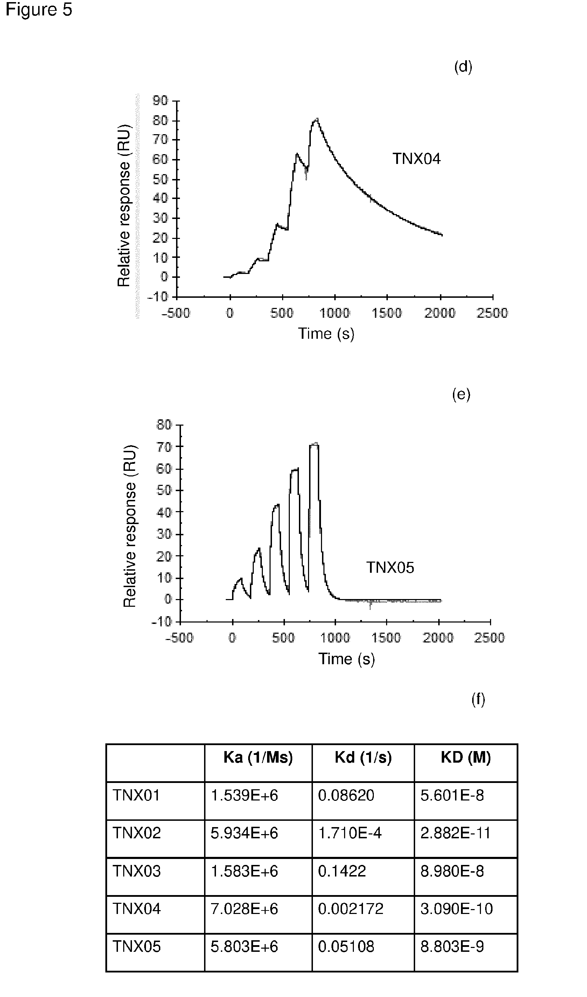

Figures 5(a)-5(e) provide representative CD154 sensorgrams for TNX01-TNX05, respectively, binding to FcyRIA (CD64). Avidity measurements of purified anti-CD154 antibodies were performed by surface plasmon resonance (SPR) using the BIACORE^{®} T200 instrument and a sensorchip CM-5. The change in refractive index was plotted as response in resonance units (RUs) versus time in the sensorgram using the Biacore T200 Evaluation software. For each sensorgram, the CD154 binding data was fit using a 1:1 binding model. Figure 5(f) summarizes the resulting Ka (1/Ms), Kd (1/s) and KD (M) values.

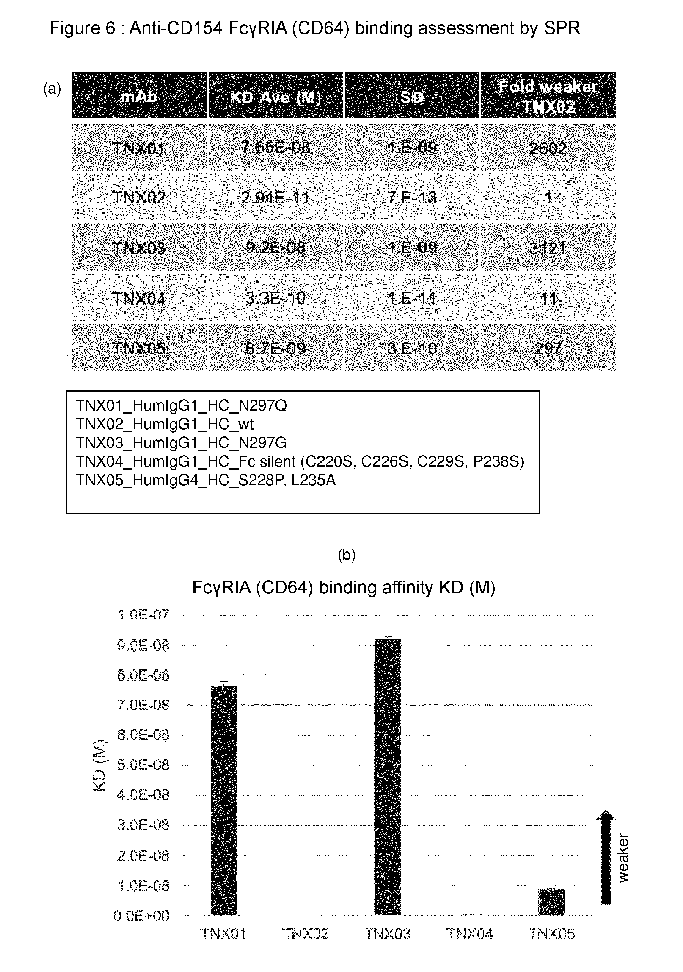

Figures 6(a) and 6(b) provide the kinetic results based on the 1:1 binding model fit to the sensorgrams of Figure 5. Average values for FcyRIA (CD64) Binding Affinity KD (M) are shown with standard deviations (n=3). A lower KD value indicates tighter binding between the antibody and FcyRIA, such that TNX02 binds FcyRIA the tightest, followed by (in order) TNX04, TNX05, TNX01, and TNX03.

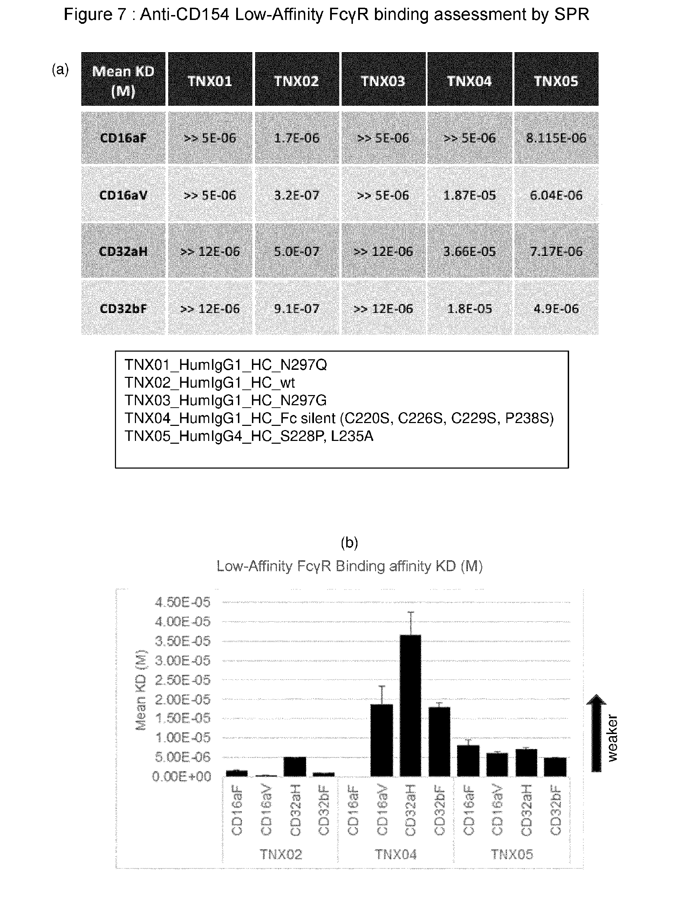

Figures 7(a) and 7(b) provide the kinetic results based on the 1:1 binding model fit to the sensorgrams of Figure 3. Average values for binding affinity of TNX02, TNX04, and TNX05 KD (M) to a low-affinity FcyR panel (CD16aF, CD16aV, CD32aH, CD32bF) are shown with standard deviations (n=3). The KD values for TNX01 and TNX03 could not be determined because these variants showed negligible binding activity. A lower KD value indicates tighter binding between the antibody and FcyR.

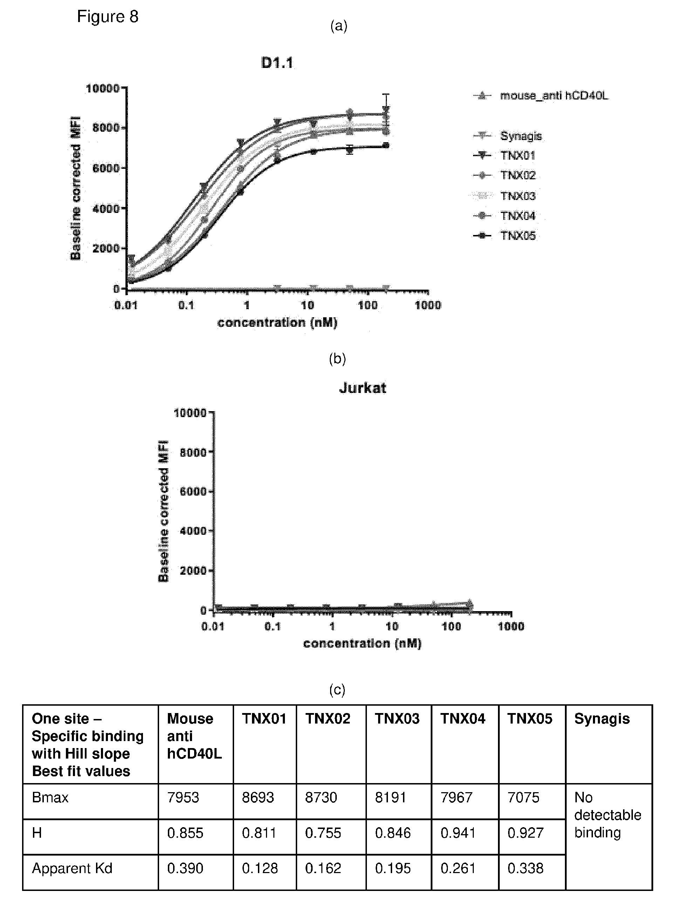

Figures 8(a) and 8(b) provide graphical representations of the mean fluorescence intensity (MFI) as a function of concentration (nM) in the binding of each antibody to the surface of (a) Jurkat D1.1 cells, which are CD154 positive, or (b) Jurkat cells, which are CD154 negative, as obtained by flow cytometry. Figure 8(c) provides the binding characteristics (Bmax (maximum number of binding sites), h (Hill slope) and apparent Kd (ligand concentration that binds to half the receptor sites at equilibrium)) of each anti-CD 154 antibody (TNX01-TNX05), a positive control antibody (mouse anti-human CD40L or CD140) and a negative control antibody (SYNAGIS^{®}) to D1.1 cells (CD154 positive) as analyzed using high-throughput FACS cell-binding assay conducted with the various antibodies.

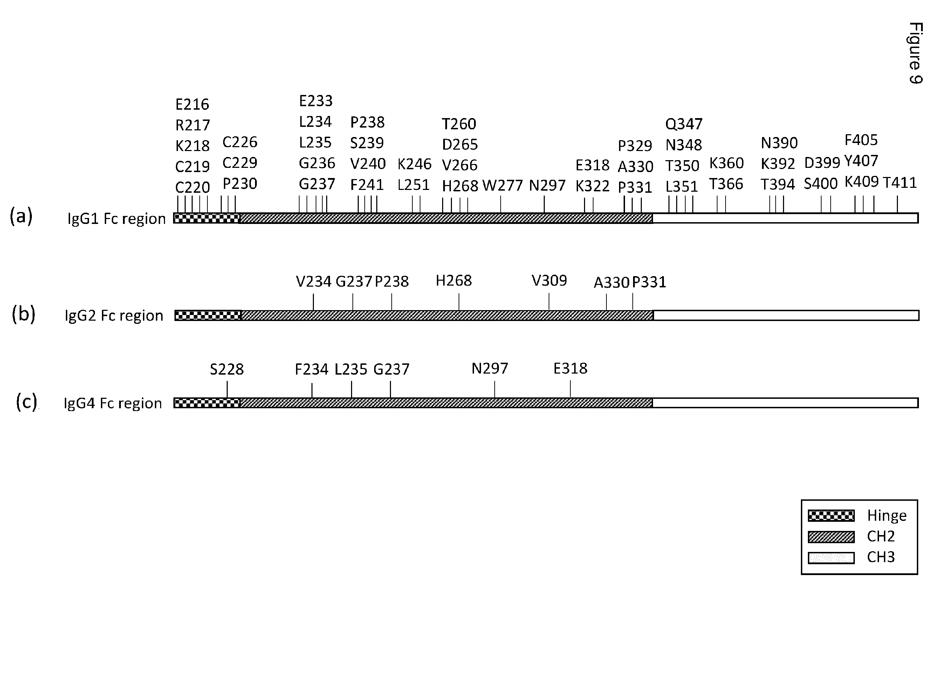

Figures 9(a), 9(b), and 9(c) provide maps of various amino acid modifications for the Fc region of IgG1 (Figure 9(a)), IgG2 (Figure 9(b)), and IgG4 (Figure 9(c)) that may modify (e.g., reduce) FcR effector functions.

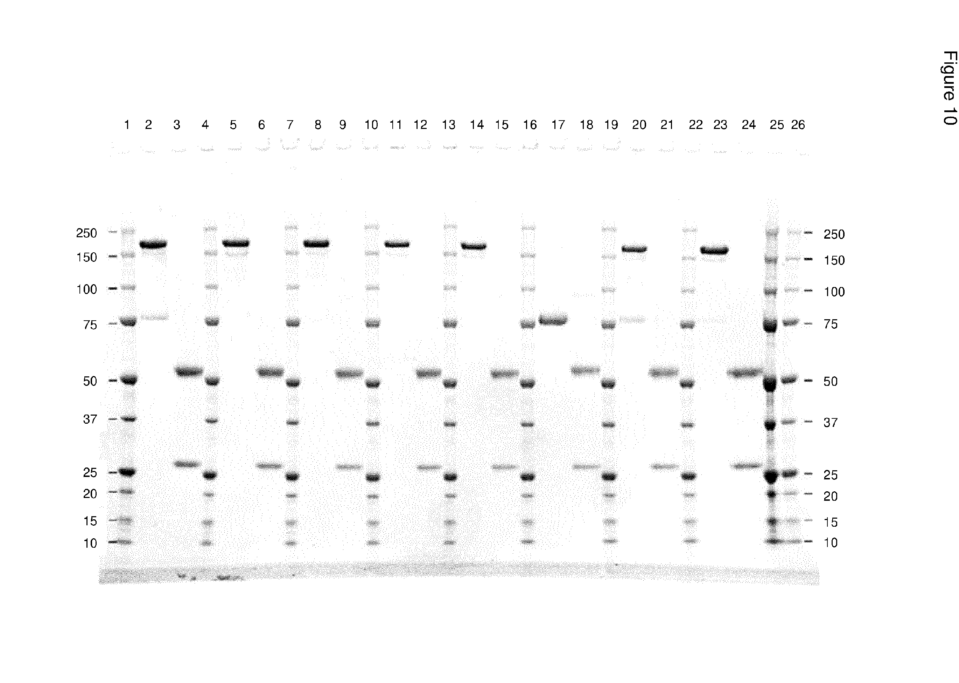

Figure 10 provides SDS-PAGE analysis of each of anti-CD154 mAbs (TNX06-TNX13) obtained during the process of protein generation and purification. STD is the protein standard which indicates the molecular weight of each band obtained on the Sypro Ruby stained gel under non-reducing (NR) or reducing (R) conditions. The lanes in the gel, from left to right, are as follows: (1) STD; (2) TNX06(NR); (3) TNX06(R); (4) STD; (5) TNX07(NR); (6) TNX07(R); (7) STD; (8) TNX08(NR); (9) TNX08(R); (10) STD; (11) TNX09(NR); (12) TNX09(R); (13) STD; (14) TNX10(NR); (15) TNX10(R); (16) STD; (17) TNX11(NR); (18) TNX11(R); (19) STD; (20) TNX12(NR); (21) TNX12(R); (22) STD; (23) TNX13(NR); (24) TNX13(R); (25) STD; (26) STD.

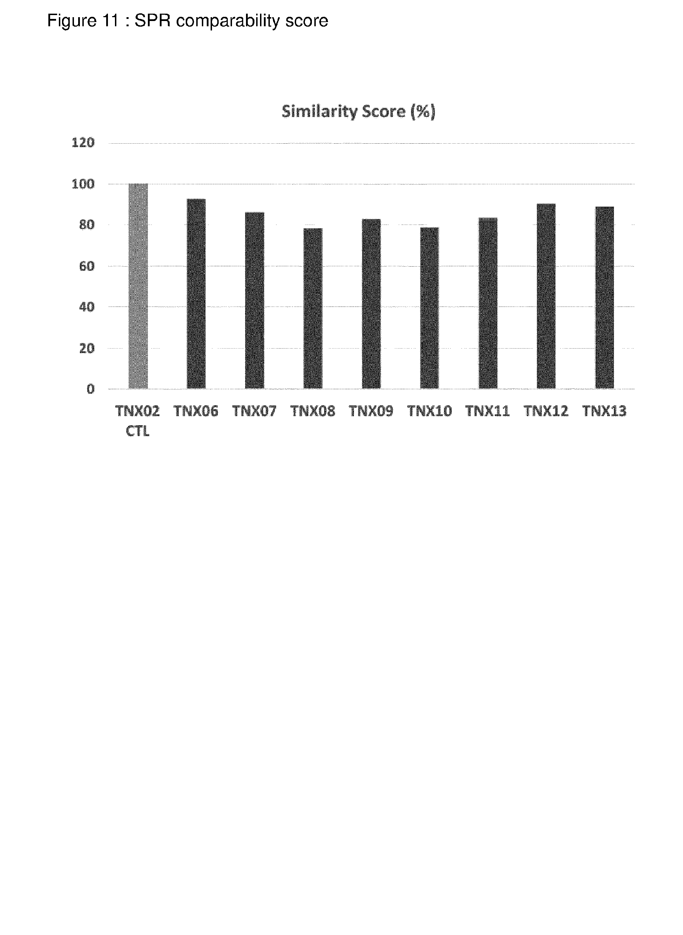

Figure 11 provides the surface plasmon resonance (SPR) comparability scores of sCD154 binding by TNX06-13 compared to that of TNX02. The scores as expressed as percentage similarity by pairwise comparison.

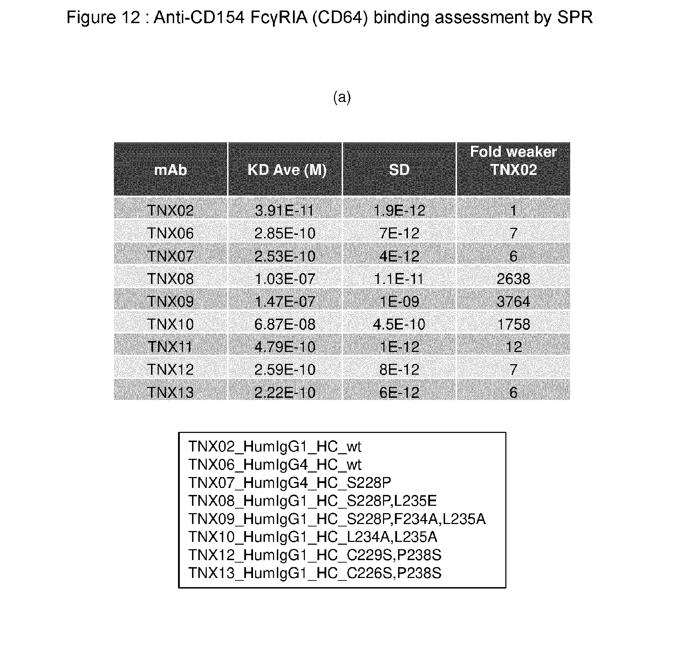

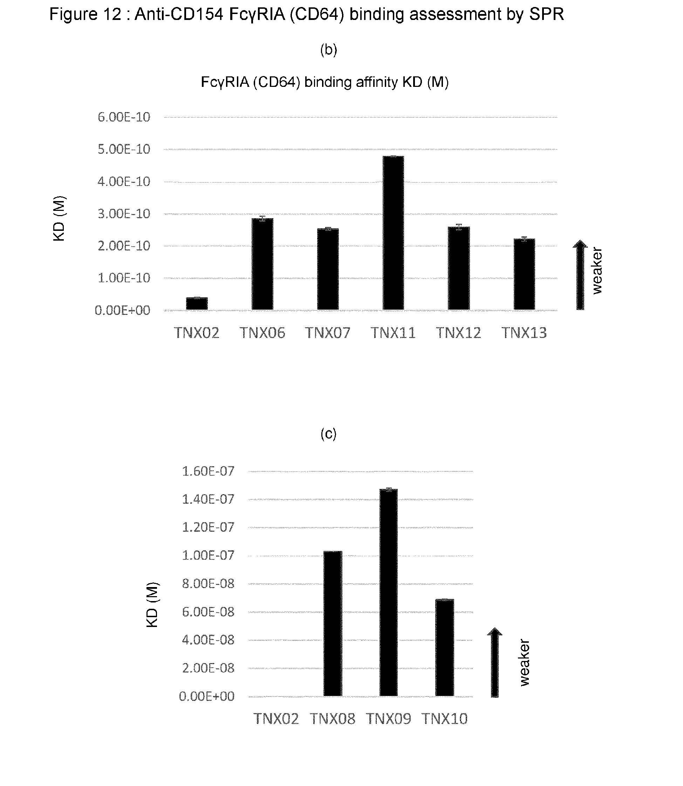

Figure 12(a) summarizes the data generated from the surface plasmon resonance (SPR) analysis and the calculated apparent KD values (affinity; ratio of kd to ka) for TNX06-13 binding to FcyRIA (CD64) and TNX02. Figures 12(b) and 12(c) show the average values for FcyRIA (CD64) Binding Affinity KD (M) with standard deviations (n=3). A lower KD value indicates tighter binding between the antibody and FcyRIA, such that TNX02 binds FcyRIA the tightest, followed by (in order) TNX13, TNX07, TNX12, TNX06, TNX10, TNX08, and TNX09.

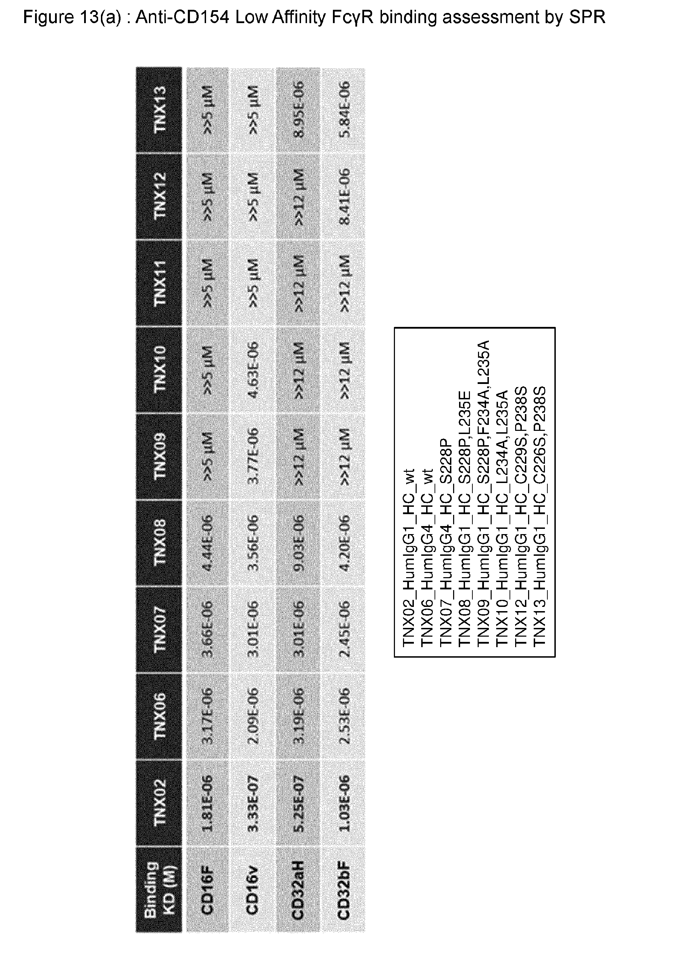

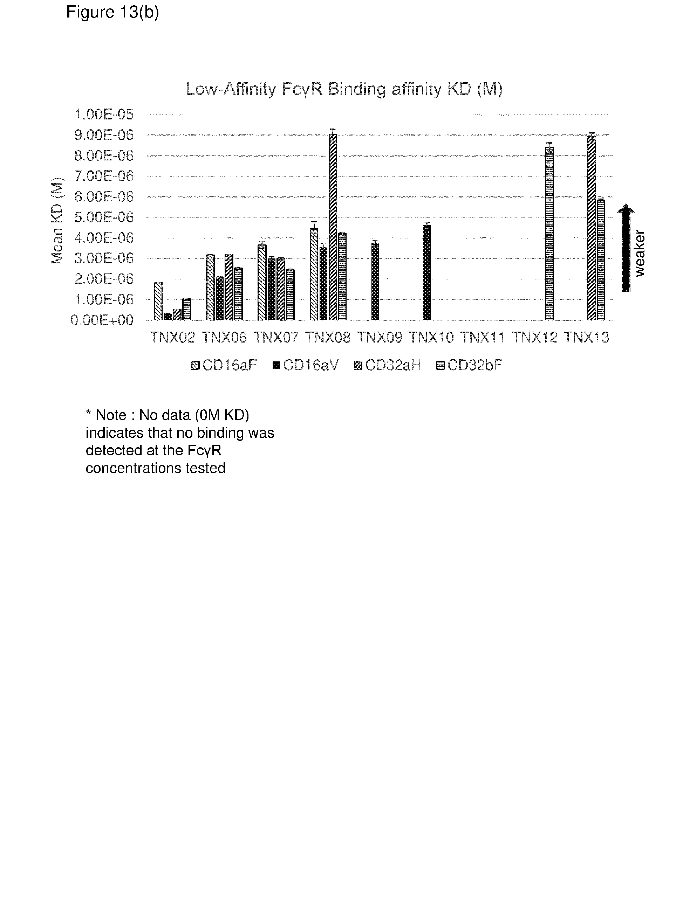

Figure 13(a) provides the mean KD values based on the sensorgram data obtained for TNX02 and TNX06-13 binding to low-affinity FcyRs. Figure 13(b) shows the average values for binding affinity of TNX02, TNX06-10, TNX12 and TNX13 KD (M) to a low-affinity FcyR panel (CD16aF, CD16aV, CD32aH, CD32bF) with standard deviations (n=3). The KD values for TNX11 and for some FcyR panel components for TNX09, TNX10, and TNX12 could not be determined because these variants showed negligible binding activity. A lower KD value indicates tighter binding between the antibody and FcyR.

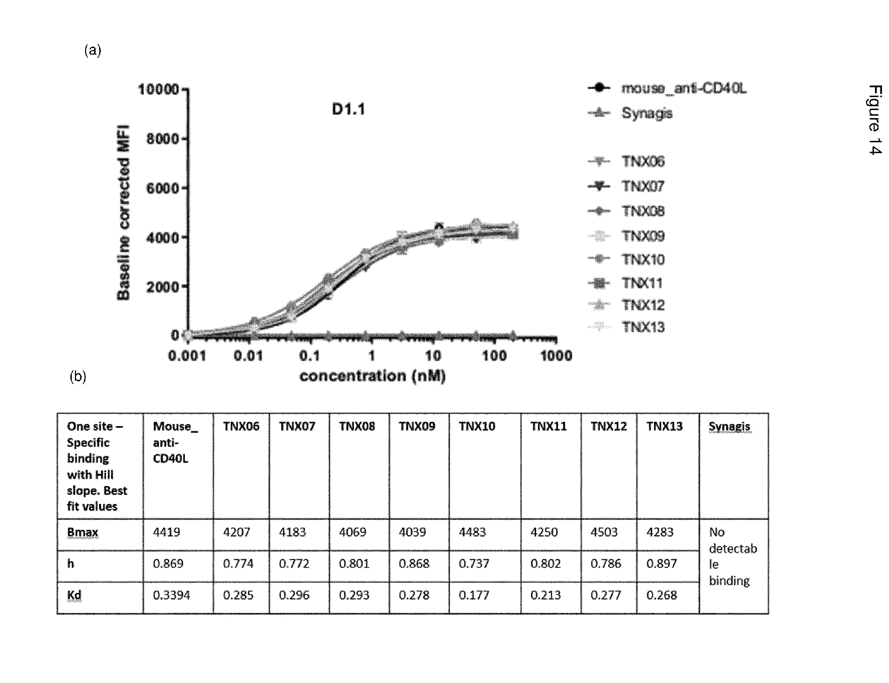

Figure 14(a) provides graphical representations of the mean fluorescence intensity (MFI) as a function of concentration (nM) in the binding of each antibody to the surface of Jurkat D1.1 cells, which are CD154 positive. Figure 14(b) provides the binding characteristics (Bmax (maximum number of binding sites), h (Hill slope) and apparent Kd (ligand concentration that binds to half the receptor sites at equilibrium)) of each anti-CD154 antibody (TNX06-TNX13), a positive control antibody (mouse anti-human CD40L or CD154) and a negative control antibody (SYNAGIS^{®}) to D1.1 cells (CD154 positive) as analyzed using high-throughput FACS cell-binding assay conducted with the various antibodies.

### Detailed Description Of The Invention

### Definitions and General Techniques

Unless otherwise defined herein, scientific and technical terms used in this application shall have the meanings that are commonly understood by those of ordinary skill in the art. Generally, nomenclature used in connection with, and techniques of, cell and tissue culture, molecular biology, immunology, microbiology, genetics and protein and nucleic acid chemistry and hybridization described herein are those well-known and commonly used in the art. In case of conflict, the present specification, including definitions, will control.

The methods and techniques of the present disclosure will employ, unless otherwise indicated, conventional techniques of molecular biology (including recombinant techniques), microbiology, cell biology, biochemistry and immunology, which are within the skill of the art. Such techniques are explained fully in the literature, such as Green MR & Sambrook J. Molecular Cloning: A Laboratory Manual, 4th ed., Cold Spring Harbor Laboratory Press, Cold Spring Harbor, N.Y. (2012); Ausubel et al., Short Protocols in Molecular Biology: A Compendium of Methods from Current Protocols in Molecular Biology, 5th ed., Wiley, John & Sons, Inc. (2002); Harlow and Lane Using Antibodies: A Laboratory Manual, Cold Spring Harbor Laboratory Press, Cold Spring Harbor, N.Y. (1998); and Coligan et al., Short Protocols in Protein Science, Wiley, John & Sons, Inc. (2003); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney, ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-1998) J. Wiley and Sons; Methods in Enzymology (Academic Press, Inc.); Gene Transfer Vectors for Mammalian Cells (J.M. Miller and M.P. Calos, eds., 1987); Current Protocols in Molecular Biology (F.M. Ausubel et al., eds., John Wiley & Sons, Inc., 2003); PCR: The Polymerase Chain Reaction, (Mullis et al., eds., 1994), each of which is incorporated herein by reference.

Enzymatic reactions and purification techniques are performed according to manufacturer's specifications, as commonly accomplished in the art or as described herein. The nomenclature used in connection with, and the laboratory procedures and techniques of, analytical chemistry, synthetic organic chemistry, and medicinal and pharmaceutical chemistry described herein are those well known and commonly used in the art.

Standard techniques are used for chemical syntheses, and chemical analyses.

Throughout this specification and embodiments, the word "comprise," or variations such as "comprises" or "comprising," will be understood to imply the inclusion of a stated integer or group of integers but not the exclusion of any other integer or group of integers. "Comprising" may be synonymous with "including" or "containing."

It is understood that wherever embodiments are described herein with the language "comprising," otherwise analogous embodiments described in terms of "consisting of' and/or "consisting essentially of' are also provided. As used herein, "consisting of' is a closed term that includes only the specific elements recited, and "consisting essentially of' includes the specific elements recited and may include additional unrecited, nonmaterial elements.

The term "including" is used to mean "including but not limited to." "Including" and "including but not limited to" are used interchangeably.

Any example(s) following the term "e.g." or "for example" is not meant to be exhaustive or limiting.

Unless otherwise required by context, singular terms shall include pluralities and plural terms shall include the singular.

The articles "a", "an" and "the" are used herein to refer to one or to more than one (i.e., to at least one) of the grammatical object of the article. By way of example, "an element" means one element or more than one element. As used herein, the term "about" modifying the quantity of an ingredient, parameter, calculation, or measurement in the compositions of the disclosure or employed in the methods of the disclosure refers to variation in the numerical quantity that can occur, for example, through typical measuring and liquid handling procedures used for making isolated polypeptides or pharmaceutical compositions in the real world; through inadvertent error in these procedures; through differences in the manufacture, source, or purity of the ingredients employed to make the compositions or carry out the methods; and the like without having a substantial effect on the chemical or physical attributes of the compositions or methods of the disclosure. Such variation can be within an order of magnitude, typically within 10%, more typically still within 5%, of a given value or range. The term "about" also encompasses amounts that differ due to different equilibrium conditions for a composition resulting from a particular initial mixture. Whether or not modified by the term "about", the paragraphs include equivalents to the quantities. Reference to "about" a value or parameter herein includes (and describes) embodiments that are directed to that value or parameter per se. For example, description referring to "about X" includes description of "X." Numeric ranges are inclusive of the numbers defining the range.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a stated range of "1 to 10" should be considered to include any and all subranges between (and inclusive of) the minimum value of 1 and the maximum value of 10; that is, all subranges beginning with a minimum value of 1 or more, *e.g.,* 1 to 6.1, and ending with a maximum value of 10 or less, *e.g.,* 5.5 to 10.

Exemplary methods and materials are described herein, although methods and materials similar or equivalent to those described herein can also be used in the practice or testing of the present application. The materials, methods, and examples are illustrative only and not intended to be limiting.

### Definitions

The following terms, unless otherwise indicated, shall be understood to have the following meanings:

As used herein, the term "antibody" or "Ab" refers to an immunoglobulin molecule (*e.g*., complete antibodies, antibody fragment or modified antibodies) capable of recognizing and binding to a specific target or antigen, such as a carbohydrate, polynucleotide, lipid, polypeptide, etc., through at least one antigen recognition site, located in the variable region of the immunoglobulin molecule. As used herein, the term "antibody" can encompass any type of antibody, including but not limited to monoclonal antibodies, polyclonal antibodies, human antibodies, engineered antibodies (including humanized antibodies, fully human antibodies, chimeric antibodies, single-chain antibodies, artificially selected antibodies, CDR-granted antibodies, etc) that specifically bind to a given antigen (*e.g*. CD 154). Further, "antibody" and/or "immunoglobulin" (Ig) refers to a polypeptide comprising at least two heavy (H) chains (about 50-70 kDa) and two light (L) chains (about 25 kDa), optionally inter-connected by disulfide bonds. There are two types of light chain: λ and κ. In humans, λ and κ light chains are similar, but only one type is present in each antibody. Heavy chains are classified as mu, delta, gamma, alpha, or epsilon, and define the antibody's isotype as IgM, IgD, IgG, IgA, and IgE, respectively. See generally, Fundamental Immunology Ch. 7 (Paul, W., ed., 2nd ed. Raven Press, N.Y. (1989)) (incorporated by reference in its entirety). The antibodies disclosed herein may be "functionally divalent" or "functionally monovalent". In other words, the antibodies disclosed herein may have one antigen-binding site (monovalent) or two antigen-binding sites that are typically linked by disulfide bonds (divalent). In some embodiments, the anti-CD154 antibody is an IgG1 antibody. The anti-CD154 antibody may be an IgG2 antibody. Optionally, the anti-CD154 antibody is an IgG4 antibody. In some embodiments, the anti-CD154 antibody does not contain the final lysine residue at its C-terminal end in order to improve antibody stability. See *e.g.,* Jiang G et al., J Pharm Sci. Jul;105(7):2066-72 (2016); and Hintersteiner B. MAbs. 2016 Nov/Dec;8(8): 1548-1560 (2016). It is known in the art that each heavy chain and light chain is expressed comprising a leader sequence (also known as a signal sequence) at its N terminus, which is used to transport the newly synthesized chains into the endoplasmic reticulum. During post-translational processing, the leader sequences are removed and, therefore, are not present in the final chain or the mature antibody.

As used herein, the term "monoclonal antibody" or "mAb" refers to an antibody that is produced by an identical set of immune cells that are each clones of a unique parent cell. Monoclonal antibodies have monovalent affinity (i.e., they bind to the same epitope).

As used herein, the term "chimeric" antibodies refers to antibodies and antigen-binding fragments thereof comprising portions from two or more different species (*e.g*., mouse and human). Chimeric antibodies can be produced with mouse variable regions of desired specificity spliced onto human constant domain gene segments (for example, U.S. patent No. 4,816,567). In this manner, non-human antibodies can be modified to make them more suitable for human clinical application. The term "chimeric" may refer to a non-native sequence that has been manipulated to have one or more changes relative a native sequence. A chimeric antibody as used herein means an antibody that comprises regions from two or more different antibodies.

As used herein, the term "humanized" antibodies refers to chimeric antibodies from a non-human species, whose amino acid sequences have been modified to increase their similarity to antibodies produced in humans. In some embodiments, humanized antibodies are chimeric immunoglobulins, immunoglobulin chains, or fragments thereof (such as Fv, Fab, Fab', F(ab')2 or other antigen binding subsequences of antibodies) that contain minimal sequence derived from a non-human immunoglobulin. Optionally, humanized antibodies are derived from human immunoglobulins (i.e., recipient antibodies) in which residues from one or more complementary determining regions (CDRs) of the recipient antibody are replaced by residues from one or more CDRs of an antibody from a non-human species (donor antibody) having the desired specificity, affinity, and capacity. In some embodiments, the non-human species is a mouse, a rat, or a rabbit. Humanized or CDR-grafted mAbs are particularly useful as therapeutic agents for humans because they are not cleared from the circulation as rapidly as mouse antibodies and do not typically provoke an adverse immune reaction. Generally, a humanized antibody has one or more amino acid residues introduced into it from a non-human source.

In some embodiments, the chimeric antibody is a humanized antibody, *e.g*., a humanized anti-CD154 antibody. A humanized anti-CD 154 antibody may comprise the amino acid sequence of one or more human framework regions and/or the amino acid sequence from at least a portion of a human constant region and further comprises sequences derived from a non-human antibody, for example non-human (*e.g*., mouse) CDR sequences. In some embodiments, the humanized antibody comprises a human constant region. Optionally, all of the framework regions in the humanized antibody are human framework regions.

Humanized antibodies can be generated by replacing non-human sequences of the Fv variable region that are not directly involved in antigen binding with equivalent sequences from human Fv variable regions. General methods for generating humanized antibodies are provided by Morrison, S. L., Science, 229:1202-1207 (1985), by Oi et al., BioTechniques, 4:214 (1986), by Jones et al., Nature 321:522-525 (1986), by Riechmann et al., Nature, 332:323-327 (1988), by Verhoeyen et al., Science, 239: 1534-1536 (1988)), by Staelens et al. 2006 Mol Immunol 43: 1243-1257, and by U.S. Pat. No. 5,225,539; U.S. Pat. No. 5,585,089; U.S. Pat. No. 5,693,761; U.S. Pat. No. 5,693,762; U.S. Pat. No. 5,859,205; and U.S. Pat. No. 6,407,213, each incorporated herein by reference. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against a predetermined target, as described above, from germline immunoglobulin genes, or from synthetic constructs. The recombinant DNA encoding the humanized antibody can then be cloned into an appropriate expression vector. Humanized antibodies are typically human antibodies in which some CDR residues and possibly some framework residues are substituted by residues from analogous sites in rodent antibodies. See, for example, U.S. Patent Nos. 5,225,539; 5,585,089; 5,693,761; 5,693,762; 5,859,205, each incorporated herein by reference. See also U.S. Patent No. 6,180,370, and PCT International Publication No. WO 01/27160 (each incorporated herein by reference), where humanized antibodies and techniques for producing humanized antibodies having improved affinity for a predetermined antigen are disclosed. Furthermore, humanized and chimeric antibodies can be modified to comprise residues that are not found in the recipient antibody or in the donor antibody in order to further improve antibody properties, such as, for example, affinity or effector function.

As used herein, the term "human antibodies" refers to antibodies having variable and constant regions derived from human germline immunoglobulin sequences. Human antibodies can include amino acid residues not encoded by human germline immunoglobulin sequences (*e.g*., mutations introduced by random or site-specific mutagenesis in vitro or by somatic mutation in vivo). However, the term "human antibody" does not include antibodies in which CDR sequences derived from the germline of another mammalian species, such as a mouse, have been grafted onto human framework sequences (i.e., humanized antibodies). The term encompasses antibodies with sequences derived from human genes, but which have been changed, *e.g.* to decrease possible immunogenicity, increase affinity, eliminate cysteines that might cause undesirable folding, etc. The term also encompasses such antibodies produced recombinantly in non-human cells, which might impart glycosylation not typical of human cells. For the generation of human antibodies, see Mendez et al. Nature Genetics 15: 146-156 (1997), Green and Jakobovits J. Exp. Med. 188:483-495 (1998), Lonberg, Nature Biotechnology, Vol. 23(5): 1117-1125 (2005); Jackobovits, "Therapeutic Antibodies from XenoMouse Transgenic Mice," Chapter 7, Recombinant Antibodies for Immunotherapy, Cambridge University Press, New York, 2009, Murphy, "VelocImmune: Immunoglobulin Variable Region Humanized Mice," Chapter 8, Recombinant Antibodies for Immunotherapy, Cambridge University Press, New York, 2009, Murphy et al., PNAS, 2014, vol. 111(14): 5153-5158, Brüggemann et al., Arch. Immunol. Ther. Exp., 2015, vol. 63: 101-108, the disclosures of which are hereby incorporated by reference in their entirety. Human antibodies and methods of making them are further discussed in U.S. patents 5,939,598; 6,673,986; 6,114,598; 6,075,181; 6,162,963; 6,150,584; 6,713,610; 6,657,103; 6,586,251; as well as U.S. patent application publications US 2006-0015957 A1; and International Patent Publication Nos. WO 98/24893; and WO 2007/117410. The disclosures of each of the above-cited patents, applications, and references are hereby incorporated by reference in their entirety.

As used herein, the term "amino acid modification" refers to at least one amino acid substitution, insertion, deletion or mutation in an amino acid sequence compared to a wild-type amino acid sequence. Such modifications are within the ordinary skill of an artisan. Certain modifications, including amino acid deletions, substitutions and additions, of the Fc region have been demonstrated to alter the binding of the Fc region to its ligands and/or receptors resulting in a concomitant change in effector function (see, *e.g.,* (Shields et al., J Biol Chem 276:6591-6604 (2001); Presta et al., Biochem Soc Trans 30:487-490 (2002); Escobar-Cabrera E et al. Antibodies. 6: 7 (2017); Duncan AR et al. Nature. 1988; 332: 738-740 (1988); Duncan AR et al. Nature. 332: 563-564 (1988); Hezareh M et al. J Virol. 75: 12161-12168 (2001); Oganesyan V et al. Acta Crystallogr D Biol Crystallogr; 64: 700-704 (2008); Schlothauer T et al. Protein Eng Des Sel. Oct; 29(10):457-466 (2016); Tao MH et al. J. Immunol. 143: 2595-2601 (1989); Von Kreudenstein TS et al. MAbs. 5(5):646-654 (2013); Wang X et al. Protein Cell. 9(1):63-73 (2018); U.S. Patent Publications 20040132101; 20070111260; 20110287032; 20180194860; U.S. Patent No. US 8409568; International Publication No. WO2017/177337, incorporated by reference in their entirety. An amino acid deletion is indicated as "Δ", and an insertion is indicated as "In". For instance, a deletion of the amino acid sequence from E216 to E222 is indicated as ΔE216-E222. An insertion of an arginine (R) between amino acid residues 234 and 235, e.g, would be indicated as InR234/235.

As used herein, the term "Fc domain" refers to the crystallizable fragment of an antibody following papain digestion. The Fc domain comprises two identical protein fragments derived from the hinge region and the second and third constant domains of IgA, IgD, and IgG antibody isotypes or the hinge region and the second, third, and fourth constant domains of IgM and IgE antibody isotypes. The Fc domain is the portion of an antibody that binds to cell surface Fc receptors and certain proteins of the complement system. The term "Fc region" refers to the Fc domain in combination with a hinge region. The hinger region is typically between the C-terminus of a variable domain and the N-terminus of the Fc domain. Although the boundaries of the Fc region may vary, the human IgG heavy chain Fc region as defined herein comprises residue E216 to its carboxyl-terminus of the CH3 domain (or the CH4 domain for IgM and IgE antibodies), wherein the numbering is in the EU format as in Edelman GM et al., (1969) Proc. Natl. Acad. USA, 63, 78-85. The "EU format as set forth in Edelman" refers to the residue numbering of the human IgG1 EU antibody as described in Edelman GM *et al. supra.* The human IgG2 and human IgG4 residue numbering is also in the EU format (See Dillon TM, et al., J Biol Chem. Jun 6;283(23):16206-15 (2008); Aalberse RC and Schuurman J et al., Immunology 105:9-19 (2002); and Scholthauer T et al, Protein Engineering, Design and Selection, 29(10): 457-466, (2016). The terms "Fc domain" and "Fc region" may refer to these sequences in isolation, or these sequences in the context of an antibody, antibody fragment, or Fc fusion protein. An Fc variant protein may be an antibody, Fc fusion, or any protein or protein domain that comprises an F domain or an Fc region. The amino acid sequence of a non-naturally occurring Fc domain or Fc region (also referred to herein as a "variant Fc domain" or a "variant Fc region," respectively) may comprise an amino acid modification. Any new amino acid residue appearing in the sequence of a variant Fc domain or a variant Fc region as a result of an insertion or substitution may be referred to as a non-naturally occurring amino acid residue. Polymorphisms have been observed at a number of Fc domain positions, including but not limited to positions 270, 272, 312, 315, 356, and 358, and thus slight differences between the presented sequence and sequences in the prior art may exist.

As used herein, the term "linker" refers to a polypeptide sequence that joins two or more antibody domains. The characteristics of linkers and their suitability for particular purposes are known in the art. See, *e.g.,* Chen et al. Adv Drug Deliv Rev. October 15; 65(10): 1357-1369 (2013) (disclosing various types of linkers, their properties, and associated linker designing tools and databases), which is incorporated herein by reference. The linker may be flexible, rigid, or *in vivo* cleavable. Preferably, the linker is flexible. Flexible linkers typically comprise small non-polar (*e.g*. Gly) or polar (*e.g*., Ser or Thr) amino acids. The most commonly used flexible linkers have sequences consisting primarily of stretches of Gly and Ser residues ("GS" linker). Optionally, flexible linkers comprise repeats of 5 Gly and Ser residues. Non-limiting examples of flexible linker include (Gly-Gly-Gly-Gly-Ser)ₙ (SEQ ID NO: 327), (Ser-Ser-Ser-Ser-Gly)ₙ (SEQ ID NO: 328), (Gly-Ser-Ser-Gly-Gly)ₙ (SEQ ID NO: 329), and (Gly-Gly-Ser-Gly-Gly)ₙ (SEQ ID NO: 330), where n may be any integer between 1 and 5. The linker is optionally between 5 and 25 amino acid residues long. Other suitable linkers may be selected from the group consisting of AS (SEQ ID NO: 214), AST (SEQ ID NO: 215), TVAAPS (SEQ ID NO: 216), TVA (SEQ ID NO: 217), ASTSGPS (SEQ ID NO: 218), KESGSVSSEQLAQFRSLD (SEQ ID NO: 219), EGKSSGSGSESKST (SEQ ID NO: 220), (Gly)6 (SEQ ID NO: 221), (Gly)8 (SEQ ID NO: 222), and GSAGSAAGSGEF (SEQ ID NO: 223). In general, a flexible linker should provide good flexibility and solubility and may serve as a passive linker to keep a distance between functional domains. The length of the flexible linkers can be adj usted to allow for proper folding or to achieve optimal biological activity of the fusion proteins.

As used herein, the twenty conventional amino acids and their abbreviations follow conventional usage. See Immunology--A Synthesis (2nd Edition, E. S. Golub and D. R. Gren, Eds., Sinauer Associates, Sunderland, Mass. (1991)), which is incorporated herein by reference.

A "conservative amino acid substitution" is one in which an amino acid residue is replaced with a different amino acid residue with similar biochemical properties (*e.g*., charge, hydrophobicity, or size). Typically, conservative amino acid substitutions do not substantially change a protein's functional properties. When comparing proteins with conservative substitutions, the percent sequence identity or degree of similarity may be adjusted to account for the conservative nature of the substitution. Such adjustments are well-known to those of skill in the art. See, *e.g.,* Pearson, Methods Mol. Biol. 243:307-31 (1994).

Groups of amino acids with similar biochemical properties that may be used in conservative substitutions include 1) amino acid residues with aliphatic side chains: glycine, alanine, valine, leucine, and isoleucine; 2) amino acid residues with aliphatic-hydroxyl side chains: serine and threonine; 3) amino acid residues with amide-containing side chains: asparagine and glutamine; 4) amino acid residues with aromatic side chains: phenylalanine, tyrosine, and tryptophan; 5) amino acid residues with basic side chains: lysine, arginine, and histidine; 6) amino acid residues with acidic side chains: aspartic acid and glutamic acid; and 7) amino acid residues with sulfur-containing side chains: cysteine and methionine. Preferred conservative amino acids substitution groups include: valine-leucine-isoleucine, phenylalanine-tyrosine, lysine-arginine, alanine-valine, glutamate-aspartate, and asparagine-glutamine.

Each heavy chain is comprised of a heavy chain variable domain (VH) and multiple heavy chain constant domains (CH). For IgA, IgD, and IgG antibodies, the heavy chain typically comprises three domains: CH1, CH2 and CH3. For IgM and IgE antibodies, the heavy chain typically comprises four domains: CH1, CH2, CH3, and CH4. In some embodiments, the antibody comprises two domains: CH2 and CH3. Each light chain comprises a light chain variable domain (VL) and a light chain constant domain. The light chain typically comprises one domain: CL. Light chain variable domains are encoded by two gene segments: a variable (V) gene segment, which encodes the first 95-101 amino acids of the light chain, and a joining (J) gene segment, which encodes about 12 or more amino acids. Heavy chain variable domains are encoded by three gene segments and include a diversity (D) gene segment, which encodes about 3 or more amino acids, between the V and J gene segments. The VH and VL domains can be further subdivided into regions of hypervariability, called "complementarity determining regions" (CDR) that are separated by more conserved "framework regions" (FR). Each VH and VL is composed of three CDRs and four FRs, arranged from amino-terminus to carboxyl-terminus in the following order: FR1, CDR1, FR2, CDR2, FR3, CDR3, FR4.

The pairing of the variable domains of the heavy chain and light chain (VH and VL) forms the antibody binding site that interacts with an antigen. Thus, each antibody typically has two binding sites. With the exception of multifunctional / multispecific (*e.g*., bifunctional or bispecific) antibodies, the two binding sites are the same. The Fc regions of the constant regions of the antibodies typically mediate the binding of antibodies to host tissues and factors, including various cells of the immune system (*e.g*., effector cells) and the first component (Clq) of the classical complement system.

Residues in a variable domain are numbered according to Edelman, also known as the EU numbering system, which is a numbering system used for heavy chain variable domains or light chain variable domains of the compilation of antibodies. See, Edelman Proc Natl Acad Sci U S A. May;63(1):78-85 (1969) and Kabat, E. A., Wu, T. T., Perry, H., Gottesman, K., and Foeller, C. (1991) Sequences of Proteins of Immunological Interest, 5th ed., NIH Publication No. 91-3242, Bethesda, MD. The Eu numbering of residues may be determined for a given antibody by alignment at regions of homology of the sequence of the antibody with a "standard" EU numbered sequence. Variable region CDRs (CDR L1, CDR L2, CDR L3, CDR H1, CDR H2, CDR H3) are identified according to contact based on crystal structures as defined in Karpusas et al. Structure. Apr 4;9(4):321-9 (2001) and numbered in accordance with Edelman.

As used herein, the term "operably linked" refers to a first structure that has been placed in a functional relationship with a second structure. In the context of an antibody, a targeting structure may be operably linked to a structure that confers effector function. For example, the antigen-binding sequence of an antibody (*e.g*., variable region or VH or VL domain) may be operatively linked to a Fc region. In the context of a polynucleotide, a coding sequence may be operatively linked to a non-coding regulatory sequence, such as a promoter, an enhance, a signal sequence, a ribosome binding sequence, a splice acceptor sequence, a splice donor sequence, a termination sequence, etc. Two operably linked structures may be directly connected. Alternatively, two operably linked structures may be connected via one or more intermediary structures. For example, the antigen-binding portion of an antibody may be operably linked to the Fc region via a CH1 domain, a hinge region and/or a linker sequence. Similarly, operably linked non-coding regulatory sequences include both sequences that are contiguous with the coding sequence and sequences that act in *trans* or at a distance to control the coding sequence.

As used herein, the term "effector function" refers to the responses triggered by the interaction of antibodies and antibody-antigen complexes with cells of the immune system. These effector functions typically involve one of three major mechanisms: antibody-dependent cell-mediated cytotoxicity (ADCC), complement-dependent cytotoxicity (CDC), and opsonization and phagocytosis. In ADCC, Fc receptors on cytotoxic T cells, natural killer (NK) cells, or macrophages bind to the Fc regions of antibodies bound to a target cell, resulting in the secretion of substances, such as lytic enzymes, perforin, granzymes and tumor necrosis factor, which mediate the destruction of the target cell. In CDC, cell death is induced via activation of the complement cascade. See Daeron, Annu. Rev. Immunol., 15:203-234 (1997); Ward and Ghetie, Therapeutic Immunol., 2:77-94 (1995); and Ravetch and Kinet, Annu. Rev. Immunol. 9:457-492 (1991)). In opsonization and phagocytosis, the Fc region of a pathogen-bound antibody binds to a Fc receptor on the surface of a phagocyte, inducing phagocytosis. Such effector functions generally require the Fc region to be combined with a binding domain (*e.g*. an antibody variable domain) and can be assessed using standard assays that are known in the art (see, *e.g*., WO 05/018572, WO 05/003175, and U.S. Pat. No. 6,242,195). The Fc domain of the antibody mediates immune effector mechanisms. IgG antibodies activate effector pathways of the immune system by binding to members of the family of cell surface Fcy receptors and to Clq of the complement system. Ligation of effector proteins by clustered antibodies triggers a variety of responses, including release of inflammatory cytokines, regulation of antigen production, endocytosis, and cell killing. These responses can provoke unwanted side effects such as inflammation and thrombosis. Accordingly, the present disclosure further relates to anti-CD 154 antibodies, with modified effector functions, including antibodies in which one or more effector functions is reduced or eliminated. Without being bound by theory, it is believed that the anti-CD 154 antibodies disclosed herein do not cause platelet activation or aggregation, because the antibodies comprising the mutated Fc regions do not bind FcyRIIa (also known as CD32a) on the platelet surface.

As used herein, the term "modified effector functions" refers to a Fc domain or an Fc region whose effector functions differ from a wild-type immunoglobulin Fc domain or Fc region. In some embodiments, one or more effector functions are reduced. Optionally, one or more effector functions are eliminated. The modified or reduced effector functions may be the result of lower binding affinity of the Fc region of the antibodies disclosed herein to effector molecules (*e.g*., FcyRs and/or C1q). For example, the anti-CD 154 antibodies disclosed herein have reduced Fc receptor binding and complement activation compared with that of wild-type anti-CD154 antibodies. In some embodiments, a variant Fc region has a reduced antibody dependent cell-mediated cytotoxicity (ADCC). Effector function of an anti-CD154 antibody may be determined using one of many known assays, including the CDC assay, the ADCC assay, and the phagocytosis assay (see Xu-Rong Jiang et al, Nature Reviews Drug Discovery 10: 101-111 (2011) and Liu et al. The Journal of Biological Chemistry 292:1876-1883 (2017)). The anti-CD154 one or more of the antibody's effector functions may be reduced by at least 10%, 20%, 30%, 40%, 50%, 60%, 70%, 80%, 90%, or 95% relative to the effector function of a wild-type anti-CD154 antibody.

As used herein, the terms "CD154," "CD40 ligand" and "CD40L" may be used interchangeably and refer to a mammalian protein that is primarily expressed on the surfaces of activated T cells, a soluble form of CD154 that is cleaved and released by activated T cells, is a member of the TNF superfamily, and binds the CD40 protein on antigen presenting cells. The term CD154 is intended to include recombinant CD154 and recombinant chimeric forms of CD154, which can be prepared by standard recombinant expression methods. In some embodiments, CD154 refers to human CD154.

As used herein, the term "inhibits" refers to the property of an antibody, or other molecule, that prevents the interaction of CD154 with CD40 or one that inhibits the binding of CD154 to CD40, or one that inhibits CD154 cleavage or shedding. In some embodiments, the antibody inhibits the binding of CD154 to CD40 by at least about 20%, preferably 40%, more preferably 60%, even more preferably 80%, or even more preferably 85%. Optionally, the antibody inhibits CD154 cleavage or shedding by at least about 20%, preferably 40%, more preferably 60%, even more preferably 80%, or even more preferably 85%. The inhibitory potential of anti-CD154 antibodies may be determined, for example, by their ability to inhibit up-regulation of a specific downstream target gene of CD40. For example, an anti-CD 154 antibody may alter the expression, activity, or activation of kinases and genes that respond to CD154-CD40 signaling. An anti-CD 154 antibody may inhibit the upregulation of CD23 expression, inhibit the upregulation of CD69 expression, inhibit the upregulation and activity of activation-induced cytidine deaminase (AID), inhibit rescue from apoptosis, inhibit upregulation of NF-κB activity, inhibit immunoglobulin isotype class switching, inhibit immunoglobulin CDR somatic hypermutation, alter the expression or activity of molecules within the TNF-receptor associated factor (TRAF) family such as TRAF-2, TRAF3 (also known as CRAF1), TRAF-5 and TRAF-6, kinase activation or inhibit the expression of other genes that respond to CD154-CD40 signaling. See, for example, Lederman, S., et al. J. Exp. Med. 175:1091-1101. (1992); Lederman, S., et al., Journal of Immunol. 149:3817-3826. (1992); Lederman, S., et al., Journal of Immunol. 152:2163. (1994); Cleary, A.M., et al., Journal of Immunol., 155:3329-3337 (1995); Cheng et al., Science. 267(5203):1494-8 (1995), Bankert KC et al., Journal of Immunol. 194:4319-4327 (2015), Ishida TK et al Proc Natl Acad Sci U S A. 93(18):9437-42 (1996). Muramatsu, MK et al. 2000. Cell 102: 553 (2000); Buchta CM and Bishop GA., Journal of Immunol. 192(1):145-50. (2014), Arcipowski KM, et al. International Immunology. 26(3): 149-58 (2014); Mambetsariev N, et al., Proc Natl Acad Sci U S A. 113(4):1032-7 (2016), Arcipowski KM, Bishop GA., PLoS One. 7(7) (2012); Bishop GA. Journal of Immunol. 91(7):3483-5. (2013); Peters AL and Bishop GA. Journal of Immunol. 185(11):6555-62. (2010); Rowland SL, et al., Journal of Immunol. 179(7):4645-53. (2007), Benson RJ, et al, European Journal of Immunol. 6(9):2535-43. (2006).

As used herein, the term "immune response" refers to reaction of body's immune system to the presence of a substance which is not recognized as a constituent of the body itself. An immune response may be a humoral immune response, a cell-mediated immune response, or a mixed humoral and cell-mediated immune response. A humoral response may be an antibody-mediated response. A cell-mediated response may be one or more of a cytotoxic T-cell mediated immune response, a macrophage mediated response, a natural killer (NK) cell mediated immune response or a cytokine mediated response. A mixed humoral and cell-mediated response may be one or more of an antibody-mediated response, a cytotoxic T-cell mediated immune response, a macrophage mediated response, a natural killer (NK) cell mediated immune response or a cytokine mediated response. The immune response can refer to an adaptive and/or an innate immune response. For the various types of immune responses, see David Chaplin J Allergy Clin Immunol February; 125(2 Suppl 2): S3-23 (2010).

As used herein, the term "affinity" of an antibody refers to the strength of interaction between the antibody's antigen-binding site and an epitope. An antibody's affinity for an antigen is typically expressed as the binding affinity equilibrium dissociation constant (K_{D}) of a particular antibody-antigen interaction. An antibody is said to specifically bind an antigen when the K_{D} is ≤ 1 mM, preferably ≤ 100 nM. High affinity antibodies are generally considered to have a K_{D} in the low nanomolar (10⁻⁹) range, and very high affinity antibodies are generally considered to have a K_{D} in picomolar (10⁻¹²) range. A K_{D} binding affinity constant can be measured by surface plasmon resonance, for example using the BIACORE^{®} system (Pharmacia Biosensor AB, Uppsala, Sweden and Piscataway, N.J.) as discussed in Example 3. See also, Jonsson et al., Ann. Biol. Clin. 51: 19-26 (1993); Jonsson et al., Biotechniques 11:620-627 (1991); Jonsson et al., J. Mol. Recognit. 8: 125-131 (1995); Johnsson et al., Anal. Biochem. 198:268-277 (1991); Hearty S et al., Methods Mol Biol. 907:411-42 (2012), each incorporated herein by reference. The K_{D} may also be measured using a KINEXA^{®} system (Sapidyne Instruments, Hanover, Germany and Boise, ID).

As used herein, the terms "ka" or "affinity constant" and "kd" or "dissociation constant" refer to the amount of antibody-antigen complex that exists at the point when equilibrium concentration between antibody and antigen is reached. K_{D} is the ratio of kd to ka.

As used herein, the term "avidity" refers to the overall strength of an antibody-antigen complex. Avidity relates to three major parameters: the affinity of the antibody for the epitope; the valency of both the antibody and antigen; and the structural arrangement of the parts that interact. As used herein, "avidity" describes the increased affinity that occurs as result of multiple antigen binding sites on an immunoglobulin.

As used herein, the term "transplantation" refers to process of surgically removing a cell or tissue or organ from a first organism (the donor) and placing it into a second organism (the recipient). The donor may be a human or a non-human organism. In some embodiments, the donor is a primate. The donor may be a non-human primate. Optionally, the donor is a human. In some embodiments, the donor is a pig or mini-swine. The recipient may be a human or a non-human organism. Preferably, the recipient is a human. Optionally, the recipient is a non-human primate. The cell, tissue or organ being transferred is referred to as the "transplant" or "graft." A "xenotransplant" refers to the transfer of a cell or tissue or organ from a donor of a certain species (such as a monkey or pig) into a recipient of a different species (such as a human).

As used herein, the term "engineered cell" refers to a cell that is modified from its natural state. An engineered cell may be modified using one or more techniques such as transduction to express a cDNA, a CRISPR/Cas9 system, RNAi technology and retroviral technology. For example, the cell may be modified to express a chimeric antigen receptor (CAR) on its surface. Examples of cells that may be transplanted include, but are not limited to a stem cell, a regulatory T (Treg) cell, a CAR-T cell (see Zhang, C et al. Biomarker Research (5)22. (2017), a CAR-B cell (Voss JE et al., Elife. Jan 17;8. (2019), and a tumor-infiltrating lymphocyte (TIL) (Zhang L et al., Clin Cancer Res. May 15;21(10):2278-88. doi: 10.1158/1078-0432.CCR-14-2085 (2015).

As used herein, the term "ex-vivo expanded cell" refers to a cell that is produced in an ex-vivo method to enhance the yield of that cell (such as a hematopoietic stem cell (HSC)) to be used in clinical applications, such as transplantation. See, *e.g.,* Xie J, and Zhang C Sci China Life Sci. Sep;58(9):839-53 (2015) which reviews the methods to expand the numbers of HSCs, including culture systems such as stroma/HSC co-culture, continuous perfusion and fed-batch cultures, and those supplemented with extrinsic ligands, membrane transportable transcription factors, complement components, protein modification enzymes, metabolites, or small molecule chemicals. The desired cell to be transplanted may also be expanded ex-vivo by applying endogenous Notch-signaling activators (see, *e.g.,* Ex vivo expansion of human hematopoietic stem and progenitor cells Dahlberg A, et al., Blood 117:6083-6090 (2011).

As used herein, the term "transplant rejection" refers to the phenomenon that occurs when a transplanted cell, tissue, or organ from a donor is rejected by the recipient's immune system. The recipient's immune system may mount an adaptive immune response (cellular immunity) mediated by killer T cells inducing apoptosis of the donor cells, a humoral immunity mediated by activated B cells secreting antibodies, and/or an innate immune response mediated by phagocytes and soluble immune proteins (see Ochanda J et al., Cell Mol Immunol. Apr;16(4):350-356 (2019); Koo J, and Wang HL. Surg Pathol Clin. Jun;11(2):431-452 (2018); Wang H, and Yang YG, Curr Opin Organ Transplant. Apr;17(2): 162-7 (2012); and da Silva MB, World J Transplant. Feb 24;7(1):1-25 (2017)).

As used herein, the term "immune-related disease" refers to a condition in which the host immune system plays an integral role in mediated the disease and contributes to the progress of the disease. The term encompasses "autoimmune disease" which is a condition that arises when a specific adaptive immune response is mounted against self-antigens, as a result of which the effector pathways of immunity cause chronic inflammatory injury to tissues. Autoimmune diseases include, but are not limited to type I diabetes, juvenile diabetes, autoimmune diabetes, autoimmune hemolytic anemia, rheumatoid arthritis, systemic lupus erythematosus (SLE), psoriasis, multiple sclerosis, inflammatory bowel disease, Addison's disease, Crohn's disease, Graves' disease, Sjögren's syndrome, Hashimoto's thyroiditis, myasthenia gravis, vasculitis, pernicious anemia, celiac diseases, Guillain-Barre syndrome, ankylosing spondylitis, primary biliary cirrhosis, lupus nephritis, Goodpasture's disease, polymyositis, dermatomyositis, psoriasis, temporal arteritis, Churg-Strauss syndrome, transverse myelitis, thyroiditis, ulcerative colitis, sarcoidosis, hemolytic anemia, idiopathic thrombocytopenic purpura, neuromyelitis optica spectrum disorder, paroxysmal nocturnal hemoglobinuria, atypical hemolytic uremic syndrome, and Behcet's disease. Immune-related disease may also refer to an allergic disease which includes, but is not limited to allergic rhinitis, asthma, atopic eczema, anaphylaxis, insect venom allergy, drug allergy, and food allergy.

An immune-related disease may also refer to a dysfunctional immune response that causes or contributes to an inflammatory condition of organs, including but not limited to the lungs, heart, and kidney. For example, acute respiratory distress syndrome (ARDS) is a form of hypoxemic respiratory failure characterized by severe impairment in gas exchange and lung mechanics with a high case fatality rate. ARDS occurs when small blood vessels in the lung leak fluid that enter and may fill up the alveoli. Multiple immunologic processes involving neutrophils, macrophages, and dendritic cells partake in mediating tissue injury in ARDS. See Han, S, and Mallampalli RK. Journal of immunology 194,3: 855-60 (2015). Other inflammatory conditions of the lungs include, but are not limited to pneumonia, bronchitis, pneumonitis, chronic obstructive pulmonary disease (COPD). See Moldoveanu, B et al. Journal of inflammation research (2): 1-11 (2009).

Further, an immune-related disease, such as a dysfunctional immune response that causes or contributes to an inflammatory condition of organs, may be due to a viral infection. For example, ARDS, pneumonia, bronchitis, pneumonitis and COPD can occur in an infection by a virus, such as a cytomegalovirus, an Epstein-Barr virus, an influenza virus, a variola virus, an orthopoxvirus, or a coronavirus, such as SARS coronavirus (SARS-CoV), SARS-CoV-2, and MERS-CoV. Such viral infections may be associated with an inflammatory condition or a dysfunctional immune response such as cytokine release syndrome (CRS), or "cytokine storm" which is a severe immune reaction in which the host releases too many cytokines into the bloodstream too quickly. For example, the host may release a high amount of pro-inflammatory cytokines such as IP-10, MCP-1, MIP-1A, IL-6 and TNF-alpha into the bloodstream. (see Prompetchara E. et al., Asian Pac J Allergy Immunol, 38(1): 1-9 (2020)). A cytokine storm can occur as a result of an infection, an autoimmune condition, or other disease. For example, a cytokine storm can occur in an infection by a virus, such as a cytomegalovirus, an Epstein-Barr virus, an influenza virus, a variola virus, SARS-CoV, SARS-CoV-2, and MERS-CoV. An immune-related disease may refer to a condition associated with a dysfunctional immune response, a cytokine storm or a cytokine storm syndrome in the host. Examples of such viral-mediated immune-related diseases include, but are not limited to, severe acute respiratory syndrome (SARS), Middle East respiratory syndrome (MERS), and coronavirus disease 2019 (COVID-19). The transition from pneumonia to ARDS has also been described as a "cytokine storm". Many patients with severe COVID-19 lung disease manifest typical ARDS, but others manifest an atypical form of ARDS with relatively high compliance and well preserved lung gas volume, which may relate to loss of lung perfusion regulation, hypoxic vasoconstriction (Gattinoni L et al., Am J. Respir Crit Care Med. (2020) and/or microvascular thromboses and potentially disseminated intravascular coagulation (Phend C. MedPage Today, Infectious Disease. April 8, 2020).

As used herein, the term "primary response" refers to the immune response of the body to an antigen that occurs on the first occasion that it is encountered. The term "secondary response" refers to the immune response that involves both B- and T cells, following a subsequent encounter with the same antigen and is more rapid leading to the activation of previously generated memory cells. A secondary response has some quantitative and qualitative differences from the primary response as reviewed in Ademokun and Dunn-Walters, Immune Responses: Primary and Secondary; John Wiley & Sons, (2010) and Kuby Immunology Macmillan; 8th edition (2018). In a primary immune response, the responding cell is a naive B-cell and T-cell while in a secondary response, the responding cell is a memory cell.

As used herein, the term "hematopoietic chimerism" refers to the coexistence of both host and donor hematopoietic cells that arises due to the engraftment of donor pluripotent hematopoietic stem cells into the host. The host and donor cells may be tolerant of each other. Mechanisms of hematopoietic chimerism are known in the art. See Pasquet L, et al. Front Immunol. 2:80 (2011) and Nikolic B, and Sykes M, Immunol Res. 16(3):217-28. (1997) incorporated herein by reference. In some embodiments, such hematopoietic chimerism results in "central tolerance." The mechanisms of "central tolerance" in such chimeras may involve central, intrathymic clonal deletion, selection of regulatory T cells and/or other related immune mechanisms. *See, e.g.,* Nikolic B, and Sykes M, Immunol Res. 16(3):217-28. (1997) and Hogquist KA et al., Nature Reviews Immunology 5:772-782 (2005), incorporated herein by reference. In some embodiments, the hematopoietic stem cells are isolated or purified. Optionally, the hematopoietic stem cells are passenger cells that are transplanted with an organ, *e.g.* a kidney or liver transplant. Stem cells may be derived from bone marrow or fat cells/adipose tissue of the donor.

As used herein, the term "conditioning" or "conditioned" refers to the preparation of a recipient for stem cell transplantation, such as a hematopoietic cell transplantation. Gyurkocza B and Sandmaier BM Blood 124:344-353 (2014) provides a review of high-dose, reduced-intensity, and nonmyeloablative conditioning regiments and the most commonly used agents, such as total body irradiation, fludarabine phosphate, cyclophosphamide, T cell-depleting antibodies, cyclosporine A (CsA). Monoclonal antibodies, such as anti-CD20 Ab, anti-CD33 Ab, and anti-CD45 Ab, may also be used alone or in combination with conventional therapies as part of a conditioning regimen to prevent transplant rejection. *See, e.g.,* Topcuoglu P et al; Progress in Stem Cell Transplantation; December (2015). Other agents that may be used in conditioning regimens include, but are not limited to, BCL-2 inhibitors (Perini GF et al., Journal of Hematology & Oncology 11:65 (2018) and anti-CTLA4 Abs (Pree I et al., Transplantation. Mar 15; 83(5): 663-667 (2007). Conditioning regimens may include chemotherapeutic agents including, but not limited to, Alemtuzumab (CAMPATH^{™}), Busulfan, Carboplatin, Carmustine, Cyclophosphamide, Cytarabine (Ara-C), Daunorubicin, Etoposide (VP-16), Fludarabine, Melphalan, Rituximab, and Vincristine.

The term "isolated antibody" refers to an antibody that is at least partially free of the other biological molecules present in the cells used to produce them. The other biological molecules from which an isolated antibody is free include nucleic acid molecules, proteins, lipids, carbohydrates, cellular debris and culture medium. The term "isolated antibody" does not require, but does encompass, a complete absence of such other biological molecules. The term "isolated antibody" also does not refer to a complete absence of other molecules, such as water, buffers, salts or the components of pharmaceutical formulations. Thus, a molecule that is chemically synthesized or synthesized in a cell-free system will be "isolated" from its naturally associated components. A molecule may also be "isolated" using purification techniques well known in the art.

As used herein, the terms "nucleic acid," "nucleic acid molecule" and "polynucleotide" are used interchangeably and refer a polymeric form of nucleotides of at least 10 bases in length. Polynucleotides may comprise ribonucleotides, deoxynucleotides, modified forms of either type of nucleotide, or a combination thereof. A polynucleotide may be single-stranded or double-stranded.

As used herein, the term "isolated polynucleotide" or "isolated nucleic acid molecule" as used herein means a polynucleotide of genomic, mRNA cDNA, or synthetic origin or some combination thereof, which (1) is not associated with all or a portion of polynucleotide with which the "isolated nucleic acid" is associated with in nature, (2) is operably linked to a polynucleotide to which it is not linked in nature, or (3) does not occur in nature as part of a larger sequence. An isolated polynucleotide "comprising" a specific sequence may also include coding sequences for other proteins or immunoglobulin chains, expression regulatory sequences or vector sequences.

Molecule purity or homogeneity may be assayed by a number of means well known in the art. For example, the purity of an antibody sample may be assayed using polyacrylamide gel electrophoresis and staining of the gel to visualize the antibody using techniques well known in the art. For certain purposes, higher resolution may be provided by using HPLC or other means well known in the art for purification. The purity of a nucleic acid sample may be assayed using the spectrophotometric absorbance of the sample 260nm to that of 280nm using techniques well known in the art. For certain purposes, higher resolution may be provided by using means well known in the art for purification.

Examples of isolated antibodies include, but are not limited to, an anti-CD154 antibody that has been affinity purified using CD154, and an anti-CD154 antibody that has been synthesized by a cell line *in vitro.*

The term "vector", as used herein, refers to a construct, which is capable of delivering, and, preferably, expressing, one or more polynucleotide sequences of interest in a host cell. Non-limiting examples of vectors include viral vectors, naked DNA or RNA expression vectors, bacterial vectors, mammalian vectors, plasmids, cosmids, phage vectors, DNA or RNA expression vectors associated with cationic condensing agents, DNA or RNA expression vectors encapsulated in liposomes, and certain eukaryotic cells, such as producer cells. In some embodiments, the vectors autonomously replicate in a host cell into which they are introduced. Optionally, the vectors integrate into the genome of a host cell and are replicated along with the host genome. Vectors may be capable of directing the expression of coding sequences to which they are operably linked. Such vectors are referred to herein as "recombinant expression vectors" or "expression vectors".

The term "host cell", as used herein, refers to a cell into which a vector or a polynucleotide has been introduced. It should be understood that "host cell" includes not only the particular subject cell but also the progeny of such a cell. Because certain modifications may occur in succeeding generations due to either mutation or environmental influences, such progeny may not, in fact, be identical to the parent cell, but are still included within the scope of the term "host cell" as used herein, provided they still contain the vector, polynucleotide, or a portion thereof, either episomally or integrated into the host-cell genome.

The term "percent sequence identity" in the context of polynucleotide (or polypeptide) sequences is defined as the percentage of nucleic acid (or amino acid) residues in a candidate sequence that are identical with the nucleic acids (or amino acid residues) in the reference nucleotide (or polypeptide) sequence, after aligning the sequences and introducing gaps, if necessary, to achieve the maximum percent sequence identity, and not considering any conservative substitutions as part of the sequence identity. Such conservative substitutions are considered (in addition to identical residues) in calculating the "percent sequence similarity" of two sequences. Residue positions that are not identical but are similar differ by conservative amino acid substitutions.

Alignment for purposes of determining percent amino acid sequence identity, sequence similarity or sequence homology, such as between a wild type protein and a mutein thereof, can be achieved in various ways that are within the skill in the art, for instance, using publicly available sequence analysis computer software such as BLAST, BLAST-2, ALIGN, Megalign (DNASTAR), Gap, BESTFIT^{®}, and other programs in programs in Wisconsin Package Version 10.0 or Genetics Computer Group (GCG), Madison, Wisconsin.software. Those skilled in the art can determine appropriate parameters for aligning sequences, including any algorithms needed to achieve maximal alignment over the full length of the sequences being compared. Polypeptide sequences also can be compared using FASTA using default or recommended parameters. In the context of polypeptide sequences, FASTA takes the query amino acid sequence and searches a sequence database using local sequence alignment to identify similar sequences within the database (Pearson, Methods Enzymol. 183:63-98 (1990); Pearson, Methods Mol. Biol. 132: 185-219 (2000); Pearson Curr Protoc Bioinformatics. Mar 24;53:3.9.1-25 (2016); each herein incorporated by reference). BLAST, especially blastp or tblastn, using default parameters may be used to compare a query sequence to a database containing sequences from different organisms. *See, e.g.,* Altschul et al., J. Mol. Biol. 215:403-410 (1990); Altschul et al., Nucleic Acids Res. 25:3389-402 (1997); Eser et al., PLoS One. 22;9(12): e115445 (2014), each herein incorporated by reference.

A reference herein to a nucleotide sequence encompasses its complement unless otherwise specified. Thus, a reference to a nucleic acid having a particular sequence should be understood to encompass its complementary strand, with its complementary sequence.

The terms "patient," "subject," and "individual" are used interchangeably herein and refer to either a human or a non-human animal in need to treatment. These terms include mammals, such as humans, and primates (*e.g*., monkey). Optionally, the subject is a human. In some embodiments, the subject is in need of inhibition or reduction of an immune response.

The term "primate" refers to a mammal of the order primates, which includes the anthropoids and prosimians, characterized by refined development of the hands and feet, a shortened snout, and a large brain. The mammalian order Primates includes humans, apes, monkeys, and prosimians, or lower primates.

As used herein, the term "therapeutically effective amount" refers to that amount of the therapeutic agent being administered which will relieve to some extent one or more of the symptoms of the condition being treated. With respect to the treatment of transplant rejection, a therapeutically effective amount refers to that amount which has at least one of the following effects: reduces, inhibits or prevents acute or chronic rejection of the transplanted cell, tissue or organ and one or more symptoms associated with the rejection, prolongs graft survival, reduces thrombosis, reduces the risk of life-threatening infections, cancers and other complications, such as cardiovascular diseases, and kidney failure. *See,* for example, Romano et al. Front Immunol. 10:43 (2019) and Ingulli E. Pediatr Nephrol. 25(1):61-74 (2010) for mechanisms of cellular rejection in transplantation. With respect to the treatment of autoimmune disease and antibody-mediated inflammatory disease, a therapeutically effective amount refers to that amount which has at least one of the following effects: reduces one or more symptoms associated with the autoimmune disease such as fatigue, muscle aches, low fever, inflammation, skin rashes, etc.

The pharmaceutical compositions may include a therapeutically effective amount or a prophylactically effective amount of an antibody disclosed herein. A therapeutically effective amount of the antibody may vary according to factors such as the disease state, age, sex, and weight of the individual, and the ability of the antibody or antibody portion to elicit a desired response in the individual. A therapeutically effective amount is also one in which any toxic or detrimental effects of the antibody are outweighed by the therapeutically beneficial effects. It is routine in the art for the skilled artisan to determine a therapeutically effective amount of an antibody disclosed herein based on these factors. A "prophylactically effective amount" refers to an amount effective, at dosages and for periods of time necessary, to achieve the desired prophylactic result. Typically, since a prophylactic dose is used in subjects prior to transplantation or at an earlier stage of transplant rejection, the prophylactically effective amount may be less than the therapeutically effective amount.

As used herein, the terms "treat", "treating" and "treatment" refer the administration of a therapeutic agent, such as a composition containing any of the antibodies disclosed herein, internally or externally to a subject or patient having one or more disease symptoms, or being suspected of having a disease, for which the agent has therapeutic activity. "Treat", "treating" and "treatment" refer to therapeutic treatments and/or prophylactic treatments. Therapeutic treatment includes, *e.g*., a method of alleviating or reducing the severity of a condition or abolishing a condition and includes alleviating or reducing the severity of one or more symptoms of the condition. If the treatment is administered prior to clinical manifestation of a condition, the treatment is considered prophylactic. The alleviation or reduction of a disease symptom can be assessed by any clinical measurement typically used by physicians or other skilled artisans to assess the severity or progression of that symptom. The terms further refer to a postponement of development of one or more disease symptoms and/or a reduction in the severity of one or more disease symptoms. The terms further include ameliorating existing uncontrolled or unwanted disease symptoms, preventing additional disease symptoms, and ameliorating or preventing the underlying causes of such disease symptoms. Thus, the terms denote that a beneficial result has been conferred on the subject.

With respect to the treatment of transplant rejection, treatment may refer to the alleviation, reduction, or delay of rejection of the transplanted cell, tissue or organ or one or more symptoms associated with the rejection. Treatment may also result in prolonging graft survival, reducing thrombosis, and/or reducing the risk of life-threatening infections, cancers and other complications, such as cardiovascular diseases, and kidney failure. With respect to the treatment of autoimmune disease, treatment may refer to dampening of the body's immune responses and controlling the autoimmune reaction. With respect to the treatment of antibody-mediated inflammatory disease, treatment may refer to reducing one or more symptoms associated with the autoimmune disease such as fatigue, muscle aches, low fever, inflammation, skin rashes, etc. With regard to treatment with the antibodies disclosed herein, these terms may simply mean that the life expectancy and quality of life of an individual receiving a transplant or an individual affected with an autoimmune or inflammatory disease will be increased or that one or more of the symptoms associated with transplant rejection or the autoimmune or inflammatory disease will be reduced.

As used herein, the terms "prevent", "preventing" and "prevention" refer to the prevention or delay of the recurrence or onset of, or a reduction in one or more symptoms of a condition in a subject as a result of the administration of an anti-CD 154 antibody of the disclosure. For example, in the context of the administration of a therapy to a subject, "prevent", "preventing" and "prevention" refer to the inhibition, reduction, or delay in the development or onset of the rejection of that transplant or associated thrombosis or the prevention or delay of the recurrence, onset, or development of one or more symptoms associated with the transplantation in a subject (*e.g*., a solid organ transplant) or the administration of a combination of therapies (*e.g*., a combination of a solid organ transplant and an immunosuppressant).

As used herein, the terms "administering" or "administration of" the antibodies or compositions of this disclosure to a subject refers to refers to contacting the antibodies or compositions to the subject or to a cell, tissue, organ, or biological fluid of the subject. Such administration can be carried out using one of a variety of methods known to those skilled in the art. For example, an antibody or a composition of this disclosure can be administered systemically or locally. In some embodiments, the composition can be administered subcutaneously, intravenously, intravitreally, orally, via inhalation, transdermally, or rectally. Administering can also be performed, for example, once, a plurality of times, and/or over one or more extended periods. In some embodiments, the administration includes both direct administration (including self-administration) and indirect administration, including the act of prescribing a drug.

As used herein, the term "highly stringent conditions" refers to hybridization to filter-bound DNA in 0.1x sodium chloride/sodium citrate (SSC) at 65 °C, followed by one or more washes in 0.1× SSC, 0.1% SDS at 50-65 °C. (Ausubel et al., eds., 1989, Current Protocols in Molecular Biology, Vol. I, Green Publishing Associates, Inc., and John Wiley & Sons, Inc., N.Y., at p. 2.10.3).

### Anti-CD154 Antibodies

A first aspect of the present disclosure provides isolated anti-CD 154 antibodies with modified effector functions that bind to mammalian CD154, more preferably human CD154. In some embodiments, one or more effector functions are reduced. Optionally, one or more effector functions are eliminated. In some embodiments, the antibodies with one or more reduced effector functions demonstrate reduced binding to Fc receptors relative to an antibody having wild-type IgG1 heavy chain. Optionally, the reduced binding to the Fc receptor is 10- to 3200-fold weaker than the binding demonstrated by a wild-type IgG heavy chain. In some embodiments, the K_{D} for FcyRIA binding the Fc domain of the antibody with reduced effector function is between 0.1-100 nM, such as 0.3-92 nM. Optionally, the K_{D} for CD 16aF binding the Fc domain of the antibody with one or more reduced effector functions is greater than 2 µM. In some embodiments, the K_{D} for CD16aV binding the Fc domain of the antibody with one or more reduced effector functions is greater than 0.4 µM. Optionally, the K_{D} for CD32aH binding the Fc domain of the antibody with one or more reduced effector functions is greater than 0.5 µM. In some embodiments, the K_{D} for CD32bF binding the Fc domain of the antibody with one or more reduced effector functions is greater than 1 µM.

In some embodiments, the isolated antibodies are fully human monoclonal antibodies. In some embodiments, the isolated antibodies are chimeric antibodies. In some embodiments, the isolated antibodies are humanized antibodies. In some embodiments, human anti-CD154 antibodies are produced by immunizing a non-human transgenic animal, *e.g.,* a rodent, whose genome comprises human immunoglobulin genes so that the transgenic animal produces human antibodies.

In some embodiments, the anti-CD154 antibody comprises a human or humanized variable region, wherein the variable region comprises a heavy chain variable domain (VH) and a light chain variable domain (VL), and wherein the VH is operably linked to a human Fc domain with modified effector functions. In some embodiments, one or more effector functions of the human Fc domain are reduced. Optionally, one or more effector functions of the human Fc domain are eliminated. Optionally, the VH is operably linked to a human Fc region, wherein the human Fc region comprises a human hinge sequence and the human Fc domain, wherein the human hinge sequence is between the VH and the human Fc domain. In some embodiments, one or more effector functions of the human Fc region are reduced. Optionally, one or more effector functions of the human Fc region are eliminated. The hinge may comprise the amino acid sequence of any one of SEQ ID NOs: 76-90. In some embodiments, the hinge comprises the amino acid sequence of SEQ ID NO: 76. Optionally, the hinge comprises the amino acid sequence of SEQ ID NO: 77. The hinge may comprise the amino acid sequence of SEQ ID NO: 78. In some embodiments, the hinge comprises the amino acid sequence of SEQ ID NO: 79. Optionally, the hinge comprises the amino acid sequence of SEQ ID NO: 80. The hinge may comprise the amino acid sequence of SEQ ID NO: 81. In some embodiments, the hinge comprises the amino acid sequence of SEQ ID NO: 82. Optionally, the hinge comprises the amino acid sequence of SEQ ID NO: 83. The hinge may comprise the amino acid sequence of SEQ ID NO: 84. In some embodiments, the hinge comprises the amino acid sequence of SEQ ID NO: 85. Optionally, the hinge comprises the amino acid sequence of SEQ ID NO: 86. The hinge may comprise the amino acid sequence of SEQ ID NO: 87. In some embodiments, the hinge comprises the amino acid sequence of SEQ ID NO: 88. Optionally, the hinge comprises the amino acid sequence of SEQ ID NO: 89. The hinge may comprise the amino acid sequence of SEQ ID NO: 90.

In some embodiments, the human Fc domain is derived from an IgG1 Fc (or crystallizable fragment) region. Optionally, the human Fc domain is derived from an IgG1 constant region. The human Fc domain may be derived from an IgG2 Fc (or crystallizable fragment) region. In some embodiments, the Fc domain is derived from an IgG2 constant region. The human Fc domain may be derived from an IgG4 Fc (or crystallizable fragment) region. In some embodiments, the Fc domain is derived from an IgG4 constant region. In some embodiments, the human Fc region is derived from an IgG1 Fc (or crystallizable fragment) region. Optionally, the human Fc region is derived from an IgG1 constant region. In some embodiments, the human Fc region is derived from an IgG2 Fc (or crystallizable fragment) region. In some embodiments, the Fc region is derived from an IgG2 constant region. Optionally, the human Fc region is derived from an IgG4 Fc (or crystallizable fragment) region. The human Fc region may be derived from an IgG4 constant region.

In some embodiments, the Fc domain of the antibody disclosed herein comprises one or more amino acid modifications that modify effector functions, including reducing or eliminating one or more effector functions. In some embodiments, the Fc domain comprises an amino acid sequence that is at least 70%, 75%, 80%, 85%, 90%, 95%, 97%, 98% or 99% identical to the amino acid sequence of any one of SEQ ID NOs: 3-9, 12-18, and 238-241. Optionally, the Fc domain comprises an amino acid sequence that is at least 70%, 75%, 80%, 85%, 90%, 95%, 97%, 98% or 99% similar to the amino acid sequence of any one of SEQ ID NOs: 3-9, 12-18, and 238-241. The Fc domain may comprise an amino acid sequence selected from the group consisting of SEQ ID NOs÷ 3-9, 12-18, and 238-241. In some embodiments, the Fc domain comprises an amino acid sequence that is at least 70%, 75%, 80%, 85%, 90%, 95%, 97%, 98% or 99% identical to the amino acid sequence of any one of SEQ ID NOs: 3-9, 12-18, and 236-241. Optionally, the Fc domain comprises an amino acid sequence that is at least 70%, 75%, 80%, 85%, 90%, 95%, 97%, 98% or 99% similar to the amino acid sequence of any one of SEQ ID NOs: 3-9, 12-18, and 236-241. In some embodiments, the Fc domain comprises the amino acid sequence of SEQ ID NO: 3. Optionally, the Fc domain comprises the amino acid sequence of SEQ ID NO: 4. In some embodiments, the Fc domain comprises the amino acid sequence of SEQ ID NO: 5. Optionally, the Fc domain comprises the amino acid sequence of SEQ ID NO: 6. In some embodiments, the Fc domain comprises the amino acid sequence of SEQ ID NO: 7. Optionally, the Fc domain comprises the amino acid sequence of SEQ ID NO: 8. In some embodiments, the Fc domain comprises the amino acid sequence of SEQ ID NO: 9. Optionally, the Fc domain comprises the amino acid sequence of SEQ ID NO: 12. In some embodiments, the Fc domain comprises the amino acid sequence of SEQ ID NO: 13. Optionally, the Fc domain comprises the amino acid sequence of SEQ ID NO: 14. In some embodiments, the Fc domain comprises the amino acid sequence of SEQ ID NO: 15. Optionally, the Fc domain comprises the amino acid sequence of SEQ ID NO: 16. In some embodiments, the Fc domain comprises the amino acid sequence of SEQ ID NO: 17. Optionally, the Fc domain comprises the amino acid sequence of SEQ ID NO: 18. In some embodiments, the Fc domain comprises the amino acid sequence of SEQ ID NO: 236. Optionally, the Fc domain comprises the amino acid sequence of SEQ ID NO: 237. In some embodiments, the Fc domain comprises the amino acid sequence of SEQ ID NO: 238. Optionally, the Fc domain comprises the amino acid sequence of SEQ ID NO: 239. In some embodiments, the Fc domain comprises the amino acid sequence of SEQ ID NO: 240. Optionally, the Fc domain comprises the amino acid sequence of SEQ ID NO: 241. In some embodiments, the Fc domain does not comprise the amino acid sequence of SEQ ID NO: 236. Optionally, the Fc domain does not comprise the amino acid sequence of SEQ ID NO: 237. In some embodiments, the Fc domain does not comprise the amino acid sequence of SEQ ID NOs: 236 and 237.

In some embodiments, the Fc region of the antibody of an antibody disclosed herein comprises one or more amino acid modifications that modify effector functions, including reducing or eliminating one or more effector functions. In some embodiments, the Fc region comprises an amino acid sequence that is at least 70%, 75%, 80%, 85%, 90%, 95%, 97%, 98% or 99% identical to the amino acid sequence of any one of SEQ ID NOs: 21-37, 40-56 and 243-251. Optionally, the Fc region comprises an amino acid sequence that is at least 70%, 75%, 80%, 85%, 90%, 95%, 97%, 98% or 99% similar to the amino acid sequence of any one of SEQ ID NOs: 21-37, 40-56 and 243-251. The Fc region may comprise an amino acid sequence selected from the group consisting of SEQ ID NOs÷ 21-37, 40-56 and 243-251. In some embodiments, the Fc region comprises an amino acid sequence that is at least 70%, 75%, 80%, 85%, 90%, 95%, 97%, 98% or 99% identical to the amino acid sequence of any one of SEQ ID NOs: 21-37, 40-56 and 242-251. Optionally, the Fc region comprises an amino acid sequence that is at least 70%, 75%, 80%, 85%, 90%, 95%, 97%, 98% or 99% similar to the amino acid sequence of any one of SEQ ID NOs: 21-37, 40-56 and 242-251. The Fc region may comprise an amino acid sequence selected from the group consisting of SEQ ID NOs: 21-37, 40-56 and 242-251. In some embodiments, the Fc region comprises the amino acid sequence of SEQ ID NO: 21. Optionally, the Fc region comprises the amino acid sequence of SEQ ID NO: 22. In some embodiments, the Fc region comprises the amino acid sequence of SEQ ID NO: 23. Optionally, the Fc region comprises the amino acid sequence of SEQ ID NO: 24. In some embodiments, the Fc region comprises the amino acid sequence of SEQ ID NO: 25. Optionally, the Fc region comprises the amino acid sequence of SEQ ID NO: 26. In some embodiments, the Fc region comprises the amino acid sequence of SEQ ID NO: 27. Optionally, the Fc region comprises the amino acid sequence of SEQ ID NO: 28. In some embodiments, the Fc region comprises the amino acid sequence of SEQ ID NO: 29. Optionally, the Fc region comprises the amino acid sequence of SEQ ID NO: 30. In some embodiments, the Fc region comprises the amino acid sequence of SEQ ID NO: 31. Optionally, the Fc region comprises the amino acid sequence of SEQ ID NO: 32. In some embodiments, the Fc region comprises the amino acid sequence of SEQ ID NO: 33. Optionally, the Fc region comprises the amino acid sequence of SEQ ID NO: 34. In some embodiments, the Fc region comprises the amino acid sequence of SEQ ID NO: 35. Optionally, the Fc region comprises the amino acid sequence of SEQ ID NO: 36. In some embodiments, the Fc region comprises the amino acid sequence of SEQ ID NO: 37. In some embodiments, the Fc region comprises the amino acid sequence of SEQ ID NO: 40. Optionally, the Fc region comprises the amino acid sequence of SEQ ID NO: 41. In some embodiments, the Fc region comprises the amino acid sequence of SEQ ID NO: 42. Optionally, the Fc region comprises the amino acid sequence of SEQ ID NO: 43. In some embodiments, the Fc region comprises the amino acid sequence of SEQ ID NO: 44. Optionally, the Fc region comprises the amino acid sequence of SEQ ID NO: 45. In some embodiments, the Fc region comprises the amino acid sequence of SEQ ID NO: 46. Optionally, the Fc region comprises the amino acid sequence of SEQ ID NO: 47. In some embodiments, the Fc region comprises the amino acid sequence of SEQ ID NO: 48. Optionally, the Fc region comprises the amino acid sequence of SEQ ID NO: 49. In some embodiments, the Fc region comprises the amino acid sequence of SEQ ID NO: 50. Optionally, the Fc region comprises the amino acid sequence of SEQ ID NO: 51. In some embodiments, the Fc region comprises the amino acid sequence of SEQ ID NO: 52. Optionally, the Fc region comprises the amino acid sequence of SEQ ID NO: 53. In some embodiments, the Fc region comprises the amino acid sequence of SEQ ID NO: 54. Optionally, the Fc region comprises the amino acid sequence of SEQ ID NO: 55. In some embodiments, the Fc region comprises the amino acid sequence of SEQ ID NO: 56. In some embodiments, the Fc region comprises the amino acid sequence of SEQ ID NO: 242. Optionally, the Fc region comprises the amino acid sequence of SEQ ID NO: 243. In some embodiments, the Fc region comprises the amino acid sequence of SEQ ID NO: 244. Optionally, the Fc region comprises the amino acid sequence of SEQ ID NO: 245. In some embodiments, the Fc region comprises the amino acid sequence of SEQ ID NO: 246. Optionally, the Fc region comprises the amino acid sequence of SEQ ID NO: 247. In some embodiments, the Fc region comprises the amino acid sequence of SEQ ID NO: 248. Optionally, the Fc region comprises the amino acid sequence of SEQ ID NO: 249. In some embodiments, the Fc region comprises the amino acid sequence of SEQ ID NO: 250. Optionally, the Fc region comprises the amino acid sequence of SEQ ID NO: 251. In some embodiments, the Fc domain does not comprise the amino acid sequence of SEQ ID NO: 236. Optionally, the Fc domain does not comprise the amino acid sequence of SEQ ID NO: 237. In some embodiments, the Fc domain does not comprise the amino acid sequence of SEQ ID NO: 242. Optionally, the Fc domain does not comprise the amino acid sequence of SEQ ID NOs: 236, 237 and 242.

In some embodiments, the antibody comprising the IgG4-derived Fc domain or Fc region comprises an amino acid modification at any one of the positions selected from the group consisting of S228, L235, L236, G237, E318, and N297 or a combination thereof, wherein the numbering of amino acid residues is according to the EU index as set forth in Edelman GM et al., Proc. Natl. Acad. USA, 63, 78-85 (1969). In preferred embodiments, the antibody comprising the IgG4-derived Fc domain or Fc region comprises an amino acid modification selected from the group consisting of S228P, L235A, L235E, L236E, G237A, E318A, and N297Q or a combination thereof. See, *e.g*., Figure 9c.

In some embodiments, the antibody comprising the IgG1-derived Fc domain or Fc region comprises an amino acid modification at any one of the positions selected from the group consisting E216, R217, K218, C219, C220, C226, C229, P230, E233, L234, L235, G236, G237, P238, S239, V240, F241, K246, L251, T260, D265, V266, H268, W277, N297, E318, K322, P329, A330, P331, Q347, N348, T350, L351, K360, T366, N390, K392, T394, D399, S400, F405, Y407, K409, T411 or a combination such amino acid modifications, wherein the numbering of amino acid residues is according to the EU index as set forth in Edelman GM et al., Proc. Natl. Acad. USA, 63, 78-85 (1969). In preferred embodiments, the antibody comprising the IgG1-derived Fc domain or Fc region comprises an amino acid modification selected from the group consisting of C220S, C226S, C229S, P230S, E233P, L234A, L234F, L234V, L235A, L235E, L235V, G236E, G237A, P238S, D265S, D265A, H268Q, W277T, N297G, N297Q, N297D, N297A, E318A, K322A, P329G, P329A, A330S, P331S, Q347R, Q347E, Q347K, T350V, L351Y, K360D, K360E, T366A, T366I, T366L, T366M, T366V, N390R, N390K, N390D, K392V, K392M, K392R, K392L, K392F, K392E, T394W, D399R, D399W, D399K, S400E, S400D, S400R, S400K, F405A, F405I, F405M, F405T, F405S, F405V, F405W, Y407A, Y407I, Y407L, Y407V, K409F, K409I, K409S, K409W, T411N, T411R, T411Q, T411K, T411D, T411E, T411W, ΔE216-E222, K246R/L251E/T260R, InR234/235, InV235/236, InR236/237, InR237/238, InV238/239, InN238/239, InL238/239, InE238/239, InG238/239, InS239/240, InG240/241, InE240/241, InG240/241, InL238/239/P238Q, InE238/239/N348A, InS239/240/V266A, and InR237/238/G236A or a combination thereof. See, *e.g*., Figure 9a.

In some embodiments, the antibody comprising the IgG2-derived Fc domain or Fc region comprises an amino acid modification at any one of the positions selected from the group consisting of V234, G237, P238, H268, V309, A330, and P331 or a combination thereof, wherein the numbering of amino acid residues is according to the EU numbering as set forth in Edelman. In preferred embodiments, the antibody comprising the IgG2-derived Fc domain or Fc region comprises an amino acid modification selected from the group consisting of V234A, G237A, P238S, H268Q, H268A, V309L, A330S, P331S, or a combination thereof. See, *e.g*., Figure 9b.

In some embodiments, the VH comprises (a) a heavy chain CDR1 having the amino acid sequence of SEQ ID NO: 57, (b) a heavy chain CDR2 having the amino acid sequence of SEQ ID NO: 58, and (c) a heavy chain CDR3 having the amino acid sequence of SEQ ID NO: 59; and the VL comprises (a) a light chain CDR1 having the amino acid sequence of SEQ ID NO: 60, (b) a light chain CDR2 having the amino acid sequence of SEQ ID NO: 61, and (c) a light chain CDR3 having the amino acid sequence of SEQ ID NO: 62.

In some embodiments, the VH comprises an amino acid sequence that is at least 70%, 75%, 80%, 85%, 90%, 95%, 97%, 98% or 99% identical to the amino acid sequence of SEQ ID NO: 63, 64, 252 or 253. Optionally, the VH comprise an amino acid sequence that is at least 70%, 75%, 80%, 85%, 90%, 95%, 97%, 98% or 99% similar to the amino acid sequence of SEQ ID NO: 63, 64, 252 or 253. The VH may comprise the amino acid sequence of SEQ ID NO: 63, 64, 252 or 253. In some embodiments, the VH comprises the amino acid sequence of SEQ ID NO: 63. Optionally, the VH comprises the amino acid sequence of SEQ ID NO: 64. In some embodiments, the VH comprises the amino acid sequence of SEQ ID NO: 252. Optionally, the VH comprises the amino acid sequence of SEQ ID NO: 253. In some-embodiments, the VH does not comprise the amino acid sequence of SEQ ID NO: 233.

In some embodiments, the VL comprises an amino acid sequence that is at least 70%, 75%, 80%, 85%, 90%, 95%, 97%, 98% or 99% identical to the amino acid sequence of SEQ ID NO: 65 or 66. Optionally, the VL comprise an amino acid sequence that is at least 70%, 75%, 80%, 85%, 90%, 95%, 97%, 98% or 99% similar to the amino acid sequence of SEQ ID NO: 65 or 66. The VL may comprise the amino acid sequence of SEQ ID NO: 65 or 66. In some embodiments, the VL comprises the amino acid sequence of SEQ ID NO: 65. Optionally, the VL comprises the amino acid sequence of SEQ ID NO: 66.

In some embodiments, the antibody further comprises a CH1 domain, wherein the CH1 domain is operably linked to (a) the C-terminal end of the VH, and (b) the N-terminal end of the hinge. Optionally, the CH1 domain comprises an amino acid sequence that is at least 80% 85%, 90%, 95%, 97%, 98% or 99% identical to the amino acid sequence of any one of SEQ ID NOs: 67, 70, and 73. The CH1 domain may comprise an amino acid sequence that is at least 80% 85%, 90%, 95%, 97%, 98% or 99% similar to the amino acid sequence of any one of SEQ ID NOs: 67, 70, and 73. In some embodiments, the CH1 domain comprises the amino acid sequence of any one of SEQ ID NO: 67, 70, and 73. The CH1 domain may comprise the amino acid sequence of SEQ ID NO: 67. Optionally, the CH1 domain comprises the amino acid sequence of SEQ ID NO: 70. In some embodiments, the CH1 domain comprises the amino acid sequence of SEQ ID NO: 73.

In some embodiments, the antibody comprises a linker between the VH and the Fc domain. Optionally, the linker comprises the amino acid sequence of any one of SEQ ID NOs: 199-223 and 327-330. In some embodiments, the linker comprises the amino acid sequence of any one of SEQ ID NOs: 199-223. The linker may comprise the amino acid sequence of any one of SEQ ID NOs: 327-330. In some embodiments, the linker comprises the amino acid sequence of SEQ ID NO: 199. Optionally, the linker comprises the amino acid sequence of SEQ ID NO: 200. In some embodiments, the linker comprises the amino acid sequence of SEQ ID NO: 201. Optionally, the linker comprises the amino acid sequence of SEQ ID NO: 202. In some embodiments, the linker comprises the amino acid sequence of SEQ ID NO: 203. Optionally, the linker comprises the amino acid sequence of SEQ ID NO: 204. In some embodiments, the linker comprises the amino acid sequence of SEQ ID NO: 205. Optionally, the linker comprises the amino acid sequence of SEQ ID NO: 206. In some embodiments, the linker comprises the amino acid sequence of SEQ ID NO: 207. Optionally, the linker comprises the amino acid sequence of SEQ ID NO: 208. In some embodiments, the linker comprises the amino acid sequence of SEQ ID NO: 209. Optionally, the linker comprises the amino acid sequence of SEQ ID NO: 210. In some embodiments, the linker comprises the amino acid sequence of SEQ ID NO: 211. Optionally, the linker comprises the amino acid sequence of SEQ ID NO: 212. In some embodiments, the linker comprises the amino acid sequence of SEQ ID NO: 213. Optionally, the linker comprises the amino acid sequence of SEQ ID NO: 214. In some embodiments, the linker comprises the amino acid sequence of SEQ ID NO: 215. Optionally, the linker comprises the amino acid sequence of SEQ ID NO: 216. In some embodiments, the linker comprises the amino acid sequence of SEQ ID NO: 217. Optionally, the linker comprises the amino acid sequence of SEQ ID NO: 218. In some embodiments, the linker comprises the amino acid sequence of SEQ ID NO: 219. Optionally, the linker comprises the amino acid sequence of SEQ ID NO: 220. In some embodiments, the linker comprises the amino acid sequence of SEQ ID NO: 221. Optionally, the linker comprises the amino acid sequence of SEQ ID NO: 222. In some embodiments, the linker comprises the amino acid sequence of SEQ ID NO: 223. Optionally, the linker comprises the amino acid sequence of SEQ ID NO: 327. In some embodiments, the linker comprises the amino acid sequence of SEQ ID NO: 328. Optionally, the linker comprises the amino acid sequence of SEQ ID NO: 329. In some embodiments, the linker comprises the amino acid sequence of SEQ ID NO: 330. The linker may be between the VH and the hinge. In some embodiments, the linker is between the VH and the CH1 domain.

In some embodiments, the VH is operably linked to an amino acid sequence that is at least 80% 85%, 90%, 95%, 97%, 98% or 99% identical to the amino acid sequence of any one of SEQ ID NOs: 3-9, 12-18, 21-37, 40-56, 238-241, and 243-251. Optionally, the VH is operably linked to an amino acid sequence that is at least 80% 85%, 90%, 95%, 97%, 98% or 99% similar to the amino acid sequence of any one of SEQ ID NOs: 3-9, 12-18, 21-37, 40-56, 238-241, and 243-251. The VH may be operably linked to the amino acid sequence of any one of SEQ ID NOs: 3-9, 12-18, 21-37, 40-56, 238-241, and 243-251. In some embodiments, the VH is operably linked to the amino acid sequence of SEQ ID NO: 3. Optionally, the VH is operably linked to the amino acid sequence of SEQ ID NO: 4. The VH may be operably linked to the amino acid sequence of SEQ ID NO: 5. In some embodiments, the VH is operably linked to the amino acid sequence of SEQ ID NO: 6. Optionally, the VH is operably linked to the amino acid sequence of SEQ ID NO: 7. The VH may be operably linked to the amino acid sequence of SEQ ID NO: 8. In some embodiments, the VH is operably linked to the amino acid sequence of SEQ ID NO: 9. Optionally, the VH is operably linked to the amino acid sequence of SEQ ID NO: 12. The VH may be operably linked to the amino acid sequence of SEQ ID NO: 13. In some embodiments, the VH is operably linked to the amino acid sequence of SEQ ID NO: 14. Optionally, the VH is operably linked to the amino acid sequence of SEQ ID NO: 15. The VH may be operably linked to the amino acid sequence of SEQ ID NO: 16. In some embodiments, the VH is operably linked to the amino acid sequence of SEQ ID NO: 17. Optionally, the VH is operably linked to the amino acid sequence of SEQ ID NO: 18. The VH may be operably linked to the amino acid sequence of SEQ ID NO: 21. In some embodiments, the VH is operably linked to the amino acid sequence of SEQ ID NO: 22. Optionally, the VH is operably linked to the amino acid sequence of SEQ ID NO: 23. The VH may be operably linked to the amino acid sequence of SEQ ID NO: 24. In some embodiments, the VH is operably linked to the amino acid sequence of SEQ ID NO: 25. Optionally, the VH is operably linked to the amino acid sequence of SEQ ID NO: 26. The VH may be operably linked to the amino acid sequence of SEQ ID NO: 27. In some embodiments, the VH is operably linked to the amino acid sequence of SEQ ID NO: 28. Optionally, the VH is operably linked to the amino acid sequence of SEQ ID NO: 29. The VH may be operably linked to the amino acid sequence of SEQ ID NO: 30. In some embodiments, the VH is operably linked to the amino acid sequence of SEQ ID NO: 31. Optionally, the VH is operably linked to the amino acid sequence of SEQ ID NO: 32. The VH may be operably linked to the amino acid sequence of SEQ ID NO: 33. In some embodiments, the VH is operably linked to the amino acid sequence of SEQ ID NO: 34. Optionally, the VH is operably linked to the amino acid sequence of SEQ ID NO: 35. The VH may be operably linked to the amino acid sequence of SEQ ID NO: 36. In some embodiments, the VH is operably linked to the amino acid sequence of SEQ ID NO: 37. Optionally, the VH is operably linked to the amino acid sequence of SEQ ID NO: 40. The VH may be operably linked to the amino acid sequence of SEQ ID NO: 41. In some embodiments, the VH is operably linked to the amino acid sequence of SEQ ID NO: 42. Optionally, the VH is operably linked to the amino acid sequence of SEQ ID NO: 43. The VH may be operably linked to the amino acid sequence of SEQ ID NO: 44. In some embodiments, the VH is operably linked to the amino acid sequence of SEQ ID NO: 45. Optionally, the VH is operably linked to the amino acid sequence of SEQ ID NO: 46. The VH may be operably linked to the amino acid sequence of SEQ ID NO: 47. In some embodiments, the VH is operably linked to the amino acid sequence of SEQ ID NO: 48. Optionally, the VH is operably linked to the amino acid sequence of SEQ ID NO: 49. The VH may be operably linked to the amino acid sequence of SEQ ID NO: 50. In some embodiments, the VH is operably linked to the amino acid sequence of SEQ ID NO: 51. Optionally, the VH is operably linked to the amino acid sequence of SEQ ID NO: 52. The VH may be operably linked to the amino acid sequence of SEQ ID NO: 53. In some embodiments, the VH is operably linked to the amino acid sequence of SEQ ID NO: 54. Optionally, the VH is operably linked to the amino acid sequence of SEQ ID NO: 55. The VH may be operably linked to the amino acid sequence of SEQ ID NO: 56. Optionally, the VH is operably linked to the amino acid sequence of SEQ ID NO: 236. The VH may be operably linked to the amino acid sequence of SEQ ID NO: 237. Optionally, the VH is operably linked to the amino acid sequence of SEQ ID NO: 238. The VH may be operably linked to the amino acid sequence of SEQ ID NO: 239. In some embodiments, the VH is operably linked to the amino acid sequence of SEQ ID NO: 240. Optionally, the VH is operably linked to the amino acid sequence of SEQ ID NO: 241. In some embodiments, the VH is operably linked to the amino acid sequence of SEQ ID NO: 242. The VH may be operably linked to the amino acid sequence of SEQ ID NO: 243. Optionally, the VH is operably linked to the amino acid sequence of SEQ ID NO: 244. The VH may be operably linked to the amino acid sequence of SEQ ID NO: 245. Optionally, the VH is operably linked to the amino acid sequence of SEQ ID NO: 246. The VH may be operably linked to the amino acid sequence of SEQ ID NO: 247. Optionally, the VH is operably linked to the amino acid sequence of SEQ ID NO: 248. The VH may be operably linked to the amino acid sequence of SEQ ID NO: 249. Optionally, the VH is operably linked to the amino acid sequence of SEQ ID NO: 250. The VH may be operably linked to the amino acid sequence of SEQ ID NO: 251. Optionally, the VH is not operably linked to the amino acid sequence of SEQ ID NO: 236. In some embodiments, the VH is not be operably linked to the amino acid sequence of SEQ ID NO: 237. Optionally, the VH is not be operably linked to the amino acid sequence of SEQ ID NO: 242. In some embodiments, the VH is not be operably linked to the amino acid sequence of SEQ ID NOs: 236 and 237. Optionally, the VH is not be operably linked to the amino acid sequence of SEQ ID NOs: 236, 237 and 242.

In some embodiments, the heavy chain comprises an amino acid sequence that is at least 70%, 75%, 80%, 85%, 90%, 95%, 97%, 98% or 99% identical to the amino acid sequence of any one of SEQ ID NOs: 121-132, 135-146, 149-160, 163-174 and 266-288. Optionally, the heavy chain comprises an amino acid sequence that is at least 70%, 75%, 80%, 85%, 90%, 95%, 97%, 98% or 99% similar to the amino acid sequence of any one of SEQ ID NOs: 121-132, 135-146, 149-160, 163-174 and 266-288. The heavy chain may comprise the amino acid sequence of any one of SEQ ID NOs: 121-132, 135-146, 149-160, 163-174 and 266-288. In some embodiments, the heavy chain comprises an amino acid sequence that is at least 70%, 75%, 80%, 85%, 90%, 95%, 97%, 98% or 99% identical to the amino acid sequence of any one of SEQ ID NOs: 121-132, 135-146, 149-160, 163-174, 266-277, and 279-288. Optionally, the heavy chain comprises an amino acid sequence that is at least 70%, 75%, 80%, 85%, 90%, 95%, 97%, 98% or 99% similar to the amino acid sequence of any one of SEQ ID NOs: 121-132, 135-146, 149-160, 163-174, 266-277, and 279-288. The heavy chain may comprise the amino acid sequence of any one of SEQ ID NOs: 121-132, 135-146, 149-160, 163-174, 266-277, and 279-288. In some embodiments, the heavy chain comprises an amino acid sequence that is at least 70%, 75%, 80%, 85%, 90%, 95%, 97%, 98% or 99% identical to the amino acid sequence of any one of SEQ ID NOs: 121-132, 135-146, 149, 151-160, 163, 165-174 and 266-288. Optionally, the heavy chain comprises an amino acid sequence that is at least 70%, 75%, 80%, 85%, 90%, 95%, 97%, 98% or 99% similar to the amino acid sequence of any one of SEQ ID NOs: 121-132, 135-146, 149, 151-160, 163, 165-174 and 266-288. The heavy chain may comprise the amino acid sequence of any one of SEQ ID NOs: 121-132, 135-146, 149, 151-160, 163, 165-174 and 266-288. In some embodiments, the heavy chain comprises an amino acid sequence that is at least 70%, 75%, 80%, 85%, 90%, 95%, 97%, 98% or 99% identical to the amino acid sequence of any one of SEQ ID NOs: 121-132, 135-146, 149, 151-160, 163, 165-174, 266-277, and 279-288. Optionally, the heavy chain comprises an amino acid sequence that is at least 70%, 75%, 80%, 85%, 90%, 95%, 97%, 98% or 99% similar to the amino acid sequence of any one of SEQ ID NOs: 121-132, 135-146, 149, 151-160, 163, 165-174, 266-277, and 279-288. The heavy chain may comprise the amino acid sequence of any one of SEQ ID NOs: 121-132, 135-146, 149, 151-160, 163, 165-174, 266-277, and 279-288. In some embodiments, the heavy chain comprises an amino acid sequence that is at least 70%, 75%, 80%, 85%, 90%, 95%, 97%, 98% or 99% identical to the amino acid sequence of any one of SEQ ID NOs: 121-132, 135-146, 149, 151-160, 163, 165-174, 267-271, 273-277, and 279-288. Optionally, the heavy chain comprises an amino acid sequence that is at least 70%, 75%, 80%, 85%, 90%, 95%, 97%, 98% or 99% similar to the amino acid sequence of any one of SEQ ID NOs: 121-132, 135-146, 149, 151-160, 163, 165-174, 267-271, 273-277, and 279-288. The heavy chain may comprise the amino acid sequence of any one of SEQ ID NOs: 121-132, 135-146, 149, 151-160, 163, 165-174, 267-271, 273-277, and 279-288. In some embodiments, the heavy chain comprises the amino acid sequence of SEQ ID NO: 121. Optionally, the heavy chain comprises the amino acid sequence of SEQ ID NO: 122. The heavy chain may comprise the amino acid sequence of SEQ ID NO: 123. In some embodiments, the heavy chain comprises the amino acid sequence of SEQ ID NO: 124. Optionally, the heavy chain comprises the amino acid sequence of SEQ ID NO: 125. The heavy chain may comprise the amino acid sequence of SEQ ID NO: 126. In some embodiments, the heavy chain comprises the amino acid sequence of SEQ ID NO: 127. Optionally, the heavy chain comprises the amino acid sequence of SEQ ID NO: 128. The heavy chain may comprise the amino acid sequence of SEQ ID NO: 129. In some embodiments, the heavy chain comprises the amino acid sequence of SEQ ID NO: 130. Optionally, the heavy chain comprises the amino acid sequence of SEQ ID NO: 131. The heavy chain may comprise the amino acid sequence of SEQ ID NO: 132. In some embodiments, the heavy chain comprises the amino acid sequence of SEQ ID NO: 135. Optionally, the heavy chain comprises the amino acid sequence of SEQ ID NO: 136. The heavy chain may comprise the amino acid sequence of SEQ ID NO: 137. In some embodiments, the heavy chain comprises the amino acid sequence of SEQ ID NO: 138. Optionally, the heavy chain comprises the amino acid sequence of SEQ ID NO: 139. The heavy chain may comprise the amino acid sequence of SEQ ID NO: 140. In some embodiments, the heavy chain comprises the amino acid sequence of SEQ ID NO: 141. Optionally, the heavy chain comprises the amino acid sequence of SEQ ID NO: 142. The heavy chain may comprise the amino acid sequence of SEQ ID NO: 143. In some embodiments, the heavy chain comprises the amino acid sequence of SEQ ID NO: 144. Optionally, the heavy chain comprises the amino acid sequence of SEQ ID NO: 145. The heavy chain may comprise the amino acid sequence of SEQ ID NO: 146. In some embodiments, the heavy chain comprises the amino acid sequence of SEQ ID NO: 149. Optionally, the heavy chain comprises the amino acid sequence of SEQ ID NO: 150. The heavy chain may comprise the amino acid sequence of SEQ ID NO: 151. In some embodiments, the heavy chain comprises the amino acid sequence of SEQ ID NO: 152. Optionally, the heavy chain comprises the amino acid sequence of SEQ ID NO: 153. The heavy chain may comprise the amino acid sequence of SEQ ID NO: 154. In some embodiments, the heavy chain comprises the amino acid sequence of SEQ ID NO: 155. Optionally, the heavy chain comprises the amino acid sequence of SEQ ID NO: 156. The heavy chain may comprise the amino acid sequence of SEQ ID NO: 157. In some embodiments, the heavy chain comprises the amino acid sequence of SEQ ID NO: 158. Optionally, the heavy chain comprises the amino acid sequence of SEQ ID NO: 159. The heavy chain may comprise the amino acid sequence of SEQ ID NO: 160. In some embodiments, the heavy chain comprises the amino acid sequence of SEQ ID NO: 163. Optionally, the heavy chain comprises the amino acid sequence of SEQ ID NO: 164. The heavy chain may comprise the amino acid sequence of SEQ ID NO: 165. In some embodiments, the heavy chain comprises the amino acid sequence of SEQ ID NO: 166. Optionally, the heavy chain comprises the amino acid sequence of SEQ ID NO: 167. The heavy chain may comprise the amino acid sequence of SEQ ID NO: 168. In some embodiments, the heavy chain comprises the amino acid sequence of SEQ ID NO: 169. Optionally, the heavy chain comprises the amino acid sequence of SEQ ID NO: 170. The heavy chain may comprise the amino acid sequence of SEQ ID NO: 171. In some embodiments, the heavy chain comprises the amino acid sequence of SEQ ID NO: 172. Optionally, the heavy chain comprises the amino acid sequence of SEQ ID NO: 173. The heavy chain may comprise the amino acid sequence of SEQ ID NO: 174. In some embodiments, the heavy chain comprises the amino acid sequence of SEQ ID NO: 266. Optionally, the heavy chain comprises the amino acid sequence of SEQ ID NO: 267. The heavy chain may comprise the amino acid sequence of SEQ ID NO: 268. In some embodiments, the heavy chain comprises the amino acid sequence of SEQ ID NO: 269. Optionally, the heavy chain comprises the amino acid sequence of SEQ ID NO: 270. The heavy chain may comprise the amino acid sequence of SEQ ID NO: 271. The heavy chain may comprise the amino acid sequence of SEQ ID NO: 272. In some embodiments, the heavy chain comprises the amino acid sequence of SEQ ID NO: 273. Optionally, the heavy chain comprises the amino acid sequence of SEQ ID NO: 274. The heavy chain may comprise the amino acid sequence of SEQ ID NO: 275. Optionally, the heavy chain comprises the amino acid sequence of SEQ ID NO: 276. The heavy chain may comprise the amino acid sequence of SEQ ID NO: 277. In some embodiments, the heavy chain comprises the amino acid sequence of SEQ ID NO: 278. In some embodiments, the heavy chain comprises the amino acid sequence of SEQ ID NO: 279. Optionally, the heavy chain comprises the amino acid sequence of SEQ ID NO: 280. The heavy chain may comprise the amino acid sequence of SEQ ID NO: 281. In some embodiments, the heavy chain comprises the amino acid sequence of SEQ ID NO: 282. Optionally, the heavy chain comprises the amino acid sequence of SEQ ID NO: 283. The heavy chain may comprise the amino acid sequence of SEQ ID NO: 284. In some embodiments, the heavy chain comprises the amino acid sequence of SEQ ID NO: 285. Optionally, the heavy chain comprises the amino acid sequence of SEQ ID NO: 286. The heavy chain may comprise the amino acid sequence of SEQ ID NO: 287. In some embodiments, the heavy chain comprises the amino acid sequence of SEQ ID NO: 288. Optionally, the heavy chain does not comprise the amino acid sequence of SEQ ID NO: 150. In some embodiments, the heavy chain does not comprise the amino acid sequence of SEQ ID NO: 164. Optionally, the heavy chain does not comprise the amino acid sequence of SEQ ID NOs: 150 and 164. In some embodiments, the heavy chain does not comprise the amino acid sequence of SEQ ID NO: 234. Optionally, the heavy chain does not comprise the amino acid sequence of SEQ ID NO: 266. In some embodiments, the heavy chain does not comprise the amino acid sequence of SEQ ID NO: 272. In some embodiments, the heavy chain does not comprise the amino acid sequence of SEQ ID NO: 278. Optionally, the heavy chain does not comprise the amino acid sequence of SEQ ID NOs: 234 and 278. In some embodiments, the heavy chain does not comprise the amino acid sequence of SEQ ID NOs: 234, 266, 272 and 278. Optionally, the heavy chain does not comprise the amino acid sequence of SEQ ID NOs: 150, 164, 234 and 278. In some embodiments, the heavy chain does not comprise the amino acid sequence of SEQ ID NOs: 150, 164, 234, 266, 272 and 278.

In some embodiments, the light chain comprises an amino acid sequence that is at least 70%, 75%, 80%, 85%, 90%, 95%, 97%, 98% or 99% identical to the amino acid sequence of any one of SEQ ID NOs: 195 and 196. Optionally, the light chain comprises an amino acid sequence that is at least 70%, 75%, 80%, 85%, 90%, 95%, 97%, 98% or 99% similar to the amino acid sequence of any one of SEQ ID NOs: 195 and 196. The light chain may comprise the amino acid sequence of SEQ ID NO: 195 or 196. In some embodiments, the light chain comprises the amino acid sequence of SEQ ID NO: 195. Optionally, the light chain comprises the amino acid sequence of SEQ ID NO: 196.

In some embodiments, the antibody is monoclonal. Optionally, the antibody is a chimeric antibody. The antibody may be a humanized antibody. In some embodiments, the antibody is a human antibody.

In some embodiments, the binding of the antibody to human CD154 inhibits the interaction between human CD154 and human CD40. Optionally, the antibody blocks the activation of one or more of B cells, macrophages, dendritic cells, or endothelial cells by inhibiting binding of CD154 to CD40. In some embodiments, a reduced level of thrombosis is observed after administration of the antibody compared to level of thrombosis after administration of the 5c8 or hu5c8 antibody. Optionally, the antibody does not cause thrombosis when administered to a subject.

In some embodiments, the antibody has one or more of the following effects when administered to a subject: (a) decreased risk of thrombosis or thromboembolic events compared to hu5c8 antibody; (b) decreased activation of platelets expressing CD154; (c) inhibition of CD154 shedding; and (d) alteration of the expression or activity of downstream targets of CD154-CD40 signaling. Optionally, the administration of the antibody results in a decreased risk for thrombosis or thromboembolic events, compared to a subject where a 5c8 or hu5c8 antibody has been administered. In some embodiments, the administration of the antibody results in a decreased activation of platelets expressing CD154. Optionally, the administration of the antibody results in the inhibition of CD154 shedding. In some embodiments, the administration of the antibody results in the alteration the expression of activity of downstream targets of CD154-CD40 signaling.

In some embodiments, the human Fc domain with modified effector functions does not comprise the amino acid sequence consisting of any one of SEQ ID NOs: 1, 2, 10, 11, 231, and 236. Optionally, the human Fc domain with modified effector functions does not comprise the amino acid sequence consisting of SEQ ID NO: 1. In some embodiments, the human Fc domain with modified effector functions does not comprise the amino acid sequence consisting of SEQ ID NO: 2. Optionally, the human Fc domain with modified effector functions does not comprise the amino acid sequence consisting of SEQ ID NO: 10. In some embodiments, the human Fc domain with modified effector functions does not comprise the amino acid sequence consisting of SEQ ID NO: 11. Optionally, the human Fc domain with modified effector functions does not comprise the amino acid sequence consisting of SEQ ID NO: 231. In some embodiments, the human Fc domain with modified effector functions does not comprise the amino acid sequence consisting of SEQ ID NO: 236. In some embodiments, the antibody does not comprise the amino acid sequence consisting of any one of SEQ ID NOs: 1, 2, 10, 11, 231, and 236. Optionally, the antibody does not comprise the amino acid sequence consisting of SEQ ID NO: 1. In some embodiments, the antibody does not comprise the amino acid sequence consisting of SEQ ID NO: 2. Optionally, the antibody does not comprise the amino acid sequence consisting of SEQ ID NO: 10. In some embodiments, the antibody does not comprise the amino acid sequence consisting of SEQ ID NO: 11. Optionally, the antibody does not comprise the amino acid sequence consisting of SEQ ID NO: 231. In some embodiments, the antibody does not comprise the amino acid sequence consisting of SEQ ID NO: 236.

Optionally, the human Fc region with modified effector functions does not comprise the amino acid sequence consisting of any one of SEQ ID NOs: 19, 20, 38, 39, 232, and 235. In some embodiments, the human Fc region with modified effector functions does not comprise the amino acid sequence consisting of SEQ ID NO: 19. Optionally, the human Fc region with modified effector functions does not comprise the amino acid sequence consisting of SEQ ID NO: 20. In some embodiments, the human Fc region with modified effector functions does not comprise the amino acid sequence consisting of SEQ ID NO: 20. Optionally, the human Fc region with modified effector functions does not comprise the amino acid sequence consisting of SEQ ID NO: 38. In some embodiments, the human Fc region with modified effector functions does not comprise the amino acid sequence consisting of SEQ ID NO: 39. Optionally, the human Fc region with modified effector functions does not comprise the amino acid sequence consisting of SEQ ID NO: 232. In some embodiments, the human Fc region with modified effector functions does not comprise the amino acid sequence consisting of SEQ ID NO: 235. Optionally, the antibody does not comprise the amino acid sequence consisting of any one of SEQ ID NOs: 19, 20, 38, 39, 232, and 235. In some embodiments, the antibody does not comprise the amino acid sequence consisting of SEQ ID NO: 19. Optionally, the antibody does not comprise the amino acid sequence consisting of SEQ ID NO: 20. In some embodiments, the antibody does not comprise the amino acid sequence consisting of SEQ ID NO: 20. Optionally, the antibody does not comprise the amino acid sequence consisting of SEQ ID NO: 38. In some embodiments, antibody does not comprise the amino acid sequence consisting of SEQ ID NO: 39. Optionally, the antibody does not comprise the amino acid sequence consisting of SEQ ID NO: 232. In some embodiments, the antibody does not comprise the amino acid sequence consisting of SEQ ID NO: 235.

In some embodiments, the heavy chain with modified effector functions does not comprise the amino acid sequence consisting of any one of SEQ ID NOs: 119, 120, 133, 134, 147, 148, 150, 161, 162, 164, 230, 234, and 278. Optionally, the heavy chain with modified effector functions does not comprise the amino acid sequence consisting of SEQ ID NO: 119. In some embodiments, the heavy chain with modified effector functions does not comprise the amino acid sequence consisting of SEQ ID NO: 120. Optionally, the heavy chain with modified effector functions does not comprise the amino acid sequence consisting of SEQ ID NO: 133. In some embodiments, the heavy chain with modified effector functions does not comprise the amino acid sequence consisting of SEQ ID NO: 134. Optionally, the heavy chain with modified effector functions does not comprise the amino acid sequence consisting of SEQ ID NO: 147. In some embodiments, the heavy chain with modified effector functions does not comprise the amino acid sequence consisting of SEQ ID NO: 148. In some embodiments, the heavy chain with modified effector functions does not comprise the amino acid sequence consisting of SEQ ID NO: 150. Optionally, the heavy chain with modified effector functions does not comprise the amino acid sequence consisting of SEQ ID NO: 161. In some embodiments, the heavy chain with modified effector functions does not comprise the amino acid sequence consisting of SEQ ID NO: 162. In some embodiments, the heavy chain with modified effector functions does not comprise the amino acid sequence consisting of SEQ ID NO: 164. Optionally, the heavy chain with modified effector functions does not comprise the amino acid sequence consisting of SEQ ID NO: 230. In some embodiments, the heavy chain with modified effector functions does not comprise the amino acid sequence consisting of SEQ ID NO: 234. Optionally, the heavy chain with modified effector functions does not comprise the amino acid sequence consisting of SEQ ID NO: 278. In some embodiments, the antibody does not comprise the amino acid sequence consisting of any one of SEQ ID NOs: 119, 120, 133, 134, 147, 148, 150, 161, 162, 164, 230, 234, and 278. Optionally, the antibody does not comprise the amino acid sequence consisting of SEQ ID NO: 119. In some embodiments, the antibody does not comprise the amino acid sequence consisting of SEQ ID NO: 120. Optionally, the antibody does not comprise the amino acid sequence consisting of SEQ ID NO: 133. In some embodiments, the antibody does not comprise the amino acid sequence consisting of SEQ ID NO: 134. Optionally, the antibody does not comprise the amino acid sequence consisting of SEQ ID NO: 147. In some embodiments, the antibody does not comprise the amino acid sequence consisting of SEQ ID NO: 148. Optionally, the antibody does not comprise the amino acid sequence consisting of SEQ ID NO: 161. In some embodiments, the antibody does not comprise the amino acid sequence consisting of SEQ ID NO: 162. In some embodiments, the heavy chain with modified effector functions does not comprise the amino acid sequence consisting of SEQ ID NO: 164. Optionally, the antibody does not comprise the amino acid sequence consisting of SEQ ID NO: 230. In some embodiments, the antibody does not comprise the amino acid sequence consisting of SEQ ID NO: 234. Optionally, the heavy chain with modified effector functions does not comprise the amino acid sequence consisting of SEQ ID NO: 278. In some embodiments, the antibody does not comprise the amino acid sequence consisting of any one of SEQ ID NOs: 1, 2, 10, 11, 19, 20, 38, 39, 119, 120, 133, 134, 147, 148, 150, 161, 162, 164, 230, 231, 232, 234, 235, 236, and 278.

### Properties of the Anti-CD 154 Antibodies

### Binding Affinity of Anti-CD154 Antibodies to CD154

The binding affinity (K_{D}) and dissociation rate (k_{off}) of an anti-CD154 antibody to CD154 can be determined by methods known in the art. The binding affinity can be measured by ELISA, RIA, flow cytometry, or surface plasmon resonance (SPR), such as with the BIACORE^{®} system. The dissociation rate can be measured by SPR. Optionally, the binding affinity and dissociation rate are measured by SPR. In some embodiments, the binding affinity and dissociation rate are measured using BIACORE^{®}. The skilled artisan can determine whether an antibody disclosed herein has substantially the same K_{D} as another anti-CD154 antibody by using methods known in the art. Such methods of determining K_{D} and k_{off} can be used during the initial screening stage, as well as during subsequent optimization stages. In some embodiments, the antibody has a K_{D} for CD154 of less than 50 pM. Optionally, the antibody has a K_{D} for CD154 of less than 25 pM. In some embodiments, the antibody has a K_{D} for CD154 of 5-25 pM. Optionally, the antibody has a K_{D} for CD154 of 9.5-23 pM.

### Inhibition of CD154 Activity by Anti-CD154 Antibody

Anti-CD154 antibodies that inhibit CD154 binding to CD40 can be identified using any one of a number of assays, e.g. competitive binding assays, FACS analysis, B cell activation assays, B cell proliferation assays, T cell activation assays, T cell proliferation assays. See, *e.g.,* Barr et al., Immunology, 102(1):39-43 (2001); and Blair et al., J. Exp. Med., 191(4):651-660 (2001). For example, neutralizing anti-CD 154 antibodies can be identified by their inhibition of up-regulation of a specific downstream target gene of CD154, such as CD23, CD44H, CD54, TRAF-3 and NFκB In some embodiments, the anti-CD154 antibodies have an IC₅₀ of no greater than 500 nM, 300 nM, 200 nM, 150 nM, 100 nM, 50 nM, 20 nM, 10 nM, or 1 nM.

### Effector function - Platelet assays

The effector function of the anti-CD 154 can be identified using any one of a number of assays, *e.g. in vitro* platelet activation and/or aggregation assays. See, *e.g.,* U.S. Patent No. 9,765,150; Langer F et al., Thromb Haemost. Jun;93(6): 1137-46 (2005); McKenzie, S.E. et al., J Immunol 162 (7) 4311-4318 (1999); and Scholthauer T et al, Protein Engineering, Design and Selection, 29(10): 457-466, (2016). Blood from human donors or mice expressing FcγRIIA (CD32a) on platelets may be used to assay platelet function. Platelet activation may be detected by flow cytometry using antibodies against platelet activation markers P-selectin (CD62P) and PAC-1 (activated GPIIb/IIIa). Platelet aggregation analysis may be analyzed using a minicell impedance device and quantifying the area-under-the-curve as a measure of the platelet aggregation impedance curve.

### Nucleic Acids, Vectors, Host Cells, and Recombinant Methods of Making Antibodies

### Nucleic Acids

A second aspect of the present disclosure provides nucleic acid molecules encoding the anti-CD154 antibodies disclosed herein. In some embodiments, separate nucleic acid molecules encode a heavy chain and a light chain of an anti-CD154 immunoglobulin. In other embodiments, the same nucleic acid molecule encodes both a heavy chain and a light chain of an anti-CD 154 immunoglobulin.

In some embodiments, the nucleic acid molecule comprises a sequence encoding the VL of an anti-CD154 antibody disclosed herein.

In some embodiments, the nucleic acid molecule comprises a nucleotide sequence that encodes a VL comprising the amino acid sequence of any one of SEQ ID NOs: 65 and 66. In some embodiments, the nucleic acid molecule comprises the nucleotide sequence that encodes a VL comprising the amino acid sequence of any one of SEQ ID NOS: 65 and 66 or a portion thereof. In some embodiments, the nucleic acid encodes the amino acid sequence of one, two or all three light chain CDRs of said antibody. In some embodiments, said portion encodes a contiguous region from CDR1-CDR3 of the light chain of an anti-CD 154 antibody.

In some embodiments, the nucleic acid molecule encodes a VL comprising an amino acid sequence that is at least 70%, 75%, 80%, 85%, 90%, 95%, 97%, 98% or 99% identical to the VL amino acid sequence of SEQ ID NO: 65 or 66. Optionally, the nucleic acid molecule encodes a VL comprising an amino acid sequence that is at least 70%, 75%, 80%, 85%, 90%, 95%, 97%, 98% or 99% similar to the VL amino acid sequence of SEQ ID NO: 65 or 66. Nucleic acid molecules may include nucleotide sequences that hybridize under highly stringent conditions to the complement of a nucleic acid encoding the amino acid sequence the VL region of SEQ ID NO: 65 or 66.

In some embodiments, the nucleic acid molecule comprises a sequence encoding the VH of an anti-CD154 antibody disclosed herein.

In some embodiments, the nucleic acid molecule comprises a nucleotide sequence that encodes a VH comprising the amino acid sequence of any one of SEQ ID NOs: 63, 64, 252 and 253. In some embodiments, the nucleic acid molecule comprises the nucleotide sequence that encodes a VH comprising the amino acid sequence of any one of SEQ ID NOS: 63, 64, 252 and 253 or a portion thereof. In some embodiments, the nucleic acid encodes the amino acid sequence of one, two or all three heavy chain CDRs of said antibody. In some embodiments, said portion encodes a contiguous region from CDR1-CDR3 of the heavy chain of an anti-CD 154 antibody.

In some embodiments, the nucleic acid molecule encodes a VH comprising an amino acid sequence that is at least 70%, 75%, 80%, 85%, 90%, 95%, 97%, 98% or 99% identical to the VH amino acid sequence of SEQ ID NO: 63, 64, 252 or 253. Optionally, the nucleic acid molecule encodes a VH comprising an amino acid sequence that is at least 70%, 75%, 80%, 85%, 90%, 95%, 97%, 98% or 99% similar to the VH amino acid sequence of SEQ ID NO: 63, 64, 252 or 253. Nucleic acid molecules may include nucleotide sequences that hybridize under highly stringent conditions to the complement of a nucleic acid encoding the amino acid sequence the VH region of SEQ ID NOs: 63, 64, 252 or 253.

In some embodiments, the nucleic acid molecule comprises a sequence encoding the Fc domain of an anti-CD154 antibody disclosed herein.

In some embodiments, the nucleic acid molecule comprises a nucleotide sequence that encodes an Fc domain comprising the amino acid sequence of one of SEQ ID NOs: 3-9, 12-18 and 236-241. In some embodiments, the nucleic acid molecule comprises the nucleotide sequence that encodes an Fc domain comprising the amino acid sequence of any one of SEQ ID NOS: 3-9, 12-18 and 236-241 or a portion thereof.

In some embodiments, the nucleic acid molecule encodes an Fc domain comprising an amino acid sequence that is at least 70%, 75%, 80%, 85%, 90%, 95%, 97%, 98% or 99% identical to any one of the amino acid sequences of any one of SEQ ID NO: 3-9, 12-18 and 238-241. Optionally, the nucleic acid molecule encodes an Fc domain comprising an amino acid sequence that is at least 70%, 75%, 80%, 85%, 90%, 95%, 97%, 98% or 99% similar to any one of the amino acid sequences of any one of SEQ ID NO: 3-9, 12-18 and 238-241. Nucleic acid molecules may include nucleotide sequences that hybridize under highly stringent conditions to the complement of a nucleic acid encoding any one of the amino acid sequences of any one of SEQ ID NOs: 3-9, 12-18 and 238-241 or to the complement of a nucleic acid comprising the Fc region portion of the heavy chain nucleotide sequence of any one of SEQ ID NO: 177-179, 192-194, 289, 291-297, and 316, 318-324. In some embodiments, the nucleic acid molecule encodes an Fc domain comprising an amino acid sequence that is at least 70%, 75%, 80%, 85%, 90%, 95%, 97%, 98% or 99% identical to any one of the amino acid sequences of any one of SEQ ID NO: 3-9, 12-18 and 236-241. Optionally, the nucleic acid molecule encodes an Fc domain comprising an amino acid sequence that is at least 70%, 75%, 80%, 85%, 90%, 95%, 97%, 98% or 99% similar to any one of the amino acid sequences of any one of SEQ ID NO: 3-9, 12-18 and 236-241. Nucleic acid molecules may include nucleotide sequences that hybridize under highly stringent conditions to the complement of a nucleic acid encoding any one of the amino acid sequences of any one of SEQ ID NOs: 3-9, 12-18 and 236-241 or to the complement of a nucleic acid comprising the Fc region portion of the heavy chain nucleotide sequence of any one of SEQ ID NO: 177-179, 192-194, 289-297, and 316-324. In some embodiments, the nucleic acid molecule encoding an Fc domain comprises the nucleotide sequence that is at least 70%, 75%, 80%, 85%, 90%, 95%, 97%, 98% or 99% identical to the Fc region portion of any one of SEQ ID NO: 177-179, 192-194, 289-297, and 316-324. Optionally, the nucleic acid molecule encoding an Fc domain comprises the nucleotide sequence that is at least 70%, 75%, 80%, 85%, 90%, 95%, 97%, 98% or 99% similar to the Fc region portion of any one of SEQ ID NO: 177-179, 192-194, 289-297, and 316-324. In some embodiments, the nucleic acid molecule encoding an Fc domain comprises the nucleotide sequence of the Fc region portion of any one of SEQ ID NO: 177-179, 192-194, 289-297, and 316-324.

In some embodiments, the nucleic acid molecule comprises a sequence encoding the Fc region of an anti-CD 154 antibody disclosed herein.

In some embodiments, the nucleic acid molecule comprises a nucleotide sequence that encodes an Fc region comprising the amino acid sequence of any one of SEQ ID NOs: 3-9, 12-18, 21-37, 40-56, 238-241 and 243-251. In some embodiments, the nucleic acid molecule comprises the nucleotide sequence that encodes an Fc region comprising the amino acid sequence of any one of SEQ ID NOS: 3-9, 12-18, 21-37, 40-56, 238-241 and 243-251 or a portion thereof. In some embodiments, the nucleic acid molecule comprises a nucleotide sequence that encodes an Fc region comprising the amino acid sequence of any one of SEQ ID NOs: 3-9, 12-18, 21-37, 40-56 and 236-251. In some embodiments, the nucleic acid molecule comprises the nucleotide sequence that encodes an Fc region comprising the amino acid sequence of any one of SEQ ID NOS: 3-9, 12-18, 21-37, 40-56 and 236-251 or a portion thereof. In some embodiments, the nucleic acid molecule comprises a nucleotide sequence that encodes an Fc region comprising the amino acid sequence of any one of SEQ ID NOs: 21-37, 40-56, and 243-251. In some embodiments, the nucleic acid molecule comprises the nucleotide sequence that encodes an Fc region comprising the amino acid sequence of any one of SEQ ID NOS: 21-37, 40-56, and 243-251 or a portion thereof. In some embodiments, the nucleic acid molecule comprises a nucleotide sequence that encodes an Fc region comprising the amino acid sequence of any one of SEQ ID NOs: 21-37, 40-56 and 242-251. In some embodiments, the nucleic acid molecule comprises the nucleotide sequence that encodes an Fc region comprising the amino acid sequence of any one of SEQ ID NOS: 21-37, 40-56 and 242-251 or a portion thereof.

In some embodiments, the nucleic acid molecule encodes an Fc region comprising an amino acid sequence that is at least 70%, 75%, 80%, 85%, 90%, 95%, 97%, 98% or 99% identical to any one of the amino acid sequences of any one of SEQ ID NO: 3-9, 12-18, 21-37, 40-56, 238-241, and 243-251. Optionally, the nucleic acid molecule encodes an Fc region comprising an amino acid sequence that is at least 70%, 75%, 80%, 85%, 90%, 95%, 97%, 98% or 99% similar to any one of the amino acid sequences of any one of SEQ ID NO: 3-9, 12-18, 21-37, 40-56, 238-241 and 243-251. Nucleic acid molecules may include nucleotide sequences that hybridize under highly stringent conditions to the complement of a nucleic acid encoding any one of the amino acid sequences of any one of SEQ ID NOs: 3-9, 12-18, 21-37, 40-56, 238-241 and 243-251. In some embodiments, the nucleic acid molecule encodes an Fc region comprising an amino acid sequence that is at least 70%, 75%, 80%, 85%, 90%, 95%, 97%, 98% or 99% identical to any one of the amino acid sequences of any one of SEQ ID NO: 3-9, 12-18, 21-37, 40-56, and 236-251. Optionally, the nucleic acid molecule encodes an Fc region comprising an amino acid sequence that is at least 70%, 75%, 80%, 85%, 90%, 95%, 97%, 98% or 99% similar to any one of the amino acid sequences of any one of SEQ ID NO: 3-9, 12-18, 21-37, 40-56, and 236-251. Nucleic acid molecules may include nucleotide sequences that hybridize under highly stringent conditions to the complement of a nucleic acid encoding any one of the amino acid sequences of any one of SEQ ID NOs: 3-9, 12-18, 21-37, 40-56, and 236-251. In some embodiments, the nucleic acid molecule encodes an Fc region comprising an amino acid sequence that is at least 70%, 75%, 80%, 85%, 90%, 95%, 97%, 98% or 99% identical to any one of the amino acid sequences of any one of SEQ ID NO: 21-37, 40-56, and 243-251. Optionally, the nucleic acid molecule encodes an Fc region comprising an amino acid sequence that is at least 70%, 75%, 80%, 85%, 90%, 95%, 97%, 98% or 99% similar to any one of the amino acid sequences of any one of SEQ ID NO: 21-37, 40-56, and 243-251. Nucleic acid molecules may include nucleotide sequences that hybridize under highly stringent conditions to the complement of a nucleic acid encoding any one of the amino acid sequences of any one of SEQ ID NOs: 21-37, 40-56, and 243-251. In some embodiments, the nucleic acid molecule encodes an Fc region comprising an amino acid sequence that is at least 70%, 75%, 80%, 85%, 90%, 95%, 97%, 98% or 99% identical to any one of the amino acid sequences of any one of SEQ ID NO: 21-37, 40-56, and 242-251. Optionally, the nucleic acid molecule encodes an Fc region comprising an amino acid sequence that is at least 70%, 75%, 80%, 85%, 90%, 95%, 97%, 98% or 99% similar to any one of the amino acid sequences of any one of SEQ ID NO: 21-37, 40-56, and 242-251. Nucleic acid molecules may include nucleotide sequences that hybridize under highly stringent conditions to the complement of a nucleic acid encoding any one of the amino acid sequences of any one of SEQ ID NOs: 21-37, 40-56, and 242-251.

In some embodiments, the nucleic acid molecule comprises a sequence encoding the light chain of an anti-CD 154 antibody disclosed herein. Optionally, the nucleic acid molecule encoding the light chain comprises a nucleotide sequence selected from the group consisting of SEQ ID NOs: 197 and 198. In some embodiments, the nucleic acid molecule encoding the light chain comprises a nucleotide sequence that is at least 70%, 75%, 80%, 85%, 90%, 95%, 97%, 98% or 99% identical to a nucleotide sequence selected from the group consisting of SEQ ID NOs: 197 and 198. In some embodiments, the nucleic acid molecule encoding the light chain comprises a nucleotide sequence that hybridizes under highly stringent conditions to the complement of a nucleotide sequence selected from the group consisting of SEQ ID NOs: 197 and 198.

In some embodiments, the nucleic acid molecule comprises a nucleotide sequence that encodes a light chain comprising the amino acid sequence of any one of SEQ ID NOs: 195 and 196. In some embodiments, the nucleic acid molecule comprises the nucleotide sequence that encodes a light chain comprising the amino acid sequence of any one of SEQ ID NOS: 195 and 196 or a portion thereof.

In some embodiments, the nucleic acid molecule encodes a light chain amino acid sequence that is at least 70%, 75%, 80%, 85%, 90%, 95%, 97%, 98% or 99% identical to any one of the amino acid sequences of any one of SEQ ID NO: 195 and 196. Optionally, the nucleic acid molecule encodes a light chain amino acid sequence that is at least 70%, 75%, 80%, 85%, 90%, 95%, 97%, 98% or 99% similar to any one of the amino acid sequences of any one of SEQ ID NO: 195 and 196. Nucleic acid molecules may include nucleotide sequences that hybridize under highly stringent conditions to the complement of a nucleic acid encoding any one of the light chain amino acid sequences of any one of SEQ ID NOs: 195 and 196or to the complement of a nucleic acid comprising the light chain nucleotide sequence of any one of SEQ ID NO: 197 and 198.

In some embodiments, the nucleic acid molecule comprises a sequence encoding the heavy chain of an anti-CD 154 antibody disclosed herein. Optionally, the nucleic acid molecule encoding the heavy chain comprises a nucleotide sequence selected from the group consisting of any one of SEQ ID NOs: 177-179, 182-184, 187-189, and 192-194. In some embodiments, the nucleic acid molecule encoding the heavy chain comprises a nucleotide sequence that is at least 70%, 75%, 80%, 85%, 90%, 95%, 97%, 98% or 99% identical to a nucleotide sequence selected from the group consisting of any one of SEQ ID NOs: 177-179, 182-184, 187-189, and 192-194. In some embodiments, the nucleic acid molecule encoding the heavy chain comprises a nucleotide sequence that hybridizes under highly stringent conditions to the complement of a nucleotide sequence selected from the group consisting of SEQ ID NOs: 177-179, 182-184, 187-189, and 192-194.

In some embodiments, the nucleic acid molecule comprises a nucleotide sequence that encodes a heavy chain comprising the amino acid sequence of any one of SEQ ID NOs: 121-132, 135-146, 149-160, 163-174 and 266-288. In some embodiments, the nucleic acid molecule comprises the nucleotide sequence that encodes a heavy chain comprising the amino acid sequence of any one of SEQ ID NOS: 121-132, 135-146, 149-160, 163-174, and 266-288 or a portion thereof. In some embodiments, the nucleic acid molecule comprises a nucleotide sequence that encodes a heavy chain comprising the amino acid sequence of any one of SEQ ID NOs: 121-132, 135-146, 149-160, 163-174, 266-277 and 279-288. In some embodiments, the nucleic acid molecule comprises the nucleotide sequence that encodes a heavy chain comprising the amino acid sequence of any one of SEQ ID NOS: 121-132, 135-146, 149-160, 163-174, 266-277 and 279-288 or a portion thereof. In some embodiments, the nucleic acid molecule comprises a nucleotide sequence that encodes a heavy chain comprising the amino acid sequence of any one of SEQ ID NOs: 121-132, 135-146, 149, 151-160, 163, 165-174 and 266-288. In some embodiments, the nucleic acid molecule comprises the nucleotide sequence that encodes a heavy chain comprising the amino acid sequence of any one of SEQ ID NOS: 121-132, 135-146, 149, 151-160, 163, 165-174, and 266-288 or a portion thereof. In some embodiments, the nucleic acid molecule comprises a nucleotide sequence that encodes a heavy chain comprising the amino acid sequence of any one of SEQ ID NOs: 121-132, 135-146, 149, 151-160, 163, 165-174, 266-277 and 279-288. In some embodiments, the nucleic acid molecule comprises the nucleotide sequence that encodes a heavy chain comprising the amino acid sequence of any one of SEQ ID NOS: 121-132, 135-146, 149, 151-160, 163, 165-174, 266-277 and 279-288 or a portion thereof. In some embodiments, the nucleic acid molecule comprises a nucleotide sequence that encodes a heavy chain comprising the amino acid sequence of any one of SEQ ID NOs: 121-132, 135-146, 149, 151-160, 163, 165-174, 267-271, 273-277, and 279-288. In some embodiments, the nucleic acid molecule comprises the nucleotide sequence that encodes a heavy chain comprising the amino acid sequence of any one of SEQ ID NOS: 121-132, 135-146, 149, 151-160, 163, 165-174, 267-271, 273-277, and 279-288 or a portion thereof.

In some embodiments, the nucleic acid molecule encodes a heavy chain amino acid sequence that is at least 70%, 75%, 80%, 85%, 90%, 95%, 97%, 98% or 99% identical to any one of the amino acid sequences of any one of SEQ ID NO: 121-132, 135-146, 149-160, 163-174 and 266-288. Optionally, the nucleic acid molecule encodes a heavy chain amino acid sequence that is at least 70%, 75%, 80%, 85%, 90%, 95%, 97%, 98% or 99% similar to any one of the amino acid sequences of any one of SEQ ID NO: 121-132, 135-146, 149-160, 163-174 and 266-288. Nucleic acid molecules may include nucleotide sequences that hybridize under highly stringent conditions to the complement of a nucleic acid encoding any one of the heavy chain amino acid sequences of any one of SEQ ID NOs: 121-132, 135-146, 149-160, 163-174 and 266-288 or to the complement of a nucleic acid comprising the heavy chain nucleotide sequence of any one of SEQ ID NO: 177-179, 182-184, 187-189, 192-194, and 289-324. In some embodiments, the nucleic acid molecule encodes a heavy chain amino acid sequence that is at least 70%, 75%, 80%, 85%, 90%, 95%, 97%, 98% or 99% identical to any one of the amino acid sequences of any one of SEQ ID NO: 121-132, 135-146, 149-160, 163-174, 266-277, and 279-288. Optionally, the nucleic acid molecule encodes a heavy chain amino acid sequence that is at least 70%, 75%, 80%, 85%, 90%, 95%, 97%, 98% or 99% similar to any one of the amino acid sequences of any one of SEQ ID NO: 121-132, 135-146, 149-160, 163-174, 266-277, and 279-288. Nucleic acid molecules may include nucleotide sequences that hybridize under highly stringent conditions to the complement of a nucleic acid encoding any one of the heavy chain amino acid sequences of any one of SEQ ID NOs: 121-132, 135-146, 149-160, 163-174, 266-277, and 279-288 or to the complement of a nucleic acid comprising the heavy chain nucleotide sequence of any one of SEQ ID NO: 177-179, 182-184, 187-189, 192-194 and 289-324. In some embodiments, the nucleic acid molecule encodes a heavy chain amino acid sequence that is at least 70%, 75%, 80%, 85%, 90%, 95%, 97%, 98% or 99% identical to any one of the amino acid sequences of any one of SEQ ID NO: 121-132, 135-146, 149, 151-160, 163, 165-174, 266-277, and 279-288. Optionally, the nucleic acid molecule encodes a heavy chain amino acid sequence that is at least 70%, 75%, 80%, 85%, 90%, 95%, 97%, 98% or 99% similar to any one of the amino acid sequences of any one of SEQ ID NO: 121-132, 135-146, 149, 151-160, 163, 165-174, 266-277, and 279-288. Nucleic acid molecules may include nucleotide sequences that hybridize under highly stringent conditions to the complement of a nucleic acid encoding any one of the heavy chain amino acid sequences of any one of SEQ ID NOs: 121-132, 135-146, 149, 151-160, 163, 165-174, 266-277, and 279-288 or to the complement of a nucleic acid comprising the heavy chain nucleotide sequence of any one of SEQ ID NO: 177-179, 182-184, 187-189, 192-194 and 289-324. In some embodiments, the nucleic acid molecule encodes a heavy chain amino acid sequence that is at least 70%, 75%, 80%, 85%, 90%, 95%, 97%, 98% or 99% identical to any one of the amino acid sequences of any one of SEQ ID NO: 121-132, 135-146, 149, 151-160, 163, 165-174, 266-277, and 279-288. Optionally, the nucleic acid molecule encodes a heavy chain amino acid sequence that is at least 70%, 75%, 80%, 85%, 90%, 95%, 97%, 98% or 99% similar to any one of the amino acid sequences of any one of SEQ ID NO: 121-132, 135-146, 149, 151-160, 163, 165-174, 266-277, and 279-288. Nucleic acid molecules may include nucleotide sequences that hybridize under highly stringent conditions to the complement of a nucleic acid encoding any one of the heavy chain amino acid sequences of any one of SEQ ID NOs: 121-132, 135-146, 149, 151-160, 163, 165-174, 266-277, and 279-288 or to the complement of a nucleic acid comprising the heavy chain nucleotide sequence of any one of SEQ ID NO: 177-179, 182-184, 187-189, 192-194 and 289-324. In some embodiments, the nucleic acid molecule encodes a heavy chain amino acid sequence that is at least 70%, 75%, 80%, 85%, 90%, 95%, 97%, 98% or 99% identical to any one of the amino acid sequences of any one of SEQ ID NO: 121-132, 135-146, 149, 151-160, 163, 165-174, 267-271, and 273-288. Optionally, the nucleic acid molecule encodes a heavy chain amino acid sequence that is at least 70%, 75%, 80%, 85%, 90%, 95%, 97%, 98% or 99% similar to any one of the amino acid sequences of any one of SEQ ID NO: 121-132, 135-146, 149, 151-160, 163, 165-174, 267-271, and 273-288. Nucleic acid molecules may include nucleotide sequences that hybridize under highly stringent conditions to the complement of a nucleic acid encoding any one of the heavy chain amino acid sequences of any one of SEQ ID NOs: 121-132, 135-146, 149, 151-160, 163, 165-174, 267-271, and 273-288 or to the complement of a nucleic acid comprising the heavy chain nucleotide sequence of any one of SEQ ID NO: 177-179, 182-184, 187-189, 192-194 and 289-324. In some embodiments, the nucleic acid molecule does not encode a heavy chain comprising the amino acid sequence of SEQ ID NO: 164 or SEQ ID NO: 150. Optionally, the nucleic acid molecule does not comprise the nucleotide sequence of SEQ ID NO: 188 or SEQ ID NO: 183. In some embodiments, the nucleic acid molecule does not encode a heavy chain comprising the amino acid sequence of SEQ ID NO: 278 or SEQ ID NO: 234. Optionally, the nucleic acid molecule does not comprise the nucleotide sequence of SEQ ID NO: 299 or SEQ ID NO: 308. In some embodiments, the nucleic acid molecule encoding a heavy chain comprises the nucleotide sequence that is at least 70%, 75%, 80%, 85%, 90%, 95%, 97%, 98% or 99% identical to any one of SEQ ID NO: 177-179, 192-194, 289-297, and 316-324. Optionally, the nucleic acid molecule encoding a heavy chain comprises the nucleotide sequence that is at least 70%, 75%, 80%, 85%, 90%, 95%, 97%, 98% or 99% similar to any one of SEQ ID NO: 177-179, 192-194, 289-297, and 316-324. In some embodiments, the nucleic acid molecule encoding a heavy chain comprises the nucleotide sequence of any one of SEQ ID NO: 177-179, 192-194, 289-297, and 316-324.

A nucleic acid molecule encoding the heavy chain or light chain of an anti-CD 154 antibody can be isolated from any source that produces such antibody. In various embodiments, the nucleic acid molecules are isolated from a B cell that expresses an anti-CD154 antibody isolated from an animal immunized with CD 154 or from an immortalized cell derived from such a B cell. Methods of isolating nucleic acids encoding an antibody are well-known in the art. See, *e.g*., Sambrook J. & Russell D., Molecular Cloning: A Laboratory Manual, 3rd ed., Cold Spring Harbor Laboratory Press, Cold Spring Harbor, N.Y. (2000). In another embodiment, the nucleic acid is isolated from a non-human, non-transgenic animal. The nucleic acid molecules isolated from a non-human, non-transgenic animal may be used, *e.g.,* for humanized antibodies that comprise one or more amino acid sequences from a human anti-CD154 antibody disclosed herein.

### Vectors

A third aspect of the present disclosure provides vectors comprising the disclosed nucleic acid molecules. In some embodiments, the vector comprises a nucleotide sequence that encodes the heavy chain of a disclosed anti-CD 154 antibody. Optionally, the vector comprises a nucleotide sequence that encodes the light chain of a disclosed antibody. The vector may comprise a nucleotide sequence encoding the heavy chain and the light chain of a disclosed anti-CD 154 antibody.

In some embodiments, the anti-CD154 antibodies are expressed by inserting DNAs encoding partial or full-length light and/or heavy chains, obtained as described above, into expression vectors such that the genes are operatively linked to expression control sequences, such as transcriptional and translational control sequences. In some embodiments, the DNAs encoding partial light and/or heavy chains may contain VH and/or VL sequences. Expression vectors include plasmids, retroviruses, adenoviruses, adeno-associated viruses (AAV), plant viruses such as cauliflower mosaic virus, tobacco mosaic virus, cosmids, YACs, EBV derived episomes, and the like. The polynucleotide encoding the heavy and/or light chain may be ligated into a vector such that transcriptional and translational control sequences within the vector serve their intended function of regulating the transcription and translation of the polynucleotide. The expression vector and expression control sequences may be chosen to be compatible with the expression host cell used. The antibody light chain gene and the antibody heavy chain gene may be inserted into separate vectors. In some embodiments, both genes are inserted into the same expression vector. In some embodiments, the polynucleotides encoding VH and/or VL sequences are inserted into an expression vector comprising CH and/or CL nucleotide sequences. Optionally, the polynucleotides encoding the VH sequence is inserted into an expression vector comprising an Fc domain nucleotide sequence. In some embodiments, the polynucleotides encoding the VH sequence is inserted into an expression vector comprising an Fc region nucleotide sequence. The polynucleotides encoding the heavy and/or light chain are inserted into the expression vector by standard methods (e.g., ligation of complementary restriction sites on the antibody gene fragment and vector, or blunt end ligation if no restriction sites are present).

A convenient vector may be one that encodes a functionally complete CH or CL immunoglobulin sequence, with appropriate restriction sites engineered so that any VH or VL nucleotide sequence can easily be inserted 5' of the CH or CL nucleotide sequence and expressed, as described above. In such vectors, splicing usually occurs between the splice donor site in the inserted J region and the splice acceptor site preceding the C domain, and also at the splice regions that occur within the CH exons. Optionally, the vector encodes an Fc domain with modified effector functions with appropriate restriction sites engineered so that a VH nucleotide sequence can be easily inserted 5' of the Fc domain nucleotide sequence and expressed. In some embodiments, the vector comprises a sequence encoding a linker that is 5' to the sequence encoding the Fc domain with appropriate restriction sites engineered so that a VH nucleotide sequence can be easily inserted 5' of the linker sequence and expressed. Optionally, the vector encodes an Fc region with modified effector functions with appropriate restriction sites engineered so that a VH nucleotide sequence can be easily inserted 5' of the Fc region nucleotide sequence and expressed. In some embodiments, the vector comprises a sequence encoding a linker that is 5' to the sequence encoding the Fc region with appropriate restriction sites engineered so that a VH nucleotide sequence can be easily inserted 5' of the linker polynucleotide sequence and expressed. In some embodiments, the vector comprises a sequence encoding a CH1 domain that is 5' to the sequence encoding the Fc region with appropriate restriction sites engineered so that a VH nucleotide sequence can be easily inserted 5' of the CH1 domain polynucleotide sequence and expressed.

In some embodiments, the vector comprises a polyadenylation and/or transcription termination sequence downstream of the coding regions. The recombinant expression vector also may encode a signal peptide that facilitates secretion of the antibody chain from a host cell. The antibody chain gene may be cloned into the vector such that the signal peptide is linked in-frame to the amino terminus of the immunoglobulin chain. Optionally, the signal peptide is an immunoglobulin signal peptide or a heterologous signal peptide (i.e., a signal peptide from a non-immunoglobulin protein).

In addition to the antibody chain coding sequences, the recombinant expression vectors may optionally carry regulatory sequences that control the expression of the antibody chain coding sequences in a host cell. It will be appreciated by those skilled in the art that the design of the expression vector, including the selection of regulatory sequences may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from retroviral long terminal repeats (LTRs), cytomegalovirus (CMV) (such as the CMV promoter/enhancer), Simian Virus 40 (SV40) (such as the SV40 promoter/enhancer), adenovirus, (e.g., the adenovirus major late promoter (AdMLP)), polyoma and strong mammalian promoters such as native immunoglobulin and actin promoters. For further description of viral regulatory elements, and sequences thereof, see *e.g.,* U.S. Patent No. 5,168,062, U.S. Patent No. 4,510,245 and U.S. Patent No. 4,968,615. Methods for expressing antibodies in plants, including a description of promoters and vectors, as well as transformation of plants is known in the art. See, *e.g.,* United States Patent 6,517,529, incorporated herein by reference. Methods of expressing polypeptides in bacterial cells or fungal cells, *e.g.*, yeast cells, are also well known in the art.

In addition to the antibody chain coding sequences and regulatory sequences, the recombinant expression vectors disclosed herein may carry additional sequences, such as sequences that regulate replication of the vector in host cells (e.g., origins of replication) and selectable marker genes. Optionally, the vector comprises a selectable marker gene to facilitate selection of host cells into which the vector has been introduced (see *e.g.,* U.S. Patent Nos. 4,399,216, 4,634,665 and 5,179,017, incorporated herein by reference). For example, the selectable marker gene may confer resistance to a drug, such as Geneticin (G418), hygromycin or methotrexate, on a host cell into which the vector has been introduced. For example, selectable marker genes include the dihydrofolate reductase (DHFR) gene (for use in DHFR-deficient host cells with methotrexate selection/amplification), the neomycin resistance (neo) gene (for G418 selection), and the glutamate synthetase gene.

### Host Cells and Methods of Recombinantly Producing Protein

Any of the nucleic acid molecules encoding anti-CD154 antibodies disclosed herein and vectors comprising these nucleic acid molecules can be used for transfection of a suitable mammalian, plant, or yeast host cell or transformation of a bacterial host cell. Methods for transfecting/transforming a host cell with a polynucleotide are well known in the art. For example, methods for introduction of heterologous polynucleotides into mammalian cells are well known in the art and include, without limitation, dextran-mediated transfection, calcium phosphate precipitation, polybrene-mediated transfection, protoplast fusion, electroporation, encapsulation of the polynucleotide(s) in liposomes, and direct microinjection of the DNA into nuclei. In addition, nucleic acid molecules may be introduced into mammalian cells by viral vectors. Methods of transforming cells are well known in the art. See, *e.g.,* U.S. Patent Nos. 4,399,216, 4,912,040, 4,740,461, and 4,959,455, each incorporated herein by reference. Methods of transforming plant cells are well known in the art, including, *e.g.*, Agrobacterium-mediated transformation, biolistic transformation, direct injection, electroporation, and viral transformation. Methods of transforming bacterial and yeast cells are also well known in the art.

Mammalian cell lines available as hosts for expression are well known in the art and include many immortalized cell lines available from the American Type Culture Collection (ATCC). Non-limiting examples include, *inter alia*, Chinese hamster ovary (CHO) cells, NS0 cells, SP2 cells, HEK-293T cells, 293 Freestyle cells (Invitrogen), NIH-3T3 cells, HeLa cells, baby hamster kidney (BHK) cells, African green monkey kidney cells (COS), human hepatocellular carcinoma cells (e.g., Hep G2), A549 cells, and a number of other cell lines. Other cell lines that may be used are insect cell lines, such as Sf9 or Sf21 cells. Plant host cells include, *e.g.*, Nicotiana, Arabidopsis, duckweed, corn, wheat, potato, etc. Bacterial host cells include E. *coli* and *Streptomyces* species. Yeast host cells include *Schizosaccharomyces pombe, Saccharomyces cerevisiae* and *Pichia pastoris.* It is within the skill in the art to select cell lines based on particular characteristics, such as expression level and glycosylation pattern. When recombinant expression vectors encoding antibody chains are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium or the host cell using standard protein purification methods.

Further, expression of the disclosed antibodies from production cell lines can be enhanced using a number of known techniques. For example, the glutamine synthetase gene expression system (the GS system) is a common approach for enhancing expression under certain conditions. See, *e.g.*, European PatentNos. 0216846, 0256055, 0323997 and 0338841, each incorporated herein by reference.

Antibodies expressed by different cell lines or in transgenic animals may have different glycosylation patterns. The antibodies disclosed herein may be expressed by any suitable host cell line and, therefore, may have any glycosylation pattern.

### Pharmaceutical Compositions and Administration

A fourth aspect of the present disclosure provides a pharmaceutical composition comprising an anti-CD154 antibody described herein and a pharmaceutically acceptable carrier. In some embodiments, the pharmaceutical composition comprises a therapeutically effective amount of the anti-CD154 antibody described herein.

The antibodies disclosed herein may be incorporated into pharmaceutical compositions suitable for administration to a subject. Typically, the pharmaceutical composition comprises an antibody disclosed herein and a pharmaceutically acceptable carrier. As used herein, "pharmaceutically acceptable carrier" and "pharmaceutically acceptable excipient" are used interchangeably refer to any and all solvents, dispersion media, coatings, antibacterial and antifungal agents, isotonic and absorption delaying agents, and the like that are physiologically compatible. Pharmaceutically acceptable carriers are well known in the art. See, *e.g.*, Remington's Pharmaceutical Sciences and U.S. Pharmacopeia: National Formulary, Mack Publishing Company, Easton, PA (1984), incorporated herein by reference. Some examples of pharmaceutically acceptable carriers are water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Additional examples of pharmaceutically acceptable substances are wetting agents or minor amounts of auxiliary substances such as wetting or emulsifying agents, preservatives, or buffers, which enhance the shelf life or effectiveness of the antibody. Pharmaceutical compositions may be prepared by mixing an antibody disclosed herein with acceptable carriers, excipients, or stabilizers in the form of, *e.g.,* lyophilized powders, slurries, aqueous solutions or suspensions (see, *e.g.,* Hardman, et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY; each incorporated herein by reference).

The pharmaceutical compositions may be in a variety of forms, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. In some embodiments, the pharmaceutical compositions are in the form of injectable or infusible solutions, such as compositions similar to those used for passive immunization of humans. Optionally, mode of administration is parenteral (e.g., intravenous, subcutaneous, intraperitoneal, intramuscular). In some embodiments, the mode of administration is intravitreal injection. The pharmaceutical composition may be administered by intravenous infusion or injection. In some embodiments, the antibody is administered by intramuscular or subcutaneous injection. Formulations for injection may be presented in unit dosage form, *e.g.,* in ampoules, pre-filled syringes, or in multi-dose containers, with or without an added preservative. The pharmaceutical compositions may take such forms as suspensions, solutions, or emulsions in oily or aqueous vehicles, and may contain formulatory agents such as suspending, stabilizing and/or dispersing agents. Alternatively, the active ingredient may be prepared in powder form for reconstitution with a suitable vehicle, *e.g.*, sterile pyrogen-free water, before use.

Therapeutic compositions typically must be sterile and stable under the conditions of manufacture and storage. The composition may be formulated as a solution, microemulsion, dispersion, liposome, or other ordered structure suitable to high drug concentration. Sterile injectable solutions may be prepared by incorporating the anti-CD154 antibody in the required amount in an appropriate solvent with one or a combination of ingredients enumerated above, as required, followed by filtered sterilization. Dispersions may be prepared by incorporating the anti-CD154 antibody into a sterile vehicle that contains a basic dispersion medium and the required other ingredients from those enumerated above. In the case of sterile powders for the preparation of sterile injectable solutions, the preferred methods of preparation include vacuum drying and freeze-drying that yield a powder of the anti-CD154 antibody and any additional desired ingredient from a previously sterile-filtered solution thereof. The proper fluidity of a solution can be maintained, for example, by the use of a coating such as lecithin, by the maintenance of the required particle size in the case of dispersion and/or by the use of surfactants. Prolonged absorption of injectable compositions can be brought about by including in the composition an agent that delays absorption, for example, monostearate salts and gelatin.

The pharmaceutical compositions may be administered by a variety of methods known in the art. In some embodiments, the preferred route/mode of administration is subcutaneous, intramuscular, or intravenous infusion. In some embodiments, the mode of administration is intravitreal. As will be appreciated by the skilled artisan, the route and/or mode of administration will vary depending upon the desired results.

In some embodiments, the pharmaceutical compositions may be prepared with a carrier that will protect the antibody against rapid release, such as a controlled release formulation, including implants, transdermal patches, and microencapsulated delivery systems. Biodegradable, biocompatible polymers, such as ethylene vinyl acetate, polyanhydrides, polyglycolic acid, collagen, polyorthoesters, and polylactic acid, may be used. Methods for the preparation of such formulations are generally known to those skilled in the art. See, *e.g.*, Sustained and Controlled Release Drug Delivery Systems J. R. Robinson, ed., Marcel Dekker, Inc., New York, 1978, which is incorporated herein by reference.

Additional active compounds also can be incorporated into the compositions. In certain embodiments, an anti-CD 154 antibody disclosed herein is co-formulated with and/or co-administered with one or more additional therapeutic agents. These agents include, without limitation, antibodies that bind other targets, anti-thrombotic drugs, anti-platelet drugs, non-steroidal anti-inflammatory drugs (NSAIDs) and anti-allergy drugs. Such combination therapies may require lower dosages of the anti-CD154 antibody as well as the co-administered agents, thus avoiding possible toxicities or complications associated with the various monotherapies.

Dosage regimens may be adjusted to provide the optimum desired response (e.g., a therapeutic or prophylactic response). For example, a single bolus can be administered, several divided doses can be administered over time or the dose can be proportionally reduced or increased as indicated by the exigencies of the therapeutic situation. It may be advantageous to formulate parenteral compositions in dosage unit form for ease of administration and uniformity of dosage. The term "dosage unit form" as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms may be dictated by and directly dependent on (a) the unique characteristics of the anti-CD 154 antibody and the particular therapeutic or prophylactic effect to be achieved, and (b) the limitations inherent in the art of compounding such an antibody for the treatment of sensitivity in individuals.

An exemplary, non-limiting range for a therapeutically or prophylactically effective amount of an antibody disclosed herein is 5 to 50 mg/kg. The therapeutically or prophylactically effective amount of an antibody disclosed herein may be about 5 to about 50 mg/kg. In some embodiments, a therapeutically or prophylactically effective amount of an antibody disclosed herein is 5 to 30 mg/kg. The therapeutically or prophylactically effective amount of an antibody disclosed herein may be about 5 to about 30 mg/kg. Optionally, the therapeutically or prophylactically effective amount of an antibody disclosed herein is 5 mg/kg, 10 mg/kg, 15 mg/kg, 20 mg/kg, 25 mg/kg, 30 mg/kg, 35 mg/kg, 40 mg/kg, 45 mg/kg or 50 mg/kg. In some embodiments, the therapeutically or prophylactically effective amount of an antibody disclosed herein is about 5 mg/kg, about 10 mg/kg, about 15 mg/kg, about 20 mg/kg, about 25 mg/kg, about 30 mg/kg, about 35 mg/kg, about 40 mg/kg, about 45 mg/kg or about 50 mg/kg. Dosage values may vary with the type and severity of the condition to be alleviated. For any particular subject, specific dosage regimens may be adjusted over time according to the individual need and the professional judgment of the person administering or supervising the administration of the compositions, and that dosage ranges set forth herein are exemplary only and are not intended to limit the scope or practice of the paragraphed composition.

### Therapeutic Methods of Use

A fifth aspect of the present disclosure provides a method for inhibiting CD154 activity. In some embodiments, CD154 activity is inhibit by contacting CD154 with an anti-CD154 antibody disclosed herein. Optionally, CD154 is inhibited by administering an anti-CD154 antibody disclosed herein to a subject in need thereof. In some embodiments, a therapeutically effective amount of the anti-CD154 antibody is administered. Optionally, the anti-CD154 is administered in a pharmaceutical composition disclosed herein.

A sixth aspect of the present disclosure provides a method of inhibiting an immune response in a subject. In some embodiments, the immune response is inhibited by administering an anti-CD154 antibody disclosed herein to a subject in need thereof. Optionally, a therapeutically effective amount of the anti-CD154 antibody is administered. In some embodiments, the anti-CD154 is administered in a pharmaceutical composition disclosed herein. The immune response may be a humoral response, such as an antibody-mediated response. The immune response may be a cell-mediated response, such as one or more of a cytotoxic T-cell mediated immune response, a macrophage mediated response, a natural killer (NK) cell mediated immune response or a cytokine mediated response. The immune response could be a mixed humoral and cell-mediated response. The immune response may be a primary response or a secondary response.

Any of the antibodies disclosed herein may be used therapeutically. In some embodiments, the anti-CD154 antibody is a human, chimeric or humanized antibody. Optionally, the subject is a human. The subject may be a non-human, such as a monkey. Optionally, the anti-CD154 antibody is human antibody, and the subject is human. In some embodiments, the anti-CD154 antibody is a humanized antibody, and the subject is human. Alternatively, the subject may be a mammal (e.g. a monkey) that expresses CD154 that the anti-CD154 antibody cross-reacts with. The antibody may be administered to a non-human mammal expressing CD154 with which the antibody cross-reacts (e.g. a cynomolgus monkey) for veterinary purposes or as an animal model of human transplantation or disease. Such animal models may be useful for evaluating the therapeutic efficacy of antibodies disclosed herein.

The antibody may be administered once. Optionally, the antibody is administered multiple times. The antibody may be administered from three times daily to once every six months or longer. The administering may be on a schedule such as three times daily, twice daily, once daily, once every two days, once every three days, once weekly, once every two weeks, once every month, once every two months, once every three months, once every six months, twice weekly, three times weekly, four times weekly, twice every two weeks, three times every two weeks and four times every two weeks. The antibody may also be administered continuously via a minipump. The antibody may be administered via a mucosal, buccal, intranasal, inhalable, intravenous, intravitreal, subcutaneous, intramuscular, parenteral, or intratumor route. In some embodiments, the anti-CD154 antibody is administered systemically, such as subcutaneously, intravenously, orally, via inhalation, transdermally, or rectally. Optionally, the anti-CD154 antibody is administered locally. In some embodiments, the anti-CD154 antibody is administered intravitreally. The antibody may be administered once, at least twice or for at least the period of time until the condition is treated, palliated or cured. The antibody generally will be administered for as long as the condition is present. The antibody will generally be administered as part of a pharmaceutical composition as described *supra.* The dosage of antibody will generally be in the range of 5 to 50 mg/kg. The dosage of antibody may be about 5 to about 50 mg/kg. In some embodiments, dosage of antibody is 5 to 30 mg/kg. The dosage of antibody may be about 5 to about 30 mg/kg. Optionally, the dosage of antibody is 5 mg/kg, 10 mg/kg, 15 mg/kg, 20 mg/kg, 25 mg/kg, 30 mg/kg, 35 mg/kg, 40 mg/kg, 45 mg/kg or 50 mg/kg. In some embodiments, the dosage of antibody is about 5 mg/kg, about 10 mg/kg, about 15 mg/kg, about 20 mg/kg, about 25 mg/kg, about 30 mg/kg, about 35 mg/kg, about 40 mg/kg, about 45 mg/kg or about 50 mg/kg.

In some embodiments, an anti-CD154 antibody disclosed herein is administered to a subject who expresses inappropriately high levels of CD154. Optionally, the subject has received or will receive a cell, tissue, or organ transplant. The transplant may be an allogeneic transplant, autologous transplant or a xenogeneic transplant of a cell, tissue, or organ. The cell may be an engineered cell or an ex-vivo expanded cell. For example, the cell may be modified using one or more techniques such as transduction to express a cDNA, a CRISPR/Cas9 system, RNAi technology and retroviral technology. Optionally, the cell is modified to express a chimeric antigen receptor (CAR) on its surface. Examples of cells that may be transplanted include, but are not limited to a stem cell, a regulatory T (Treg) cell, a CAR-T cell, a CAR-B cell, and a tumor-infiltrating lymphocyte (TIL).

In some embodiments, any of the anti-CD154 antibodies of the disclosure disclosed herein may be administered to a subject in order to prevent a transplant rejection in the subject. For example, the anti-CD154 antibody may be administered to prevent an acute or a chronic humoral rejection of a grafted cell, tissue, or organ. The rejection may be an acute or chronic graft rejection in a graft recipient of an allogeneic transplant or xenotransplant. The methods disclosed herein may promote a long-term graft survival of the grafted cell, tissue, or organ. Optionally, the long-term graft survival is at least 6 months post-transplant, at least 1year post-transplant or at least 5 years post-transplant.

The transplant rejection may be associated with the transplantation of hematopoietic cell or bone marrow, an allogeneic transplant of pancreatic islet cells, graft vs host disease, or a solid organ transplant selected from the group consisting of a heart transplant, a kidney transplant, a liver transplant, a lung transplant, a pancreas transplant, a kidney-pancreas transplant, a heart-lung transplant, kidney-heart transplant, a kidney-heart-pancreas transplant, a heart-liver transplant, a heart-liver-kidney transplant, a heart-lung-kidney transplant, a heart-lung-liver transplant, a lung-kidney transplant, a lung-liver transplant, a liver-intestines-pancreas transplant, an intestines-pancreas transplant, a liver-kidney-intestines-pancreas transplant, and a kidney-intestines transplant.

In some embodiments, any of the anti-CD154 antibodies disclosed herein may be administered to a subject having one or more of an immune-related disease, atherosclerotic disorder, or neurodegenerative disorder. A subject may also have had or is at risk of having one or more of a stroke, a transient ischemic attack (TIA), an aneurysm, or a dissecting aneurysm.

Examples of immune-related diseases that may be treated/prevented by the compositions/methods disclosed herein include, but are not limited to, type I diabetes, juvenile diabetes, autoimmune diabetes, autoimmune hemolytic anemia, rheumatoid arthritis, systemic lupus erythematosus (SLE), psoriasis, multiple sclerosis, inflammatory bowel disease, Addison's disease, Crohn's disease, Graves' disease, Sjögren's syndrome, Hashimoto's thyroiditis, myasthenia gravis, vasculitis, pernicious anemia, celiac diseases, Guillain-Barre syndrome, ankylosing spondylitis, primary biliary cirrhosis, lupus nephritis, Goodpasture's disease, polymyositis, dermatomyositis, psoriasis, temporal arteritis, Churg-Strauss syndrome, transverse myelitis, thyroiditis, ulcerative colitis, sarcoidosis, hemolytic anemia, idiopathic thrombocytopenic purpura, neuromyelitis optica spectrum disorder, paroxysmal nocturnal hemoglobinuria, atypical hemolytic uremic syndrome, dysfunctional immune responses associated with viral-mediated diseases, cytokine release syndrome (CRS), cytokine storm syndrome, Behcet's disease, diabetic retinopathy (DR), diabetic macular edema (DME), wet age-related macular degeneration (AMD), and macular edema following retinal vein occlusion (MEfRVO). An immune-related disease may also include allergic conditions which include, but not limited to, allergic rhinitis, asthma, atopic eczema, anaphylaxis, insect venom allergy, drug allergy, and food allergy. Optionally, the immune-related disease is an inflammatory condition of an organ. In some embodiments, the organ is selected from the group consisting of lung, heart, and kidney. Optionally, the organ is lung. In some embodiments, the immune-related disease is acute respiratory distress syndrome (ARDS), pneumonia, bronchitis, pneumonitis, and chronic obstructive pulmonary disease (COPD). The immune-related disease may be selected from the group consisting of ARDS, pneumonia, and pneumonitis. Optionally, the immune-related disease is ARDS. In some embodiments, the immune-related disease is a condition associated with a cytokine storm in the host, cytokine release syndrome (CRS), or a cytokine storm syndrome.

In some embodiments, the immune-related disease is a dysfunctional immune response associated with a viral infection. Optionally, the virus is selected from the group consisting of a cytomegalovirus, an Epstein-Barr virus, an influenza virus, a variola virus, an orthopoxvirus, a coronavirus, such as SARS coronavirus (SARS-CoV), SARS-CoV-2, and MERS-CoV. In some embodiments, the virus is selected from the group consisting of SARS-CoV, SARS-CoV-2, and MERS-CoV. The virus may be SARS-CoV-2. In some embodiments, the immune-related disease is selected from the group consisting of severe acute respiratory syndrome (SARS), Middle East respiratory syndrome (MERS), coronavirus disease 2019 (COVID-19), cytokine release syndrome (CRS), and cytokine storm syndrome. The immune-related disease may be selected from the group consisting of severe acute respiratory syndrome (SARS), Middle East respiratory syndrome (MERS), coronavirus disease 2019 (COVID-19) and influenza. Optionally, the immune-related disease is COVID-19.

Examples of neurodegenerative disorders that may be treated/prevented by the compositions/methods disclosed herein include, but are not limited to Alzheimer's disease, traumatic brain injury (TBI), chronic traumatic encephalitis (CTE), and Parkinson's disease.

Examples of atherosclerotic disorder that may be treated/prevented by the compositions/methods disclosed herein include, but are not limited to angina pectoris myocardial infarction, carotid stenosis, transient ischemic attacks and cerebral vascular accident (CVA).

In some embodiments, the method comprises administering to said mammal a therapeutically effective amount of an anti-CD154 antibody disclosed herein in combination with one or more additional agents. Optionally, the one or more additional agents is selected from the group consisting of anti-thrombotic drugs, anti-platelet drugs and non-steroidal anti-inflammatory drugs (NSAIDs). The anti-CD154 antibody may be administered simultaneously with the one or more additional agents. In some embodiments, the anti-CD154 antibody is administered sequentially with the one or more additional agents. Optionally, the anti-CD154 antibody is administered prior to the one or more additional agents. The anti-CD154 antibody may be administered subsequent to the one or more additional agents. In some embodiments, the anti-CD154 antibody is administered in the same composition as the one or more additional agents. Optionally, the anti-CD 154 antibody and the one or more additional agents are administered in separate compositions.

Examples of anti-thrombotic drugs include but are not limited to a glycoprotein IIb/IIIa receptor antagonist, a direct or indirect factor Xa inhibitor and an anticoagulant. Examples of anticoagulants include, but are not limited to, heparin, warfarin, rivaroxaban (XARELTO^{®}), ximelgatran (EXANTA^{®}), dabigatran (PRADAXA^{®}), apixaban (ELIQUIS^{®}), edoxaban (SAVAYSA^{®}), enoxaparin (LOVENOX^{®}), and fondaparinux (ARIXTRA^{®}). Examples of anti-thrombotic drugs include, but are not limited to, those disclosed in US Patent Nos. 4,782,069; 5,332,822; 5,492,895; 5,612,363, 5,691,364 5,693,641; 5,721,214; 5,726,173; 5,753,635; 5,846,970; 5,849,759; 5,889,005; 6,107,280; 6,140,351; 6,150,329; 6,180,627; 6,200,976; 6,242,432; 6,248,770; 6,271,215; 6,280,731; 6,287,794; 6,300,330; 6,300,342; 6,333,338; 6,395,731; 6,417,203; 6,432,955; 6,444,672; 6,451,832; 6,458,793; 6,486,129; 6,500,803; 6,583,173; 6,599,881; 6,723,723; 6,730,672; 6,753,331; 6,774,110; 6,797,710; and 6,924,296, incorporated in their entirety for all purposes. Examples of glycoprotein IIb/IIIa receptor antagonists include, but are not limited to, abciximab (REOPRO^{®}), rivaroxaban (XARELTO^{®}), apixaban (ELIQUIS^{®}), edoxaban (SAVAYSA^{®}), idrabiotaparinux, tirofiban (AGGRASTAT^{®}), and eptifibatide (INTEGRILIN^{®}). The direct or indirect factor Xa inhibitors include, but are not limited to, apixaban (ELIQUIS^{®}), idrabiotaparinux, fondaparinux (ARIXTRA^{®}), and rivaroxaban (XARELTO^{®}).

Examples of anti-platelet drugs include, but are not limited to, TXA2 pathway inhibitors, the adenosine diphosphate (ADP) pathway inhibitors, thrombin inhibitors, Protease activated receptor-1 (PAR-1) inhibitors and phosphodiesterase (PDE) inhibitors. Examples of ADP pathway inhibitors include, but are not limited, to clopidogrel (PLAVIX^{®}, ticlopidine (TICLID^{®}), prasugrel (EFFIENT^{®}), ticagrelor (BRILINTA^{®}), cangrelor (KENGREAL^{®}) and elinogrel. Non-limiting examples of PDE inhibitors include dipyridamole (PERSANTINE^{®}) and cilostazol (PLETAL^{®}).

Examples of NSAIDs include, but are not limited to, acetylsalicylic acid, celecoxib (CELEBREX^{®}), diclofenac (VOLTAREN^{®}, PENNSAID^{®}, SOLARAZE^{®}, ZIPSOR^{®}, CATAFLAM^{®}, ZORVOLEX^{®}), diflunisal (DOLOBID^{®}), etodolac (LODINE SR^{®}, ECCOXOLAC^{®}), ibuprofen (BRUFEN^{®}, ADVIL^{®}, MOTRIN^{®}), indomethacin (INDOCIN^{®}), ketoprofen (ORUDIS^{®}), ketorolac (TORADOL^{®}, ACULAR^{®}, SPRIX^{®}), nabumetone (RELAFEN^{®}), naproxen (AFLAXEN^{®}, ALEVE^{®}, ANAPROX^{®}, NAPRELAN^{®}), oxaprozin (DAYPRO^{®}, DAYRUN^{®}, DURAPROX^{®}), piroxicam (FELDENE^{®}), salsalate (MONO-GESIC^{®}, SALFLEX^{®}, DISALCID^{®}, SALSITAB^{®}), sulindac (CLINORIL^{®}), tolmetin (TOLECTIN^{®}), prasugrel (EFFIENT^{®}), ticagrelor (BRILINTA^{®}) and cangrelor (KENGREAL^{®}).

In some embodiments, the anti-CD 154 antibody may be administered in combination with one or more supplemental agents including but not limited to immunosuppressive drugs, immunomodulatory drugs, and monoclonal and/or polyclonal antibodies. The anti-CD154 antibody may be administered simultaneously with the one or more supplemental agents. In some embodiments, the anti-CD154 antibody is administered sequentially with the one or more supplemental agents. Optionally, the anti-CD154 antibody is administered prior to the one or more supplemental agents. The anti-CD154 antibody may be administered subsequent to the one or more supplemental agents. In some embodiments, the anti-CD154 antibody is administered in the same composition as the one or more supplemental agents. Optionally, the anti-CD154 antibody and the one or more supplemental agents are administered in separate compositions. Examples of such one or more supplemental agents include, but not limited to, anti-CD2 antibodies, anti-CD3 antibodies, anti-CD4 antibodies, anti-CD28 antibodies, anti-CD52 antibodies, anti-C5 antibodies, mTOR inhibitors, calcineurin inhibitors, antiviral drugs, and fusion peptides that bind to and block the function of CD28. Non-limiting examples of fusion peptides that bind to and block the function of CD28 include abatacept and belatocept (NULOJIX^{®}). For example, the anti-CD52 antibody may be alemtuzumab (CAMPATH^{®}). Optionally, the anti-C5 antibody is eculizumab (SOLIRIS^{®}).

Non-limiting examples of immunosuppressive drug or immunomodulatory drugs include cyclosporine A, tacrolimus (FK-506), doxorubicin (ADRIAMYCIN^{®}), azathioprine (IMURAN^{®}), busulfan (BUSULFEX^{®}), cyclophosphamide (CYTOXAN^{®}), fludarabine, 5- fluorouracil, methotrexate (OTREXUP^{®}, RASUVO^{®}, RHEUMATREX^{®}, TREXALL^{™}), mycophenolate mofetil (CELLCEPT^{®}), mizoribine (BREDININ^{™}), leflunomide, a nonsteroidal anti-inflammatory, adrenocortical steroids, rapamycin (RAPAMUNE^{®}), deoxyspergualin, FTY720, muromonab-CD3 (ORTHOCLONE OKT3^{®}), alemtuzumab (CAMPATH^{®}, MABCAMPATH^{®}, CAMPATH-1H^{®}, LEMTRADA^{®}), basiliximab (SIMULECT^{®}), daclizumab (ZINBRYTA^{®}), eculizumab (SOLIRIS^{®}), rituximab (RITUXAN^{®}, MABTHERA^{®}), bortezomib (VELCADE^{®}, CHEMOBORT^{®}, BORTECAD^{®}), siplizumab, anti-thymocyte globulin (THYMOGLOBULIN^{®}, ATGAM^{®}), leronlimab, siltuximbab (SYLVANT^{®}), sarilumab (KEVZARA^{®}), tocilizumab (ACTEMRA^{®}), bevacizumab (AVASTIN^{®}), ranibizumab (LUCENTIS^{®}), aflibercept (EYLEA^{®}) and inhibitors of Bruton's tyrosine kinase (BTK), including zanubrutinib (BRUKINSA^{®}), acalabrutinib (CALQUENCE^{®}) and ibrutinib (IMBRUVICA^{®}).

Examples of mTOR inhibitors include, but are not limited to, rapamycin (Rapamune^{®}), everolimus (AFINITOR^{®}), temsirolimus (TORISEL^{®}), ridaforolimus, and deforolimus. Examples of calcineurin inhibitors include, but are not limited to, cyclosporine (NEORAL^{®}, SANDIMMUNE^{®}, GENGRAF^{®}, RESTASIS^{®}), tacrolimus (FK506, ENVARSUS^{®}, HECORIA^{®}, PROGRAF^{®} PROTOPIC^{®}, ASTRAGRAF^{®}), and pimecrolimus (ELIDEL^{®}).

Examples of anti-allergy drugs include, but are not limited to, antihistamines, decongestants, corticosteroids, mast cell stabilizers, leukotriene inhibitors, epinephrine shots, azelastine eyedrops (OPTIVAR^{®}), azelastine nasal sprays (ASTELIN^{®}, ASTEPRO^{®}), beclomethasone (QNASL^{®} and QVAR^{®}), betamethasone (DERMABET^{®}, DIPROLENE^{®}), brompheniramine (DIMETANE^{®}), budesonide (RHINOCORT^{®} and PULMICORT FLEXHALER^{®}), carbinoxamine (PALGIC^{®}), cetirizine (ZYRTEC^{®}), ciclesonide (ALVESCO^{®}, ZETONNA^{®}), cyproheptadine, chlorpheniramine (CHLOR-TRIMETON^{®}), clemastine (TAVIST^{®}), cromolyn (CROLOM^{®}), desloratadine (CLARINEX^{®}), desonide (DESONATE^{®}, DESOWEN^{®}), diphenhydramine (BENADRYL^{®}), emedastine eyedrops (EMADINE^{®}), epinastine ophthalmic (ELESTAT^{®}), fexofenadine (ALLEGRA^{®}), fluorometholone (FLAREX^{®}, FML^{®}), fluticasone furoate (FLONASE SENSIMIST^{®}, VERAMYST^{®}), fluticasone propionate (FLONASE ALLERGY RELIEF^{®}), hydrocortisone (CORTAID^{®}, MICORT-HC^{®}), hydroxyzine (ATARAX^{®}, VISTARIL^{®}), ketotifen (ZADITOR^{®}), levocabastine eyedrops (LIVOSTIN^{®}), levocabastine oral (XYZAL^{®}), lodoxamide (ALOMIDE^{®}), loratadine (ALAVERT^{®}, CLARITIN^{®}), loteprednol (ALREX^{®}, LOTEMAX^{®}), methylprednisolone (MEDROL^{®}), mometasone (ASMANEX TWISTHALER^{®}, NASONEX^{®}, ELOCON^{®}), naphazoline and pheniramine combination ophthalmic (OCUHIST^{®}), nedocromil (ALOCRIL^{®}), olopatadine ophthalmic (PATANOL^{®}), oxymetazoline (AFRIN^{®}, DRISTAN^{®}), pemirolast (ALAMAST^{®}), prednisolone (PRELONE^{®}, OMNIPRED^{®}, PRED FORTE^{®}), prednisone (PREDNISONE INTENSOL^{®}, RAYOS^{®}), tetrahydrozoline (TYZINE^{®}) and triamcinolone (NASACORT ALLERGY 24 HOUR^{®}).

Examples of antiviral drugs include, but are not limited to, ribavirin, interferon (alfacon-1), chloroquine, hydroxychloroquine, EIDD-2801, EIDD-1931, GS-5734, GS-441524, ivermectin, favipiravir, indomethacin, chlorpromazine, penciclovir, nafomostat, nitazoxanide and remdesivir.

### Method of inducing chimerism

A seventh aspect of the present disclosure provides a method of inducing hematopoietic chimerism in a transplant recipient. In some embodiments, the method comprises administering to the recipient an anti-CD154 antibody disclosed herein, and transplanting into the recipient hematopoietic stem cells, thereby inducing hematopoietic chimerism in the recipient. Optionally, a therapeutically effective amount of the anti-CD154 antibody is administered. In some embodiments, the anti-CD154 is administered in a pharmaceutical composition disclosed herein. The anti-CD154 antibody may be administered simultaneously with the transplantation of the hematopoietic stem cells. In some embodiments, the anti-CD154 antibody is administered sequentially with the transplantation of the hematopoietic stem cells. Optionally, the anti-CD154 antibody is administered prior to the transplantation of the hematopoietic stem cells. The anti-CD154 antibody may be administered subsequent to the transplantation of the hematopoietic stem cells. In some embodiments, the anti-CD154 is administered in a single dose. Optionally, the anti-CD154 antibody is administered in multiple doses.

In some embodiments, the anti-CD154 antibody is administered at a dose of 5-50 mg/kg. The anti-CD154 antibody may be administered at a dose of about 5 to about 50 mg/kg. In some embodiments, the anti-CD154 antibody is administered at a dose of 5-30 mg/kg. The anti-CD154 antibody may be administered at a dose of about 5 to about 30 mg/kg. Optionally, the anti-CD154 antibody is administered at a dose of 5 mg/kg, 10 mg/kg, 15 mg/kg, 20 mg/kg, 25 mg/kg, 30 mg/kg, 35 mg/kg, 40 mg/kg, 45 mg/kg or 50 mg/kg. In some embodiments, the anti-CD154 antibody is administered at a dose of about 5 mg/kg, about 10 mg/kg, about 15 mg/kg, about 20 mg/kg, about 25 mg/kg, about 30 mg/kg, about 35 mg/kg, about 40 mg/kg, about 45 mg/kg or about 50 mg/kg. In some embodiments, the anti-CD154 antibody is administered systemically. The anti-CD154 antibody may be administered subcutaneously, intravenously, intravitreally, orally, via inhalation, transdermally, or rectally. Optionally, the anti-CD154 antibody is administered locally.

In some embodiments, the transplant recipient is conditioned prior to the transplantation with stem cells. The anti-CD154 antibody may be administered simultaneously with the conditioning step. Optionally, the anti-CD154 antibody is administered sequentially with the conditioning step. In some embodiment, the anti-CD154 antibody is administered prior to the conditioning step. Optionally, the anti-CD154 antibody is administered subsequent to the conditioning step.

Methods for conditioning the recipient include, but are not limited to, total body irradiation, administration of one or more BCL-2 inhibitors, administration of busulfan, administration of fludarabine phosphate, administration of cyclophosphamide, administration of immunosuppressive one or more T cell-depleting antibodies, administration of cyclosporine A (CsA), administration of FK-506, administration of one or more interleukin-2 (IL-2) receptor inhibitors, administration of IL-15 receptor inhibitors, administration of rapamycin, administration of one or more anti-αβ T cell receptor antibodies, and administration of one or more CD122 antagonists (which block both IL2 and IL15 signaling), administration of kidney donor-derived CD34+ hematopoietic stem cells and CD3+ T-cells (MDR-101 cellular therapy) or a combination thereof. Non-limiting examples of one or more T cell-depleting antibodies include anti-CD4, anti-CD8, anti-CD45, anti-CTLA4, anti-CD20, and anti-CD33 antibodies or a combination thereof. In some embodiments, the transplant recipient has cancer. In some embodiments, the transplant is a bone marrow transplant.

### Method of inducing central tolerance

An eighth aspect of the present disclosure provides a method of inducing central tolerance in a transplant recipient. In some embodiments, the method comprises administering to the recipient an anti-CD154 antibody disclosed herein, transplanting into the recipient hematopoietic stem cells, and transplanting a donor tissue into the recipient, wherein the transplanted hematopoietic stem cells produce immune cells that are tolerant of the donor tissue, thereby inducing central tolerance in the recipient. Optionally, a therapeutically effective amount of the anti-CD154 antibody is administered. In some embodiments, the anti-CD154 is administered in a pharmaceutical composition disclosed herein. The anti-CD154 antibody may be administered simultaneously with the transplantation of the hematopoietic stem cells. In some embodiments, the anti-CD154 antibody is administered sequentially with the transplantation of the hematopoietic stem cells. Optionally, the anti-CD154 antibody is administered prior to the transplantation of the hematopoietic stem cells. The anti-CD154 antibody may be administered subsequent to the transplantation of the hematopoietic stem cells. In some embodiments, the anti-CD154 is administered in a single dose. Optionally, the anti-CD154 antibody is administered in multiple doses.

In some embodiments, the anti-CD154 antibody is administered at a dose of 5-50 mg/kg. The anti-CD154 antibody may be administered at a dose of about 5 to about 50 mg/kg. In some embodiments, the anti-CD154 antibody is administered at a dose of 5-30 mg/kg. The anti-CD154 antibody may be administered at a dose of about 5 to about 30 mg/kg. Optionally, the anti-CD154 antibody is administered at a dose of 5 mg/kg, 10 mg/kg, 15 mg/kg, 20 mg/kg, 25 mg/kg, 30 mg/kg, 35 mg/kg, 40 mg/kg, 45 mg/kg or 50 mg/kg. In some embodiments, the anti-CD154 antibody is administered at a dose of about 5 mg/kg, about 10 mg/kg, about 15 mg/kg, about 20 mg/kg, about 25 mg/kg, about 30 mg/kg, about 35 mg/kg, about 40 mg/kg, about 45 mg/kg or about 50 mg/kg. In some embodiments, the anti-CD154 antibody is administered systemically. The anti-CD154 antibody may be administered subcutaneously, intravenously, intravitreally, orally, via inhalation, transdermally, or rectally. Optionally, the anti-CD154 antibody is administered locally.

In some embodiments, the transplant recipient is conditioned prior to the transplantation with stem cells. The anti-CD154 antibody may be administered simultaneously with the conditioning step. Optionally, the anti-CD154 antibody is administered sequentially with the conditioning step. In some embodiment, the anti-CD154 antibody is administered prior to the conditioning step. Optionally, the anti-CD154 antibody is administered subsequent to the conditioning step.

Methods for conditioning the recipient include, but are not limited to, total body irradiation, administration of one or more BCL-2 inhibitors, administration of busulfan, administration of fludarabine phosphate, administration of cyclophosphamide, administration of one or more immunosuppressive T cell-depleting antibodies, administration of cyclosporine A (CsA), administration of FK-506, administration of one or more interleukin-2 (IL-2) inhibitors, administration of rapamycin, administration of one or more anti-αβ T cell receptor antibodies, and administration of one or more CD122 antagonists or a combination thereof. Non-limiting examples of one or more T cell-depleting antibodies include anti-CD4, anti-CD8, anti-CD45, anti-CTLA4, anti-CD20, and anti-CD33 antibodies or a combination thereof.

### Method of preventing xenotransplant rejection

A ninth aspect of the present disclosure provides a method of preventing xenotransplant rejection in a transplant recipient. In some embodiments, the method comprises administering to the subject an effective amount of an anti-CD154 antibody disclosed herein. Optionally, a therapeutically effective amount of the anti-CD154 antibody is administered. In some embodiments, the anti-CD154 is administered in a pharmaceutical composition disclosed herein. The anti-CD154 antibody may be administered simultaneously with the xenotransplant. In some embodiments, the anti-CD154 antibody is administered sequentially with the xenotransplant. Optionally, the anti-CD154 antibody is administered prior to the xenotransplant. The anti-CD 154 antibody may be administered subsequent to the xenotransplant. In some embodiments, the anti-CD 154 is administered in a single dose. Optionally, the anti-CD154 antibody is administered in multiple doses. Optionally, the anti-CD154 antibody is administered in four doses. Optionally, the anti-CD154 antibody is administered in four doses of 30 mg/kg. Optionally, the anti-CD154 antibody is administered in four doses within two weeks. Optionally, the first dose of anti-CD 154 antibody is administered in two times, a first half dose before the graft is revascularized and a second half dose once it is confirmed that the xenotransplant procedure has been successful.

The anti-CD 154 antibody may be administered at a dose of 5-50 mg/kg. In some embodiments, the anti-CD154 antibody is administered at a dose of about 5 to about 50 mg/kg. The anti-CD154 antibody may be administered at a dose of 5-30 mg/kg. In some embodiments, the anti-CD 154 antibody is administered at a dose of about 5 to about 30 mg/kg. Optionally, the anti-CD154 antibody is administered at a dose of 5 mg/kg, 10 mg/kg, 15 mg/kg, 20 mg/kg, 25 mg/kg, 30 mg/kg, 35 mg/kg, 40 mg/kg, 45 mg/kg or 50 mg/kg. In some embodiments, the anti-CD154 antibody is administered at a dose of about 5 mg/kg, about 10 mg/kg, about 15 mg/kg, about 20 mg/kg, about 25 mg/kg, about 30 mg/kg, about 35 mg/kg, about 40 mg/kg, about 45 mg/kg or about 50 mg/kg. In some embodiments, the anti-CD 154 antibody is administered systemically. The anti-CD 154 antibody may be administered subcutaneously, intravenously, intravitreally, orally, via inhalation, transdermally, or rectally. Optionally, the anti-CD 154 antibody is administered locally.

The transplant recipient may be human, and the xenotransplant may be from a non-human donor. In some embodiments, the non-human donor is selected from a group consisting of a pig, a mini-swine, and a non-human primate. Optionally, the non-human donor is a pig or mini-swine that has been engineered to decrease or eliminate expression of one or more genes. In some embodiments, the one or more decreased or eliminated genes include, but are not limited to, porcine endogenous retroviruses (PERV), α-1,3-galactosyltransferase (GGTA1), cytidine monophosphate-N-acetylneuraminic acid hydroxylase (CMAH), β1,4-N-acetylgalactosaminyltransferase (β4GalNT2), and MHC class I. Optionally, the one or more decreased or eliminated genes comprise PERV. The PERV may be PERV A, a PERV B, or a PERV C. The expression of all PERV genes may be eliminated in the pig or mini-swine. The expression of the one or more genes may be decreased or eliminated using CRISPR/Cas9 gene editing.

In some embodiments, the non-human donor may be engineered to express one or more human proteins. Examples of one or more human proteins include, but are not limited to, complement regulatory protein, human α-galactosidase, a coagulation regulatory protein, a human anti-inflammatory protein, and human CTLA-4-Ig or a combination thereof. The one or more human proteins may be expressed in all tissues of the non-human donor. In some embodiments, the one or more human proteins are expressed in a tissue-specific manner in the non-human donor. Examples of complement regulatory protein include, but are not limited to, human decay-accelerating factor (CD55), membrane cofactor protein (CD46) and CD59. Examples of coagulation regulatory proteins include, but are not limited to, thrombomodulin, endothelial protein C receptor, tissue factor pathway inhibitor, CD39, and CD73. Examples of human anti-inflammatory proteins include, but are not limited to, hemeoxygenase-1 (HO-1) and A20.

The xenotransplant rejection may be associated with a solid organ transplant selected from the group consisting of a heart transplant, a kidney transplant, a liver transplant, a lung transplant, a pancreas transplant, a kidney-pancreas transplant, a heart-lung transplant, kidney-heart transplant, a kidney-heart-pancreas transplant, a heart-liver transplant, a heart-liver-kidney transplant, a heart-lung-kidney transplant, a heart-lung-liver transplant, a lung-kidney transplant, a lung-liver transplant, a liver-intestines-pancreas transplant, an intestines-pancreas transplant, a liver-kidney-intestines-pancreas transplant, and a kidney-intestines transplant.

### Examples

The following examples are offered for illustrative purposes only and do not limit the scope of the present disclosure or paragraphs in any way. Indeed, various modifications of the disclosure in addition to those shown and described herein will become apparent to those skilled in the art from the foregoing description and fall within the scope of the paragraphs. The following examples and preparations use the abbreviations: "MSX " for Methionine Sulphoximine , "RU" for ratio unit; "FACS" for Fluorescence Activated Cell Sorting "MW" for molecular weight; "His-Tag" for C-terminal polyhistidine (6xHis) (SEQ ID NO: 331) tag for rapid purification with nickel-chelating resin and detection with an anti-His (C-term) antibody; "BSA" for bovine serum albumin; "EDTA" for ethylenediaminetetraacetic acid; "DMSO" for dimethyl sulfoxide; "MOPS" for 3-(N-morpholino) propane sulfonic acid; "MES" for 2-(N-Morpholino)ethanesulfonic acid; "PBS" for phosphate buffered saline; "dPBS" for Dulbecco's phosphate buffered saline; "HEMA" for 2-hydroxy-ethyl methacrylate; "DMEM" for Dulbecco's modified eagle's medium; "FBS" for fetal bovine serum; "NEAA" for non-essential amino acids; "HEPES" for N-2-hydroxyethylpiperazine-N'-2-ethanesulfonic acid; and "DMF" for dimethyl formamide.

### Example 1. Generation of CHO Stable Pools expressing anti-CD 154 monoclonal antibody

DNA molecules encoding the sequences for the TNX01-TNX05 anti-CD154 antibodies with various Fc domains (SEQ ID NOs: 175-178 and 194) were codon optimized for CHO cells with internal EcoR1 and BamH1 removed and synthesized by one of two vendors: GeneScript (GS) and ATUM (AT). SEQ ID NO: 228 (5' -GAATTCGGCCGGCCACC) and SEQ ID NO: 229 (3'-TAATGAACGCGTGGATCC) were included in the final sequences as EcoRI and BamH1 restriction sites respectively. The DNA molecules were cloned into the pTT109^{™} (National Research Council, Canada) plasmid using EcoR1 and BamH1 and stably transfected into CHO55E1^{™} cells (National Research Council, Canada). 100µg industrial grade plasmid in ddH2O was prepared for all five constructs. Stable pools were generated using 50µM of MSX as a selection agent. The stable pool generation using 50µM MSX was performed in duplicate. Stable pools were generated in 6 extra-deep well plates, fed-bath production with Feed 4 (FUJIFILM IRVINE SCIENTIFIC^{®}). Stable pools were then generated in small scale 100 ml volumes in shake flasks with Feed 12.7 (FUJIFILM IRVINE SCIENTIFIC^{®}). Figure 1 summarizes the titer and availability of the five stable pools of CHO cells expressing anti-CD154 mAb in 100 mL fed-batch production. Table 1 summarizes the anti-CD154 mAb (TNX01-05; light chain comprising SEQ ID NO: 196; heavy chain comprising SEQ ID NOs: 147-150 and 165, respectively) production from the supernatant of stably transfected CHO cells as a starting material. A similar process was used to generate TNX06-TNX13 anti-CD154 antibodies with various Fc domains (SEQ ID NOs. 316-319 and 293-296). Table 2 summarizes the anti-CD154 mAb (TNX06-13; light chain comprising SEQ ID NO: 196; heavy chain comprising SEQ ID NOs: 166, 234, 284, 285, 159, 160, 281 and 282, respectively) production from the supernatant of stably transfected CHO cells as a starting material.

**Table 1. Antibody Production from Stable CHO Cells (100 mL Fed-Batch Production)**

| Pool Name | Fc Domain | Titer, pA-HPLC (mg/L) | Harvest Volume (mL) | Total amount (mg) | Quantity in 50 mL supernatant (mg) |
|---|---|---|---|---|---|
| TNX01 | SEQ ID NO: 1 | 603 | 100 | 60.3 | 30.15 |
| TNX02 | SEQ ID NO: 2 | 861 | 100 | 86.1 | 43.05 |
| TNX03 | SEQ ID NO: 3 | 615 | 100 | 61.5 | 30.75 |
| TNX04 | SEQ ID NO: 4 | 659 | 100 | 65.9 | 32.95 |
| TNX05 | SEQ ID NO: 14 | 354 | 100 | 35.4 | *24.78 (*70mL) |

**Table 2. Antibody Production from Stable CHO Cells (100 mL Fed-Batch Production)**

| Pool Name | Fc Domain | Titer, pA-HPLC (mg/L) | Harvest Volume (mL) | Total amount (mg) | Volume (mL) (~30 mg) |
|---|---|---|---|---|---|
| TNX06 | SEQ ID NO: 17 | 496 | 132 | 65 | 61 |
| TNX07 | SEQ ID NO: 236 | 463 | 131 | 61 | 65 |
| TNX08 | SEQ ID NO: 240 | 538 | 132 | 71 | 56 |
| TNX09 | SEQ ID NO: 241 | 523 | 131 | 69 | 57 |
| TNX10 | SEQ ID NO: 6 | 595 | 131 | 78 | 50 |
| TNX11 | SEQ ID NO: 4 | 643 | 131 | 84 | 47 |
| TNX12 | SEQ ID NO: 4 | 630 | 131 | 82 | 48 |
| TNX13 | SEQ ID NO: 4 | 639 | 131 | 84 | 47 |

### Example 2. Purification of anti-CD 154 monoclonal antibody

The anti-CD154 monoclonal antibodies (TNX01-TNX13) from Example 1 were purified by Protein A Affinity Purification using 4x1 mL HiTrap MabSelect SuRe columns. The following steps were performed according to the manufacturer's instructions. (1) Equilibration/Wash was conducted using DPBS; (2) 4x10 mL of the samples were loaded at a linear flow rate for binding set at approximately 45 cm/h (0.3 mL/min) to get a residence time of approximately 3.3 minutes; (3) Elution was conducted using 0.1M Citrate at a pH of 3.0; (4) Neutralization was conducted using 10% (v/v) 1M HEPES Buffer. The samples were buffer exchanged in DPBS using 5 mL Zeba Spin desalting columns (ThermoFisher Scientific, Waltham MA) and aseptically filtered on 0.22µm. No precipitation was observed during elution or neutralization. Table 3 below summarizes the antibody characteristics obtained after Protein A purification and desalting, and the yields of the thirteen purified anti-CD154 monoclonal antibodies samples obtained from the CHOSSE1 stable pools. Note that TNX04 and TNX05 required further purification using preparative size exclusion chromatography (prep-SEC) to remove aggregates.

**Table 3. Antibody Recovery Following Purification**

| Sample ID/Lot# | Fc Domain | Quantity (mg) | Concentration (mg/mL) | Volume (mL) | % Monomer (UPLC-SEC) |
|---|---|---|---|---|---|
| TNX01 | SEQ ID NO: 1 | 22.84 | 11.77 | 1.94 | 98.3 |
| TNX02 | SEQ ID NO: 2 | 29.38 | 15.22 | 1.93 | 99.1 |
| TNX03 | SEQ ID NO: 3 | 20.79 | 11.49 | 1.81 | 98.3 |
| TNX04 | SEQ ID NO: 4 | 19.04 | 1.63 | 11.68 | 99.0 |
| TNX05 | SEQ ID NO: 14 | 14.54 | 1.23 | 11.8 | 99.8 |
| TNX06 | SEQ ID NO: 17 | 21.28 | 13.30 | 1.60 | 98.15 |
| TNX07 | SEQ ID NO: 236 | 22.31 | 13.86 | 1.61 | 94.61 |
| TNX08 | SEQ ID NO: 240 | 20.34 | 13.30 | 1.53 | 95.92 |
| TNX09 | SEQ ID NO: 241 | 21.52 | 13.79 | 1.56 | 94.82 |
| TNX10 | SEQ ID NO: 6 | 21.88 | 13.76 | 1.59 | 97.72 |
| TNX 11 | SEQ ID NO: 4 | 21.52 | 13.45 | 1.60 | 97.24 |
| TNX12 | SEQ ID NO: 4 | 23.22 | 14.79 | 1.57 | 97.44 |
| TNX 13 | SEQ ID NO: 4 | 19.01 | 12.34 | 1.54 | 97.62 |

The samples were analyzed by SDS PAGE during each step of the purification process using Sypro Ruby stained gel under non-reducing conditions. Samples were also analyzed once the final product was obtained. As shown in Figure 2, under non-reducing condition, the mAbs TNX01-TNX05 were estimated to have a molecular weight of 160-170 kDa, except for TNX04 which exists as a free heavy chain (HC) and free light chain (LC). In TNX04, the heavy and light chains run separately in a denaturing SDS-PAGE, under non-reducing conditions, given that the cysteines that form the di-sulfide bonds (C220, C226, and C229) are mutated. UPLC-SEC results suggest that the non-covalent interactions form an intact mAb under physiological conditions. Lower molecular weight (LMW) bands are likely due to artefactual fragmentation of mAbs during SDS-PAGE samples preparation. As shown in Figure 10, the mAbs TNX06-TNX13 were estimated to have a molecular weight of 160-170 kDa, except for TNX11 which behaves as a half antibody and presumably forms a non-covalent 2HC-2LC structure.

The final products were also analyzed using size-exclusion ultra-performance liquid chromatography (UPLC-SEC) according to the manufacturer's instructions. *Results:* These results demonstrate that highly pure samples of the anti-CD 154 antibodies of the disclosure were obtained.

### Example 3. Determination of Anti-CD 154 Monoclonal Antibody Binding to CD40L by Surface Plasmon Resonance (SPR) using BIACORE^{®}

The binding of the purified anti-CD154 antibodies from Example 2 to CD40L was measured by surface plasmon resonance using the BIACORE^{®} T200 instrument and a sensorchip CM-5 according to the manufacturer's instructions.

Specifically, commercial CD40L (SINO BIOLOGICAL^{®} Cat#LC12AP2301) was injected over anti-CD154 antibody pools (TNX01-05) captured onto a Protein A surface (2,000 Rus; Cat#Z02201 GENSCRIPT^{®}, Piscataway, NJ), using PBST pH 7.4 (PBS with 0.05% Tween 20) as the running buffer. The SPR antibody capture surface was prepared using Protein A (Cat#Z02201 GENSCRIPT^{®}, Piscataway, NJ), 2000 Rus. The ligand was 1.25-2.5 µg/mL mAb (TNX01-TNX05) captured for 10s at a flow of 10µL/min to generate approximately 50 RU antibody surfaces. The analyte was CD40L (SINO BIOLOGICAL^{®} 10239-H08E) resuspended according to manufacturer's recommendations. Single cycle kinetics were conducted with 4 concentrations (6.0 / 1.5 / 0.38 / nM 3-fold dilution, Flow 75 µL/min, s association, 1800s dissociation. The regeneration was conducted with 1 x 60s 10mM glycine pH 1.5, flow 30µL/min.

The raw data (n=3) were processed using the BIACORE^{®} T200 Evaluation software. Figure 3 provides representative CD40L sensorgrams for TNX01-TNX05 and demonstrated a 1:1 binding model. As CD40L is a trimer, the sensorgrams deviated somewhat from ideal binding behavior due to the possibility of avid binding (multiple binding sites) by the trimer antigen. All of the fits should be considered "apparent" K_{D} values because the sensorgrams were deviating from 1:1 binding behavior. CD40L binding is based on the calculated fit of the sensorgrams.

Figure 4 summarizes the data generated from the SPR analysis and the calculated apparent K_{D} values (affinity; ratio of kd to ka). TNX05 did not to bind to the protein A surface as efficiently as TNX01-TNX04. This weaker binding may represent a smaller component of avidity in these sensorgrams. A lower surface density indicates that the TNX05 molecules are further apart which could equate to fewer sCD40L molecules engaging multiple antibody paratopes at any one time. TNX01-TNX05 were comparable in terms of K_{D} and approached the lower limit of affinity measurement for the instrument platform in terms of the measured on-rate.

For TNX06-TNX13, the sensorgrams were generated using the BIACORE^{®}T200 Evaluation software and compared using the sensorgram comparability approach. This method is a statistical comparison of sensorgram shape only, independent of kinetic analysis, developed by GEHealthcare for the Biacore T200 platform. An average 3SD window of variation in the sensorgram scatter is generated for one batch as a reference (TNX02), which was then compared pairwise to the other variants. Each pairwise comparison was assessed a similarity score based on the number of points inside the window of variation, and distance of points outside the reference window. Using this method, all of the samples were compared to the TNX02 scatter window collected in triplicate. All of the variants (TNX06-TNX13) tested showed approximately 80% similarity to the TNX02 sensorgrams for sCD40L binding with no obvious large deviations in the on- or off-rate, which indicates they are all binding in a similar manner. *See* Figure 11.

*Results* : These results demonstrate that each of the anti-CD154 antibodies of the disclosure bind to CD40L. TNX01-TNX04 (purified anti-CD154 antibodies) bind to CD40L with an affinity of about 10pM binding (see Figure 4). TNX05 had slightly weaker binding, at around 20 pM (see Figure 4). TNX06-13 are approximately 80% similar to TNX02 in the manner in which they bind to CD40L with no obvious large deviations.

### Example 4. Determination of Anti-CD154 Monoclonal Antibody Binding to FcyRIA by Surface Plasmon Resonance (SPR) using BIACORE^{®}

The binding of the purified anti-CD154 antibodies from Example 2 to high affinity FcyRIA (CD64) was measured by surface plasmon resonance (SPR) using the BIACORE^{®} T200 instrument and a sensorchip CM-5 according to the manufacturer's instructions.

Specifically, FcγRIA was injected over anti-CD 154 antibody pools immobilized onto a Protein A surface (2,000 Rus; Cat#Z02201 GENSCRIPT^{®}, Piscataway, NJ), using PBST pH 7.4 (PBS with 0.05% Tween 20) as the running buffer. The ligand was 1.25-2.5 µg/mL mAb (TNX01-TNX05) captured for 10s at a flow of 10µL/min to generate approximately 250-400 RUs antibody surfaces. The analyte FcγRIA (CD64; National Research Council, Canada) was resuspended in PBST containing 3.4 mM EDTA and 0.05% Tween20 and used at a concentration range of 300 to 0.14 nM, at a flow rate of 100µL/min, 90s association time, 1,200s dissociation time. The regeneration was conducted with 1 x 60s 10mM glycine pH 1.5, flow 30µL/min.

The raw data (n=3) were processed using the BIACORE^{®}T200 Evaluation software. Figure 5 provides representative FcγRIA sensorgrams for TNX01-TNX05 and demonstrates a 1:1 binding model.

Figure 6 summarizes the data generated from the SPR analysis and the calculated apparent K_{D} values (affinity; ratio of kd to ka) for TNX01-05. Due to the high affinity of the ligand CD64 (TNX02 K_{D} ~ 30 pM), accurate K_{D} values could be determined for all of the loss-of-function variants. TNX04 showed an 11-fold loss of activity followed by TNX05 with a 300-fold loss. Both TNX01 and TNX03 had a 3000-fold loss of activity (fast-on and -off), which necessitated a steady-state end-point analysis rather than kinetic analysis.

Figures 12a-12c summarize the data generated from the SPR analysis and the calculated apparent K_{D} values (affinity; ratio of kd to ka) for TNX06-13 binding to FcγRIA. TNX06 to TNX13 were all successfully assayed for binding to FcgRIa. Due to the high affinity of the ligand CD64 (TNX02 K_{D} ~ 40 pM), accurate K_{D} values could be determined for all of the loss-of-function variants. TNX06, 07, 11,12 and 13 showed between 6-12-fold weaker binding than TNX02. TNX08, 09 and 10 had 2600, 3800 and 1800-fold weaker binding, respectively, (fast-on and -off). *Results* : These results demonstrate that, as desired, the anti-CD154 antibodies of the disclosure demonstrate reduced binding to FcγRIA.

### Example 5. Determination of Anti-CD 154 Monoclonal Antibody Binding to low-affinity FcyR by Surface Plasmon Resonance (SPR) using BIACORE^{®}

The binding of the purified anti-CD154 antibodies from Example 2 to a low affinity FcyR panel (CD16aF, CD16aV, CD32aH, CD32bF) was measured by surface plasmon resonance (SPR) using the BIACORE^{®} T200 instrument and a sensorchip CM-5 according to the manufacturer's instructions. This low affinity FcyR panel of antigens represents most common allotypes of the different receptors that have been used to assess the effector function of antibodies of interest. See *e.g.*, Geuijen et al., FEBS Open Bio 7 (2017) 1557-1574 (2017); Hanson and Barb, Biochemistry, 54, 2931-2942 (2015); Smith and Clatworthy Nature Reviews Immunol (10):328-344 (2010); Weiner et al., Nature Reviews Immunol (10):317-327 (2010).

Specifically, antigens in an FcyR panel (CD16aF, CD16aV, CD32aH, CD32bF) were each injected over anti-CD154 antibody pools (TNX01-TNX05) onto a Protein A surface (2,000 Rus; Cat#Z02201 GENSCRIPT^{®}, Piscataway, NJ), using PBST pH 7.4 (PBS with 0.05% Tween 20) as the running buffer. The ligand was 1.25 - 2.5 µg/mL mAb (TNX01-TNX05) captured for 10s at a flow of 10µL/min to generate approximately 200-500 RUs antibody surfaces (surface dependent). The analytes were antigens in the FcyR panel (CD16aF, CD16aV, CD32aH, CD32bF; National Research Council, Canada), which were resuspended in PBST containing 3.4mM EDTA and 0.05% Tween20. Single cycle kinetics were conducted at concentration ranging from 12 µM to 0.062 µM, Flow rate 25 µL/min, 15s association (CD32aH and CD32bF) and 40 s association (CD16aF and CD16aV), dissociation time 120s. The regeneration was conducted with 1 x 60s 10mM glycine pH 1.5, flow 30µL/min (SPR data not shown). Thus, TNX01-TNX05 were assayed for binding to the National Research Council Canada, Human Health Therapeutics Research Centre low-affinity FcyR panel (CD16aF, CD16aV, CD32aH, CD32bF) by measuring steady-state affinity.

Figure 7 summarizes the data generated from the SPR analysis and the calculated apparent K_{D} values (affinity; ratio of kd to ka) for TNX01-05. In particular, TNX02 displayed the expected wild-type binding, with the other variants showing significantly reduced binding. In particular, TNX01 and TNX03 demonstrated almost no binding activity at the FcyR concentrations tested. TNX05 showed ~5- to 20-fold loss of binding to the various receptors. TNX04 showed 20- to 60-fold loss of binding to CD16aV, CD32aH, CD32bF and undetectable binding to CD16aF (FcyRIIIaF).

Figures 13a-13b summarize the data generated from the SPR analysis and the calculated apparent K_{D} values (affinity; ratio of kd to ka) for TNX06-13 binding to low-affinity FcyR. TNX02 displayed the expected wild-type binding, with the other variants showing significant losses of function. In particular, TNX11, showed almost no binding activity at the FcyR concentrations tested to any members of the low-affinity FcyR panel. TNX06 and 07 showed ~2 to 10-fold loss of activity against the various low-affinity receptors. TNX08 showed 2 to 17-fold loss of activity against the various low-affinity receptors. TNX09 and 10 showed undetectable activity against all low-affinity receptors except CD16aV (FcyRIIIaV) to which they both had ~12-fold loss. TNX12 showed undetectable activity against all low-affinity receptors except CD32bF (FcyRIIbF) for which it had an 8-fold loss. TNX13 showed undetectable activity against both CD16aV (FcyRIIIaV) and CD16aF (FcyRIIIaF) and had 6 and 17-fold loss of binding activity against CD32bF (FcyRIIbF) and CD32aH (FcγRIIaH), respectively. *Results:* These results demonstrate that the anti-CD154 antibodies of the disclosure do not bind or demonstrate reduced binding to various FcyR low-affinity receptors.

### Example 6. Anti-CD154 Monoclonal Antibodies Binding in Jurkat D1.1 and Jurkat cells

The binding of the purified anti-CD 154 antibodies from Example 2 to Jurkat D1.1 cells (CD40L positive) and Jurkat cells (CD40L negative) was evaluated. Yellin, MJ, et al; J. Immunol. 147:3389-3395 (1991); Lederman, S, et al. J. Exp. Med. 175:1091-1101 (1992); U.S. Patent No. 6,331,433. High-throughput FACS cell-binding assays were conducted using the test antibodies (TNX01-TNX05), and a control antibody (mouse anti-human CD40L from (R&D MAB617)). SYNAGIS^{®} was used as a human IgG1 isotype negative control antibody. The detection reagents were AF488 F(ab')₂ donkey anti-human IgG, Fcy fragment specific (Jackson ImmunoResearch, Westgrove, PA), and goat anti-mouse IgG, Fcy fragment specific (Jackson ImmunoResearch, Westgrove, PA). Briefly, 10⁵ Jurkat or D1.1 cells cells were incubated for 2 hours at 4°C with 0-200 nM primary antibody, and 1 hour at 4°C with the secondary detection antibody followed by viability staining with propidium iodide (1% solution). Flow cytometric acquisition of signals in samples were performed the same day in a LSR FORTESSA^{™} (BD Biosciences). The cells were washed and the AlexaFluor488 fluorescence was measured using the 488 nm laser as an excitation source and the 530/30nm band pass filter. The median fluorescence intensity (MFI) was determined for 5000 alive, single cells per sample. The FACS cell-binding assays were similarly conducted for the test antibodies TNX06-TNX13.

As shown in Figure 8(a), the anti-CD154 antibodies (TNX01-TNX05) all bind with high affinity to D1.1 cells, whereas the negative control antibody (SYNAGIS^{®}) had no detectable binding to D1.1 cells. The K_{D} values were calculated using the specific binding parameter with hill slope fit. In contrast to Figure 8(a), Figure 8(b) demonstrates that all of the anti-CD154 antibodies and the negative control antibody (SYNAGIS^{®}) failed to bind to Jurkat cells, which do not express CD40L on their surfaces. As shown in Figure 14(a), the anti-CD154 antibodies (TNX06-TNX13) all bind with high affinity to D1.1 cells, whereas the negative control antibody (SYNAGIS^{®}) had no detectable binding to D1.1 cells. The K_{D} values were calculated using the specific binding parameter with hill slope fit. *Results:* These results demonstrate that the purified anti-CD154 antibodies of the disclosure bind to CD40L-expressing D1.1 cells with high affinity.

### Example 7. Anti-CD154 Monoclonal Antibodies Platelet Activation Study

The ability of purified anti-CD 154 antibodies from Example 2 to activate platelets is evaluated. CD40L (CD154) is a strong activator of nuclear factor kappa B (NF-κB) in platelets that primes and enhances platelet activation in response to thrombotic stimuli. Further, binding of the Fc domain of anti-CD40L mAbs (such as hu5c8) to the activating FcgRIIa (CD32a) receptor on platelets results in activation and aggregation of platelets. Kojok K et. al.; J Am Heart Assoc. 7:e009636 (2018); Shock et al., Arthritis Res Ther. Sep 3;17:234 (2015); Xie et al., The Journal of Immunology, 192: 4083-4092 (2014).

Blood samples from healthy human blood donors, 18 years or older, is collected in sterile vacuum blood collection tubes (yellow top BD Vacutainer B364606, Canada), mixed gently four times by inversion, and tested within 60 mins. A portion of the blood is pre-treated with anti-CD32a (FcgRIIa) antibody and incubated at room temperature (RT) for 30 min. A portion of untreated blood (i.e., no incubation with an anti-CD32a antibody) from each donor is also tested. The test conditions include: (1) soluble recombinant human CD40L (sCD40L) alone; (2) sCD40L complexed to human IgG1 anti-CD40L; (3) a direct platelet agonist adenosine 5'-diphosphate sodium salt; along with detection antibodies to platelet phenotyping cell surface markers CD41a and CD61, and the platelet activation markers P-selectin (CD62P) and activated GPIIb/IIIa (PAC1). IC ratios of the HuIgG1 anti-CD40L: sCD40L of 1:1, 1:2, 1:3.5, 1:4, 1:5 and 1:6 are tested for activating platelets. To determine absolute counts of each cell type, 50 µL of 123count eBeads (Invitrogen, NJ) are added to the wells. All the conditions are tested in triplicates. Platelets are analyzed for activation by flow cytometry.

**Table 4 below summarizes the final concentrations of the molecules used in the platelet assay:**

| **Molecule** | **Description** | **Final concentration used** |
|---|---|---|
| Media | Basal control | -- |
| Excipient | PBS | -- |
| TI 1 (sCD40L) | sCD40L, tested alone at 10 µg/mL and in IC | 10 µg/mL, 20 µg/mL, 35 µg/mL, 40 µg/mL, 50 µg/mL and 60 µg/mL |
| TI 2 Hu IgG1 anti-CD40L (hu5c8-IgG1) | anti-CD40L (hu5c8-IgG1) (Absolute antibody Cedarlane, Canada) | 10 µg/mL |
| TI 3 Mouse IgG1 anti-Hu CD40L | Mouse IgG1 anti-Hu CD40L | 10 µg/mL |
| ADP | Adenosine 5'-diphosphate sodium salt, Positive control | 10 µg/mL ≈ 22 µM |
| Hu IgG1 anti-CD40L with reduced FcR binding (TNX01-TNX13) | Fc domain containing key point mutations that decrease/abrogate binding to Fc gamma receptors | 10 µg/mL |

Once stained and fixed, the samples are acquired on Cytoflex flow cytometer using Cytexpert version 1.2 software (Beckman Coulter, MN) and analyzed using FlowJo version 10.1 software (FlowJo LLC, OR). Platelet cells are defined based on the forward scatter (FSC; an indication of size) and side scatter (SSC; an indication of granularity) profile. Further, the activated and non-activated status of platelets identified based on gating are recorded. Absolute Count using 123count eBeads is calculated using the following formula: (cells/µL) = (Cell count/eBead Count) X eBead Concentration.

From the recorded data, all of the wells from the same sample are grouped, averaged and the standard deviation as well as the % Coefficient of Variation (CV) is calculated between replicates using LibreOffice^{™} Calc software. From the Calc files generated, tables including the percentage of each cell population are generated.

Next, the fold induction over control is computed for each treatment group in the platelet assay conducted at the optimal molar ratio for each test antibody:sCD40L. Fold induction over control is the ratio of median fluorescence intensity (MFI) of each treatment group for each parameter (CD62P or PAC-1) over MFI of the control blood group. The anti-CD32a antibody, which selectively blocks 5c8-IgG1/sCD40L IC-induced platelet activation, is expected to show no effect on platelet activation induced by ADP, a direct platelet agonist. 5c8-IgG1 (the positive control) is expected to show platelet activation. The test antibodies of the present disclosure (TNX01-TNX13) are expected to show diminished or no activity in the platelet activation assay.

*Results:* These results are expected to demonstrate that the purified anti-CD154 antibodies of the disclosure have a diminished ability to activate or aggregate platelets unlike the positive control hu5c8-IgG1.

### Example 8. Anti-CD 154 Monoclonal Antibodies for the treatment of heart transplant rejection

A heterotopic heart transplantation was performed in a Cynomolgus macaque monkey. The heart was transplanted into the abdomen and connected to descending aortic artery and vena cava. Anti-CD154 antibody TNX05 (hu5c8 mutated-IgG4 (IgG4 S228P/L235A)) was administered intravenously to the monkey. The monkey was administered a 30 mg/kg dose four times within the first two weeks. Half of the first dose of the Anti-CD154 antibody TNX05 was given before the graft was revascularized and the other half when it was confirmed that the procedure was successful. After the first two weeks, the monkey was administered a 10 mg/kg dose once a week for four weeks. Then, the monkey was administered 20 mg/kg once every four weeks. The dose on Day 84 was reduced from 20 mg/kg to 15 mg/kg.

Blood was collected at days 70, 77 and 84 and a Complete Blood Count was performed. All the parameters tested were within normal ranges, as seen in Table 5 below for the 30 mg/kg dose monkey. The monkey's transplanted heart was strongly beating through palpation, and contractility was also maintained, as observed by Ultrasound. No sign of rejection of the transplanted heart was detected through Day 84. No signs of thrombosis or thromboembolic events were detected through Day 84.

**Table 5.**

| **Normal Ranges** | **Parameter*** | **Day 70** | **Day 77** | **Day 84** |
|---|---|---|---|---|
| 6.9 - 14.5 | WBC | 7.2 | 11 | 5.6 |
| 4.4 - 7.0 | LYM | 3.4 | 4 | 2.1 |
| 0.0 - 0.2 | MONO | 0.6 | 1.1 | 0.6 |
| 3.3 - 6.3 | GRAN | 3.2 | 5.9 | 2.9 |
| | LYM % | 47.9 | 36.9 | 37.9 |
| | MONO % | 7.3 | 9.6 | 8.8 |
| | GRAN % | 44.8 | 53.5 | 53.3 |
| 40.7 - 47.3 | HCT | 36.1 | 38.1 | 38.3 |
| 72.3 - 83.7 | MCV | 67.5 | 68.1 | 66.9 |
| 0.0 - 99.9 | RDWa | 56.6 | 54.4 | 53.2 |
| 0.0 - 99.9 | RDW % | 20.1 | 19.2 | 19 |
| 12.8 - 15.0 | HGB | 11.8 | 12.2 | 12.4 |
| 31.1 - 32.9 | MCHC | 32.8 | 32 | 32.6 |
| 23.1 - 26.9 | MCH | 22.1 | 21.8 | 21.8 |
| 5.10 - 6.30 | RBC | 5.35 | 5.59 | 5.72 |
| 302 - 734 | PLT | 236 | 129 | 201 |
| 0.0 - 99.9 | MPV | 8.5 | 8.9 | 9.2 |

| | | | | |
|---|---|---|---|---|
| *WBC: white blood cell; LYM: Lymphocytes (%: percentage of total cells); MONO: Monocytes (%: percentage of total cells); GRAN: Granulocytes (%: percentage of total cells); HCT: Hematocrit; MCV: Mean corpuscular volume; RDW: Red cell distribution width (a: absolute value and % percentage); HGB: Hemoglobin; MCHC: Mean corpuscular hemoglobin concentration; MCH: Mean corpuscular hemoglobin; RBC: Red blood cell count; PLT: Platelet count; MPV: Mean platelet volume. | | | | |

A biopsy was performed on day 91. Two days after the biopsy, a telemetry device showed decreasing heart graft function. An ultrasound demonstrated that graft contractility was reduced. An exploration of the abdomen confirmed that the graft was beating weakly. The graft was removed, and the monkey was euthanized.

An autopsy did not show any evidence of thromboembolism outside of the heart graft, where fresh thrombus confined to the ventricular cavities was found, as expected after a graft stops beating, and blood in the heart becomes static. The lungs had no infarcts, and the brain and intestine were entirely normal. Those are sites where thrombi were mainly observed following administration with prior art antibodies. The graft anastomoses were widely open and without any clots.

*Results:* These results demonstrate that the antibodies of the invention are capable of preventing transplant rejection, while avoiding the problems with thrombosis caused by prior art antibodies.

**Table 6. Sequences of humanized anti-CD154 antibodies.**

| SEQ ID | Description | Sequences |
|---|---|---|
| **Fc (CH2 - CH3) WITH AND WITHOUT TERMINAL LYSINE AND WITH/WITHOUT HINGE** | | |
| **Fc domain, no terminal lysine** | | |
| 1 | TNX01 IgG1 Fc (N297Q) | APELLGGPSVFLFPPKPKDTLMISRTPEVTCVVVDVSHEDPEVKFNWYV DGVEVHNAKTKPREEQYQSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPG |
| 2 | TNX02 IgG1 Fc (wildtype) | APELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPG |
| 3 | TNX03 IgG1 Fc (N297G) | APELLGGPSVFLFPPKPKDTLMISRTPEVTCVVVDVSHEDPEVKFNWYV DGVEVHNAKTKPREEQYGSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPG |
| 4 | TNX04 TNX11 TNX12 TNX13 IgG1 Fc (P238S) | APELLGGSSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPG |
| 5 | TNX05 TNX14 IgG4 Fc (L235A) | APEFAGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLG |
| 6 | TNX10 IgG1 Fc (L234A, L235A) | APEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPG |
| 7 | IgG1 Fc (P238S, N297Q) | APELLGGSSVFLFPPKPKDTLMISRTPEVTCVVVDVSHEDPEVKFNWYV DGVEVHNAKTKPREEQYQSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPG |
| 8 | TNX06 Wildtype IgG4 Fc | APEFLGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLG |
| 9 | Wildtype IgG2 Fc | APPVAGPSVFLFPPKPKDTLMISRTPEVTCVVVDVSHEDPEVQFNWYVD GMEVHNAKTKPREEQFNSTFRVVSVLTVVHQDWLNGKEYKCKVSNKGLP APIEKTISKTKGQPREPQVYTLPPSREEMTKNQVSLTCLVKGFYPSDIA VEWESNGQPENNYKTTPPMLDSDGSFFLYSKLTVDKSRWQQGNVFSCSV MHEALHNHYTQKSLSLSPG |
| 237 | TNX07 IgG4 Fc (S228P) | APEFLGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLG |
| 238 | TNX08 IgG4 Fc (L235E) | APEFEGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLG |
| 239 | TNX09 IgG4 Fc(F234A, L235A) | APEAAGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLG |

| **Fc domain +terminal lysine** | | |
|---|---|---|
| 10 | TNX01 IgG1 Fc (N297Q) | APELLGGPSVFLFPPKPKDTLMISRTPEVTCVVVDVSHEDPEVKFNWYV DGVEVHNAKTKPREEQYQSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPGK |
| 11 | TNX02 IgG1 Fc (wildtype) | APELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPGK |
| 12 | TNX03 IgG1 Fc (N297G) | APELLGGPSVFLFPPKPKDTLMISRTPEVTCVVVDVSHEDPEVKFNWYV DGVEVHNAKTKPREEQYGSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPGK |
| 13 | TNX04 TNX11 TNX12 TNX13 IgG1 Fc (P238S) | APELLGGSSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPGK |
| 14 | TNX05 TNX14 IgG4 Fc (L235A) | APEFAGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLGK |
| 15 | TNX10 IgG1 Fc (L234A, L235A) | APEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPGK |
| 16 | IgG1 Fc (P238S, N297Q) | APELLGGSSVFLFPPKPKDTLMISRTPEVTCVVVDVSHEDPEVKFNWYV DGVEVHNAKTKPREEQYQSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPGK |
| 17 | TNX06 Wildtype IgG4 Fc | APEFLGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLGK |
| 18 | Wildtype IgG2 Fc | APPVAGPSVFLFPPKPKDTLMISRTPEVTCVVVDVSHEDPEVQFNWYVD GMEVHNAKTKPREEQFNSTFRVVSVLTVVHQDWLNGKEYKCKVSNKGLP APIEKTISKTKGQPREPQVYTLPPSREEMTKNQVSLTCLVKGFYPSDIA VEWESNGQPENNYKTTPPMLDSDGSFFLYSKLTVDKSRWQQGNVFSCSV MHEALHNHYTQKSLSLSPGK |
| 240 | TNX08 IgG4 Fc (L235E) | APEFEGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLGK |
| 241 | TNX09 IgG4 Fc(F234A, L235A) | APEAAGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLGK |

| **Fc region, no terminal lysine** | | |
|---|---|---|
| 19 | TNX01 IgG1 hinge-Fc (N297Q) | EPKSCDKTHTCPPCPAPELLGGPSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 20 | TNX02 IgG1 hinge-Fc (wildtype) | EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 21 | TNX03 IgG1 hinge-Fc (N297G) | EPKSCDKTHTCPPCPAPELLGGPSVFLFPPKPKDTLMISRTPEVTCVVV DVSHEDPEVKFNWYVDGVEVHNAKTKPREEQYGSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 22 | TNX04 IgG1 hinge-Fc (C220S, C226S, C229S, P238S) | EPKSSDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 23 | TNX05 TNX14 hinge-Fc IgG4 (S228P, L235A) | ESKYGPPCPPCPAPEFAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 24 | TNX10 IgG1 hinge-Fc (L234A, L235A) | EPKSCDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 25 | IgG1 hinge-Fc (P238S) | EPKSCDKTHTCPPCPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 26 | IgG1 hinge-Fc (P238S, N297Q) | EPKSCDKTHTCPPCPAPELLGGSSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 27 | TNX06 Wildtype hinge-IgG4 Fc | ESKYGPPCPSCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 28 | Wildtype hinge-IgG2 Fc | ERKCCVECPPCPAPPVAGPSVFLFPPKPKDTLMISRTPEVTCVVVDVSH EDPEVQFNWYVDGMEVHNAKTKPREEQFNSTFRVVSVLTVVHQDWLNGK EYKCKVSNKGLPAPIEKTISKTKGQPREPQVYTLPPSREEMTKNQVSLT CLVKGFYPSDIAVEWESNGQPENNYKTTPPMLDSDGSFFLYSKLTVDKS RWQQGNVFSCSVMHEALHNHYTQKSLSLSPG |
| 29 | IgG1 hinge-Fc (C220S, C226S, C229S, P238S, N297Q) | EPKSSDKTHTSPPSPAPELLGGSSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 30 | IgG1 hinge-Fc (C220S, C226S, C229S) | EPKSSDKTHTSPPSPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 31 | IgG1 hinge-Fc (C220S, N297Q) | EPKSSDKTHTCPPCPAPELLGGPSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 32 | IgG1 short-hinge-Fc (C226S, C229S, P238S) | THTSPPSPAPELLGGSSVFLFPPKPKDTLMISRTPEVTCVVVDVSHEDP EVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| 33 | IgG1 short-hinge-Fc (C226S, C229S) | THTSPPSPAPELLGGPSVFLFPPKPKDTLMISRTPEVTCVVVDVSHEDP EVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| 34 | IgG1 short-hinge-Fc (C226S, C229S, P238S, N297Q) | THTSPPSPAPELLGGSSVFLFPPKPKDTLMISRTPEVTCVVVDVSHEDP EVKFNWYVDGVEVHNAKTKPREEQYQSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| 35 | IgG1 short-hinge-Fc (C226S, C229S, N297Q) | THTSPPSPAPELLGGPSVFLFPPKPKDTLMISRTPEVTCVVVDVSHEDP EVKFNWYVDGVEVHNAKTKPREEQYQSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| 36 | IgG1 short-hinge-Fc (N297Q) | THTCPPCPAPELLGGPSVFLFPPKPKDTLMISRTPEVTCVVVDVSHEDP EVKFNWYVDGVEVHNAKTKPREEQYQSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| 37 | TNX11 IgG1 hinge-Fc (C226S, C229S, P238S) | EPKSCDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 242 | TNX07 IgG4 hinge-Fc (S228P) | ESKYGPPCPPCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 243 | TNX08 IgG4 hinge-Fc (L235E) | ESKYGPPCPPCPAPEFEGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 244 | TNX09 IgG4 hinge-Fc (F234A, L235A) | ESKYGPPCPPCPAPEAAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 245 | TNX12 IgG1 hinge-Fc (C229S, P238S) | EPKSCDKTHTCPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 246 | TNX13 IgG1 hinge-Fc (C226S, P238S) | EPKSCDKTHTSPPCPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |

| **Fc region + terminal lysine** | | |
|---|---|---|
| 38 | TNX01 IgG1 hinge-Fc (N297Q) | EPKSCDKTHTCPPCPAPELLGGPSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 39 | TNX02 IgG1 hinge-Fc (wildtype) | EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 40 | TNX03 IgG1 hinge-Fc (N297G) | EPKSCDKTHTCPPCPAPELLGGPSVFLFPPKPKDTLMISRTPEVTCVVV DVSHEDPEVKFNWYVDGVEVHNAKTKPREEQYGSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 41 | TNX04 IgG1 hinge-Fc (C220S, C226S, C229S, P238S) | EPKSSDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 42 | TNX05 hinge-Fc IgG4 (S228P, L235A) | ESKYGPPCPPCPAPEFAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 43 | TNX10 IgG1 hinge-Fc (L234A, L235A) | EPKSCDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 44 | IgG1 hinge-Fc (P238S) | EPKSCDKTHTCPPCPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 45 | IgG1 hinge-Fc (P238S, N297Q) | EPKSCDKTHTCPPCPAPELLGGSSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 46 | TNX06 Wildtype hinge-IgG4 Fc | ESKYGPPCPSCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 47 | Wildtype hinge-IgG2 Fc | ERKCCVECPPCPAPPVAGPSVFLFPPKPKDTLMISRTPEVTCVVVDVSH EDPEVQFNWYVDGMEVHNAKTKPREEQFNSTFRVVSVLTVVHQDWLNGK EYKCKVSNKGLPAPIEKTISKTKGQPREPQVYTLPPSREEMTKNQVSLT CLVKGFYPSDIAVEWESNGQPENNYKTTPPMLDSDGSFFLYSKLTVDKS RWQQGNVFSCSVMHEALHNHYTQKSLSLSPGK |
| 48 | IgG1 hinge-Fc (C220S, C226S, C229S, P238S, N297Q) | EPKSSDKTHTSPPSPAPELLGGSSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 49 | IgG1 hinge-Fc (C220S, C226S, C229S) | EPKSSDKTHTSPPSPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 50 | IgG1 hinge-Fc (C220S, N297Q) | EPKSSDKTHTCPPCPAPELLGGPSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 51 | IgG1 short-hinge-Fc (C226S, C229S, P238S) | THTSPPSPAPELLGGSSVFLFPPKPKDTLMISRTPEVTCVVVDVSHEDP EVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPGK |
| 52 | IgG1 short-hinge-Fc (C226S, C229S) | THTSPPSPAPELLGGPSVFLFPPKPKDTLMISRTPEVTCVVVDVSHEDP EVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPGK |
| 53 | IgG1 short-hinge-Fc (C226S, C229S, P238S, N297Q) | THTSPPSPAPELLGGSSVFLFPPKPKDTLMISRTPEVTCVVVDVSHEDP EVKFNWYVDGVEVHNAKTKPREEQYQSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPGK |
| 54 | IgG1 short-hinge-Fc (C226S, C229S, N297Q) | THTSPPSPAPELLGGPSVFLFPPKPKDTLMISRTPEVTCVVVDVSHEDP EVKFNWYVDGVEVHNAKTKPREEQYQSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPGK |
| 55 | IgG1 short-hinge-Fc (N297Q) | THTCPPCPAPELLGGPSVFLFPPKPKDTLMISRTPEVTCVVVDVSHEDP EVKFNWYVDGVEVHNAKTKPREEQYQSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPGK |
| 56 | TNX11 IgG1 hinge-Fc (C226S, C229S, P238S) | EPKSCDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 247 | TNX08 IgG4 hinge-Fc (S228P, L235E) | ESKYGPPCPPCPAPEFEGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 248 | TNX09 IgG4 hinge-Fc (S228P, F234A, L235A) | ESKYGPPCPPCPAPEAAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 249 | TNX12 IgG1 hinge-Fc (C229S, P238S) | EPKSCDKTHTCPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 250 | TNX13 IgG1 hinge-Fc (C226S, P238S) | EPKSCDKTHTSPPCPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 251 | TNX14 IgG4 hinge-Fc (L115T, S228P, L235A) | ESKYGPPCPPCPAPEFAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |

| **Heavy and light chain components (CDRs, VH, VL CH1, CH2, CH3)** | | |
|---|---|---|
| 57 | CDR H1 | SYYMY |
| 58 | CDR H2 | EINPSNGDTNFNEKFKS |
| 59 | CDR H3 | SDGRNDMDS |
| 60 | CDR L1 | ISCRASQRVSSSTYSYMH |
| 61 | CDR L2 | YASNLES |
| 62 | CDR L3 | QHSWEIPPT |
| 63 | VH full (with leader) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSS |
| 64 | VH full (no leader) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSS |
| 252 | VH full (with leader) (L115T) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTTVTVSS |
| 253 | VH full (no leader) (L115T) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTTVTVSS |
| 65 | VL full (with leader) | MRLPAQLLGLLMLWVSGSSGDIVLTQSPATLSVSPGERATISCRASQRV SSSTYSYMHWYQQKPGQPPKLLIKYASNLESGVPARFSGSGSGTDFTLT ISSVEPEDFATYYCQHSWEIPPTFGGGTKLEIK |
| 66 | VL full (no leader) | DIVLTQSPATLSVSPGERATISCRASQRVSSSTYSYMHWYQQKPGQPPK LLIKYASNLESGVPARFSGSGSGTDFTLTISSVEPEDFATYYCQHSWEI PPTFGGGTKLEIK |
| 67 | IgG1 CH1 (wildtype) (118-215 EU Numbering) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV |
| 68 | IgG1 CH2 (wildtype) (231-340) | APELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAK |
| 69 | IgG1 CH3 (wildtype) (341-446) | GQPREPQVYTLPPSRDELTKNQVSLTCLVKGFYPSDIAVEWESNGQPEN NYKTTPPVLDSDGSFFLYSKLTVDKSRWQQGNVFSCSVMHEALHNHYTQ KSLSLSPGK |
| 70 | IgG2 CH1 (wildtype) | ASTKGPSVFPLAPCSRSTSESTAALGCLVKDYFPEPVTVSWNSGALTSG VHTFPAVLQSSGLYSLSSVVTVPSSNFGTQTYTCNVDHKPSNTKVDKTV |
| 71 | IgG2 CH2 (wildtype) (231-339 EU) | APPVAGPSVFLFPPKPKDTLMISRTPEVTCVVVDVSHEDPEVQFNWYVD GVEVHNAKTKPREEQFNSTFRVVSVLTVVHQDWLNGKEYKCKVSNKGLP APIEKTISKTK |
| 72 | IgG2 CH3 (wildtype) (340-446 EU) | GQPREPQVYTLPPSREEMTKNQVSLTCLVKGFYPSDISVEWESNGQPEN NYKTTPPMLDSDGSFFLYSKLTVDKSRWQQGNVFSCSVMHEALHNHYTQ KSLSLSPGK |
| 73 | IgG4 CH1 (wildtype) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV |
| 74 | IgG4 CH2 (wildtype) (231-339 EU) | APEFLGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAK |
| 75 | IgG4 CH3 (wildtype) (341-447 EU) | GQPREPQVYTLPPSQEEMTKNQVSLTCLVKGFYPSDIAVEWESNGQPEN NYKTTPPVLDSDGSFFLYSRLTVDKSRWQEGNVFSCSVMHEALHNHYTQ KSLSLSLGK |

| **HINGES** | | |
|---|---|---|
| 76 | IgG1 hinge (216-230) | EPKS**C**DKTHT**C**PP**C**P |
| 77 | IgG1 hinge (C229S) | EPKS**C**DKTHT**C**PPSP |
| 78 | IgG1 hinge (C226S) | EPKS**C**DKTHTSPP**C**P |
| 79 | IgG1 hinge (C220S) | EPKSSDKTHT**C**PP**C**P |
| 80 | IgG1 hinge (C220S, C229S) | EPKSSDKTHT**C**PPSP |
| 81 | IgG1 hinge (C226S, C229S) | EPKS**C**DKTHTSPPSP |
| 82 | IgG1 hinge (C220S, C226S) | EPKSSDKTHTSPP**C**P |
| 83 | IgG1 hinge-Fc (C220S, C226S, C229S) | EPKSSDKTHTSPPSP |
| 84 | IgG1 short-hinge-Fc (C226S, C229S) | THTSPPSP |
| 85 | IgG1 short-hinge-Fc | THT**C**PP**C**P |
| 86 | IgG1 short-hinge-Fc (C226S) | THTSPP**C**P |
| 87 | IgG1 short-hinge-Fc (C229S) | THT**C**PPSP |
| 88 | IgG4 hinge-Fc (S228P) | ESKYGPPCPPCP |
| 89 | Wildtype hinge-IgG4 Fc (216-230) | ESKYGPPCP**S**CP |
| 90 | Wildtype hinge-IgG2 Fc (216-230) | ERKCCVECPPCP |

| **Heavy chain constant domains (ASTlinker-CH1-hinge-CH2-CH3) with and without terminal lysine residue** | | |
|---|---|---|
| **ASTlinker-CH1-hinge-CH2-CH3 without terminal K** | | |
| 91 | TNX01 CH1-CH2-CH3 IgG1(N297Q) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 92 | TNX02 CH1-CH2-CH3 IgG1 (wt) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 93 | TNX03 CH1-CH2-CH3 IgG1 (N297G) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYGSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 94 | TNX04 CH1-CH2-CH3 IgG1 (C220S, C226S, C229S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSSDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 95 | TNX05 CH1-CH2-CH3 IgG4 (S228P, L235A) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEFAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 96 | TNX06 CH1-CH2-CH3 IgG4 Wild Type | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPSCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 97 | CH1-CH2-CH3 IgG2 Wild Type | ASTKGPSVFPLAPCSRSTSESTAALGCLVKDYFPEPVTVSWNSGALTSG VHTFPAVLQSSGLYSLSSVVTVTSSNFGTQTYTCNVDHKPSNTKVDKTV ERKCCVECPPCPAPPVAGPSVFLFPPKPKDTLMISRTPEVTCVVVDVSH EDPEVQFNWYVDGMEVHNAKTKPREEQFNSTFRVVSVLTVVHQDWLNGK EYKCKVSNKGLPAPIEKTISKTKGQPREPQVYTLPPSREEMTKNQVSLT CLVKGFYPSDIAVEWESNGQPENNYKTTPPMLDSDGSFFLYSKLTVDKS RWQQGNVFSCSVMHEALHNHYTQKSLSLSPG |
| 98 | CH1-CH2-CH3 IgG1 (ΔE216-K222 IN HINGE) (C226S, C229S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV THTSPPSPAPELLGGSSVFLFPPKPKDTLMISRTPEVTCVVVDVSHEDP EVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| 99 | CH1-CH2-CH3 IgG1 (C220S, C226S, C229S, P238S, N297Q) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSSDKTHTSPPSPAPELLGGSSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 100 | CH1-CH2-CH3 IgG1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV THTSPPSPAPELLGGSSVFLFPPKPKDTLMISRTPEVTCVVVDVSHEDP EVKFNWYVDGVEVHNAKTKPREEQYQSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| | (ΔE216-K222 IN HINGE) (C226S, C229S, P238S, N297Q) | |
| 101 | CH1-CH2-CH3 IgG1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSSDKTHTSPPSPAPELLGGSSVFLFPPKPKDTLMISRTPEVTCVVV DVSHEDPEVKFNWYVDGVEVHNAKTKPREEQYGSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| | (C220S, C226S, C229S, P238S, N297G) | |
| 102 | CH1-CH2-CH3 IgG1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV THTSPPSPAPELLGGSSVFLFPPKPKDTLMISRTPEVTCVVVDVSHEDP EVKFNWYVDGVEVHNAKTKPREEQYGSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| | (ΔE216-K222 IN HINGE) (C226S, C229S, P238S, N297G) | |
| 103 | TNX10 CH1-CH2-CH3 IgG1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| | (L234A, L235A) | |
| 104 | TNX11 CH1-CH2-CH3 IgG1 (C226S, C229S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 254 | TNX07 CH1-CH2-CH3 IgG4 (S228P) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 255 | TNX08 CH1-CH2-CH3 IgG4 (S228P, L235E) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEFEGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 256 | TNX09 CH1-CH2-CH3 IgG4 (S228P, F234A, L235A) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEAAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |
| 257 | TNX12 IgG1 CH1-CH2-CH3 IgG1 (C229S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 258 | TNX13 IgG1 VH-CH1-CH2-CH3 IgG1 (C226S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTSPPCPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPG |
| 259 | TNX14 VH-CH1-CH2-CH3 IgG4 (L115T, S228P, L235A) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEFAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLG |

| ASTlinker-CH1-hinge-CH2-CH3 with terminal K | | |
|---|---|---|
| 105 | TNX01 CH1-CH2-CH3 IgG1(N297Q) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 106 | TNX02 CH1-CH2-CH3 IgG1 (wt) | |
| 107 | TNX03 CH1-CH2-CH3 IgG1 (N297G) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVV DVSHEDPEVKFNWYVDGVEVHNAKTKPREEQYGSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 108 | TNX04 CH1-CH2-CH3 IgG1 (C220S, C226S, C229S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSSDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 109 | TNX05 CH1-CH2-CH3 IgG4 (S228P, L235A) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEFAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 110 | TNX06 CH1-CH2-CH3 IgG4 Wild Type | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPSCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 111 | CH1-CH2-CH3 IgG2 Wild Type | ASTKGPSVFPLAPCSRSTSESTAALGCLVKDYFPEPVTVSWNSGALTSG VHTFPAVLQSSGLYSLSSVVTVTSSNFGTQTYTCNVDHKPSNTKVDKTV ERKCCVECPPCPAPPVAGPSVFLFPPKPKDTLMISRTPEVTCVVVDVSH EDPEVQFNWYVDGMEVHNAKTKPREEQFNSTFRVVSVLTVVHQDWLNGK EYKCKVSNKGLPAPIEKTISKTKGQPREPQVYTLPPSREEMTKNQVSLT CLVKGFYPSDIAVEWESNGQPENNYKTTPPMLDSDGSFFLYSKLTVDKS RWQQGNVFSCSVMHEALHNHYTQKSLSLSPGK |
| 112 | CH1-CH2-CH3 IgG1 (ΔE216-K222 IN HINGE) (C226S, C229S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV THTSPPSPAPELLGGSSVFLFPPKPKDTLMISRTPEVTCVVVDVSHEDP EVKFNWYVDGVEVHNAKTKPREEQYNSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPGK |
| 113 | CH1-CH2-CH3 IgG1 (C220S, C226S, C229S, P238S, N297Q) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSSDKTHTSPPSPAPELLGGSSVFLFPPKPKDTLMISRTPEVTCVVV DVSHEDPEVKFNWYVDGVEVHNAKTKPREEQYQSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 114 | CH1-CH2-CH3 IgG1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV THTSPPSPAPELLGGSSVFLFPPKPKDTLMISRTPEVTCVVVDVSHEDP EVKFNWYVDGVEVHNAKTKPREEQYQSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPG |
| | (ΔE216-K222 IN HINGE) (C226S, C229S, P238S, N297Q) | |
| 115 | CH1-CH2-CH3 IgG1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSSDKTHTSPPSPAPELLGGSSVFLFPPKPKDTLMISRTPEVTCVVV DVSHEDPEVKFNWYVDGVEVHNAKTKPREEQYGSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| | (C220S, C226S, C229S, P238S, N297G) | |
| 116 | CH1-CH2-CH3 IgG1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV THTSPPSPAPELLGGSSVFLFPPKPKDTLMISRTPEVTCVVVDVSHEDP EVKFNWYVDGVEVHNAKTKPREEQYGSTYRVVSVLTVLHQDWLNGKEYK CKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQ QGNVFSCSVMHEALHNHYTQKSLSLSPGK |
| | (ΔE216-K222 IN HINGE) (C226S, C229S, P238S, N297G) | |
| 117 | TNX10 CH1-CH2-CH3 IgG1 | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| | (L234A, L235A) | |
| 118 | TNX11 CH1-CH2-CH3 IgG1 (C226S, C229S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 260 | TNX07 CH1-CH2-CH3 IgG4 (S228P) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 261 | TNX08 CH1-CH2-CH3 IgG4 (S228P, L235E) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEFEGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 262 | TNX09 CH1-CH2-CH3 IgG4 (S228P, F234A, L235A) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEAAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 263 | TNX12 IgG1 CH1-CH2-CH3 IgG1 (C229S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTCPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 264 | TNX13 IgG1 VH-CH1-CH2-CH3 IgG1 (C226S, P238S) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKV EPKSCDKTHTSPPCPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| 265 | TNX14 VH-CH1-CH2-CH3 IgG4 (L115T, S228P, L235A) | ASTKGPSVFPLAPCSRSTSESTAALGCLVKDYFPEPVTVSWNSGALTSG VHTFPAVLQSSGLYSLSSVVTVPSSSLGTKTYTCNVDHKPSNTKVDKRV ESKYGPPCPPCPAPEFAGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |

| **Full length heavy chain with and without leader; with and without terminal lysine residue** | | |
|---|---|---|
| **Heavy chain full length sequences with leader and no terminal K** | | |
| 119 | TNX01 VH-CH1-CH2-CH3 IgG1(N297Q) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNW YVDGVEVHNAKTKPREEQYQSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| 120 | TNX02 VH-CH1-CH2-CH3 IgG1 (wt) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| 121 | TNX03 VH-CH1-CH2-CH3 IgG1 (N297G) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNW YVDGVEVHNAKTKPREEQYGSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| 122 | TNX04 VH-CH1-CH2-CH3 IgG1 (C220S, C226S, C229S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSSDKTHTSPP SPAPELLGGSSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| 123 | TNX05 VH-CH1-CH2-CH3 IgG4 (S228P, L235A) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFAGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLG |
| 124 | TNX06 VH-CH1-CH2-CH3 IgG4 Wild Type | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPSCPA PEFLGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLG |
| 125 | VH-CH1-CH2-CH3 IgG2 Wild Type | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVTSSNFGTQTYTCNVDHKPSNTKVDKTVERKCCVECPPCPA PPVAGPSVFLFPPKPKDTLMISRTPEVTCVVVDVSHEDPEVQFNWYVDG MEVHNAKTKPREEQFNSTFRVVSVLTVVHQDWLNGKEYKCKVSNKGLPA PIEKTISKTKGQPREPQVYTLPPSREEMTKNQVSLTCLVKGFYPSDIAV EWESNGQPENNYKTTPPMLDSDGSFFLYSKLTVDKSRWQQGNVFSCSVM HEALHNHYTQKSLSLSPG |
| 126 | VH-CH1-CH2-CH3 IgG1 (ΔE216-K222 IN HINGE) (C226S, C229S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVTHTSPPSPAPELL GGSSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEA LHNHYTQKSLSLSPG |
| 127 | VH-CH1-CH2-CH3 IgG1 (C220S, C226S, C229S, P238S, N297Q) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSSDKTHTSPP SPAPELLGGSSVFLFPPKPKDTLMISRTPEVTCVVVDVSHEDPEVKFNW YVDGVEVHNAKTKPREEQYQSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| 128 | VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVTHTSPPSPAPELL GGSSVFLFPPKPKDTLMISRTPEVTCVVVDVSHEDPEVKFNWYVDGVEV HNAKTKPREEQYQSTYRVVSVLTVLHQDWLNGKEYKCKVSNKALPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEA LHNHYTQKSLSLSPG |
| | (ΔE216-K222 IN HINGE) (C226S, C229S, P238S, N297Q) | |
| 129 | VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSSDKTHTSPP SPAPELLGGSSVFLFPPKPKDTLMISRTPEVTCVVVDVSHEDPEVKFNW YVDGVEVHNAKTKPREEQYGSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| | (C220S, C226S, C229S, P238S, N297G) | |
| 130 | VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVTHTSPPSPAPELL GGSSVFLFPPKPKDTLMISRTPEVTCVVVDVSHEDPEVKFNWYVDGVEV HNAKTKPREEQYGSTYRVVSVLTVLHQDWLNGKEYKCKVSNKALPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEA LHNHYTQKSLSLSPG |
| | (ΔE216-K222 IN HINGE) (C226S, C229S, P238S, N297G) | |
| 131 | TNX10 VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP CPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| | (L234A, L235A) | |
| 132 | TNX11 VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTSPP SPAPELLGGSSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| | (C226S, C229S, P238S) | |
| 266 | TNX07 VH-CH1-CH2-CH3 IgG4 (S228P) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFLGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLG |
| 267 | TNX08 VH-CH1-CH2-CH3 IgG4 (S228P, L235E) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFEGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLG |
| 268 | TNX09 VH-CH1-CH2-CH3 IgG4 (S228P, F234A, L235A) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEAAGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLG |
| 269 | TNX12 IgG1 VH-CH1-CH2-CH3 IgG1 (C229S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP SPAPELLGGSSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| 270 | TNX13 IgG1 VH-CH1-CH2-CH3 IgG1 (C226S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTSPP CPAPELLGGSSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPG |
| 271 | TNX14 VH-CH1-CH2-CH3 IgG4 (L115T, S228P, L235A) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTTVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFAGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLG |

| **Heavy chain full length sequences above with leader and terminal K** | | |
|---|---|---|
| 133 | TNX01 VH-CH1-CH2-CH3 IgG1(N297Q) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNW YVDGVEVHNAKTKPREEQYQSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 134 | TNX02 VH-CH1-CH2-CH3 IgG1 (wt) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 135 | TNX03 VH-CH1-CH2-CH3 IgG1 (N297G) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNW YVDGVEVHNAKTKPREEQYGSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 136 | TNX04 VH-CH1-CH2-CH3 IgG1 (C220S, C226S, C229S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSSDKTHTSPP SPAPELLGGSSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 137 | TNX05 VH-CH1-CH2-CH3 IgG4 (S228P, L235A) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFAGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLGK |
| 138 | TNX06 VH-CH1-CH2-CH3 IgG4 Wild Type | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPSCPA PEFLGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLGK |
| 139 | VH-CH1-CH2-CH3 IgG2 Wild Type | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVTSSNFGTQTYTCNVDHKPSNTKVDKTVERKCCVECPPCPA PPVAGPSVFLFPPKPKDTLMISRTPEVTCVVVDVSHEDPEVQFNWYVDG MEVHNAKTKPREEQFNSTFRVVSVLTVVHQDWLNGKEYKCKVSNKGLPA PIEKTISKTKGQPREPQVYTLPPSREEMTKNQVSLTCLVKGFYPSDIAV EWESNGQPENNYKTTPPMLDSDGSFFLYSKLTVDKSRWQQGNVFSCSVM HEALHNHYTQKSLSLSPGK |
| 140 | VH-CH1-CH2-CH3 IgG1 (ΔE216-K222 IN HINGE) (C226S, C229S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVTHTSPPSPAPELL GGSSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEA LHNHYTQKSLSLSPGK |
| 141 | VH-CH1-CH2-CH3 IgG1 (C220S, C226S, C229S, P238S, N297Q) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSSDKTHTSPP SPAPELLGGSSVFLFPPKPKDTLMISRTPEVTCVVVDVSHEDPEVKFNW YVDGVEVHNAKTKPREEQYQSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 142 | VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVTHTSPPSPAPELL GGSSVFLFPPKPKDTLMISRTPEVTCVVVDVSHEDPEVKFNWYVDGVEV HNAKTKPREEQYQSTYRVVSVLTVLHQDWLNGKEYKCKVSNKALPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEA LHNHYTQKSLSLSPGK |
| | (ΔE216-K222 IN HINGE) (C226S, C229S, P238S, N297Q) | |
| 143 | VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSSDKTHTSPP SPAPELLGGSSVFLFPPKPKDTLMISRTPEVTCVVVDVSHEDPEVKFNW YVDGVEVHNAKTKPREEQYGSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| | (C220S, C226S, C229S, P238S, N297G) | |
| 144 | VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVTHTSPPSSPAPELL GGSSVFLFPPKPKDTLMISRTPEVTCVVVDVSHEDPEVKFNWYVDGVEV HNAKTKPREEQYGSTYRVVSVLTVLHQDWLNGKEYKCKVSNKALPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEA LHNHYTQKSLSLSPGK |
| | (ΔE216-K222 IN HINGE) (C226S, C229S, P238S, N297G) | |
| 145 | TNX10 VH-CH1-CH2-CH3 IgG1 | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP CPAPEAAGGPSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| | (L234A, L235A) | |
| 146 | TNX11 VH-CH1-CH2-CH3 IgG1 (C226S, C229S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTSPP SPAPELLGGSSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 272 | TNX07 VH-CH1-CH2-CH3 IgG4 (S228P) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFLGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLGK |
| 273 | TNX08 VH-CH1-CH2-CH3 IgG4 (S228P, L235E) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFEGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLGK |
| 274 | TNX09 VH-CH1-CH2-CH3 IgG4 (S228P, F234A, L235A) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEAAGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLGK |
| 275 | TNX12 IgG1 VH-CH1-CH2-CH3 IgG1 (C229S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPP SPAPELLGGSSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 276 | TNX13 IgG1 VH-CH1-CH2-CH3 IgG1 (C226S, P238S) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTLVTVSSASTKGPSVFPLAP SSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTSPP CPAPELLGGSSVFLFPPKPKDTLMISRTPEVTCVVVDVSHEDPEVKFNW YVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 277 | TNX14 VH-CH1-CH2-CH3 IgG4 (L115T, S228P, L235A) | MPLLLLLPLLWAGALAQVQLVQSGAEVVKPGASVKLSCKASGYIFTSYY MYWVKQAPGQGLEWIGEINPSNGDTNFNEKFKSKATLTVDKSASTAYME LSSLRSEDTAVYYCTRSDGRNDMDSWGQGTTVTVSSASTKGPSVFPLAP CSRSTSESTAALGCLVKDYFPEPVTVSWNSGALTSGVHTFPAVLQSSGL YSLSSVVTVPSSSLGTKTYTCNVDHKPSNTKVDKRVESKYGPPCPPCPA PEFAGGPSVFLFPPKPKDTLMISRTPEVTCVVVDVSQEDPEVQFNWYVD GVEVHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSCSV MHEALHNHYTQKSLSLSLGK |

| **Full-length heavy chain sequences without leader and no terminal K** | | |
|---|---|---|
| 147 | TNX01 VH-CH1-CH2-CH3 IgG1(N297Q) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPRE EQYQSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| 148 | TNX02 VH-CH1-CH2-CH3 IgG1 (wt) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| 149 | TNX03 VH-CH1-CH2-CH3 IgG1 (N297G) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPRE EQYGSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| 150 | TNX04 VH-CH1-CH2-CH3 IgG1 (C220S, C226S, C229S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSSDKTHTSPPSPAPELLGGSSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| 151 | TNX05 VH-CH1-CH2-CH3 IgG4 (S228P, L235A) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEFAGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLG |
| 152 | TNX06 VH-CH1-CH2-CH3 IgG4 Wild Type | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPSCPAPEFLGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLG |
| 153 | VH-CH1-CH2-CH3 IgG2 Wild Type | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVTSSNFGT QTYTCNVDHKPSNTKVDKTVERKCCVECPPCPAPPVAGPSVFLFPPKPK DTLMISRTPEVTCVVVDVSHEDPEVQFNWYVDGMEVHNAKTKPREEQFN STFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTP PMLDSDGSFFLYSKLTVDKSRWQQGNVFSCSVMHEALHNHYTQKSLSLS PG |
| 154 | VH-CH1-CH2-CH3 IgG1 (ΔE216-K222 IN HINGE) (C226S, C229S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVTHTSPPSPAPELLGGSSVFLFPPKPKDTL MISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPG |
| 155 | VH-CH1-CH2-CH3 IgG1 (C220S, C226S, C229S, P238S, N297Q) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSSDKTHTSPPSPAPELLGGSSVFLFP PKPKDTLMISRTPEVTCVVVDVSHEDPEVKFNWYVDGVEVHNAKTKPRE EQYQSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| 156 | VH-CH1-CH2-CH3 IgG1 | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVTHTSPPSPAPELLGGSSVFLFPPKPKDTL MISRTPEVTCVVVDVSHEDPEVKFNWYVDGVEVHNAKTKPREEQYQSTY RVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPG |
| | (ΔE216-K222 IN HINGE) (C226S, C229S, P238S, N297Q) | |
| 157 | VH-CH1-CH2-CH3 IgG1 | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSSDKTHTSPPSPAPELLGGSSVFLFP PKPKDTLMISRTPEVTCVVVDVSHEDPEVKFNWYVDGVEVHNAKTKPRE EQYGSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| | (C220S, C226S, C229S, P238S, N297G) | |
| 158 | VH-CH1-CH2-CH3 IgG1 | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVTHTSPPSPAPELLGGSSVFLFPPKPKDTL MISRTPEVTCVVVDVSHEDPEVKFNWYVDGVEVHNAKTKPREEQYGSTY RVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFCSVMHEALHNHYTQKSLSLSPG |
| | (ΔE216-K222 IN HINGE) (C226S, C229S, P238S, N297G) | |
| 159 | TNX10 VH-CH1-CH2-CH3 IgG1 | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPEAAGGPSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| | (L234A, L235A) | |
| 160 | TNX11 VH-CH1-CH2-CH3 IgG1 (C226S, C229S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTSPPSPAPELLGGSSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| 278 | TNX07 VH-CH1-CH2-CH3 IgG4 (S228P) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEFLGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLG |
| 279 | TNX08 VH-CH1-CH2-CH3 IgG4 (S228P, L235E) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEFEGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLG |
| 280 | TNX09 VH-CH1-CH2-CH3 IgG4 (S228P, F234A, L235A) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEAAGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLG |
| 281 | TNX12 IgG1 VH-CH1-CH2-CH3 IgG1 (C229S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPSPAPELLGGSSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| 282 | TNX13 IgG1 VH-CH1-CH2-CH3 IgG1 (C226S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTSPPCPAPELLGGSSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPG |
| 283 | TNX14 VH-CH1-CH2-CH3 IgG4 (L115T, S228P, L235A) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTIVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEFAGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLG |

| **Full-le ngth heavy chain sequences without leader and with terminal K** | | |
|---|---|---|
| 161 | TNX01 VH-CH1-CH2-CH3 IgG1(N297Q) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPRE EQYQSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 162 | TNX02 VH-CH1-CH2-CH3 IgG1 (wt) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 163 | TNX03 VH-CH1-CH2-CH3 IgG1 (N297G) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPRE EQYGSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 164 | TNX04 VH-CH1-CH2-CH3 IgG1 (C220S, C226S, C229S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSSDKTHTSPPSPAPELLGGSSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 165 | TNX05 VH-CH1-CH2-CH3 IgG4 (S228P, L235A) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEFAGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLGK |
| 166 | TNX06 VH-CH1-CH2-CH3 IgG4 Wild Type | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPSCPAPEFLGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLGK |
| 167 | VH-CH1-CH2-CH3 IgG2 Wild Type | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVTSSNFGT QTYTCNVDHKPSNTKVDKTVERKCCVECPPCPAPPVAGPSVFLFPPKPK DTLMISRTPEVTCVVVDVSHEDPEVQFNWYVDGMEVHNAKTKPREEQFN STFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTP PMLDSDGSFFLYSKLTVDKSRWQQGNVFSCSVMHEALHNHYTQKSLSLS PGK |
| 168 | VH-CH1-CH2-CH3 IgG1 (ΔE216-K222 IN HINGE) (C226S, C229S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVTHTSPPSPAPELLGGSSVFLFPPKPKDTL MISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| 169 | VH-CH1-CH2-CH3 IgG1 (C220S, C226S, C229S, P238S, N297Q) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSSDKTHTSPPSPAPELLGGSSVFLFP PKPKDTLMISRTPEVTCVVVDVSHEDPEVKFNWYVDGVEVHNAKTKPRE EQYQSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 170 | VH-CH1-CH2-CH3 IgG1 | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVTHTSPPSPAPELLGGSSVFLFPPKPKDTL MISRTPEVTCVVVDVSHEDPEVKFNWYVDGVEVHNAKTKPREEQYQSTY RVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| | (ΔE216-K222 IN HINGE) (C226S, C229S, P238S, N297Q) | |
| 171 | VH-CH1-CH2-CH3 IgG1 | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSSDKTHTSPPSPAPELLGGSSVFLFP PKPKDTLMISRTPEVTCVVVDVSHEDPEVKFNWYVDGVEVHNAKTKPRE EQYGSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| | (C220S, C226S, C229S, P238S, N297G) | |
| 172 | VH-CH1-CH2-CH3 IgG1 | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVTHTSPPSPAPELLGGSSVFLFPPKPKDTL MISRTPEVTCVVVDVSHEDPEVKFNWYVDGVEVHNAKTKPREEQYGSTY RVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVL DSDGSFFLYSKLTVDKSRWQQGNVFSCSVMHEALHNHYTQKSLSLSPGK |
| | (ΔE216-K222 IN HINGE) (C226S, C229S, P238S, N297G) | |
| 173 | TNX10 VH-CH1-CH2-CH3 IgG1 | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPCPAPEAAGGPSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| | (L234A, L235A) | |
| 174 | TNX11 VH-CH1-CH2-CH3 IgG1 (C226S, C229S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTSPPSPAPELLGGSSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 284 | TNX08 VH-CH1-CH2-CH3 IgG4 (S228P, L235E) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEFEGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLGK |
| 285 | TNX09 VH-CH1-CH2-CH3 IgG4 (S228P, F234A, L235A) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEAAGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLGK |
| 286 | TNX12 IgG1 VH-CH1-CH2-CH3 IgG1 (C229S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTCPPSPAPELLGGSSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 287 | TNX13 IgG1 VH-CH1-CH2-CH3 IgG1 (C226S, P238S) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPSSKSTSGGTAALGCLV KDYFPEPVTVSWNSGALTSGVHTFPAVLQSSGLYSLSSVVTVPSSSLGT QTYICNVNHKPSNTKVDKKVEPKSCDKTHTSPPCPAPELLGGSSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 288 | TNX14 VH-CH1-CH2-CH3 IgG4 (L115T, S228P, L235A) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTTVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEFAGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLGK |

| Heavy chain DNA sequences corresponding WITH LEADER, no terminal K | | |
|---|---|---|
| 175 | TNX01 VH-CH1-CH2-CH3 IgG1(N297Q) (no terminal K) | ATGCCACTGCTGCTGCTGCTGCCACTGCTGTGGGCTGGCGCTCTGGCTC AGGTGCAGCTGGTGCAGTCTGGAGCTGAGGTGGTGAAGCCAGGCGCCTC TGTGAAGCTGTCCTGCAAGGCTAGCGGCTACATCTTCACCTCCTACTAT ATGTATTGGGTGAAGCAGGCTCCTGGACAGGGACTGGAGTGGATCGGCG AGATCAACCCATCTAATGGCGACACCAACTTCAATGAGAAGTTTAAGTC CAAGGCTACCCTGACAGTGGATAAGTCCGCCTCTACAGCCTACATGGAG CTGTCCAGCCTGAGAAGCGAGGACACCGCCGTGTACTATTGCACAAGGT CTGATGGCCGGAACGACATGGATTCCTGGGGCCAGGGCACCCTGGTGAC AGTGTCTTCCGCTTCTACCAAGGGCCCTTCCGTGTTTCCACTGGCTCCA AGCTCTAAGTCCACCAGCGGAGGAACAGCCGCTCTGGGCTGTCTGGTGA AGGACTATTTCCCTGAGCCAGTGACAGTGAGCTGGAACTCTGGCGCTCT GACCTCCGGCGTGCACACATTTCCAGCCGTGCTGCAGTCCAGCGGCCTG TACAGCCTGTCTTCCGTGGTGACCGTGCCAAGCTCTTCCCTGGGCACCC AGACATATATCTGCAACGTGAATCATAAGCCCTCTAATACAAAGGTGGA CAAGAAGGTGGAGCCCAAGTCTTGTGATAAAACACATACTTGCCCCCCT TGTCCTGCACCAGAACTGCTGGGAGGACCTAGCGTGTTCCTGTTTCCAC CCAAGCCAAAAGACACCCTGATGATTAGTAGAACCCCTGAGGTCACATG CGTGGTCGTGGACGTGAGCCACGAGGACCCCGAGGTGAAGTTCAACTGG TACGTGGACGGCGTGGAGGTGCACAATGCTAAGACCAAGCCCAGAGAGG AGCAGTACCAGAGCACCTATCGCGTGGTGTCTGTGCTGACAGTGCTGCA TCAGGATTGGCTGAACGGCAAGGAGTATAAGTGCAAGGTGAGCAATAAG GCTCTGCCCGCCCCTATCGAGAAGACCATCTCTAAGGCTAAGGGCCAGC CTAGGGAGCCACAGGTGTACACACTGCCCCCTTCCCGGGACGAGCTGAC CAAGAACCAGGTGAGCCTGACATGTCTGGTGAAGGGCTTCTATCCCAGC GATATCGCCGTGGAGTGGGAGTCTAATGGCCAGCCTGAGAACAATTACA AGACCACACCACCCGTGCTGGACTCCGATGGCAGCTTCTTTCTGTATTC CAAGCTGACCGTGGATAAGAGCCGCTGGCAGCAGGGCAACGTGTTTTCT TGTTCCGTGATGCACGAGGCCCTGCACAATCATTACACACAGAAGAGCC TGTCTCTGTCCCCTGGC |
| 176 | TNX02 VH-CH1-CH2-CH3 IgG1 (wt) (no terminal K) | ATGCCCCTGCTGCTGCTGCTGCCTCTGCTGTGGGCTGGCGCTCTGGCTC AGGTGCAGCTGGTGCAGTCCGGAGCTGAGGTGGTGAAGCCAGGAGCCTC TGTGAAGCTGTCCTGCAAGGCTAGCGGCTACATCTTCACCAGCTACTAT ATGTATTGGGTGAAGCAGGCTCCAGGACAGGGCCTGGAGTGGATCGGCG AGATCAACCCTTCTAATGGCGACACCAACTTCAATGAGAAGTTTAAGTC CAAGGCTACCCTGACAGTGGATAAGTCCGCCTCTACAGCCTACATGGAG CTGTCCAGCCTGAGGTCTGAGGACACCGCCGTGTACTATTGCACAAGGT CTGATGGCCGGAACGACATGGATTCCTGGGGCCAGGGCACCCTGGTGAC AGTGTCTTCCGCTTCTACCAAGGGACCATCCGTGTTTCCACTGGCTCCA AGCTCTAAGTCCACCAGCGGAGGAACAGCCGCTCTGGGCTGTCTGGTGA AGGACTATTTCCCAGAGCCCGTGACAGTGAGCTGGAACTCTGGCGCTCT GACCAGCGGCGTGCACACATTTCCAGCCGTGCTGCAGTCCAGCGGCCTG TACTCTCTGTCTTCCGTGGTGACCGTGCCTAGCTCTTCCCTGGGCACCC AGACATATATCTGCAACGTGAATCACAAGCCTAGCAATACAAAGGTGGA CAAGAAGGTGGAGCCAAAGTCTTGTGATAAGACCCATACATGCCCCCCT TGTCCTGCTCCAGAGCTGCTGGGAGGACCATCCGTGTTCCTGTTTCCAC CCAAGCCCAAGGACACCCTGATGATCTCCAGAACCCCTGAGGTGACATG CGTGGTGGTGGACGTGAGCCACGAGGACCCCGAGGTGAAGTTCAACTGG TACGTGGATGGCGTGGAGGTGCATAATGCCAAGACCAAGCCTAGAGAGG AGCAGTACAATAGCACCTATCGCGTGGTGTCTGTGCTGACAGTGCTGCA CCAGGACTGGCTGAACGGCAAGGAGTATAAGTGCAAGGTGTCTAATAAG GCTCTGCCCGCCCCTATCGAGAAGACCATCTCCAAGGCTAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCAAGC GATATCGCCGTGGAGTGGGAGTCTAATGGCCAGCCCGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTTTCT TGTTCCGTGATGCATGAGGCTCTGCACAATCATTACACACAGAAGAGCC TGTCTCTGTCCCCAGGC |
| 177 | TNX03 VH-CH1-CH2-CH3 IgG1 (N297G) (no terminal K) | ATGCCCCTGCTGCTGCTGCTGCCTCTGCTGTGGGCTGGCGCTCTGGCTC AGGTGCAGCTGGTGCAGTCCGGAGCTGAGGTGGTGAAGCCAGGAGCCTC TGTGAAGCTGTCCTGCAAGGCTAGCGGCTACATCTTCACCAGCTACTAT ATGTATTGGGTGAAGCAGGCTCCAGGACAGGGCCTGGAGTGGATCGGCG AGATCAACCCTTCTAATGGCGACACCAACTTCAATGAGAAGTTTAAGTC CAAGGCTACCCTGACAGTGGATAAGTCCGCCTCTACAGCCTACATGGAG CTGTCCAGCCTGAGGTCTGAGGACACCGCCGTGTACTATTGCACAAGGT CTGATGGCCGGAACGACATGGATTCCTGGGGCCAGGGCACCCTGGTGAC AGTGTCTTCCGCTTCTACCAAGGGACCATCCGTGTTTCCACTGGCTCCA AGCTCTAAGTCCACCAGCGGAGGAACAGCCGCTCTGGGCTGTCTGGTGA AGGACTATTTCCCAGAGCCCGTGACAGTGAGCTGGAACTCTGGCGCTCT GACCAGCGGCGTGCACACATTTCCAGCCGTGCTGCAGTCCAGCGGCCTG TACTCTCTGTCTTCCGTGGTGACCGTGCCTAGCTCTTCCCTGGGCACCC AGACATATATCTGCAACGTGAATCACAAGCCTAGCAATACAAAGGTGGA CAAGAAGGTGGAGCCAAAGTCTTGTGATAAGACCCATACATGCCCCCCT TGTCCTGCTCCAGAGCTGCTGGGAGGACCATCCGTGTTCCTGTTTCCAC CCAAGCCCAAGGACACCCTGATGATCTCCAGAACCCCTGAGGTGACATG CGTGGTGGTGGACGTGAGCCACGAGGACCCCGAGGTGAAGTTCAACTGG TACGTGGATGGCGTGGAGGTGCATAATGCCAAGACCAAGCCTAGAGAGG AGCAGTACGGCAGCACCTATCGCGTGGTGTCTGTGCTGACAGTGCTGCA CCAGGACTGGCTGAACGGCAAGGAGTATAAGTGCAAGGTGTCTAATAAG GCTCTGCCCGCCCCTATCGAGAAGACCATCTCCAAGGCTAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCAAGC GATATCGCCGTGGAGTGGGAGTCTAATGGCCAGCCCGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTTTCT TGTTCCGTGATGCATGAGGCTCTGCACAATCATTACACACAGAAGAGCC TGTCTCTGTCCCCAGGC |
| 178 | TNX04 VH-CH1-CH2-CH3 IgG1 (C220S, C226S, C229S, P238S) (no terminal K) | ATGCCCCTGCTGCTGCTGCTGCCTCTGCTGTGGGCTGGCGCTCTGGCTC AGGTGCAGCTGGTGCAGAGCGGAGCTGAGGTGGTGAAGCCAGGAGCCTC TGTGAAGCTGTCCTGCAAGGCTAGCGGCTACATCTTCACCTCTTACTAT ATGTATTGGGTGAAGCAGGCTCCAGGACAGGGCCTGGAGTGGATCGGCG AGATCAACCCTTCTAATGGCGACACCAACTTCAATGAGAAGTTTAAGTC CAAGGCTACCCTGACAGTGGATAAGTCTGCTTCCACAGCCTACATGGAG CTGTCCAGCCTGAGGTCCGAGGACACCGCCGTGTACTATTGCACAAGGT CTGATGGCCGGAACGACATGGATTCCTGGGGCCAGGGCACCCTGGTGAC AGTGTCTTCCGCTTCTACCAAGGGACCATCCGTGTTTCCACTGGCTCCA AGCTCTAAGAGCACCTCTGGAGGAACAGCCGCTCTGGGATGTCTGGTGA AGGACTATTTCCCAGAGCCCGTGACAGTGTCTTGGAACTCCGGCGCTCT GACCTCTGGCGTGCACACATTTCCTGCCGTGCTGCAGTCCAGCGGCCTG TACTCCCTGTCTTCCGTGGTGACCGTGCCAAGCTCTTCCCTGGGCACCC AGACATATATCTGCAACGTGAATCACAAGCCTTCCAATACAAAGGTGGA CAAGAAGGTGGAGCCAAAGAGCTCTGATAAGACCCATACAAGCCCCCCT TCTCCTGCTCCAGAGCTGCTGGGAGGCTCCAGCGTGTTCCTGTTTCCAC CCAAGCCAAAGGACACCCTGATGATCAGCAGAACCCCCGAGGTGACATG CGTGGTGGTGGACGTGTCTCACGAGGACCCCGAGGTGAAGTTCAACTGG TACGTGGATGGCGTGGAGGTGCATAATGCCAAGACCAAGCCTAGAGAGG AGCAGTACAATAGCACCTATCGCGTGGTGTCTGTGCTGACAGTGCTGCA CCAGGACTGGCTGAACGGCAAGGAGTATAAGTGCAAGGTGTCCAATAAG GCTCTGCCCGCCCCTATCGAGAAGACCATCAGCAAGGCTAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCAAGC GATATCGCCGTGGAGTGGGAGTCTAATGGCCAGCCCGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTTTCC TGTAGCGTGATGCATGAGGCTCTGCACAATCATTACACACAGAAGTCTC TGTCCCTGAGCCCTGGC |
| 179 | TNX05 VH-CH1-CH2-CH3 IgG4 (S228P, L235A) (no terminal K) | ATGCCCCTGCTGCTGCTGCTGCCTCTGCTGTGGGCTGGCGCTCTGGCTC AGGTGCAGCTGGTGCAGTCTGGAGCTGAGGTGGTGAAGCCTGGAGCTAG CGTGAAGCTGTCTTGCAAGGCTTCCGGCTACATCTTCACCTCCTACTAT ATGTATTGGGTGAAGCAGGCTCCTGGACAGGGCCTGGAGTGGATCGGCG AGATCAACCCATCCAATGGCGACACAAACTTCAATGAGAAGTTTAAGAG CAAGGCTACCCTGACAGTGGATAAGTCCGCCTCTACCGCCTACATGGAG CTGTCCAGCCTGAGGAGCGAGGACACAGCCGTGTACTATTGTACCAGGA GCGATGGCCGGAACGACATGGATTCTTGGGGCCAGGGCACACTGGTGAC CGTGTCTTCCGCTAGCACAAAGGGACCATCCGTGTTCCCACTGGCTCCA TGCTCCAGGAGCACATCTGAGTCCACCGCCGCTCTGGGCTGTCTGGTGA AGGACTATTTCCCTGAGCCAGTGACCGTGTCCTGGAATAGCGGCGCTCT GACATCCGGAGTGCACACCTTTCCAGCCGTGCTGCAGAGCTCTGGCCTG TACAGCCTGTCCAGCGTGGTGACAGTGCCTTCTTCCAGCCTGGGCACCA AGACATATACCTGCAACGTGGACCATAAGCCATCTAATACCAAGGTGGA TAAGAGAGTGGAGTCCAAGTACGGACCACCTTGCCCACCATGTCCAGCT CCTGAGTTCGCTGGAGGACCATCCGTGTTCCTGTTTCCTCCAAAGCCCA AGGACACCCTGATGATCTCTCGCACACCAGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCCAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCT TCTTCCATCGAGAAGACAATCTCTAAGGCTAAGGGCCAGCCTCGGGAGC CACAGGTGTACACCCTGCCCCCTAGCCAGGAGGAGATGACAAAGAACCA GGTGTCTCTGACCTGTCTGGTGAAGGGCTTCTATCCCTCCGACATCGCC GTGGAGTGGGAGAGCAATGGCCAGCCTGAGAACAATTACAAGACCACAC CACCCGTGCTGGACTCTGATGGCTCCTTCTTTCTGTATTCTAGACTGAC CGTCGATAAGTCCCGCTGGCAGGAGGGCAACGTGTTTAGCTGCTCTGTG ATGCACGAGGCCCTGCACAATCATTACACCCAGAAGTCCCTGAGCCTGT CTCTGGGC |
| 289 | TNX06 VH-CH1-CH2-CH3 IgG4 (wild type) (no terminal K) | ATGCCTCTGCTGCTGCTGCTGCCTCTGCTGTGGGCCGGTGCTCTGGCTC AGGTCCAGCTGGTCCAGTCAGGGGCTGAAGTGGTCAAACCCGGCGCCAG CGTGAAGCTGTCTTGCAAGGCTTCCGGCTACATCTTCACCAGCTACTAT ATGTATTGGGTGAAGCAGGCTCCAGGACAGGGCCTGGAGTGGATCGGCG AGATCAACCCTTCCAATGGCGACACAAACTTCAATGAGAAGTTTAAGAG CAAGGCCACCCTGACAGTGGATAAGAGCGCCTCTACCGCTTACATGGAG CTGTCCAGCCTGAGGTCTGAGGACACAGCCGTGTACTATTGTACCAGGA GCGATGGCCGGAACGACATGGATTCTTGGGGCCAGGGCACACTGGTGAC CGTGTCTTCCGCCAGCACAAAGGGCCCTTCCGTGTTCCCCCTGGCTCCT TGCTCCAGGAGCACATCTGAGTCCACCGCCGCTCTGGGCTGTCTGGTGA AGGACTACTTCCCAGAGCCCGTGACCGTGTCCTGGAATAGCGGCGCCCT GACATCCGGAGTGCACACCTTTCCAGCTGTGCTGCAGAGCTCTGGCCTG TACAGCCTGTCCAGCGTGGTGACAGTGCCCTCTTCCAGCCTGGGCACCA AGACATATACCTGCAACGTGGACCATAAGCCTTCTAATACCAAGGTGGA TAAGAGAGTGGAGTCCAAGTACGGACCACCTTGCCCTAGCTGTCCTGCT CCAGAGTTCCTGGGAGGACCTTCCGTGTTCCTGTTTCCACCCAAGCCAA AGGACACACTGATGATCTCTCGCACACCTGAGGTGACCTGCGTGGTGGT GGACGTGTCCCAGGAgGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGC |
| 290 | TNX07 VH-CH1-CH2-CH3 IgG4 (S228P) (no terminal K) | ATGCCTCTGCTGCTGCTGCTGCCTCTGCTGTGGGCCGGTGCTCTGGCTC AGGTCCAGCTGGTCCAGTCAGGGGCTGAAGTGGTCAAACCCGGCGCCAG CGTGAAGCTGTCTTGCAAGGCTTCCGGCTACATCTTCACCAGCTACTAT ATGTATTGGGTGAAGCAGGCTCCAGGACAGGGCCTGGAGTGGATCGGCG AGATCAACCCTTCCAATGGCGACACAAACTTCAATGAGAAGTTTAAGAG CAAGGCCACCCTGACAGTGGATAAGAGCGCCTCTACCGCTTACATGGAG CTGTCCAGCCTGAGGTCTGAGGACACAGCCGTGTACTATTGTACCAGGA GCGATGGCCGGAACGACATGGATTCTTGGGGCCAGGGCACACTGGTGAC CGTGTCTTCCGCCAGCACAAAGGGCCCTTCCGTGTTCCCCCTGGCTCCT TGCTCCAGGAGCACATCTGAGTCCACCGCCGCTCTGGGCTGTCTGGTGA AGGACTACTTCCCAGAGCCCGTGACCGTGTCCTGGAATAGCGGCGCCCT GACATCCGGAGTGCACACCTTTCCAGCTGTGCTGCAGAGCTCTGGCCTG TACAGCCTGTCCAGCGTGGTGACAGTGCCCTCTTCCAGCCTGGGCACCA AGACATATACCTGCAACGTGGACCATAAGCCTTCTAATACCAAGGTGGA TAAGAGAGTGGAGTCCAAGTACGGACCACCTTGCCCTCCCTGTCCTGCT CCAGAGTTCCTGGGAGGACCTTCCGTGTTCCTGTTTCCACCCAAGCCAA AGGACACACTGATGATCTCTCGCACACCTGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCAAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCC TCTTCCATCGAGAAGACAATCTCTAAGGCTAAGGGACAGCCAAGGGAGC CACAGGTGTACACCCTGCCTCCAAGCCAGGAGGAGATGACAAAGAACCA GGTGTCTCTGACCTGTCTGGTGAAGGGCTTCTATCCATCTGACATCGCT GTGGAGTGGGAGTCCAATGGCCAGCCCGAGAACAATTACAAGACCACAC CCCCTGTGCTGGACTCTGATGGCTCCTTCTTTCTGTATTCTAGACTGAC CGTGGATAAGTCCCGCTGGCAGGAGGGCAACGTGTTCTCCTGCTCTGTG ATGCACGAAGCACTGCACAATCATTACACTCAGAAGAGCCTGTCACTGT CCCTGGGC |
| 291 | TNX08 VH-CH1-CH2-CH3 (IgG4 S228P/L235E) (no terminal K) | ATGCCTCTGCTGCTGCTGCTGCCTCTGCTGTGGGCCGGTGCTCTGGCTC AGGTCCAGCTGGTCCAGTCAGGGGCTGAAGTGGTCAAACCCGGCGCCAG CGTGAAGCTGTCTTGCAAGGCTTCCGGCTACATCTTCACCAGCTACTAT ATGTATTGGGTGAAGCAGGCTCCAGGACAGGGCCTGGAGTGGATCGGCG AGATCAACCCTTCCAATGGCGACACAAACTTCAATGAGAAGTTTAAGAG CAAGGCCACCCTGACAGTGGATAAGAGCGCCTCTACCGCTTACATGGAG CTGTCCAGCCTGAGGTCTGAGGACACAGCCGTGTACTATTGTACCAGGA GCGATGGCCGGAACGACATGGATTCTTGGGGCCAGGGCACACTGGTGAC CGTGTCTTCCGCCAGCACAAAGGGCCCTTCCGTGTTCCCCCTGGCTCCT TGCTCCAGGAGCACATCTGAGTCCACCGCCGCTCTGGGCTGTCTGGTGA AGGACTACTTCCCAGAGCCCGTGACCGTGTCCTGGAATAGCGGCGCCCT GACATCCGGAGTGCACACCTTTCCAGCTGTGCTGCAGAGCTCTGGCCTG TACAGCCTGTCCAGCGTGGTGACAGTGCCCTCTTCCAGCCTGGGCACCA AGACATATACCTGCAACGTGGACCATAAGCCTTCTAATACCAAGGTGGA TAAGAGAGTGGAGTCCAAGTACGGACCACCTTGCCCTCCCTGTCCTGCT CCAGAGTTCGAGGGAGGACCTTCCGTGTTCCTGTTTCCACCCAAGCCAA AGGACACACTGATGATCTCTCGCACACCTGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCAAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCC TCTTCCATCGAGAAGACAATCTCTAAGGCTAAGGGACAGCCAAGGGAGC CACAGGTGTACACCCTGCCTCCAAGCCAGGAGGAGATGACAAAGAACCA GGTGTCTCTGACCTGTCTGGTGAAGGGCTTCTATCCATCTGACATCGCT GTGGAGTGGGAGTCCAATGGCCAGCCCGAGAACAATTACAAGACCACAC CCCCTGTGCTGGACTCTGATGGCTCCTTCTTTCTGTATTCTAGACTGAC CGTGGATAAGTCCCGCTGGCAGGAGGGCAACGTGTTCTCCTGCTCTGTG ATGCACGAAGCACTGCACAATCATTACACTCAGAAGAGCCTGTCACTGT CCCTGGGC |
| 292 | TNX09 VH-CH1-CH2-CH3 IgG4 (S228P/F234A/ L235A) (no terminal K) | ATGCCTCTGCTGCTGCTGCTGCCTCTGCTGTGGGCCGGTGCTCTGGCTC AGGTCCAGCTGGTCCAGTCAGGGGCTGAAGTGGTCAAACCCGGCGCCAG CGTGAAGCTGTCTTGCAAGGCTTCCGGCTACATCTTCACCAGCTACTAT ATGTATTGGGTGAAGCAGGCTCCAGGACAGGGCCTGGAGTGGATCGGCG AGATCAACCCTTCCAATGGCGACACAAACTTCAATGAGAAGTTTAAGAG CAAGGCCACCCTGACAGTGGATAAGAGCGCCTCTACCGCTTACATGGAG CTGTCCAGCCTGAGGTCTGAGGACACAGCCGTGTACTATTGTACCAGGA GCGATGGCCGGAACGACATGGATTCTTGGGGCCAGGGCACACTGGTGAC CGTGTCTTCCGCCAGCACAAAGGGCCCTTCCGTGTTCCCCCTGGCTCCT TGCTCCAGGAGCACATCTGAGTCCACCGCCGCTCTGGGCTGTCTGGTGA AGGACTACTTCCCAGAGCCCGTGACCGTGTCCTGGAATAGCGGCGCCCT GACATCCGGAGTGCACACCTTTCCAGCTGTGCTGCAGAGCTCTGGCCTG TACAGCCTGTCCAGCGTGGTGACAGTGCCCTCTTCCAGCCTGGGCACCA AGACATATACCTGCAACGTGGACCATAAGCCTTCTAATACCAAGGTGGA TAAGAGAGTGGAGTCCAAGTACGGACCACCTTGCCCTCCCTGTCCTGCT CCAGAGGCCGCCGGAGGACCTTCCGTGTTCCTGTTTCCACCCAAGCCAA AGGACACACTGATGATCTCTCGCACACCTGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCAAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCC TCTTCCATCGAGAAGACAATCTCTAAGGCTAAGGGACAGCCAAGGGAGC CACAGGTGTACACCCTGCCTCCAAGCCAGGAGGAGATGACAAAGAACCA GGTGTCTCTGACCTGTCTGGTGAAGGGCTTCTATCCATCTGACATCGCT GTGGAGTGGGAGTCCAATGGCCAGCCCGAGAACAATTACAAGACCACAC CCCCTGTGCTGGACTCTGATGGCTCCTTCTTTCTGTATTCTAGACTGAC CGTGGATAAGTCCCGCTGGCAGGAGGGCAACGTGTTCTCCTGCTCTGTG ATGCACGAAGCACTGCACAATCATTACACTCAGAAGAGCCTGTCACTGT CCCTGGGC |
| 293 | TNX10 VH-CH1-CH2-CH3 IgG1 (L234A/L235A) (no terminal K) | ATGCCTCTGCTGCTGCTGCTGCCTCTGCTGTGGGCCGGTGCCCTGGCTC AGGTCCAGCTGGTCCAGTCAGGTGCCGAAGTGGTCAAGCCCGGCGCCTC TGTGAAGCTGTCCTGCAAGGCTAGCGGCTACATCTTCACCTCCTACTAT ATGTATTGGGTGAAGCAGGCTCCTGGACAGGGCCTGGAGTGGATCGGCG AGATCAACCCATCTAATGGCGACACCAACTTCAATGAGAAGTTTAAGTC CAAGGCCACCCTGACAGTGGATAAGAGCGCCTCTACAGCTTACATGGAG CTGTCCAGCCTGAGGAGCGAGGACACCGCCGTGTACTATTGCACAAGGT CTGATGGCCGGAACGACATGGATTCCTGGGGCCAGGGCACCCTGGTGAC AGTGTCTTCCGCCTCTACCAAGGGCCCTTCCGTGTTTCCACTGGCTCCC AGCTCTAAGTCCACCAGCGGAGGAACAGCCGCTCTGGGCTGTCTGGTGA AGGACTACTTCCCAGAGCCCGTGACAGTGAGCTGGAACTCTGGCGCCCT GACCAGCGGAGTGCACACATTTCCTGCTGTGCTGCAGTCCAGCGGCCTG TACTCTCTGTCTTCCGTGGTGACCGTGCCAAGCTCTTCCCTGGGCACCC AGACATATATCTGCAACGTGAATCACAAGCCAAGCAATACAAAGGTGGA CAAGAAGGTGGAGCCCAAGTCTTGTGATAAGACCCATACATGCCCCCCT TGTCCTGCTCCAGAGGCTGCTGGAGGACCATCCGTGTTCCTGTTTCCAC CCAAGCCTAAGGACACCCTGATGATCTCCAGAACCCCAGAGGTGACATG CGTGGTGGTGGACGTGAGCCACGAGGACCCCGAGGTGAAGTTCAACTGG TACGTGGATGGCGTGGAGGTGCATAATGCTAAGACCAAGCCAAGAGAGG AGCAGTACAACAGCACCTATCGCGTGGTGTCTGTGCTGACAGTGCTGCA TCAGGACTGGCTGAACGGCAAGGAGTATAAGTGCAAGGTGTCTAATAAG GCCCTGCCCGCTCCTATCGAGAAGACCATCTCCAAGGCCAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCCAGC GATATCGCTGTGGAGTGGGAGTCTAATGGCCAGCCTGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTCTCC TGTTCCGTCATGCACGAGGCACTGCACAATCATTACACCCAGAAGTCAC TGTCACTGTCACCAGGA |
| 294 | TNX11 VH-CH1-CH2-CH3 IgG1 (C226S, C229S, P238S) (no terminal K) | ATGCCTCTGCTGCTGCTGCCTCTGCTGTGGGCCGGTGCCCTGGCTC AGGTCCAGCTGGTCCAGTCAGGTGCCGAAGTGGTCAAGCCCGGCGCCTC TGTGAAGCTGTCCTGCAAGGCTAGCGGCTACATCTTCACCTCCTACTAT ATGTATTGGGTGAAGCAGGCTCCTGGACAGGGCCTGGAGTGGATCGGCG AGATCAACCCATCTAATGGCGACACCAACTTCAATGAGAAGTTTAAGTC CAAGGCCACCCTGACAGTGGATAAGAGCGCCTCTACAGCTTACATGGAG CTGTCCAGCCTGAGGAGCGAGGACACCGCCGTGTACTATTGCACAAGGT CTGATGGCCGGAACGACATGGATTCCTGGGGCCAGGGCACCCTGGTGAC AGTGTCTTCCGCCTCTACCAAGGGCCCTTCCGTGTTTCCACTGGCTCCC AGCTCTAAGTCCACCAGCGGAGGAACAGCCGCTCTGGGCTGTCTGGTGA AGGACTACTTCCCAGAGCCCGTGACAGTGAGCTGGAACTCTGGCGCCCT GACCAGCGGAGTGCACACATTTCCTGCTGTGCTGCAGTCCAGCGGCCTG TACTCTCTGTCTTCCGTGGTGACCGTGCCAAGCTCTTCCCTGGGCACCC AGACATATATCTGCAACGTGAATCACAAGCCAAGCAATACAAAGGTGGA CAAGAAGGTGGAGCCCAAGTCTTGTGATAAGACCCATACAtccCCCCCT TccCCTGCTCCAGAGCTGCTGGGAGGAAGCTCCGTGTTCCTGTTTCCAC CCAAGCCTAAGGACACCCTGATGATCTCCAGAACCCCAGAGGTGACATG CGTGGTGGTGGACGTGAGCCACGAGGACCCCGAGGTGAAGTTCAACTGG TACGTGGATGGCGTGGAGGTGCATAATGCTAAGACCAAGCCAAGAGAGG AGCAGTACAACAGCACCTATCGCGTGGTGTCTGTGCTGACAGTGCTGCA TCAGGACTGGCTGAACGGCAAGGAGTATAAGTGCAAGGTGTCTAATAAG GCCCTGCCCGCTCCTATCGAGAAGACCATCTCCAAGGCCAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCCAGC GATATCGCTGTGGAGTGGGAGTCTAATGGCCAGCCTGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTCTCC TGTTCCGTCATGCACGAGGCACTGCACAATCATTACACCCAGAAGTCAC TGTCACTGTCACCAGGA |
| 295 | TNX12 VH-CH1-CH2-CH3 IgG1 (C229S, P238S) (no terminal K) | ATGCCTCTGCTGCTGCTGCTGCCTCTGCTGTGGGCCGGTGCCCTGGCTC AGGTCCAGCTGGTCCAGTCAGGTGCCGAAGTGGTCAAGCCCGGCGCCTC TGTGAAGCTGTCCTGCAAGGCTAGCGGCTACATCTTCACCTCCTACTAT ATGTATTGGGTGAAGCAGGCTCCTGGACAGGGCCTGGAGTGGATCGGCG AGATCAACCCATCTAATGGCGACACCAACTTCAATGAGAAGTTTAAGTC CAAGGCCACCCTGACAGTGGATAAGAGCGCCTCTACAGCTTACATGGAG CTGTCCAGCCTGAGGAGCGAGGACACCGCCGTGTACTATTGCACAAGGT CTGATGGCCGGAACGACATGGATTCCTGGGGCCAGGGCACCCTGGTGAC AGTGTCTTCCGCCTCTACCAAGGGCCCTTCCGTGTTTCCACTGGCTCCC AGCTCTAAGTCCACCAGCGGAGGAACAGCCGCTCTGGGCTGTCTGGTGA AGGACTACTTCCCAGAGCCCGTGACAGTGAGCTGGAACTCTGGCGCCCT GACCAGCGGAGTGCACACATTTCCTGCTGTGCTGCAGTCCAGCGGCCTG TACTCTCTGTCTTCCGTGGTGACCGTGCCAAGCTCTTCCCTGGGCACCC AGACATATATCTGCAACGTGAATCACAAGCCAAGCAATACAAAGGTGGA CAAGAAGGTGGAGCCCAAGTCTTGTGATAAGACCCATACATGCCCCCCT TCCCCTGCTCCAGAGCTGCTGGGAGGAAGcTCCGTGTTCCTGTTTCCAC CCAAGCCTAAGGACACCCTGATGATCTCCAGAACCCCAGAGGTGACATG CGTGGTGGTGGACGTGAGCCACGAGGACCCCGAGGTGAAGTTCAACTGG TACGTGGATGGCGTGGAGGTGCATAATGCTAAGACCAAGCCAAGAGAGG AGCAGTACAACAGCACCTATCGCGTGGTGTCTGTGCTGACAGTGCTGCA TCAGGACTGGCTGAACGGCAAGGAGTATAAGTGCAAGGTGTCTAATAAG GCCCTGCCCGCTCCTATCGAGAAGACCATCTCCAAGGCCAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCCAGC GATATCGCTGTGGAGTGGGAGTCTAATGGCCAGCCTGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTCTCC TGTTCCGTCATGCACGAGGCACTGCACAATCATTACACCCAGAAGTCAC TGTCACTGTCACCAGGA |
| 296 | TNX13 VH-CH1-CH2-CH3 IgG1 (C226S, P238S) (no terminal K) | ATGCCTCTGCTGCTGCTGCTGCCTCTGCTGTGGGCCGGTGCCCTGGCTC AGGTCCAGCTGGTCCAGTCAGGTGCCGAAGTGGTCAAGCCCGGCGCCTC TGTGAAGCTGTCCTGCAAGGCTAGCGGCTACATCTTCACCTCCTACTAT ATGTATTGGGTGAAGCAGGCTCCTGGACAGGGCCTGGAGTGGATCGGCG AGATCAACCCATCTAATGGCGACACCAACTTCAATGAGAAGTTTAAGTC CAAGGCCACCCTGACAGTGGATAAGAGCGCCTCTACAGCTTACATGGAG CTGTCCAGCCTGAGGAGCGAGGACACCGCCGTGTACTATTGCACAAGGT CTGATGGCCGGAACGACATGGATTCCTGGGGCCAGGGCACCCTGGTGAC AGTGTCTTCCGCCTCTACCAAGGGCCCTTCCGTGTTTCCACTGGCTCCC AGCTCTAAGTCCACCAGCGGAGGAACAGCCGCTCTGGGCTGTCTGGTGA AGGACTACTTCCCAGAGCCCGTGACAGTGAGCTGGAACTCTGGCGCCCT GACCAGCGGAGTGCACACATTTCCTGCTGTGCTGCAGTCCAGCGGCCTG TACTCTCTGTCTTCCGTGGTGACCGTGCCAAGCTCTTCCCTGGGCACCC AGACATATATCTGCAACGTGAATCACAAGCCAAGCAATACAAAGGTGGA CAAGAAGGTGGAGCCCAAGTCTTGTGATAAGACCCATACATCCCCCCCT TGCCCTGCTCCAGAGCTGCTGGGAGGAAGCTCCGTGTTCCTGTTTCCAC CCAAGCCTAAGGACACCCTGATGATCTCCAGAACCCCAGAGGTGACATG CGTGGTGGTGGACGTGAGCCACGAGGACCCCGAGGTGAAGTTCAACTGG TACGTGGATGGCGTGGAGGTGCATAATGCTAAGACCAAGCCAAGAGAGG AGCAGTACAACAGCACCTATCGCGTGGTGTCTGTGCTGACAGTGCTGCA TCAGGACTGGCTGAACGGCAAGGAGTATAAGTGCAAGGTGTCTAATAAG GCCCTGCCCGCTCCTATCGAGAAGACCATCTCCAAGGCCAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCCAGC GATATCGCTGTGGAGTGGGAGTCTAATGGCCAGCCTGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTCTCC TGTTCCGTCATGCACGAGGCACTGCACAATCATTACACCCAGAAGTCAC TGTCACTGTCACCAGGA |
| 297 | TNX14 VH-CH1-CH2-CH3 IgG4 (L115T, S228P, L235A) (no terminal K) | ATGCCTCTGCTGCTGCTGCCTCTGCTGTGGGCCGGTG CTCTGGCTCAGGTCCAGCTGGTCCAGTCAGGGGCTGAAGT GGTCAAACCCGGCGCCAGCGTGAAGCTGTCTTGCAAGGCT TCCGGCTACATCTTCACCAGCTACTATATGTATTGGGTGA AGCAGGCTCCAGGACAGGGCCTGGAGTGGATCGGCGAGAT CAACCCTTCCAATGGCGACACAAACTTCAATGAGAAGTTT AAGAGCAAGGCCACCCTGACAGTGGATAAGAGCGCCTCTA CCGCTTACATGGAGCTGTCCAGCCTGAGGTCTGAGGACAC AGCCGTGTACTATTGTACCAGGAGCGATGGCCGGAACGAC ATGGATTCTTGGGGCCAGGGCACAACAGTGACCGTGTCTT CCGCCAGCACAAAGGGCCCTTCCGTGTTCCCCCTGGCTCC TTGCTCCAGGAGCACATCTGAGTCCACCGCCGCTCTGGGC TGTCTGGTGAAGGACTACTTCCCAGAGCCCGTGACCGTGT CCTGGAATAGCGGCGCCCTGACATCCGGAGTGCACACCTT TCCAGCTGTGCTGCAGAGCTCTGGCCTGTACAGCCTGTCC AGCGTGGTGACAGTGCCCTCTTCCAGCCTGGGCACCAAGA CATATACCTGCAACGTGGACCATAAGCCTTCTAATACCAA GGTGGATAAGAGAGTGGAGTCCAAGTACGGACCACCTTGC CCTCCTTGTCCTGCTCCAGAGTTCGCCGGAGGACCTTCCG TGTTCCTGTTTCCACCCAAGCCAAAGGACACACTGATGAT CTCTCGCACACCTGAGGTGACCTGCGTGGTGGTGGACGTG TCCCAGGAGGACCCCGAGGTGCAGTTCAACTGGTACGTGG ATGGCGTGGAGGTGCACAATGCTAAGACCAAGCCAAGGGA GGAGCAGTTTAACTCCACATACCGGGTGGTGAGCGTGCTG ACCGTGCTGCATCAGGATTGGCTGAACGGCAAGGAGTATA AGTGCAAGGTGAGCAATAAGGGCCTGCCCTCTTCCATCGA GAAGACAATCTCTAAGGCTAAGGGACAGCCAAGGGAGCCA CAGGTGTACACCCTGCCTCCAAGCCAGGAGGAGATGACAA AGAACCAGGTGTCTCTGACCTGTCTGGTGAAGGGCTTCTA TCCATCTGACATCGCTGTGGAGTGGGAGTCCAATGGCCAG CCCGAGAACAATTACAAGACCACACCCCCTGTGCTGGACT CTGATGGCTCCTTCTTTCTGTATTCTAGACTGACCGTGGA TAAGTCCCGCTGGCAGGAGGGCAACGTGTTCTCCTGCTCT GTGATGCACGAAGCACTGCACAATCATTACACTCAGAAGA GCCTGTCACTGTCCCTGGGC |

| **Heavy chain DNA sequences WITHOUT LEADER; no terminal K** | | |
|---|---|---|
| 180 | TNX01 VH-CH1-CH2-CH3 IgG1(N297Q) (no terminal K) | CAGGTGCAGCTGGTGCAGTCTGGAGCTGAGGTGGTGAAGCCAGGCGCCT CTGTGAAGCTGTCCTGCAAGGCTAGCGGCTACATCTTCACCTCCTACTA TATGTATTGGGTGAAGCAGGCTCCTGGACAGGGACTGGAGTGGATCGGC GAGATCAACCCATCTAATGGCGACACCAACTTCAATGAGAAGTTTAAGT CCAAGGCTACCCTGACAGTGGATAAGTCCGCCTCTACAGCCTACATGGA GCTGTCCAGCCTGAGAAGCGAGGACACCGCCGTGTACTATTGCACAAGG TCTGATGGCCGGAACGACATGGATTCCTGGGGCCAGGGCACCCTGGTGA CAGTGTCTTCCGCTTCTACCAAGGGCCCTTCCGTGTTTCCACTGGCTCC AAGCTCTAAGTCCACCAGCGGAGGAACAGCCGCTCTGGGCTGTCTGGTG AAGGACTATTTCCCTGAGCCAGTGACAGTGAGCTGGAACTCTGGCGCTC TGACCTCCGGCGTGCACACATTTCCAGCCGTGCTGCAGTCCAGCGGCCT GTACAGCCTGTCTTCCGTGGTGACCGTGCCAAGCTCTTCCCTGGGCACC CAGACATATATCTGCAACGTGAATCATAAGCCCTCTAATACAAAGGTGG ACAAGAAGGTGGAGCCCAAGTCTTGTGATAAAACACATACTTGCCCCCC TTGTCCTGCACCAGAACTGCTGGGAGGACCTAGCGTGTTCCTGTTTCCA CCCAAGCCAAAAGACACCCTGATGATTAGTAGAACCCCTGAGGTCACAT GCGTGGTCGTGGACGTGAGCCACGAGGACCCCGAGGTGAAGTTCAACTG GTACGTGGACGGCGTGGAGGTGCACAATGCTAAGACCAAGCCCAGAGAG GAGCAGTACCAGAGCACCTATCGCGTGGTGTCTGTGCTGACAGTGCTGC ATCAGGATTGGCTGAACGGCAAGGAGTATAAGTGCAAGGTGAGCAATAA GGCTCTGCCCGCCCCTATCGAGAAGACCATCTCTAAGGCTAAGGGCCAG CCTAGGGAGCCACAGGTGTACACACTGCCCCCTTCCCGGGACGAGCTGA CCAAGAACCAGGTGAGCCTGACATGTCTGGTGAAGGGCTTCTATCCCAG CGATATCGCCGTGGAGTGGGAGTCTAATGGCCAGCCTGAGAACAATTAC AAGACCACACCACCCGTGCTGGACTCCGATGGCAGCTTCTTTCTGTATT CCAAGCTGACCGTGGATAAGAGCCGCTGGCAGCAGGGCAACGTGTTTTC TTGTTCCGTGATGCACGAGGCCCTGCACAATCATTACACACAGAAGAGC CTGTCTCTGTCCCCTGGC |
| 181 | TNX02 VH-CH1-CH2-CH3 IgG1 (wt) (no terminal K) | CAGGTGCAGCTGGTGCAGTCCGGAGCTGAGGTGGTGAAGCCAGGAGCCT CTGTGAAGCTGTCCTGCAAGGCTAGCGGCTACATCTTCACCAGCTACTA TATGTATTGGGTGAAGCAGGCTCCAGGACAGGGCCTGGAGTGGATCGGC GAGATCAACCCTTCTAATGGCGACACCAACTTCAATGAGAAGTTTAAGT CCAAGGCTACCCTGACAGTGGATAAGTCCGCCTCTACAGCCTACATGGA GCTGTCCAGCCTGAGGTCTGAGGACACCGCCGTGTACTATTGCACAAGG TCTGATGGCCGGAACGACATGGATTCCTGGGGCCAGGGCACCCTGGTGA CAGTGTCTTCCGCTTCTACCAAGGGACCATCCGTGTTTCCACTGGCTCC AAGCTCTAAGTCCACCAGCGGAGGAACAGCCGCTCTGGGCTGTCTGGTG AAGGACTATTTCCCAGAGCCCGTGACAGTGAGCTGGAACTCTGGCGCTC TGACCAGCGGCGTGCACACATTTCCAGCCGTGCTGCAGTCCAGCGGCCT GTACTCTCTGTCTTCCGTGGTGACCGTGCCTAGCTCTTCCCTGGGCACC CAGACATATATCTGCAACGTGAATCACAAGCCTAGCAATACAAAGGTGG ACAAGAAGGTGGAGCCAAAGTCTTGTGATAAGACCCATACATGCCCCCC TTGTCCTGCTCCAGAGCTGCTGGGAGGACCATCCGTGTTCCTGTTTCCA CCCAAGCCCAAGGACACCCTGATGATCTCCAGAACCCCTGAGGTGACAT GCGTGGTGGTGGACGTGAGCCACGAGGACCCCGAGGTGAAGTTCAACTG GTACGTGGATGGCGTGGAGGTGCATAATGCCAAGACCAAGCCTAGAGAG GAGCAGTACAATAGCACCTATCGCGTGGTGTCTGTGCTGACAGTGCTGC ACCAGGACTGGCTGAACGGCAAGGAGTATAAGTGCAAGGTGTCTAATAA GGCTCTGCCCGCCCCTATCGAGAAGACCATCTCCAAGGCTAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCAAG CGATATCGCCGTGGAGTGGGAGTCTAATGGCCAGCCCGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTTTC TTGTTCCGTGATGCATGAGGCTCTGCACAATCATTACACACAGAAGAGC CTGTCTCTGTCCCCAGGC |
| 182 | TNX03 VH-CH1-CH2-CH3 IgG1 (N297G) (no terminal K) | CAGGTGCAGCTGGTGCAGTCCGGAGCTGAGGTGGTGAAGCCAGGAGCCT CTGTGAAGCTGTCCTGCAAGGCTAGCGGCTACATCTTCACCAGCTACTA TATGTATTGGGTGAAGCAGGCTCCAGGACAGGGCCTGGAGTGGATCGGC GAGATCAACCCTTCTAATGGCGACACCAACTTCAATGAGAAGTTTAAGT CCAAGGCTACCCTGACAGTGGATAAGTCCGCCTCTACAGCCTACATGGA GCTGTCCAGCCTGAGGTCTGAGGACACCGCCGTGTACTATTGCACAAGG TCTGATGGCCGGAACGACATGGATTCCTGGGGCCAGGGCACCCTGGTGA CAGTGTCTTCCGCTTCTACCAAGGGACCATCCGTGTTTCCACTGGCTCC AAGCTCTAAGTCCACCAGCGGAGGAACAGCCGCTCTGGGCTGTCTGGTG AAGGACTATTTCCCAGAGCCCGTGACAGTGAGCTGGAACTCTGGCGCTC TGACCAGCGGCGTGCACACATTTCCAGCCGTGCTGCAGTCCAGCGGCCT GTACTCTCTGTCTTCCGTGGTGACCGTGCCTAGCTCTTCCCTGGGCACC CAGACATATATCTGCAACGTGAATCACAAGCCTAGCAATACAAAGGTGG ACAAGAAGGTGGAGCCAAAGTCTTGTGATAAGACCCATACATGCCCCCC TTGTCCTGCTCCAGAGCTGCTGGGAGGACCATCCGTGTTCCTGTTTCCA CCCAAGCCCAAGGACACCCTGATGATCTCCAGAACCCCTGAGGTGACAT GCGTGGTGGTGGACGTGAGCCACGAGGACCCCGAGGTGAAGTTCAACTG GTACGTGGATGGCGTGGAGGTGCATAATGCCAAGACCAAGCCTAGAGAG GAGCAGTACGGCAGCACCTATCGCGTGGTGTCTGTGCTGACAGTGCTGC ACCAGGACTGGCTGAACGGCAAGGAGTATAAGTGCAAGGTGTCTAATAA GGCTCTGCCCGCCCCTATCGAGAAGACCATCTCCAAGGCTAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCAAG CGATATCGCCGTGGAGTGGGAGTCTAATGGCCAGCCCGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTTTC TTGTTCCGTGATGCATGAGGCTCTGCACAATCATTACACACAGAAGAGC CTGTCTCTGTCCCCAGGC |
| 183 | TNX04 VH-CH1-CH2-CH3 IgG1 (C220S, C226S, C229S, P238S) (no terminal K) | CAGGTGCAGCTGGTGCAGAGCGGAGCTGAGGTGGTGAAGCCAGGAGCCT CTGTGAAGCTGTCCTGCAAGGCTAGCGGCTACATCTTCACCTCTTACTA TATGTATTGGGTGAAGCAGGCTCCAGGACAGGGCCTGGAGTGGATCGGC GAGATCAACCCTTCTAATGGCGACACCAACTTCAATGAGAAGTTTAAGT CCAAGGCTACCCTGACAGTGGATAAGTCTGCTTCCACAGCCTACATGGA GCTGTCCAGCCTGAGGTCCGAGGACACCGCCGTGTACTATTGCACAAGG TCTGATGGCCGGAACGACATGGATTCCTGGGGCCAGGGCACCCTGGTGA CAGTGTCTTCCGCTTCTACCAAGGGACCATCCGTGTTTCCACTGGCTCC AAGCTCTAAGAGCACCTCTGGAGGAACAGCCGCTCTGGGATGTCTGGTG AAGGACTATTTCCCAGAGCCCGTGACAGTGTCTTGGAACTCCGGCGCTC TGACCTCTGGCGTGCACACATTTCCTGCCGTGCTGCAGTCCAGCGGCCT GTACTCCCTGTCTTCCGTGGTGACCGTGCCAAGCTCTTCCCTGGGCACC CAGACATATATCTGCAACGTGAATCACAAGCCTTCCAATACAAAGGTGG ACAAGAAGGTGGAGCCAAAGAGCTCTGATAAGACCCATACAAGCCCCCC TTCTCCTGCTCCAGAGCTGCTGGGAGGCTCCAGCGTGTTCCTGTTTCCA CCCAAGCCAAAGGACACCCTGATGATCAGCAGAACCCCCGAGGTGACAT GCGTGGTGGTGGACGTGTCTCACGAGGACCCCGAGGTGAAGTTCAACTG GTACGTGGATGGCGTGGAGGTGCATAATGCCAAGACCAAGCCTAGAGAG GAGCAGTACAATAGCACCTATCGCGTGGTGTCTGTGCTGACAGTGCTGC ACCAGGACTGGCTGAACGGCAAGGAGTATAAGTGCAAGGTGTCCAATAA GGCTCTGCCCGCCCCTATCGAGAAGACCATCAGCAAGGCTAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCAAG CGATATCGCCGTGGAGTGGGAGTCTAATGGCCAGCCCGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTTTC CTGTAGCGTGATGCATGAGGCTCTGCACAATCATTACACACAGAAGTCT CTGTCCCTGAGCCCTGGC |
| 184 | TNX05 VH-CH1-CH2-CH3 IgG4 (S228P, L235A) (no terminal K) | CAGGTGCAGCTGGTGCAGTCTGGAGCTGAGGTGGTGAAGCCTGGAGCTA GCGTGAAGCTGTCTTGCAAGGCTTCCGGCTACATCTTCACCTCCTACTA TATGTATTGGGTGAAGCAGGCTCCTGGACAGGGCCTGGAGTGGATCGGC GAGATCAACCCATCCAATGGCGACACAAACTTCAATGAGAAGTTTAAGA GCAAGGCTACCCTGACAGTGGATAAGTCCGCCTCTACCGCCTACATGGA GCTGTCCAGCCTGAGGAGCGAGGACACAGCCGTGTACTATTGTACCAGG AGCGATGGCCGGAACGACATGGATTCTTGGGGCCAGGGCACACTGGTGA CCGTGTCTTCCGCTAGCACAAAGGGACCATCCGTGTTCCCACTGGCTCC ATGCTCCAGGAGCACATCTGAGTCCACCGCCGCTCTGGGCTGTCTGGTG AAGGACTATTTCCCTGAGCCAGTGACCGTGTCCTGGAATAGCGGCGCTC TGACATCCGGAGTGCACACCTTTCCAGCCGTGCTGCAGAGCTCTGGCCT GTACAGCCTGTCCAGCGTGGTGACAGTGCCTTCTTCCAGCCTGGGCACC AAGACATATACCTGCAACGTGGACCATAAGCCATCTAATACCAAGGTGG ATAAGAGAGTGGAGTCCAAGTACGGACCACCTTGCCCACCATGTCCAGC TCCTGAGTTCGCTGGAGGACCATCCGTGTTCCTGTTTCCTCCAAAGCCC AAGGACACCCTGATGATCTCTCGCACACCAGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCCAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC TTCTTCCATCGAGAAGACAATCTCTAAGGCTAAGGGCCAGCCTCGGGAG CCACAGGTGTACACCCTGCCCCCTAGCCAGGAGGAGATGACAAAGAACC AGGTGTCTCTGACCTGTCTGGTGAAGGGCTTCTATCCCTCCGACATCGC CGTGGAGTGGGAGAGCAATGGCCAGCCTGAGAACAATTACAAGACCACA CCACCCGTGCTGGACTCTGATGGCTCCTTCTTTCTGTATTCTAGACTGA CCGTCGATAAGTCCCGCTGGCAGGAGGGCAACGTGTTTAGCTGCTCTGT GATGCACGAGGCCCTGCACAATCATTACACCCAGAAGTCCCTGAGCCTG TCTCTGGGC |
| 298 | TNX06 VH-CH1-CH2-CH3 IgG4 (wild type) (no terminal K) | CAGGTCCAGCTGGTCCAGTCAGGGGCTGAAGTGGTCAAACCCGGCGCCA GCGTGAAGCTGTCTTGCAAGGCTTCCGGCTACATCTTCACCAGCTACTA TATGTATTGGGTGAAGCAGGCTCCAGGACAGGGCCTGGAGTGGATCGGC GAGATCAACCCTTCCAATGGCGACACAAACTTCAATGAGAAGTTTAAGA GCAAGGCCACCCTGACAGTGGATAAGAGCGCCTCTACCGCTTACATGGA GCTGTCCAGCCTGAGGTCTGAGGACACAGCCGTGTACTATTGTACCAGG AGCGATGGCCGGAACGACATGGATTCTTGGGGCCAGGGCACACTGGTGA CCGTGTCTTCCGCCAGCACAAAGGGCCCTTCCGTGTTCCCCCTGGCTCC TTGCTCCAGGAGCACATCTGAGTCCACCGCCGCTCTGGGCTGTCTGGTG AAGGACTACTTCCCAGAGCCCGTGACCGTGTCCTGGAATAGCGGCGCCC TGACATCCGGAGTGCACACCTTTCCAGCTGTGCTGCAGAGCTCTGGCCT GTACAGCCTGTCCAGCGTGGTGACAGTGCCCTCTTCCAGCCTGGGCACC AAGACATATACCTGCAACGTGGACCATAAGCCTTCTAATACCAAGGTGG ATAAGAGAGTGGAGTCCAAGTACGGACCACCTTGCCCTAGCTGTCCTGC TCCAGAGTTCCTGGGAGGACCTTCCGTGTTCCTGTTTCCACCCAAGCCA AAGGACACACTGATGATCTCTCGCACACCTGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGC |
| 299 | TNX07 VH-CH1-CH2-CH3 IgG4 (S228P) (no terminal K) | CAGGTCCAGCTGGTCCAGTCAGGGGCTGAAGTGGTCAAACCCGGCGCCA GCGTGAAGCTGTCTTGCAAGGCTTCCGGCTACATCTTCACCAGCTACTA TATGTATTGGGTGAAGCAGGCTCCAGGACAGGGCCTGGAGTGGATCGGC GAGATCAACCCTTCCAATGGCGACACAAACTTCAATGAGAAGTTTAAGA GCAAGGCCACCCTGACAGTGGATAAGAGCGCCTCTACCGCTTACATGGA GCTGTCCAGCCTGAGGTCTGAGGACACAGCCGTGTACTATTGTACCAGG AGCGATGGCCGGAACGACATGGATTCTTGGGGCCAGGGCACACTGGTGA CCGTGTCTTCCGCCAGCACAAAGGGCCCTTCCGTGTTCCCCCTGGCTCC TTGCTCCAGGAGCACATCTGAGTCCACCGCCGCTCTGGGCTGTCTGGTG AAGGACTACTTCCCAGAGCCCGTGACCGTGTCCTGGAATAGCGGCGCCC TGACATCCGGAGTGCACACCTTTCCAGCTGTGCTGCAGAGCTCTGGCCT GTACAGCCTGTCCAGCGTGGTGACAGTGCCCTCTTCCAGCCTGGGCACC AAGACATATACCTGCAACGTGGACCATAAGCCTTCTAATACCAAGGTGG ATAAGAGAGTGGAGTCCAAGTACGGACCACCTTGCCCTCCCTGTCCTGC TCCAGAGTTCCTGGGAGGACCTTCCGTGTTCCTGTTTCCACCCAAGCCA AAGGACACACTGATGATCTCTCGCACACCTGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGC |
| 300 | TNX08 VH-CH1-CH2-CH3 (IgG4 S228P/L235E) (no terminal K) | CAGGTCCAGCTGGTCCAGTCAGGGGCTGAAGTGGTCAAACCCGGCGCCA GCGTGAAGCTGTCTTGCAAGGCTTCCGGCTACATCTTCACCAGCTACTA TATGTATTGGGTGAAGCAGGCTCCAGGACAGGGCCTGGAGTGGATCGGC GAGATCAACCCTTCCAATGGCGACACAAACTTCAATGAGAAGTTTAAGA GCAAGGCCACCCTGACAGTGGATAAGAGCGCCTCTACCGCTTACATGGA GCTGTCCAGCCTGAGGTCTGAGGACACAGCCGTGTACTATTGTACCAGG AGCGATGGCCGGAACGACATGGATTCTTGGGGCCAGGGCACACTGGTGA CCGTGTCTTCCGCCAGCACAAAGGGCCCTTCCGTGTTCCCCCTGGCTCC TTGCTCCAGGAGCACATCTGAGTCCACCGCCGCTCTGGGCTGTCTGGTG AAGGACTACTTCCCAGAGCCCGTGACCGTGTCCTGGAATAGCGGCGCCC TGACATCCGGAGTGCACACCTTTCCAGCTGTGCTGCAGAGCTCTGGCCT GTACAGCCTGTCCAGCGTGGTGACAGTGCCCTCTTCCAGCCTGGGCACC AAGACATATACCTGCAACGTGGACCATAAGCCTTCTAATACCAAGGTGG ATAAGAGAGTGGAGTCCAAGTACGGACCACCTTGCCCTCCCTGTCCTGC TCCAGAGTTCGAGGGAGGACCTTCCGTGTTCCTGTTTCCACCCAAGCCA AAGGACACACTGATGATCTCTCGCACACCTGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGC |
| 301 | TNX09 VH-CH1-CH2-CH3 IgG4 (S228P/F234A/ L235A) (no terminal K) | CAGGTCCAGCTGGTCCAGTCAGGGGCTGAAGTGGTCAAACCCGGCGCCA GCGTGAAGCTGTCTTGCAAGGCTTCCGGCTACATCTTCACCAGCTACTA TATGTATTGGGTGAAGCAGGCTCCAGGACAGGGCCTGGAGTGGATCGGC GAGATCAACCCTTCCAATGGCGACACAAACTTCAATGAGAAGTTTAAGA GCAAGGCCACCCTGACAGTGGATAAGAGCGCCTCTACCGCTTACATGGA GCTGTCCAGCCTGAGGTCTGAGGACACAGCCGTGTACTATTGTACCAGG AGCGATGGCCGGAACGACATGGATTCTTGGGGCCAGGGCACACTGGTGA CCGTGTCTTCCGCCAGCACAAAGGGCCCTTCCGTGTTCCCCCTGGCTCC TTGCTCCAGGAGCACATCTGAGTCCACCGCCGCTCTGGGCTGTCTGGTG AAGGACTACTTCCCAGAGCCCGTGACCGTGTCCTGGAATAGCGGCGCCC TGACATCCGGAGTGCACACCTTTCCAGCTGTGCTGCAGAGCTCTGGCCT GTACAGCCTGTCCAGCGTGGTGACAGTGCCCTCTTCCAGCCTGGGCACC AAGACATATACCTGCAACGTGGACCATAAGCCTTCTAATACCAAGGTGG ATAAGAGAGTGGAGTCCAAGTACGGACCACCTTGCCCTCCCTGTCCTGC TCCAGAGGCCGCCGGAGGACCTTCCGTGTTCCTGTTTCCACCCAAGCCA AAGGACACACTGATGATCTCTCGCACACCTGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGC |
| 302 | TNX10 VH-CH1-CH2-CH3 IgG1 (L234A/L235A) (no terminal K) | CAGGTCCAGCTGGTCCAGTCAGGTGCCGAAGTGGTCAAGCCCGGCGCCT CTGTGAAGCTGTCCTGCAAGGCTAGCGGCTACATCTTCACCTCCTACTA TATGTATTGGGTGAAGCAGGCTCCTGGACAGGGCCTGGAGTGGATCGGC GAGATCAACCCATCTAATGGCGACACCAACTTCAATGAGAAGTTTAAGT CCAAGGCCACCCTGACAGTGGATAAGAGCGCCTCTACAGCTTACATGGA GCTGTCCAGCCTGAGGAGCGAGGACACCGCCGTGTACTATTGCACAAGG TCTGATGGCCGGAACGACATGGATTCCTGGGGCCAGGGCACCCTGGTGA CAGTGTCTTCCGCCTCTACCAAGGGCCCTTCCGTGTTTCCACTGGCTCC CAGCTCTAAGTCCACCAGCGGAGGAACAGCCGCTCTGGGCTGTCTGGTG AAGGACTACTTCCCAGAGCCCGTGACAGTGAGCTGGAACTCTGGCGCCC TGACCAGCGGAGTGCACACATTTCCTGCTGTGCTGCAGTCCAGCGGCCT GTACTCTCTGTCTTCCGTGGTGACCGTGCCAAGCTCTTCCCTGGGCACC CAGACATATATCTGCAACGTGAATCACAAGCCAAGCAATACAAAGGTGG ACAAGAAGGTGGAGCCCAAGTCTTGTGATAAGACCCATACATGCCCCCC TTGTCCTGCTCCAGAGGCTGCTGGAGGACCATCCGTGTTCCTGTTTCCA CCCAAGCCTAAGGACACCCTGATGATCTCCAGAACCCCAGAGGTGACAT GCGTGGTGGTGGACGTGAGCCACGAGGACCCCGAGGTGAAGTTCAACTG GTACGTGGATGGCGTGGAGGTGCATAATGCTAAGACCAAGCCAAGAGAG GAGCAGTACAACAGCACCTATCGCGTGGTGTCTGTGCTGACAGTGCTGC ATCAGGACTGGCTGAACGGCAAGGAGTATAAGTGCAAGGTGTCTAATAA GGCCCTGCCCGCTCCTATCGAGAAGACCATCTCCAAGGCCAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCCAG CGATATCGCTGTGGAGTGGGAGTCTAATGGCCAGCCTGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTCTC CTGTTCCGTCATGCACGAGGCACTGCACAATCATTACACCCAGAAGTCA CTGTCACTGTCACCAGGA |
| 303 | TNX11 VH-CH1-CH2-CH3 IgG1 (C226S, C229S, P238S) (no terminal K) | CAGGTCCAGCTGGTCCAGTCAGGTGCCGAAGTGGTCAAGCCCGGCGCCT CTGTGAAGCTGTCCTGCAAGGCTAGCGGCTACATCTTCACCTCCTACTA TATGTATTGGGTGAAGCAGGCTCCTGGACAGGGCCTGGAGTGGATCGGC GAGATCAACCCATCTAATGGCGACACCAACTTCAATGAGAAGTTTAAGT CCAAGGCCACCCTGACAGTGGATAAGAGCGCCTCTACAGCTTACATGGA GCTGTCCAGCCTGAGGAGCGAGGACACCGCCGTGTACTATTGCACAAGG TCTGATGGCCGGAACGACATGGATTCCTGGGGCCAGGGCACCCTGGTGA CAGTGTCTTCCGCCTCTACCAAGGGCCCTTCCGTGTTTCCACTGGCTCC CAGCTCTAAGTCCACCAGCGGAGGAACAGCCGCTCTGGGCTGTCTGGTG AAGGACTACTTCCCAGAGCCCGTGACAGTGAGCTGGAACTCTGGCGCCC TGACCAGCGGAGTGCACACATTTCCTGCTGTGCTGCAGTCCAGCGGCCT GTACTCTCTGTCTTCCGTGGTGACCGTGCCAAGCTCTTCCCTGGGCACC CAGACATATATCTGCAACGTGAATCACAAGCCAAGCAATACAAAGGTGG ACAAGAAGGTGGAGCCCAAGTCTTGTGATAAGACCCATACAtccCCCCC TTccCCTGCTCCAGAGCTGCTGGGAGGAAGCTCCGTGTTCCTGTTTCCA CCCAAGCCTAAGGACACCCTGATGATCTCCAGAACCCCAGAGGTGACAT GCGTGGTGGTGGACGTGAGCCACGAGGACCCCGAGGTGAAGTTCAACTG GTACGTGGATGGCGTGGAGGTGCATAATGCTAAGACCAAGCCAAGAGAG GAGCAGTACAACAGCACCTATCGCGTGGTGTCTGTGCTGACAGTGCTGC ATCAGGACTGGCTGAACGGCAAGGAGTATAAGTGCAAGGTGTCTAATAA GGCCCTGCCCGCTCCTATCGAGAAGACCATCTCCAAGGCCAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCCAG CGATATCGCTGTGGAGTGGGAGTCTAATGGCCAGCCTGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTCTC CTGTTCCGTCATGCACGAGGCACTGCACAATCATTACACCCAGAAGTCA CTGTCACTGTCACCAGGA |
| 304 | TNX12 VH-CH1-CH2-CH3 IgG1 (C229S, P238S) (no terminal K) | CAGGTCCAGCTGGTCCAGTCAGGTGCCGAAGTGGTCAAGCCCGGCGCCT CTGTGAAGCTGTCCTGCAAGGCTAGCGGCTACATCTTCACCTCCTACTA TATGTATTGGGTGAAGCAGGCTCCTGGACAGGGCCTGGAGTGGATCGGC GAGATCAACCCATCTAATGGCGACACCAACTTCAATGAGAAGTTTAAGT CCAAGGCCACCCTGACAGTGGATAAGAGCGCCTCTACAGCTTACATGGA GCTGTCCAGCCTGAGGAGCGAGGACACCGCCGTGTACTATTGCACAAGG TCTGATGGCCGGAACGACATGGATTCCTGGGGCCAGGGCACCCTGGTGA CAGTGTCTTCCGCCTCTACCAAGGGCCCTTCCGTGTTTCCACTGGCTCC CAGCTCTAAGTCCACCAGCGGAGGAACAGCCGCTCTGGGCTGTCTGGTG AAGGACTACTTCCCAGAGCCCGTGACAGTGAGCTGGAACTCTGGCGCCC TGACCAGCGGAGTGCACACATTTCCTGCTGTGCTGCAGTCCAGCGGCCT GTACTCTCTGTCTTCCGTGGTGACCGTGCCAAGCTCTTCCCTGGGCACC CAGACATATATCTGCAACGTGAATCACAAGCCAAGCAATACAAAGGTGG ACAAGAAGGTGGAGCCCAAGTCTTGTGATAAGACCCATACATGCCCCCC TTCCCCTGCTCCAGAGCTGCTGGGAGGAAGcTCCGTGTTCCTGTTTCCA CCCAAGCCTAAGGACACCCTGATGATCTCCAGAACCCCAGAGGTGACAT GCGTGGTGGTGGACGTGAGCCACGAGGACCCCGAGGTGAAGTTCAACTG GTACGTGGATGGCGTGGAGGTGCATAATGCTAAGACCAAGCCAAGAGAG GAGCAGTACAACAGCACCTATCGCGTGGTGTCTGTGCTGACAGTGCTGC ATCAGGACTGGCTGAACGGCAAGGAGTATAAGTGCAAGGTGTCTAATAA GGCCCTGCCCGCTCCTATCGAGAAGACCATCTCCAAGGCCAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCCAG CGATATCGCTGTGGAGTGGGAGTCTAATGGCCAGCCTGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTCTC CTGTTCCGTCATGCACGAGGCACTGCACAATCATTACACCCAGAAGTCA CTGTCACTGTCACCAGGA |
| 305 | TNX13 VH-CH1-CH2-CH3 IgG1 (C226S, P238S) (no terminal K) | CAGGTCCAGCTGGTCCAGTCAGGTGCCGAAGTGGTCAAGCCCGGCGCCT CTGTGAAGCTGTCCTGCAAGGCTAGCGGCTACATCTTCACCTCCTACTA TATGTATTGGGTGAAGCAGGCTCCTGGACAGGGCCTGGAGTGGATCGGC GAGATCAACCCATCTAATGGCGACACCAACTTCAATGAGAAGTTTAAGT CCAAGGCCACCCTGACAGTGGATAAGAGCGCCTCTACAGCTTACATGGA GCTGTCCAGCCTGAGGAGCGAGGACACCGCCGTGTACTATTGCACAAGG TCTGATGGCCGGAACGACATGGATTCCTGGGGCCAGGGCACCCTGGTGA CAGTGTCTTCCGCCTCTACCAAGGGCCCTTCCGTGTTTCCACTGGCTCC CAGCTCTAAGTCCACCAGCGGAGGAACAGCCGCTCTGGGCTGTCTGGTG AAGGACTACTTCCCAGAGCCCGTGACAGTGAGCTGGAACTCTGGCGCCC TGACCAGCGGAGTGCACACATTTCCTGCTGTGCTGCAGTCCAGCGGCCT GTACTCTCTGTCTTCCGTGGTGACCGTGCCAAGCTCTTCCCTGGGCACC CAGACATATATCTGCAACGTGAATCACAAGCCAAGCAATACAAAGGTGG ACAAGAAGGTGGAGCCCAAGTCTTGTGATAAGACCCATACATCCCCCCC TTGCCCTGCTCCAGAGCTGCTGGGAGGAAGCTCCGTGTTCCTGTTTCCA CCCAAGCCTAAGGACACCCTGATGATCTCCAGAACCCCAGAGGTGACAT GCGTGGTGGTGGACGTGAGCCACGAGGACCCCGAGGTGAAGTTCAACTG GTACGTGGATGGCGTGGAGGTGCATAATGCTAAGACCAAGCCAAGAGAG GAGCAGTACAACAGCACCTATCGCGTGGTGTCTGTGCTGACAGTGCTGC ATCAGGACTGGCTGAACGGCAAGGAGTATAAGTGCAAGGTGTCTAATAA GGCCCTGCCCGCTCCTATCGAGAAGACCATCTCCAAGGCCAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCCAG CGATATCGCTGTGGAGTGGGAGTCTAATGGCCAGCCTGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTCTC CTGTTCCGTCATGCACGAGGCACTGCACAATCATTACACCCAGAAGTCA CTGTCACTGTCACCAGGA |
| 306 | TNX14 VH-CH1-CH2-CH3 IgG4 (L115T, S228P, L235A) (no terminal K) | CAGGTCCAGCTGGTCCAGTCAGGGGCTGAAGTGGTCAAAC CCGGCGCCAGCGTGAAGCTGTCTTGCAAGGCTTCCGGCTA CATCTTCACCAGCTACTATATGTATTGGGTGAAGCAGGCT CCAGGACAGGGCCTGGAGTGGATCGGCGAGATCAACCCTT CCAATGGCGACACAAACTTCAATGAGAAGTTTAAGAGCAA GGCCACCCTGACAGTGGATAAGAGCGCCTCTACCGCTTAC ATGGAGCTGTCCAGCCTGAGGTCTGAGGACACAGCCGTGT ACTATTGTACCAGGAGCGATGGCCGGAACGACATGGATTC TTGGGGCCAGGGCACAACAGTGACCGTGTCTTCCGCCAGC ACAAAGGGCCCTTCCGTGTTCCCCCTGGCTCCTTGCTCCA GGAGCACATCTGAGTCCACCGCCGCTCTGGGCTGTCTGGT GAAGGACTACTTCCCAGAGCCCGTGACCGTGTCCTGGAAT AGCGGCGCCCTGACATCCGGAGTGCACACCTTTCCAGCTG TGCTGCAGAGCTCTGGCCTGTACAGCCTGTCCAGCGTGGT GACAGTGCCCTCTTCCAGCCTGGGCACCAAGACATATACC TGCAACGTGGACCATAAGCCTTCTAATACCAAGGTGGATA AGAGAGTGGAGTCCAAGTACGGACCACCTTGCCCTCCTTG TCCTGCTCCAGAGTTCGCCGGAGGACCTTCCGTGTTCCTG TTTCCACCCAAGCCAAAGGACACACTGATGATCTCTCGCA CACCTGAGGTGACCTGCGTGGTGGTGGACGTGTCCCAGGA GGACCCCGAGGTGCAGTTCAACTGGTACGTGGATGGCGTG GAGGTGCACAATGCTAAGACCAAGCCAAGGGAGGAGCAGT TTAACTCCACATACCGGGTGGTGAGCGTGCTGACCGTGCT GCATCAGGATTGGCTGAACGGCAAGGAGTATAAGTGCAAG GTGAGCAATAAGGGCCTGCCCTCTTCCATCGAGAAGACAA TCTCTAAGGCTAAGGGACAGCCAAGGGAGCCACAGGTGTA CACCCTGCCTCCAAGCCAGGAGGAGATGACAAAGAACCAG GTGTCTCTGACCTGTCTGGTGAAGGGCTTCTATCCATCTG ACATCGCTGTGGAGTGGGAGTCCAATGGCCAGCCCGAGAA CAATTACAAGACCACACCCCCTGTGCTGGACTCTGATGGC TCCTTCTTTCTGTATTCTAGACTGACCGTGGATAAGTCCC GCTGGCAGGAGGGCAACGTGTTCTCCTGCTCTGTGATGCA CGAAGCACTGCACAATCATTACACTCAGAAGAGCCTGTCA CTGTCCCTGGGC |

| **Heavy chain DNA sequences WITHOUT LEADER; with terminal K** | | |
|---|---|---|
| 185 | TNX01 VH-CH1-CH2-CH3 IgG1(N297Q) (with terminal K) | CAGGTGCAGCTGGTGCAGTCTGGAGCTGAGGTGGTGAAGCCAGGCGCCT CTGTGAAGCTGTCCTGCAAGGCTAGCGGCTACATCTTCACCTCCTACTA TATGTATTGGGTGAAGCAGGCTCCTGGACAGGGACTGGAGTGGATCGGC GAGATCAACCCATCTAATGGCGACACCAACTTCAATGAGAAGTTTAAGT CCAAGGCTACCCTGACAGTGGATAAGTCCGCCTCTACAGCCTACATGGA GCTGTCCAGCCTGAGAAGCGAGGACACCGCCGTGTACTATTGCACAAGG TCTGATGGCCGGAACGACATGGATTCCTGGGGCCAGGGCACCCTGGTGA CAGTGTCTTCCGCTTCTACCAAGGGCCCTTCCGTGTTTCCACTGGCTCC AAGCTCTAAGTCCACCAGCGGAGGAACAGCCGCTCTGGGCTGTCTGGTG AAGGACTATTTCCCTGAGCCAGTGACAGTGAGCTGGAACTCTGGCGCTC TGACCTCCGGCGTGCACACATTTCCAGCCGTGCTGCAGTCCAGCGGCCT GTACAGCCTGTCTTCCGTGGTGACCGTGCCAAGCTCTTCCCTGGGCACC CAGACATATATCTGCAACGTGAATCATAAGCCCTCTAATACAAAGGTGG ACAAGAAGGTGGAGCCCAAGTCTTGTGATAAAACACATACTTGCCCCCC TTGTCCTGCACCAGAACTGCTGGGAGGACCTAGCGTGTTCCTGTTTCCA CCCAAGCCAAAAGACACCCTGATGATTAGTAGAACCCCTGAGGTCACAT GCGTGGTCGTGGACGTGAGCCACGAGGACCCCGAGGTGAAGTTCAACTG GTACGTGGACGGCGTGGAGGTGCACAATGCTAAGACCAAGCCCAGAGAG GAGCAGTACCAGAGCACCTATCGCGTGGTGTCTGTGCTGACAGTGCTGC ATCAGGATTGGCTGAACGGCAAGGAGTATAAGTGCAAGGTGAGCAATAA GGCTCTGCCCGCCCCTATCGAGAAGACCATCTCTAAGGCTAAGGGCCAG CCTAGGGAGCCACAGGTGTACACACTGCCCCCTTCCCGGGACGAGCTGA CCAAGAACCAGGTGAGCCTGACATGTCTGGTGAAGGGCTTCTATCCCAG CGATATCGCCGTGGAGTGGGAGTCTAATGGCCAGCCTGAGAACAATTAC AAGACCACACCACCCGTGCTGGACTCCGATGGCAGCTTCTTTCTGTATT CCAAGCTGACCGTGGATAAGAGCCGCTGGCAGCAGGGCAACGTGTTTTC TTGTTCCGTGATGCACGAGGCCCTGCACAATCATTACACACAGAAGAGC CTGTCTCTGTCCCCTGGCAAG |
| 186 | TNX02 VH-CH1-CH2-CH3 IgG1 (wt) (with terminal K) | CAGGTGCAGCTGGTGCAGTCCGGAGCTGAGGTGGTGAAGCCAGGAGCCT CTGTGAAGCTGTCCTGCAAGGCTAGCGGCTACATCTTCACCAGCTACTA TATGTATTGGGTGAAGCAGGCTCCAGGACAGGGCCTGGAGTGGATCGGC GAGATCAACCCTTCTAATGGCGACACCAACTTCAATGAGAAGTTTAAGT CCAAGGCTACCCTGACAGTGGATAAGTCCGCCTCTACAGCCTACATGGA GCTGTCCAGCCTGAGGTCTGAGGACACCGCCGTGTACTATTGCACAAGG TCTGATGGCCGGAACGACATGGATTCCTGGGGCCAGGGCACCCTGGTGA CAGTGTCTTCCGCTTCTACCAAGGGACCATCCGTGTTTCCACTGGCTCC AAGCTCTAAGTCCACCAGCGGAGGAACAGCCGCTCTGGGCTGTCTGGTG AAGGACTATTTCCCAGAGCCCGTGACAGTGAGCTGGAACTCTGGCGCTC TGACCAGCGGCGTGCACACATTTCCAGCCGTGCTGCAGTCCAGCGGCCT GTACTCTCTGTCTTCCGTGGTGACCGTGCCTAGCTCTTCCCTGGGCACC CAGACATATATCTGCAACGTGAATCACAAGCCTAGCAATACAAAGGTGG ACAAGAAGGTGGAGCCAAAGTCTTGTGATAAGACCCATACATGCCCCCC TTGTCCTGCTCCAGAGCTGCTGGGAGGACCATCCGTGTTCCTGTTTCCA CCCAAGCCCAAGGACACCCTGATGATCTCCAGAACCCCTGAGGTGACAT GCGTGGTGGTGGACGTGAGCCACGAGGACCCCGAGGTGAAGTTCAACTG GTACGTGGATGGCGTGGAGGTGCATAATGCCAAGACCAAGCCTAGAGAG GAGCAGTACAATAGCACCTATCGCGTGGTGTCTGTGCTGACAGTGCTGC ACCAGGACTGGCTGAACGGCAAGGAGTATAAGTGCAAGGTGTCTAATAA GGCTCTGCCCGCCCCTATCGAGAAGACCATCTCCAAGGCTAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCAAG CGATATCGCCGTGGAGTGGGAGTCTAATGGCCAGCCCGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTTTC TTGTTCCGTGATGCATGAGGCTCTGCACAATCATTACACACAGAAGAGC CTGTCTCTGTCCCCAGGCAAG |
| 187 | TNX03 VH-CH1-CH2-CH3 IgG1 (N297G) (with terminal K) | CAGGTGCAGCTGGTGCAGTCCGGAGCTGAGGTGGTGAAGCCAGGAGCCT CTGTGAAGCTGTCCTGCAAGGCTAGCGGCTACATCTTCACCAGCTACTA TATGTATTGGGTGAAGCAGGCTCCAGGACAGGGCCTGGAGTGGATCGGC GAGATCAACCCTTCTAATGGCGACACCAACTTCAATGAGAAGTTTAAGT CCAAGGCTACCCTGACAGTGGATAAGTCCGCCTCTACAGCCTACATGGA GCTGTCCAGCCTGAGGTCTGAGGACACCGCCGTGTACTATTGCACAAGG TCTGATGGCCGGAACGACATGGATTCCTGGGGCCAGGGCACCCTGGTGA CAGTGTCTTCCGCTTCTACCAAGGGACCATCCGTGTTTCCACTGGCTCC AAGCTCTAAGTCCACCAGCGGAGGAACAGCCGCTCTGGGCTGTCTGGTG AAGGACTATTTCCCAGAGCCCGTGACAGTGAGCTGGAACTCTGGCGCTC TGACCAGCGGCGTGCACACATTTCCAGCCGTGCTGCAGTCCAGCGGCCT GTACTCTCTGTCTTCCGTGGTGACCGTGCCTAGCTCTTCCCTGGGCACC CAGACATATATCTGCAACGTGAATCACAAGCCTAGCAATACAAAGGTGG ACAAGAAGGTGGAGCCAAAGTCTTGTGATAAGACCCATACATGCCCCCC TTGTCCTGCTCCAGAGCTGCTGGGAGGACCATCCGTGTTCCTGTTTCCA CCCAAGCCCAAGGACACCCTGATGATCTCCAGAACCCCTGAGGTGACAT GCGTGGTGGTGGACGTGAGCCACGAGGACCCCGAGGTGAAGTTCAACTG GTACGTGGATGGCGTGGAGGTGCATAATGCCAAGACCAAGCCTAGAGAG GAGCAGTACGGCAGCACCTATCGCGTGGTGTCTGTGCTGACAGTGCTGC ACCAGGACTGGCTGAACGGCAAGGAGTATAAGTGCAAGGTGTCTAATAA GGCTCTGCCCGCCCCTATCGAGAAGACCATCTCCAAGGCTAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCAAG CGATATCGCCGTGGAGTGGGAGTCTAATGGCCAGCCCGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTTTC TTGTTCCGTGATGCATGAGGCTCTGCACAATCATTACACACAGAAGAGC CTGTCTCTGTCCCCAGGCAAG |
| 188 | TNX04 VH-CH1-CH2-CH3 IgG1 (C220S, C226S, C229S, P238S) (with terminal K) | CAGGTGCAGCTGGTGCAGAGCGGAGCTGAGGTGGTGAAGCCAGGAGCCT CTGTGAAGCTGTCCTGCAAGGCTAGCGGCTACATCTTCACCTCTTACTA TATGTATTGGGTGAAGCAGGCTCCAGGACAGGGCCTGGAGTGGATCGGC GAGATCAACCCTTCTAATGGCGACACCAACTTCAATGAGAAGTTTAAGT CCAAGGCTACCCTGACAGTGGATAAGTCTGCTTCCACAGCCTACATGGA GCTGTCCAGCCTGAGGTCCGAGGACACCGCCGTGTACTATTGCACAAGG TCTGATGGCCGGAACGACATGGATTCCTGGGGCCAGGGCACCCTGGTGA CAGTGTCTTCCGCTTCTACCAAGGGACCATCCGTGTTTCCACTGGCTCC AAGCTCTAAGAGCACCTCTGGAGGAACAGCCGCTCTGGGATGTCTGGTG AAGGACTATTTCCCAGAGCCCGTGACAGTGTCTTGGAACTCCGGCGCTC TGACCTCTGGCGTGCACACATTTCCTGCCGTGCTGCAGTCCAGCGGCCT GTACTCCCTGTCTTCCGTGGTGACCGTGCCAAGCTCTTCCCTGGGCACC CAGACATATATCTGCAACGTGAATCACAAGCCTTCCAATACAAAGGTGG ACAAGAAGGTGGAGCCAAAGAGCTCTGATAAGACCCATACAAGCCCCCC TTCTCCTGCTCCAGAGCTGCTGGGAGGCTCCAGCGTGTTCCTGTTTCCA CCCAAGCCAAAGGACACCCTGATGATCAGCAGAACCCCCGAGGTGACAT GCGTGGTGGTGGACGTGTCTCACGAGGACCCCGAGGTGAAGTTCAACTG GTACGTGGATGGCGTGGAGGTGCATAATGCCAAGACCAAGCCTAGAGAG GAGCAGTACAATAGCACCTATCGCGTGGTGTCTGTGCTGACAGTGCTGC ACCAGGACTGGCTGAACGGCAAGGAGTATAAGTGCAAGGTGTCCAATAA GGCTCTGCCCGCCCCTATCGAGAAGACCATCAGCAAGGCTAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCAAG CGATATCGCCGTGGAGTGGGAGTCTAATGGCCAGCCCGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTTTC CTGTAGCGTGATGCATGAGGCTCTGCACAATCATTACACACAGAAGTCT CTGTCCCTGAGCCCTGGCAAG |
| 189 | TNX05 VH-CH1-CH2-CH3 IgG4 (S228P, L235A) (with terminal K) | CAGGTGCAGCTGGTGCAGTCTGGAGCTGAGGTGGTGAAGCCTGGAGCTA GCGTGAAGCTGTCTTGCAAGGCTTCCGGCTACATCTTCACCTCCTACTA TATGTATTGGGTGAAGCAGGCTCCTGGACAGGGCCTGGAGTGGATCGGC GAGATCAACCCATCCAATGGCGACACAAACTTCAATGAGAAGTTTAAGA GCAAGGCTACCCTGACAGTGGATAAGTCCGCCTCTACCGCCTACATGGA GCTGTCCAGCCTGAGGAGCGAGGACACAGCCGTGTACTATTGTACCAGG AGCGATGGCCGGAACGACATGGATTCTTGGGGCCAGGGCACACTGGTGA CCGTGTCTTCCGCTAGCACAAAGGGACCATCCGTGTTCCCACTGGCTCC ATGCTCCAGGAGCACATCTGAGTCCACCGCCGCTCTGGGCTGTCTGGTG AAGGACTATTTCCCTGAGCCAGTGACCGTGTCCTGGAATAGCGGCGCTC TGACATCCGGAGTGCACACCTTTCCAGCCGTGCTGCAGAGCTCTGGCCT GTACAGCCTGTCCAGCGTGGTGACAGTGCCTTCTTCCAGCCTGGGCACC AAGACATATACCTGCAACGTGGACCATAAGCCATCTAATACCAAGGTGG ATAAGAGAGTGGAGTCCAAGTACGGACCACCTTGCCCACCATGTCCAGC TCCTGAGTTCGCTGGAGGACCATCCGTGTTCCTGTTTCCTCCAAAGCCC AAGGACACCCTGATGATCTCTCGCACACCAGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCCAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC TTCTTCCATCGAGAAGACAATCTCTAAGGCTAAGGGCCAGCCTCGGGAG CCACAGGTGTACACCCTGCCCCCTAGCCAGGAGGAGATGACAAAGAACC AGGTGTCTCTGACCTGTCTGGTGAAGGGCTTCTATCCCTCCGACATCGC CGTGGAGTGGGAGAGCAATGGCCAGCCTGAGAACAATTACAAGACCACA CCACCCGTGCTGGACTCTGATGGCTCCTTCTTTCTGTATTCTAGACTGA CCGTCGATAAGTCCCGCTGGCAGGAGGGCAACGTGTTTAGCTGCTCTGT GATGCACGAGGCCCTGCACAATCATTACACCCAGAAGTCCCTGAGCCTG TCTCTGGGCAAG |
| 307 | TNX06 VH-CH1-CH2-CH3 IgG4 (wild type) (with terminal K) | CAGGTCCAGCTGGTCCAGTCAGGGGCTGAAGTGGTCAAACCCGGCGCCA GCGTGAAGCTGTCTTGCAAGGCTTCCGGCTACATCTTCACCAGCTACTA TATGTATTGGGTGAAGCAGGCTCCAGGACAGGGCCTGGAGTGGATCGGC GAGATCAACCCTTCCAATGGCGACACAAACTTCAATGAGAAGTTTAAGA GCAAGGCCACCCTGACAGTGGATAAGAGCGCCTCTACCGCTTACATGGA GCTGTCCAGCCTGAGGTCTGAGGACACAGCCGTGTACTATTGTACCAGG AGCGATGGCCGGAACGACATGGATTCTTGGGGCCAGGGCACACTGGTGA CCGTGTCTTCCGCCAGCACAAAGGGCCCTTCCGTGTTCCCCCTGGCTCC TTGCTCCAGGAGCACATCTGAGTCCACCGCCGCTCTGGGCTGTCTGGTG AAGGACTACTTCCCAGAGCCCGTGACCGTGTCCTGGAATAGCGGCGCCC TGACATCCGGAGTGCACACCTTTCCAGCTGTGCTGCAGAGCTCTGGCCT GTACAGCCTGTCCAGCGTGGTGACAGTGCCCTCTTCCAGCCTGGGCACC AAGACATATACCTGCAACGTGGACCATAAGCCTTCTAATACCAAGGTGG ATAAGAGAGTGGAGTCCAAGTACGGACCACCTTGCCCTAGCTGTCCTGC TCCAGAGTTCCTGGGAGGACCTTCCGTGTTCCTGTTTCCACCCAAGCCA AAGGACACACTGATGATCTCTCGCACACCTGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGCAAA |
| 308 | TNX07 VH-CH1-CH2-CH3 IgG4 (S228P) (with terminal K) | CAGGTCCAGCTGGTCCAGTCAGGGGCTGAAGTGGTCAAACCCGGCGCCA GCGTGAAGCTGTCTTGCAAGGCTTCCGGCTACATCTTCACCAGCTACTA TATGTATTGGGTGAAGCAGGCTCCAGGACAGGGCCTGGAGTGGATCGGC GAGATCAACCCTTCCAATGGCGACACAAACTTCAATGAGAAGTTTAAGA GCAAGGCCACCCTGACAGTGGATAAGAGCGCCTCTACCGCTTACATGGA GCTGTCCAGCCTGAGGTCTGAGGACACAGCCGTGTACTATTGTACCAGG AGCGATGGCCGGAACGACATGGATTCTTGGGGCCAGGGCACACTGGTGA CCGTGTCTTCCGCCAGCACAAAGGGCCCTTCCGTGTTCCCCCTGGCTCC TTGCTCCAGGAGCACATCTGAGTCCACCGCCGCTCTGGGCTGTCTGGTG AAGGACTACTTCCCAGAGCCCGTGACCGTGTCCTGGAATAGCGGCGCCC TGACATCCGGAGTGCACACCTTTCCAGCTGTGCTGCAGAGCTCTGGCCT GTACAGCCTGTCCAGCGTGGTGACAGTGCCCTCTTCCAGCCTGGGCACC AAGACATATACCTGCAACGTGGACCATAAGCCTTCTAATACCAAGGTGG ATAAGAGAGTGGAGTCCAAGTACGGACCACCTTGCCCTCCCTGTCCTGC TCCAGAGTTCCTGGGAGGACCTTCCGTGTTCCTGTTTCCACCCAAGCCA AAGGACACACTGATGATCTCTCGCACACCTGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGCAAA |
| 309 | TNX08 VH-CH1-CH2-CH3 (IgG4 S228P/L235E) (with terminal K) | CAGGTCCAGCTGGTCCAGTCAGGGGCTGAAGTGGTCAAACCCGGCGCCA GCGTGAAGCTGTCTTGCAAGGCTTCCGGCTACATCTTCACCAGCTACTA TATGTATTGGGTGAAGCAGGCTCCAGGACAGGGCCTGGAGTGGATCGGC GAGATCAACCCTTCCAATGGCGACACAAACTTCAATGAGAAGTTTAAGA GCAAGGCCACCCTGACAGTGGATAAGAGCGCCTCTACCGCTTACATGGA GCTGTCCAGCCTGAGGTCTGAGGACACAGCCGTGTACTATTGTACCAGG AGCGATGGCCGGAACGACATGGATTCTTGGGGCCAGGGCACACTGGTGA CCGTGTCTTCCGCCAGCACAAAGGGCCCTTCCGTGTTCCCCCTGGCTCC TTGCTCCAGGAGCACATCTGAGTCCACCGCCGCTCTGGGCTGTCTGGTG AAGGACTACTTCCCAGAGCCCGTGACCGTGTCCTGGAATAGCGGCGCCC TGACATCCGGAGTGCACACCTTTCCAGCTGTGCTGCAGAGCTCTGGCCT GTACAGCCTGTCCAGCGTGGTGACAGTGCCCTCTTCCAGCCTGGGCACC AAGACATATACCTGCAACGTGGACCATAAGCCTTCTAATACCAAGGTGG ATAAGAGAGTGGAGTCCAAGTACGGACCACCTTGCCCTCCCTGTCCTGC TCCAGAGTTCGAGGGAGGACCTTCCGTGTTCCTGTTTCCACCCAAGCCA AAGGACACACTGATGATCTCTCGCACACCTGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGCAAA |
| 310 | TNX09 VH-CH1-CH2-CH3 IgG4 (S228P/F234A/ L235A) (with terminal K) | CAGGTCCAGCTGGTCCAGTCAGGGGCTGAAGTGGTCAAACCCGGCGCCA GCGTGAAGCTGTCTTGCAAGGCTTCCGGCTACATCTTCACCAGCTACTA TATGTATTGGGTGAAGCAGGCTCCAGGACAGGGCCTGGAGTGGATCGGC GAGATCAACCCTTCCAATGGCGACACAAACTTCAATGAGAAGTTTAAGA GCAAGGCCACCCTGACAGTGGATAAGAGCGCCTCTACCGCTTACATGGA GCTGTCCAGCCTGAGGTCTGAGGACACAGCCGTGTACTATTGTACCAGG AGCGATGGCCGGAACGACATGGATTCTTGGGGCCAGGGCACACTGGTGA CCGTGTCTTCCGCCAGCACAAAGGGCCCTTCCGTGTTCCCCCTGGCTCC TTGCTCCAGGAGCACATCTGAGTCCACCGCCGCTCTGGGCTGTCTGGTG AAGGACTACTTCCCAGAGCCCGTGACCGTGTCCTGGAATAGCGGCGCCC TGACATCCGGAGTGCACACCTTTCCAGCTGTGCTGCAGAGCTCTGGCCT GTACAGCCTGTCCAGCGTGGTGACAGTGCCCTCTTCCAGCCTGGGCACC AAGACATATACCTGCAACGTGGACCATAAGCCTTCTAATACCAAGGTGG ATAAGAGAGTGGAGTCCAAGTACGGACCACCTTGCCCTCCCTGTCCTGC TCCAGAGGCCGCCGGAGGACCTTCCGTGTTCCTGTTTCCACCCAAGCCA AAGGACACACTGATGATCTCTCGCACACCTGAGGTGACCTGCGTGGTGG TGGACGTGTCCCAGGAGGACCCCGAGGTGCAGTTCAACTGGTACGTGGA TGGCGTGGAGGTGCACAATGCTAAGACCAAGCCAAGGGAGGAGCAGTTT AACTCCACATACCGGGTGGTGAGCGTGCTGACCGTGCTGCATCAGGATT GGCTGAACGGCAAGGAGTATAAGTGCAAGGTGAGCAATAAGGGCCTGCC CTCTTCCATCGAGAAGACAATCTCTAAGGCTAAGGGACAGCCAAGGGAG CCACAGGTGTACACCCTGCCTCCAAGCCAGGAGGAGATGACAAAGAACC AGGTGTCTCTGACCTGTCTGGTGAAGGGCTTCTATCCATCTGACATCGC TGTGGAGTGGGAGTCCAATGGCCAGCCCGAGAACAATTACAAGACCACA CCCCCTGTGCTGGACTCTGATGGCTCCTTCTTTCTGTATTCTAGACTGA CCGTGGATAAGTCCCGCTGGCAGGAGGGCAACGTGTTCTCCTGCTCTGT GATGCACGAAGCACTGCACAATCATTACACTCAGAAGAGCCTGTCACTG TCCCTGGGCAAA |
| 311 | TNX10 VH-CH1-CH2-CH3 IgG1 (L234A/L235A) (with terminal K) | CAGGTCCAGCTGGTCCAGTCAGGTGCCGAAGTGGTCAAGCCCGGCGCCT CTGTGAAGCTGTCCTGCAAGGCTAGCGGCTACATCTTCACCTCCTACTA TATGTATTGGGTGAAGCAGGCTCCTGGACAGGGCCTGGAGTGGATCGGC GAGATCAACCCATCTAATGGCGACACCAACTTCAATGAGAAGTTTAAGT CCAAGGCCACCCTGACAGTGGATAAGAGCGCCTCTACAGCTTACATGGA GCTGTCCAGCCTGAGGAGCGAGGACACCGCCGTGTACTATTGCACAAGG TCTGATGGCCGGAACGACATGGATTCCTGGGGCCAGGGCACCCTGGTGA CAGTGTCTTCCGCCTCTACCAAGGGCCCTTCCGTGTTTCCACTGGCTCC CAGCTCTAAGTCCACCAGCGGAGGAACAGCCGCTCTGGGCTGTCTGGTG AAGGACTACTTCCCAGAGCCCGTGACAGTGAGCTGGAACTCTGGCGCCC TGACCAGCGGAGTGCACACATTTCCTGCTGTGCTGCAGTCCAGCGGCCT GTACTCTCTGTCTTCCGTGGTGACCGTGCCAAGCTCTTCCCTGGGCACC CAGACATATATCTGCAACGTGAATCACAAGCCAAGCAATACAAAGGTGG ACAAGAAGGTGGAGCCCAAGTCTTGTGATAAGACCCATACATGCCCCCC TTGTCCTGCTCCAGAGGCTGCTGGAGGACCATCCGTGTTCCTGTTTCCA CCCAAGCCTAAGGACACCCTGATGATCTCCAGAACCCCAGAGGTGACAT GCGTGGTGGTGGACGTGAGCCACGAGGACCCCGAGGTGAAGTTCAACTG GTACGTGGATGGCGTGGAGGTGCATAATGCTAAGACCAAGCCAAGAGAG GAGCAGTACAACAGCACCTATCGCGTGGTGTCTGTGCTGACAGTGCTGC ATCAGGACTGGCTGAACGGCAAGGAGTATAAGTGCAAGGTGTCTAATAA GGCCCTGCCCGCTCCTATCGAGAAGACCATCTCCAAGGCCAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCCAG CGATATCGCTGTGGAGTGGGAGTCTAATGGCCAGCCTGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTCTC CTGTTCCGTCATGCACGAGGCACTGCACAATCATTACACCCAGAAGTCA CTGTCACTGTCACCAGGAAAx |
| | x = A or G | |
| 312 | TNX11 VH-CH1-CH2-CH3 IgG1 (C226S, C229S, P238S) (with terminal K) | CAGGTCCAGCTGGTCCAGTCAGGTGCCGAAGTGGTCAAGCCCGGCGCCT CTGTGAAGCTGTCCTGCAAGGCTAGCGGCTACATCTTCACCTCCTACTA TATGTATTGGGTGAAGCAGGCTCCTGGACAGGGCCTGGAGTGGATCGGC GAGATCAACCCATCTAATGGCGACACCAACTTCAATGAGAAGTTTAAGT CCAAGGCCACCCTGACAGTGGATAAGAGCGCCTCTACAGCTTACATGGA GCTGTCCAGCCTGAGGAGCGAGGACACCGCCGTGTACTATTGCACAAGG TCTGATGGCCGGAACGACATGGATTCCTGGGGCCAGGGCACCCTGGTGA CAGTGTCTTCCGCCTCTACCAAGGGCCCTTCCGTGTTTCCACTGGCTCC CAGCTCTAAGTCCACCAGCGGAGGAACAGCCGCTCTGGGCTGTCTGGTG AAGGACTACTTCCCAGAGCCCGTGACAGTGAGCTGGAACTCTGGCGCCC TGACCAGCGGAGTGCACACATTTCCTGCTGTGCTGCAGTCCAGCGGCCT GTACTCTCTGTCTTCCGTGGTGACCGTGCCAAGCTCTTCCCTGGGCACC CAGACATATATCTGCAACGTGAATCACAAGCCAAGCAATACAAAGGTGG ACAAGAAGGTGGAGCCCAAGTCTTGTGATAAGACCCATACAtccCCCCC TTccCCTGCTCCAGAGCTGCTGGGAGGAAGCTCCGTGTTCCTGTTTCCA CCCAAGCCTAAGGACACCCTGATGATCTCCAGAACCCCAGAGGTGACAT GCGTGGTGGTGGACGTGAGCCACGAGGACCCCGAGGTGAAGTTCAACTG GTACGTGGATGGCGTGGAGGTGCATAATGCTAAGACCAAGCCAAGAGAG GAGCAGTACAACAGCACCTATCGCGTGGTGTCTGTGCTGACAGTGCTGC ATCAGGACTGGCTGAACGGCAAGGAGTATAAGTGCAAGGTGTCTAATAA GGCCCTGCCCGCTCCTATCGAGAAGACCATCTCCAAGGCCAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCCAG CGATATCGCTGTGGAGTGGGAGTCTAATGGCCAGCCTGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTCTC CTGTTCCGTCATGCACGAGGCACTGCACAATCATTACACCCAGAAGTCA CTGTCACTGTCACCAGGAAAx |
| | x = A or G | |
| 313 | TNX12 VH-CH1-CH2-CH3 IgG1 (C229S, P238S) (with terminal K) | CAGGTCCAGCTGGTCCAGTCAGGTGCCGAAGTGGTCAAGCCCGGCGCCT CTGTGAAGCTGTCCTGCAAGGCTAGCGGCTACATCTTCACCTCCTACTA TATGTATTGGGTGAAGCAGGCTCCTGGACAGGGCCTGGAGTGGATCGGC GAGATCAACCCATCTAATGGCGACACCAACTTCAATGAGAAGTTTAAGT CCAAGGCCACCCTGACAGTGGATAAGAGCGCCTCTACAGCTTACATGGA GCTGTCCAGCCTGAGGAGCGAGGACACCGCCGTGTACTATTGCACAAGG TCTGATGGCCGGAACGACATGGATTCCTGGGGCCAGGGCACCCTGGTGA CAGTGTCTTCCGCCTCTACCAAGGGCCCTTCCGTGTTTCCACTGGCTCC CAGCTCTAAGTCCACCAGCGGAGGAACAGCCGCTCTGGGCTGTCTGGTG AAGGACTACTTCCCAGAGCCCGTGACAGTGAGCTGGAACTCTGGCGCCC TGACCAGCGGAGTGCACACATTTCCTGCTGTGCTGCAGTCCAGCGGCCT GTACTCTCTGTCTTCCGTGGTGACCGTGCCAAGCTCTTCCCTGGGCACC CAGACATATATCTGCAACGTGAATCACAAGCCAAGCAATACAAAGGTGG ACAAGAAGGTGGAGCCCAAGTCTTGTGATAAGACCCATACATGCCCCCC TTCCCCTGCTCCAGAGCTGCTGGGAGGAAGcTCCGTGTTCCTGTTTCCA CCCAAGCCTAAGGACACCCTGATGATCTCCAGAACCCCAGAGGTGACAT GCGTGGTGGTGGACGTGAGCCACGAGGACCCCGAGGTGAAGTTCAACTG GTACGTGGATGGCGTGGAGGTGCATAATGCTAAGACCAAGCCAAGAGAG GAGCAGTACAACAGCACCTATCGCGTGGTGTCTGTGCTGACAGTGCTGC ATCAGGACTGGCTGAACGGCAAGGAGTATAAGTGCAAGGTGTCTAATAA GGCCCTGCCCGCTCCTATCGAGAAGACCATCTCCAAGGCCAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCCAG CGATATCGCTGTGGAGTGGGAGTCTAATGGCCAGCCTGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTCTC CTGTTCCGTCATGCACGAGGCACTGCACAATCATTACACCCAGAAGTCA CTGTCACTGTCACCAGGAAAx |
| | x = A or G | |
| 314 | TNX13 VH-CH1-CH2-CH3 IgG1 (C226S, P238S) (with terminal K) | CAGGTCCAGCTGGTCCAGTCAGGTGCCGAAGTGGTCAAGCCCGGCGCCT CTGTGAAGCTGTCCTGCAAGGCTAGCGGCTACATCTTCACCTCCTACTA TATGTATTGGGTGAAGCAGGCTCCTGGACAGGGCCTGGAGTGGATCGGC GAGATCAACCCATCTAATGGCGACACCAACTTCAATGAGAAGTTTAAGT CCAAGGCCACCCTGACAGTGGATAAGAGCGCCTCTACAGCTTACATGGA GCTGTCCAGCCTGAGGAGCGAGGACACCGCCGTGTACTATTGCACAAGG TCTGATGGCCGGAACGACATGGATTCCTGGGGCCAGGGCACCCTGGTGA CAGTGTCTTCCGCCTCTACCAAGGGCCCTTCCGTGTTTCCACTGGCTCC CAGCTCTAAGTCCACCAGCGGAGGAACAGCCGCTCTGGGCTGTCTGGTG AAGGACTACTTCCCAGAGCCCGTGACAGTGAGCTGGAACTCTGGCGCCC TGACCAGCGGAGTGCACACATTTCCTGCTGTGCTGCAGTCCAGCGGCCT GTACTCTCTGTCTTCCGTGGTGACCGTGCCAAGCTCTTCCCTGGGCACC CAGACATATATCTGCAACGTGAATCACAAGCCAAGCAATACAAAGGTGG ACAAGAAGGTGGAGCCCAAGTCTTGTGATAAGACCCATACATCCCCCCC TTGCCCTGCTCCAGAGCTGCTGGGAGGAAGCTCCGTGTTCCTGTTTCCA CCCAAGCCTAAGGACACCCTGATGATCTCCAGAACCCCAGAGGTGACAT GCGTGGTGGTGGACGTGAGCCACGAGGACCCCGAGGTGAAGTTCAACTG GTACGTGGATGGCGTGGAGGTGCATAATGCTAAGACCAAGCCAAGAGAG GAGCAGTACAACAGCACCTATCGCGTGGTGTCTGTGCTGACAGTGCTGC ATCAGGACTGGCTGAACGGCAAGGAGTATAAGTGCAAGGTGTCTAATAA GGCCCTGCCCGCTCCTATCGAGAAGACCATCTCCAAGGCCAAGGGCCAG CCTAGGGAGCCACAGGTGTACACACTGCCTCCATCCCGGGACGAGCTGA CCAAGAACCAGGTGAGCCTGACATGTCTGGTGAAGGGCTTCTATCCCAG CGATATCGCTGTGGAGTGGGAGTCTAATGGCCAGCCTGAGAACAATTAC AAGACCACACCCCCTGTGCTGGACTCCGATGGCAGCTTCTTTCTGTATT CCAAGCTGACCGTGGATAAGAGCCGCTGGCAGCAGGGCAACGTGTTCTC CTGTTCCGTCATGCACGAGGCACTGCACAATCATTACACCCAGAAGTCA CTGTCACTGTCACCAGGAAAx |
| | x = A or G | |
| 315 | TNX14 VH-CH1-CH2-CH3 IgG4 (L115T, S228P, L235A) (with terminal K) | CAGGTCCAGCTGGTCCAGTCAGGGGCTGAAGTGGTCAAAC CCGGCGCCAGCGTGAAGCTGTCTTGCAAGGCTTCCGGCTA CATCTTCACCAGCTACTATATGTATTGGGTGAAGCAGGCT CCAGGACAGGGCCTGGAGTGGATCGGCGAGATCAACCCTT CCAATGGCGACACAAACTTCAATGAGAAGTTTAAGAGCAA GGCCACCCTGACAGTGGATAAGAGCGCCTCTACCGCTTAC ATGGAGCTGTCCAGCCTGAGGTCTGAGGACACAGCCGTGT ACTATTGTACCAGGAGCGATGGCCGGAACGACATGGATTC TTGGGGCCAGGGCACAACAGTGACCGTGTCTTCCGCCAGC ACAAAGGGCCCTTCCGTGTTCCCCCTGGCTCCTTGCTCCA GGAGCACATCTGAGTCCACCGCCGCTCTGGGCTGTCTGGT GAAGGACTACTTCCCAGAGCCCGTGACCGTGTCCTGGAAT AGCGGCGCCCTGACATCCGGAGTGCACACCTTTCCAGCTG TGCTGCAGAGCTCTGGCCTGTACAGCCTGTCCAGCGTGGT GACAGTGCCCTCTTCCAGCCTGGGCACCAAGACATATACC TGCAACGTGGACCATAAGCCTTCTAATACCAAGGTGGATA AGAGAGTGGAGTCCAAGTACGGACCACCTTGCCCTCCTTG TCCTGCTCCAGAGTTCGCCGGAGGACCTTCCGTGTTCCTG TTTCCACCCAAGCCAAAGGACACACTGATGATCTCTCGCA CACCTGAGGTGACCTGCGTGGTGGTGGACGTGTCCCAGGA GGACCCCGAGGTGCAGTTCAACTGGTACGTGGATGGCGTG GAGGTGCACAATGCTAAGACCAAGCCAAGGGAGGAGCAGT TTAACTCCACATACCGGGTGGTGAGCGTGCTGACCGTGCT GCATCAGGATTGGCTGAACGGCAAGGAGTATAAGTGCAAG GTGAGCAATAAGGGCCTGCCCTCTTCCATCGAGAAGACAA TCTCTAAGGCTAAGGGACAGCCAAGGGAGCCACAGGTGTA CACCCTGCCTCCAAGCCAGGAGGAGATGACAAAGAACCAG GTGTCTCTGACCTGTCTGGTGAAGGGCTTCTATCCATCTG ACATCGCTGTGGAGTGGGAGTCCAATGGCCAGCCCGAGAA CAATTACAAGACCACACCCCCTGTGCTGGACTCTGATGGC TCCTTCTTTCTGTATTCTAGACTGACCGTGGATAAGTCCC GCTGGCAGGAGGGCAACGTGTTCTCCTGCTCTGTGATGCA CGAAGCACTGCACAATCATTACACTCAGAAGAGCCTGTCA CTGTCCCTGGGCAAA |

| **Heavy Chain DNA Sequence WITH LEADER; with terminal K** | | |
|---|---|---|
| 190 | TNX01 VH-CH1-CH2-CH3 IgG1(N297Q) (with terminal K) | ATGCCACTGCTGCTGCTGCTGCCACTGCTGTGGGCTGGCGCTCTGGCTC AGGTGCAGCTGGTGCAGTCTGGAGCTGAGGTGGTGAAGCCAGGCGCCTC TGTGAAGCTGTCCTGCAAGGCTAGCGGCTACATCTTCACCTCCTACTAT ATGTATTGGGTGAAGCAGGCTCCTGGACAGGGACTGGAGTGGATCGGCG AGATCAACCCATCTAATGGCGACACCAACTTCAATGAGAAGTTTAAGTC CAAGGCTACCCTGACAGTGGATAAGTCCGCCTCTACAGCCTACATGGAG CTGTCCAGCCTGAGAAGCGAGGACACCGCCGTGTACTATTGCACAAGGT CTGATGGCCGGAACGACATGGATTCCTGGGGCCAGGGCACCCTGGTGAC AGTGTCTTCCGCTTCTACCAAGGGCCCTTCCGTGTTTCCACTGGCTCCA AGCTCTAAGTCCACCAGCGGAGGAACAGCCGCTCTGGGCTGTCTGGTGA AGGACTATTTCCCTGAGCCAGTGACAGTGAGCTGGAACTCTGGCGCTCT GACCTCCGGCGTGCACACATTTCCAGCCGTGCTGCAGTCCAGCGGCCTG TACAGCCTGTCTTCCGTGGTGACCGTGCCAAGCTCTTCCCTGGGCACCC AGACATATATCTGCAACGTGAATCATAAGCCCTCTAATACAAAGGTGGA CAAGAAGGTGGAGCCCAAGTCTTGTGATAAAACACATACTTGCCCCCCT TGTCCTGCACCAGAACTGCTGGGAGGACCTAGCGTGTTCCTGTTTCCAC CCAAGCCAAAAGACACCCTGATGATTAGTAGAACCCCTGAGGTCACATG CGTGGTCGTGGACGTGAGCCACGAGGACCCCGAGGTGAAGTTCAACTGG TACGTGGACGGCGTGGAGGTGCACAATGCTAAGACCAAGCCCAGAGAGG AGCAGTACCAGAGCACCTATCGCGTGGTGTCTGTGCTGACAGTGCTGCA TCAGGATTGGCTGAACGGCAAGGAGTATAAGTGCAAGGTGAGCAATAAG GCTCTGCCCGCCCCTATCGAGAAGACCATCTCTAAGGCTAAGGGCCAGC CTAGGGAGCCACAGGTGTACACACTGCCCCCTTCCCGGGACGAGCTGAC CAAGAACCAGGTGAGCCTGACATGTCTGGTGAAGGGCTTCTATCCCAGC GATATCGCCGTGGAGTGGGAGTCTAATGGCCAGCCTGAGAACAATTACA AGACCACACCACCCGTGCTGGACTCCGATGGCAGCTTCTTTCTGTATTC CAAGCTGACCGTGGATAAGAGCCGCTGGCAGCAGGGCAACGTGTTTTCT TGTTCCGTGATGCACGAGGCCCTGCACAATCATTACACACAGAAGAGCC TGTCTCTGTCCCCTGGCAAG |
| 191 | TNX02 VH-CH1-CH2-CH3 IgG1 (wt) (with terminal K) | ATGCCCCTGCTGCTGCTGCTGCCTCTGCTGTGGGCTGGCGCTCTGGCTC AGGTGCAGCTGGTGCAGTCCGGAGCTGAGGTGGTGAAGCCAGGAGCCTC TGTGAAGCTGTCCTGCAAGGCTAGCGGCTACATCTTCACCAGCTACTAT ATGTATTGGGTGAAGCAGGCTCCAGGACAGGGCCTGGAGTGGATCGGCG AGATCAACCCTTCTAATGGCGACACCAACTTCAATGAGAAGTTTAAGTC CAAGGCTACCCTGACAGTGGATAAGTCCGCCTCTACAGCCTACATGGAG CTGTCCAGCCTGAGGTCTGAGGACACCGCCGTGTACTATTGCACAAGGT CTGATGGCCGGAACGACATGGATTCCTGGGGCCAGGGCACCCTGGTGAC AGTGTCTTCCGCTTCTACCAAGGGACCATCCGTGTTTCCACTGGCTCCA AGCTCTAAGTCCACCAGCGGAGGAACAGCCGCTCTGGGCTGTCTGGTGA AGGACTATTTCCCAGAGCCCGTGACAGTGAGCTGGAACTCTGGCGCTCT GACCAGCGGCGTGCACACATTTCCAGCCGTGCTGCAGTCCAGCGGCCTG TACTCTCTGTCTTCCGTGGTGACCGTGCCTAGCTCTTCCCTGGGCACCC AGACATATATCTGCAACGTGAATCACAAGCCTAGCAATACAAAGGTGGA CAAGAAGGTGGAGCCAAAGTCTTGTGATAAGACCCATACATGCCCCCCT TGTCCTGCTCCAGAGCTGCTGGGAGGACCATCCGTGTTCCTGTTTCCAC CCAAGCCCAAGGACACCCTGATGATCTCCAGAACCCCTGAGGTGACATG CGTGGTGGTGGACGTGAGCCACGAGGACCCCGAGGTGAAGTTCAACTGG TACGTGGATGGCGTGGAGGTGCATAATGCCAAGACCAAGCCTAGAGAGG AGCAGTACAATAGCACCTATCGCGTGGTGTCTGTGCTGACAGTGCTGCA CCAGGACTGGCTGAACGGCAAGGAGTATAAGTGCAAGGTGTCTAATAAG GCTCTGCCCGCCCCTATCGAGAAGACCATCTCCAAGGCTAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCAAGC GATATCGCCGTGGAGTGGGAGTCTAATGGCCAGCCCGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTTTCT TGTTCCGTGATGCATGAGGCTCTGCACAATCATTACACACAGAAGAGCC TGTCTCTGTCCCCAGGCAAG |
| 192 | TNX03 VH-CH1-CH2-CH3 IgG1 (N297G) (with terminal K) | ATGCCCCTGCTGCTGCTGCTGCCTCTGCTGTGGGCTGGCGCTCTGGCTC AGGTGCAGCTGGTGCAGTCCGGAGCTGAGGTGGTGAAGCCAGGAGCCTC TGTGAAGCTGTCCTGCAAGGCTAGCGGCTACATCTTCACCAGCTACTAT ATGTATTGGGTGAAGCAGGCTCCAGGACAGGGCCTGGAGTGGATCGGCG AGATCAACCCTTCTAATGGCGACACCAACTTCAATGAGAAGTTTAAGTC CAAGGCTACCCTGACAGTGGATAAGTCCGCCTCTACAGCCTACATGGAG CTGTCCAGCCTGAGGTCTGAGGACACCGCCGTGTACTATTGCACAAGGT CTGATGGCCGGAACGACATGGATTCCTGGGGCCAGGGCACCCTGGTGAC AGTGTCTTCCGCTTCTACCAAGGGACCATCCGTGTTTCCACTGGCTCCA AGCTCTAAGTCCACCAGCGGAGGAACAGCCGCTCTGGGCTGTCTGGTGA AGGACTATTTCCCAGAGCCCGTGACAGTGAGCTGGAACTCTGGCGCTCT GACCAGCGGCGTGCACACATTTCCAGCCGTGCTGCAGTCCAGCGGCCTG TACTCTCTGTCTTCCGTGGTGACCGTGCCTAGCTCTTCCCTGGGCACCC AGACATATATCTGCAACGTGAATCACAAGCCTAGCAATACAAAGGTGGA CAAGAAGGTGGAGCCAAAGTCTTGTGATAAGACCCATACATGCCCCCCT TGTCCTGCTCCAGAGCTGCTGGGAGGACCATCCGTGTTCCTGTTTCCAC CCAAGCCCAAGGACACCCTGATGATCTCCAGAACCCCTGAGGTGACATG CGTGGTGGTGGACGTGAGCCACGAGGACCCCGAGGTGAAGTTCAACTGG TACGTGGATGGCGTGGAGGTGCATAATGCCAAGACCAAGCCTAGAGAGG AGCAGTACGGCAGCACCTATCGCGTGGTGTCTGTGCTGACAGTGCTGCA CCAGGACTGGCTGAACGGCAAGGAGTATAAGTGCAAGGTGTCTAATAAG GCTCTGCCCGCCCCTATCGAGAAGACCATCTCCAAGGCTAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCAAGC GATATCGCCGTGGAGTGGGAGTCTAATGGCCAGCCCGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTTTCT TGTTCCGTGATGCATGAGGCTCTGCACAATCATTACACACAGAAGAGCC TGTCTCTGTCCCCAGGCAAG |
| 193 | TNX04 VH-CH1-CH2-CH3 IgG1 (C220S, C226S, C229S, P238S) (with terminal K) | ATGCCCCTGCTGCTGCTGCTGCCTCTGCTGTGGGCTGGCGCTCTGGCTC AGGTGCAGCTGGTGCAGAGCGGAGCTGAGGTGGTGAAGCCAGGAGCCTC TGTGAAGCTGTCCTGCAAGGCTAGCGGCTACATCTTCACCTCTTACTAT ATGTATTGGGTGAAGCAGGCTCCAGGACAGGGCCTGGAGTGGATCGGCG AGATCAACCCTTCTAATGGCGACACCAACTTCAATGAGAAGTTTAAGTC CAAGGCTACCCTGACAGTGGATAAGTCTGCTTCCACAGCCTACATGGAG CTGTCCAGCCTGAGGTCCGAGGACACCGCCGTGTACTATTGCACAAGGT CTGATGGCCGGAACGACATGGATTCCTGGGGCCAGGGCACCCTGGTGAC AGTGTCTTCCGCTTCTACCAAGGGACCATCCGTGTTTCCACTGGCTCCA AGCTCTAAGAGCACCTCTGGAGGAACAGCCGCTCTGGGATGTCTGGTGA AGGACTATTTCCCAGAGCCCGTGACAGTGTCTTGGAACTCCGGCGCTCT GACCTCTGGCGTGCACACATTTCCTGCCGTGCTGCAGTCCAGCGGCCTG TACTCCCTGTCTTCCGTGGTGACCGTGCCAAGCTCTTCCCTGGGCACCC AGACATATATCTGCAACGTGAATCACAAGCCTTCCAATACAAAGGTGGA CAAGAAGGTGGAGCCAAAGAGCTCTGATAAGACCCATACAAGCCCCCCT TCTCCTGCTCCAGAGCTGCTGGGAGGCTCCAGCGTGTTCCTGTTTCCAC CCAAGCCAAAGGACACCCTGATGATCAGCAGAACCCCCGAGGTGACATG CGTGGTGGTGGACGTGTCTCACGAGGACCCCGAGGTGAAGTTCAACTGG TACGTGGATGGCGTGGAGGTGCATAATGCCAAGACCAAGCCTAGAGAGG AGCAGTACAATAGCACCTATCGCGTGGTGTCTGTGCTGACAGTGCTGCA CCAGGACTGGCTGAACGGCAAGGAGTATAAGTGCAAGGTGTCCAATAAG GCTCTGCCCGCCCCTATCGAGAAGACCATCAGCAAGGCTAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCAAGC GATATCGCCGTGGAGTGGGAGTCTAATGGCCAGCCCGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTTTCC TGTAGCGTGATGCATGAGGCTCTGCACAATCATTACACACAGAAGTCTC TGTCCCTGAGCCCTGGCAAG |
| 194 | TNX05 VH-CH1-CH2-CH3 IgG4 (S228P, L235A) (with terminal K) | ATGCCCCTGCTGCTGCTGCTGCCTCTGCTGTGGGCTGGCGCTCTGGCTC AGGTGCAGCTGGTGCAGTCTGGAGCTGAGGTGGTGAAGCCTGGAGCTAG CGTGAAGCTGTCTTGCAAGGCTTCCGGCTACATCTTCACCTCCTACTAT ATGTATTGGGTGAAGCAGGCTCCTGGACAGGGCCTGGAGTGGATCGGCG AGATCAACCCATCCAATGGCGACACAAACTTCAATGAGAAGTTTAAGAG CAAGGCTACCCTGACAGTGGATAAGTCCGCCTCTACCGCCTACATGGAG CTGTCCAGCCTGAGGAGCGAGGACACAGCCGTGTACTATTGTACCAGGA GCGATGGCCGGAACGACATGGATTCTTGGGGCCAGGGCACACTGGTGAC CGTGTCTTCCGCTAGCACAAAGGGACCATCCGTGTTCCCACTGGCTCCA TGCTCCAGGAGCACATCTGAGTCCACCGCCGCTCTGGGCTGTCTGGTGA AGGACTATTTCCCTGAGCCAGTGACCGTGTCCTGGAATAGCGGCGCTCT GACATCCGGAGTGCACACCTTTCCAGCCGTGCTGCAGAGCTCTGGCCTG TACAGCCTGTCCAGCGTGGTGACAGTGCCTTCTTCCAGCCTGGGCACCA AGACATATACCTGCAACGTGGACCATAAGCCATCTAATACCAAGGTGGA TAAGAGAGTGGAGTCCAAGTACGGACCACCTTGCCCACCATGTCCAGCT CCTGAGTTCGCTGGAGGACCATCCGTGTTCCTGTTTCCTCCAAAGCCCA AGGACACCCTGATGATCTCTCGCACACCAGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCCAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCT TCTTCCATCGAGAAGACAATCTCTAAGGCTAAGGGCCAGCCTCGGGAGC CACAGGTGTACACCCTGCCCCCTAGCCAGGAGGAGATGACAAAGAACCA GGTGTCTCTGACCTGTCTGGTGAAGGGCTTCTATCCCTCCGACATCGCC GTGGAGTGGGAGAGCAATGGCCAGCCTGAGAACAATTACAAGACCACAC CACCCGTGCTGGACTCTGATGGCTCCTTCTTTCTGTATTCTAGACTGAC CGTCGATAAGTCCCGCTGGCAGGAGGGCAACGTGTTTAGCTGCTCTGTG ATGCACGAGGCCCTGCACAATCATTACACCCAGAAGTCCCTGAGCCTGT CTCTGGGCAAG |
| 316 | TNX06 VH-CH1-CH2-CH3 IgG4 (wild type) (with terminal K) | ATGCCTCTGCTGCTGCTGCTGCCTCTGCTGTGGGCCGGTGCTCTGGCTC AGGTCCAGCTGGTCCAGTCAGGGGCTGAAGTGGTCAAACCCGGCGCCAG CGTGAAGCTGTCTTGCAAGGCTTCCGGCTACATCTTCACCAGCTACTAT ATGTATTGGGTGAAGCAGGCTCCAGGACAGGGCCTGGAGTGGATCGGCG AGATCAACCCTTCCAATGGCGACACAAACTTCAATGAGAAGTTTAAGAG CAAGGCCACCCTGACAGTGGATAAGAGCGCCTCTACCGCTTACATGGAG CTGTCCAGCCTGAGGTCTGAGGACACAGCCGTGTACTATTGTACCAGGA GCGATGGCCGGAACGACATGGATTCTTGGGGCCAGGGCACACTGGTGAC CGTGTCTTCCGCCAGCACAAAGGGCCCTTCCGTGTTCCCCCTGGCTCCT TGCTCCAGGAGCACATCTGAGTCCACCGCCGCTCTGGGCTGTCTGGTGA AGGACTACTTCCCAGAGCCCGTGACCGTGTCCTGGAATAGCGGCGCCCT GACATCCGGAGTGCACACCTTTCCAGCTGTGCTGCAGAGCTCTGGCCTG TACAGCCTGTCCAGCGTGGTGACAGTGCCCTCTTCCAGCCTGGGCACCA AGACATATACCTGCAACGTGGACCATAAGCCTTCTAATACCAAGGTGGA TAAGAGAGTGGAGTCCAAGTACGGACCACCTTGCCCTAGCTGTCCTGCT CCAGAGTTCCTGGGAGGACCTTCCGTGTTCCTGTTTCCACCCAAGCCAA AGGACACACTGATGATCTCTCGCACACCTGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCAAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCC TCTTCCATCGAGAAGACAATCTCTAAGGCTAAGGGACAGCCAAGGGAGC CACAGGTGTACACCCTGCCTCCAAGCCAGGAGGAGATGACAAAGAACCA GGTGTCTCTGACCTGTCTGGTGAAGGGCTTCTATCCATCTGACATCGCT GTGGAGTGGGAGTCCAATGGCCAGCCCGAGAACAATTACAAGACCACAC CCCCTGTGCTGGACTCTGATGGCTCCTTCTTTCTGTATTCTAGACTGAC CGTGGATAAGTCCCGCTGGCAGGAGGGCAACGTGTTCTCCTGCTCTGTG ATGCACGAAGCACTGCACAATCATTACACTCAGAAGAGCCTGTCACTGT CCCTGGGCAAA |
| 317 | TNX07 VH-CH1-CH2-CH3 IgG4 (S228P) (with terminal K) | ATGCCTCTGCTGCTGCTGCCTCTGCTGTGGGCCGGTGCTCTGGCTC AGGTCCAGCTGGTCCAGTCAGGGGCTGAAGTGGTCAAACCCGGCGCCAG CGTGAAGCTGTCTTGCAAGGCTTCCGGCTACATCTTCACCAGCTACTAT ATGTATTGGGTGAAGCAGGCTCCAGGACAGGGCCTGGAGTGGATCGGCG AGATCAACCCTTCCAATGGCGACACAAACTTCAATGAGAAGTTTAAGAG CAAGGCCACCCTGACAGTGGATAAGAGCGCCTCTACCGCTTACATGGAG CTGTCCAGCCTGAGGTCTGAGGACACAGCCGTGTACTATTGTACCAGGA GCGATGGCCGGAACGACATGGATTCTTGGGGCCAGGGCACACTGGTGAC CGTGTCTTCCGCCAGCACAAAGGGCCCTTCCGTGTTCCCCCTGGCTCCT TGCTCCAGGAGCACATCTGAGTCCACCGCCGCTCTGGGCTGTCTGGTGA AGGACTACTTCCCAGAGCCCGTGACCGTGTCCTGGAATAGCGGCGCCCT GACATCCGGAGTGCACACCTTTCCAGCTGTGCTGCAGAGCTCTGGCCTG TACAGCCTGTCCAGCGTGGTGACAGTGCCCTCTTCCAGCCTGGGCACCA AGACATATACCTGCAACGTGGACCATAAGCCTTCTAATACCAAGGTGGA TAAGAGAGTGGAGTCCAAGTACGGACCACCTTGCCCTCCCTGTCCTGCT CCAGAGTTCCTGGGAGGACCTTCCGTGTTCCTGTTTCCACCCAAGCCAA AGGACACACTGATGATCTCTCGCACACCTGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCAAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCC TCTTCCATCGAGAAGACAATCTCTAAGGCTAAGGGACAGCCAAGGGAGC CACAGGTGTACACCCTGCCTCCAAGCCAGGAGGAGATGACAAAGAACCA GGTGTCTCTGACCTGTCTGGTGAAGGGCTTCTATCCATCTGACATCGCT GTGGAGTGGGAGTCCAATGGCCAGCCCGAGAACAATTACAAGACCACAC CCCCTGTGCTGGACTCTGATGGCTCCTTCTTTCTGTATTCTAGACTGAC CGTGGATAAGTCCCGCTGGCAGGAGGGCAACGTGTTCTCCTGCTCTGTG ATGCACGAAGCACTGCACAATCATTACACTCAGAAGAGCCTGTCACTGT CCCTGGGCAAA |
| 318 | TNX08 VH-CH1-CH2-CH3 (IgG4 S228P/L235E) (with terminal K) | ATGCCTCTGCTGCTGCTGCTGCCTCTGCTGTGGGCCGGTGCTCTGGCTC AGGTCCAGCTGGTCCAGTCAGGGGCTGAAGTGGTCAAACCCGGCGCCAG CGTGAAGCTGTCTTGCAAGGCTTCCGGCTACATCTTCACCAGCTACTAT ATGTATTGGGTGAAGCAGGCTCCAGGACAGGGCCTGGAGTGGATCGGCG AGATCAACCCTTCCAATGGCGACACAAACTTCAATGAGAAGTTTAAGAG CAAGGCCACCCTGACAGTGGATAAGAGCGCCTCTACCGCTTACATGGAG CTGTCCAGCCTGAGGTCTGAGGACACAGCCGTGTACTATTGTACCAGGA GCGATGGCCGGAACGACATGGATTCTTGGGGCCAGGGCACACTGGTGAC CGTGTCTTCCGCCAGCACAAAGGGCCCTTCCGTGTTCCCCCTGGCTCCT TGCTCCAGGAGCACATCTGAGTCCACCGCCGCTCTGGGCTGTCTGGTGA AGGACTACTTCCCAGAGCCCGTGACCGTGTCCTGGAATAGCGGCGCCCT GACATCCGGAGTGCACACCTTTCCAGCTGTGCTGCAGAGCTCTGGCCTG TACAGCCTGTCCAGCGTGGTGACAGTGCCCTCTTCCAGCCTGGGCACCA AGACATATACCTGCAACGTGGACCATAAGCCTTCTAATACCAAGGTGGA TAAGAGAGTGGAGTCCAAGTACGGACCACCTTGCCCTCCCTGTCCTGCT CCAGAGTTCGAGGGAGGACCTTCCGTGTTCCTGTTTCCACCCAAGCCAA AGGACACACTGATGATCTCTCGCACACCTGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCAAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCC TCTTCCATCGAGAAGACAATCTCTAAGGCTAAGGGACAGCCAAGGGAGC CACAGGTGTACACCCTGCCTCCAAGCCAGGAGGAGATGACAAAGAACCA GGTGTCTCTGACCTGTCTGGTGAAGGGCTTCTATCCATCTGACATCGCT GTGGAGTGGGAGTCCAATGGCCAGCCCGAGAACAATTACAAGACCACAC CCCCTGTGCTGGACTCTGATGGCTCCTTCTTTCTGTATTCTAGACTGAC CGTGGATAAGTCCCGCTGGCAGGAGGGCAACGTGTTCTCCTGCTCTGTG ATGCACGAAGCACTGCACAATCATTACACTCAGAAGAGCCTGTCACTGT CCCTGGGCAAA |
| 319 | TNX09 VH-CH1-CH2-CH3 IgG4 (S228P/F234A/ L235A) (with terminal K) | ATGCCTCTGCTGCTGCTGCTGCCTCTGCTGTGGGCCGGTGCTCTGGCTC AGGTCCAGCTGGTCCAGTCAGGGGCTGAAGTGGTCAAACCCGGCGCCAG CGTGAAGCTGTCTTGCAAGGCTTCCGGCTACATCTTCACCAGCTACTAT ATGTATTGGGTGAAGCAGGCTCCAGGACAGGGCCTGGAGTGGATCGGCG AGATCAACCCTTCCAATGGCGACACAAACTTCAATGAGAAGTTTAAGAG CAAGGCCACCCTGACAGTGGATAAGAGCGCCTCTACCGCTTACATGGAG CTGTCCAGCCTGAGGTCTGAGGACACAGCCGTGTACTATTGTACCAGGA GCGATGGCCGGAACGACATGGATTCTTGGGGCCAGGGCACACTGGTGAC CGTGTCTTCCGCCAGCACAAAGGGCCCTTCCGTGTTCCCCCTGGCTCCT TGCTCCAGGAGCACATCTGAGTCCACCGCCGCTCTGGGCTGTCTGGTGA AGGACTACTTCCCAGAGCCCGTGACCGTGTCCTGGAATAGCGGCGCCCT GACATCCGGAGTGCACACCTTTCCAGCTGTGCTGCAGAGCTCTGGCCTG TACAGCCTGTCCAGCGTGGTGACAGTGCCCTCTTCCAGCCTGGGCACCA AGACATATACCTGCAACGTGGACCATAAGCCTTCTAATACCAAGGTGGA TAAGAGAGTGGAGTCCAAGTACGGACCACCTTGCCCTCCCTGTCCTGCT CCAGAGGCCGCCGGAGGACCTTCCGTGTTCCTGTTTCCACCCAAGCCAA AGGACACACTGATGATCTCTCGCACACCTGAGGTGACCTGCGTGGTGGT GGACGTGTCCCAGGAGGACCCCGAGGTGCAGTTCAACTGGTACGTGGAT GGCGTGGAGGTGCACAATGCTAAGACCAAGCCAAGGGAGGAGCAGTTTA ACTCCACATACCGGGTGGTGAGCGTGCTGACCGTGCTGCATCAGGATTG GCTGAACGGCAAGGAGTATAAGTGCAAGGTGAGCAATAAGGGCCTGCCC TCTTCCATCGAGAAGACAATCTCTAAGGCTAAGGGACAGCCAAGGGAGC CACAGGTGTACACCCTGCCTCCAAGCCAGGAGGAGATGACAAAGAACCA GGTGTCTCTGACCTGTCTGGTGAAGGGCTTCTATCCATCTGACATCGCT GTGGAGTGGGAGTCCAATGGCCAGCCCGAGAACAATTACAAGACCACAC CCCCTGTGCTGGACTCTGATGGCTCCTTCTTTCTGTATTCTAGACTGAC CGTGGATAAGTCCCGCTGGCAGGAGGGCAACGTGTTCTCCTGCTCTGTG ATGCACGAAGCACTGCACAATCATTACACTCAGAAGAGCCTGTCACTGT CCCTGGGCAAA |
| 320 | TNX10 VH-CH1-CH2-CH3 IgG1 (L234A/L235A) (with terminal K) | ATGCCTCTGCTGCTGCTGCTGCCTCTGCTGTGGGCCGGTGCCCTGGCTC AGGTCCAGCTGGTCCAGTCAGGTGCCGAAGTGGTCAAGCCCGGCGCCTC TGTGAAGCTGTCCTGCAAGGCTAGCGGCTACATCTTCACCTCCTACTAT ATGTATTGGGTGAAGCAGGCTCCTGGACAGGGCCTGGAGTGGATCGGCG AGATCAACCCATCTAATGGCGACACCAACTTCAATGAGAAGTTTAAGTC CAAGGCCACCCTGACAGTGGATAAGAGCGCCTCTACAGCTTACATGGAG CTGTCCAGCCTGAGGAGCGAGGACACCGCCGTGTACTATTGCACAAGGT CTGATGGCCGGAACGACATGGATTCCTGGGGCCAGGGCACCCTGGTGAC AGTGTCTTCCGCCTCTACCAAGGGCCCTTCCGTGTTTCCACTGGCTCCC AGCTCTAAGTCCACCAGCGGAGGAACAGCCGCTCTGGGCTGTCTGGTGA AGGACTACTTCCCAGAGCCCGTGACAGTGAGCTGGAACTCTGGCGCCCT GACCAGCGGAGTGCACACATTTCCTGCTGTGCTGCAGTCCAGCGGCCTG TACTCTCTGTCTTCCGTGGTGACCGTGCCAAGCTCTTCCCTGGGCACCC AGACATATATCTGCAACGTGAATCACAAGCCAAGCAATACAAAGGTGGA CAAGAAGGTGGAGCCCAAGTCTTGTGATAAGACCCATACATGCCCCCCT TGTCCTGCTCCAGAGGCTGCTGGAGGACCATCCGTGTTCCTGTTTCCAC CCAAGCCTAAGGACACCCTGATGATCTCCAGAACCCCAGAGGTGACATG CGTGGTGGTGGACGTGAGCCACGAGGACCCCGAGGTGAAGTTCAACTGG TACGTGGATGGCGTGGAGGTGCATAATGCTAAGACCAAGCCAAGAGAGG AGCAGTACAACAGCACCTATCGCGTGGTGTCTGTGCTGACAGTGCTGCA TCAGGACTGGCTGAACGGCAAGGAGTATAAGTGCAAGGTGTCTAATAAG GCCCTGCCCGCTCCTATCGAGAAGACCATCTCCAAGGCCAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCCAGC GATATCGCTGTGGAGTGGGAGTCTAATGGCCAGCCTGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTCTCC TGTTCCGTCATGCACGAGGCACTGCACAATCATTACACCCAGAAGTCAC TGTCACTGTCACCAGGAAAx |
| | x = A or G | |
| 321 | TNX11 VH-CH1-CH2-CH3 IgG1 (C226S, C229S, P238S) (with terminal K) | ATGCCTCTGCTGCTGCTGCTGCCTCTGCTGTGGGCCGGTGCCCTGGCTC AGGTCCAGCTGGTCCAGTCAGGTGCCGAAGTGGTCAAGCCCGGCGCCTC TGTGAAGCTGTCCTGCAAGGCTAGCGGCTACATCTTCACCTCCTACTAT ATGTATTGGGTGAAGCAGGCTCCTGGACAGGGCCTGGAGTGGATCGGCG AGATCAACCCATCTAATGGCGACACCAACTTCAATGAGAAGTTTAAGTC CAAGGCCACCCTGACAGTGGATAAGAGCGCCTCTACAGCTTACATGGAG CTGTCCAGCCTGAGGAGCGAGGACACCGCCGTGTACTATTGCACAAGGT CTGATGGCCGGAACGACATGGATTCCTGGGGCCAGGGCACCCTGGTGAC AGTGTCTTCCGCCTCTACCAAGGGCCCTTCCGTGTTTCCACTGGCTCCC AGCTCTAAGTCCACCAGCGGAGGAACAGCCGCTCTGGGCTGTCTGGTGA AGGACTACTTCCCAGAGCCCGTGACAGTGAGCTGGAACTCTGGCGCCCT GACCAGCGGAGTGCACACATTTCCTGCTGTGCTGCAGTCCAGCGGCCTG TACTCTCTGTCTTCCGTGGTGACCGTGCCAAGCTCTTCCCTGGGCACCC AGACATATATCTGCAACGTGAATCACAAGCCAAGCAATACAAAGGTGGA CAAGAAGGTGGAGCCCAAGTCTTGTGATAAGACCCATACAtccCCCCCT TccCCTGCTCCAGAGCTGCTGGGAGGAAGCTCCGTGTTCCTGTTTCCAC CCAAGCCTAAGGACACCCTGATGATCTCCAGAACCCCAGAGGTGACATG CGTGGTGGTGGACGTGAGCCACGAGGACCCCGAGGTGAAGTTCAACTGG TACGTGGATGGCGTGGAGGTGCATAATGCTAAGACCAAGCCAAGAGAGG AGCAGTACAACAGCACCTATCGCGTGGTGTCTGTGCTGACAGTGCTGCA TCAGGACTGGCTGAACGGCAAGGAGTATAAGTGCAAGGTGTCTAATAAG GCCCTGCCCGCTCCTATCGAGAAGACCATCTCCAAGGCCAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCCAGC GATATCGCTGTGGAGTGGGAGTCTAATGGCCAGCCTGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTCTCC TGTTCCGTCATGCACGAGGCACTGCACAATCATTACACCCAGAAGTCAC TGTCACTGTCACCAGGAAAx |
| | x = A or G | |
| 322 | TNX12 VH-CH1-CH2-CH3 IgG1 (C229S, P238S) (with terminal K) | ATGCCTCTGCTGCTGCTGCTGCCTCTGCTGTGGGCCGGTGCCCTGGCTC AGGTCCAGCTGGTCCAGTCAGGTGCCGAAGTGGTCAAGCCCGGCGCCTC TGTGAAGCTGTCCTGCAAGGCTAGCGGCTACATCTTCACCTCCTACTAT ATGTATTGGGTGAAGCAGGCTCCTGGACAGGGCCTGGAGTGGATCGGCG AGATCAACCCATCTAATGGCGACACCAACTTCAATGAGAAGTTTAAGTC CAAGGCCACCCTGACAGTGGATAAGAGCGCCTCTACAGCTTACATGGAG CTGTCCAGCCTGAGGAGCGAGGACACCGCCGTGTACTATTGCACAAGGT CTGATGGCCGGAACGACATGGATTCCTGGGGCCAGGGCACCCTGGTGAC AGTGTCTTCCGCCTCTACCAAGGGCCCTTCCGTGTTTCCACTGGCTCCC AGCTCTAAGTCCACCAGCGGAGGAACAGCCGCTCTGGGCTGTCTGGTGA AGGACTACTTCCCAGAGCCCGTGACAGTGAGCTGGAACTCTGGCGCCCT GACCAGCGGAGTGCACACATTTCCTGCTGTGCTGCAGTCCAGCGGCCTG TACTCTCTGTCTTCCGTGGTGACCGTGCCAAGCTCTTCCCTGGGCACCC AGACATATATCTGCAACGTGAATCACAAGCCAAGCAATACAAAGGTGGA CAAGAAGGTGGAGCCCAAGTCTTGTGATAAGACCCATACATGCCCCCCT TCCCCTGCTCCAGAGCTGCTGGGAGGAAGcTCCGTGTTCCTGTTTCCAC CCAAGCCTAAGGACACCCTGATGATCTCCAGAACCCCAGAGGTGACATG CGTGGTGGTGGACGTGAGCCACGAGGACCCCGAGGTGAAGTTCAACTGG TACGTGGATGGCGTGGAGGTGCATAATGCTAAGACCAAGCCAAGAGAGG AGCAGTACAACAGCACCTATCGCGTGGTGTCTGTGCTGACAGTGCTGCA TCAGGACTGGCTGAACGGCAAGGAGTATAAGTGCAAGGTGTCTAATAAG GCCCTGCCCGCTCCTATCGAGAAGACCATCTCCAAGGCCAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCCAGC GATATCGCTGTGGAGTGGGAGTCTAATGGCCAGCCTGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTCTCC TGTTCCGTCATGCACGAGGCACTGCACAATCATTACACCCAGAAGTCAC TGTCACTGTCACCAGGAAAx |
| | x = A or G | |
| 323 | TNX13 VH-CH1-CH2-CH3 IgG1 (C226S, P238S) (with terminal K) | ATGCCTCTGCTGCTGCTGCTGCCTCTGCTGTGGGCCGGTGCCCTGGCTC AGGTCCAGCTGGTCCAGTCAGGTGCCGAAGTGGTCAAGCCCGGCGCCTC TGTGAAGCTGTCCTGCAAGGCTAGCGGCTACATCTTCACCTCCTACTAT ATGTATTGGGTGAAGCAGGCTCCTGGACAGGGCCTGGAGTGGATCGGCG AGATCAACCCATCTAATGGCGACACCAACTTCAATGAGAAGTTTAAGTC CAAGGCCACCCTGACAGTGGATAAGAGCGCCTCTACAGCTTACATGGAG CTGTCCAGCCTGAGGAGCGAGGACACCGCCGTGTACTATTGCACAAGGT CTGATGGCCGGAACGACATGGATTCCTGGGGCCAGGGCACCCTGGTGAC AGTGTCTTCCGCCTCTACCAAGGGCCCTTCCGTGTTTCCACTGGCTCCC AGCTCTAAGTCCACCAGCGGAGGAACAGCCGCTCTGGGCTGTCTGGTGA AGGACTACTTCCCAGAGCCCGTGACAGTGAGCTGGAACTCTGGCGCCCT GACCAGCGGAGTGCACACATTTCCTGCTGTGCTGCAGTCCAGCGGCCTG TACTCTCTGTCTTCCGTGGTGACCGTGCCAAGCTCTTCCCTGGGCACCC AGACATATATCTGCAACGTGAATCACAAGCCAAGCAATACAAAGGTGGA CAAGAAGGTGGAGCCCAAGTCTTGTGATAAGACCCATACATCCCCCCCT TGCCCTGCTCCAGAGCTGCTGGGAGGAAGCTCCGTGTTCCTGTTTCCAC CCAAGCCTAAGGACACCCTGATGATCTCCAGAACCCCAGAGGTGACATG CGTGGTGGTGGACGTGAGCCACGAGGACCCCGAGGTGAAGTTCAACTGG TACGTGGATGGCGTGGAGGTGCATAATGCTAAGACCAAGCCAAGAGAGG AGCAGTACAACAGCACCTATCGCGTGGTGTCTGTGCTGACAGTGCTGCA TCAGGACTGGCTGAACGGCAAGGAGTATAAGTGCAAGGTGTCTAATAAG GCCCTGCCCGCTCCTATCGAGAAGACCATCTCCAAGGCCAAGGGCCAGC CTAGGGAGCCACAGGTGTACACACTGCCTCCATCCCGGGACGAGCTGAC CAAGAACCAGGTGAGCCTGACATGTCTGGTGAAGGGCTTCTATCCCAGC GATATCGCTGTGGAGTGGGAGTCTAATGGCCAGCCTGAGAACAATTACA AGACCACACCCCCTGTGCTGGACTCCGATGGCAGCTTCTTTCTGTATTC CAAGCTGACCGTGGATAAGAGCCGCTGGCAGCAGGGCAACGTGTTCTCC TGTTCCGTCATGCACGAGGCACTGCACAATCATTACACCCAGAAGTCAC TGTCACTGTCACCAGGAAAx |
| | x = A or G | |
| 324 | TNX14 VH-CH1-CH2-CH3 IgG4 (L115T, S228P, L235A) (with terminal K) | ATGCCTCTGCTGCTGCTGCCTCTGCTGTGGGCCGGTG CTCTGGCTCAGGTCCAGCTGGTCCAGTCAGGGGCTGAAGT GGTCAAACCCGGCGCCAGCGTGAAGCTGTCTTGCAAGGCT TCCGGCTACATCTTCACCAGCTACTATATGTATTGGGTGA AGCAGGCTCCAGGACAGGGCCTGGAGTGGATCGGCGAGAT CAACCCTTCCAATGGCGACACAAACTTCAATGAGAAGTTT AAGAGCAAGGCCACCCTGACAGTGGATAAGAGCGCCTCTA CCGCTTACATGGAGCTGTCCAGCCTGAGGTCTGAGGACAC AGCCGTGTACTATTGTACCAGGAGCGATGGCCGGAACGAC ATGGATTCTTGGGGCCAGGGCACAACAGTGACCGTGTCTT CCGCCAGCACAAAGGGCCCTTCCGTGTTCCCCCTGGCTCC TTGCTCCAGGAGCACATCTGAGTCCACCGCCGCTCTGGGC TGTCTGGTGAAGGACTACTTCCCAGAGCCCGTGACCGTGT CCTGGAATAGCGGCGCCCTGACATCCGGAGTGCACACCTT TCCAGCTGTGCTGCAGAGCTCTGGCCTGTACAGCCTGTCC AGCGTGGTGACAGTGCCCTCTTCCAGCCTGGGCACCAAGA CATATACCTGCAACGTGGACCATAAGCCTTCTAATACCAA GGTGGATAAGAGAGTGGAGTCCAAGTACGGACCACCTTGC CCTCCTTGTCCTGCTCCAGAGTTCGCCGGAGGACCTTCCG TGTTCCTGTTTCCACCCAAGCCAAAGGACACACTGATGAT CTCTCGCACACCTGAGGTGACCTGCGTGGTGGTGGACGTG TCCCAGGAGGACCCCGAGGTGCAGTTCAACTGGTACGTGG ATGGCGTGGAGGTGCACAATGCTAAGACCAAGCCAAGGGA GGAGCAGTTTAACTCCACATACCGGGTGGTGAGCGTGCTG ACCGTGCTGCATCAGGATTGGCTGAACGGCAAGGAGTATA AGTGCAAGGTGAGCAATAAGGGCCTGCCCTCTTCCATCGA GAAGACAATCTCTAAGGCTAAGGGACAGCCAAGGGAGCCA CAGGTGTACACCCTGCCTCCAAGCCAGGAGGAGATGACAA AGAACCAGGTGTCTCTGACCTGTCTGGTGAAGGGCTTCTA TCCATCTGACATCGCTGTGGAGTGGGAGTCCAATGGCCAG CCCGAGAACAATTACAAGACCACACCCCCTGTGCTGGACT CTGATGGCTCCTTCTTTCTGTATTCTAGACTGACCGTGGA TAAGTCCCGCTGGCAGGAGGGCAACGTGTTCTCCTGCTCT GTGATGCACGAAGCACTGCACAATCATTACACTCAGAAGA GCCTGTCACTGTCCCTGGGCAAA |

| **Full linght chain (VL-CL)** | | |
|---|---|---|
| 195 | VL-CL protein seq with leader | MRLPAQLLGLLMLWVSGSSGDIVLTQSPATLSVSPGERATISCRASQRV SSSTYSYMHWYQQKPGQPPKLLIKYASNLESGVPARFSGSGSGTDFTLT ISSVEPEDFATYYCQHSWEIPPTFGGGTKLEIKRTVAAPSVFIFPPSDE QLKSGTASVVCLLNNFYPREAKVQWKVDNALQSGNSQESVTEQDSKDST YSLSSTLTLSKADYEKHKVYACEVTHQGLSSPVTKSFNRGEC |
| 196 | VL-CL protein seq without leader | DIVLTQSPATLSVSPGERATISCRASQRVSSSTYSYMHWYQQKPGQPPK LLIKYASNLESGVPARFSGSGSGTDFTLTISSVEPEDFATYYCQHSWEI PPTFGGGTKLEIKRTVAAPSVFIFPPSDEQLKSGTASVVCLLNNFYPRE AKVQWKVDNALQSGNSQESVTEQDSKDSTYSLSSTLTLSKADYEKHKVY ACEVTHQGLSSPVTKSFNRGEC |
| | | |
| 197 | VL-CL nucleotide seq with leader | ATGAGGCTGCCAGCTCAGCTGCTGGGACTGCTGATGCTGTGGGTGTCCG GCTCCAGCGGCGACATCGTGCTGACCCAGAGCCCAGCTACACTGTCCGT GTCCCCTGGAGAGAGGGCTACCATCAGCTGCAGGGCTTCTCAGCGGGTG TCTTCCAGCACATACAGCTATATGCACTGGTACCAGCAGAAGCCAGGCC AGCCCCCTAAGCTGCTGATCAAGTATGCCTCCAACCTGGAGAGCGGAGT GCCAGCTAGATTCAGCGGCTCTGGCTCCGGCACCGACTTTACCCTGACA ATCTCTTCCGTGGAGCCTGAGGATTTCGCCACATACTATTGCCAGCATT CTTGGGAGATCCCACCCACCTTTGGCGGCGGCACAAAGCTGGAGATCAA GAGAACCGTGGCCGCTCCTAGCGTGTTCATCTTTCCTCCATCTGACGAG CAGCTGAAGTCTGGCACCGCTTCCGTGGTGTGCCTGCTGAACAATTTCT ACCCACGCGAGGCCAAGGTGCAGTGGAAGGTGGATAACGCTCTGCAGAG CGGCAATTCTCAGGAGTCCGTGACCGAGCAGGACAGCAAGGATTCTACA TATTCCCTGAGCTCTACCCTGACACTGTCTAAGGCCGATTACGAGAAGC ACAAGGTGTATGCTTGCGAGGTGACCCATCAGGGCCTGTCCAGCCCAGT GACAAAGTCCTTTAATCGCGGCGAGTGT |
| 198 | VL-CL nucleotide seq without leader | CATCGTGCTGACCCAGAGCCCAGCTACACTGTCCGTGTCCCCTGGAGAG AGGGCTACCATCAGCTGCAGGGCTTCTCAGCGGGTGTCTTCCAGCACAT ACAGCTATATGCACTGGTACCAGCAGAAGCCAGGCCAGCCCCCTAAGCT GCTGATCAAGTATGCCTCCAACCTGGAGAGCGGAGTGCCAGCTAGATTC AGCGGCTCTGGCTCCGGCACCGACTTTACCCTGACAATCTCTTCCGTGG AGCCTGAGGATTTCGCCACATACTATTGCCAGCATTCTTGGGAGATCCC ACCCACCTTTGGCGGCGGCACAAAGCTGGAGATCAAGAGAACCGTGGCC GCTCCTAGCGTGTTCATCTTTCCTCCATCTGACGAGCAGCTGAAGTCTG GCACCGCTTCCGTGGTGTGCCTGCTGAACAATTTCTACCCACGCGAGGC CAAGGTGCAGTGGAAGGTGGATAACGCTCTGCAGAGCGGCAATTCTCAG GAGTCCGTGACCGAGCAGGACAGCAAGGATTCTACATATTCCCTGAGCT CTACCCTGACACTGTCTAAGGCCGATTACGAGAAGCACAAGGTGTATGC TTGCGAGGTGACCCATCAGGGCCTGTCCAGCCCAGTGACAAAGTCCTTT AATCGCGGCGAGTGT |

| **Linkers** | | |
|---|---|---|
| 199 | L1 | GGGGS |
| 200 | L2 | GGGGSGGGGS |
| 201 | L3 | GGGGSGGGGSGGGGS |
| 202 | L4 | GGGGSGGGGSGGGGSGGGGS |
| 203 | L5 | GGGGSGGGGSGGGGSGGGGSGGGGS |
| 204 | L6 | GSSGG |
| 205 | L7 | GSSGGGSSGG |
| 206 | L8 | GSSGGGSSGGGSSGG |
| 207 | L9 | GSSGGGSSGGGSSGGGSSGG |
| 208 | L10 | GSSGGGSSGGGSSGGGSSGGGSSGG |
| 209 | L11 | GGSGG |
| 210 | L12 | GGSGGGGSGG |
| 211 | L13 | GGSGGGGSGGGGSGG |
| 212 | L14 | GGSGGGGSGGGGSGGGGSGG |
| 213 | L15 | GGSGGGGSGGGGSGGGGSGGGGSGG |
| 214 | L16 | AS |
| 215 | L17 | AST |
| 216 | L18 | TVAAPS |
| 217 | L19 | TVA |
| 218 | L20 | ASTSGPS |
| 219 | L21 | KESGSVSSEQLAQFRSLD |
| 220 | L22 | EGKSSGSGSESKST |
| 221 | L23 | GGGGGG |
| 222 | L24 | GGGGGGGG |
| 223 | L25 | GSAGSAAGSGEF |
| 325 | CT-Long | EPKSSDK |
| 326 | CT-Long | ASTEPKSSDK |

| **Leader sequences** | | |
|---|---|---|
| 224 | Heavy chain leader sequence (AA) | MPLLLLLPLLWAGALA |
| 225 | Light chain leader sequence (AA) | MRLPAQLLGLLMLWVSGSSG |
| 226 | Heavy chain leader sequence (NA) | ATGCCACTGCTGCTGCTGCTGCCACTGCTGTGGGCTGGCGCTCTGGCT |
| 227 | Light chain leader sequence (NA) | ATGAGGCTGCCAGCTCAGCTGCTGGGACTGCTGATGCTGTGGGTGTCCG GCTCCAGCGGCGA |

| Misc sequences | | |
|---|---|---|
| 228 | EcoRI site sequence | GAATTCGGCCGGCCACC |
| 229 | BamHI site sequence | TAATGAACGCGTGGATCC |
| 230 | VD-CH2-CH3 (C220S, C226S, C229S, P238S) and terminal K | EVQLLESGGGLVQPGGSLRLSCAASGFTFNWELMGWARQAPGKGLEWVS GIEGPGDVTYYADSVKGRFTISRDNSKNTLYLQMNSLRAEDTAVYYCVK VGKDAKSDYRGQGTLVTVSSASTEPKSSDKTHTSPPSPAPELLGGSSVF LFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKA KGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHYT QKSLSLSPGK |
| | Full Ab | |
| 231 | VD-CH2-CH3 (C220S, C226S, C229S, P238S) and terminal K | APELLGGSSVFLFPPKPKDTLMISRTPEVTCVVVDVSHEDPEVKFNWYV DGVEVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCS VMHEALHNHYTQKSLSLSPGK |
| | Fc domain | |
| 232 | Fc region (C220S, C226S, C229S, P238S) and terminal K | EPKSSDKTHTSPPSPAPELLGGSSVFLFPPKPKDTLMISRTPEVTCVVV DVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK |
| | Fc region | |
| 233 | VH region | EVQLLESGGGLVQPGGSLRLSCAASGFTFNWELMGWARQAPGKGLEWVS GIEGPGDVTYYADSVKGRFTISRDNSKNTLYLQMNSLRAEDTAVYYCVK VGKDAKSDYRGQGTLVTVSS |
| 234 | TNX07 IgG4 heavy chain with K (S228P) | QVQLVQSGAEVVKPGASVKLSCKASGYIFTSYYMYWVKQAPGQGLEWIG EINPSNGDTNFNEKFKSKATLTVDKSASTAYMELSSLRSEDTAVYYCTR SDGRNDMDSWGQGTLVTVSSASTKGPSVFPLAPCSRSTSESTAALGCLV KDYFPEPVTVSWNSGALTSGVHTFPAVLQSSGLYSLSSVVTVPSSSLGT KTYTCNVDHKPSNTKVDKRVESKYGPPCPPCPAPEFLGGPSVFLFPPKP KDTLMISRTPEVTCVVVDVSQEDPEVQFNWYVDGVEVHNAKTKPREEQF NSTYRVVSVLTVLHQDWLNGKEYKCKVSNKGLPSSIEKTISKAKGQPRE PQVYTLPPSQEEMTKNQVSLTCLVKGFYPSDIAVEWESNGQPENNYKTT PPVLDSDGSFFLYSRLTVDKSRWQEGNVFSCSVMHEALHNHYTQKSLSL SLGK |
| 235 | TNX07 IgG4 Fc region (S228P) and terminal K | ESKYGPPCPPCPAPEFLGGPSVFLFPPKPKDTLMISRTPEVTCVVVDVS QEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSL TCLVKGFYPSDIAVEWESNGQPENNYKTTPPVLDSDGSFFLYSRLTVDK SRWQEGNVFSCSVMHEALHNHYTQKSLSLSLGK |
| 236 | IgG4 Fc region and terminal K | APEFLGGPSVFLFPPKPKDTLMISRTPEVTCVVVDVSQEDPEVQFNWYV DGVEVHNAKTKPREEQFNSTYRVVSVLTVLHQDWLNGKEYKCKVSNKGL PSSIEKTISKAKGQPREPQVYTLPPSQEEMTKNQVSLTCLVKGFYPSDI AVEWESNGQPENNYKTTPPVLDSDGSFFLYSRLTVDKSRWQEGNVFSCS VMHEALHNHYTQKSLSLSLGK |
| | Fc domain | |
| | | |
| Note that x = A or G in the listings above | | |

## Claims

1. An isolated antibody comprising a human or humanized variable domain, wherein the variable domain comprises a heavy chain variable region (VH) and a light chain variable region (VL), and wherein the VH is operably linked to a human Fc region derived from IgG4 and comprising amino acid modifications such that the modified Fc region is provided with modified effector functions.

2. The antibody of claim 1, wherein one or more effector functions are reduced or eliminated.

3. he antibody of claim 1 or claim 2, wherein the Fc region comprises an amino acid modification at any one of the positions selected from the group consisting of S228, L235, G237, E318, and N297 or a combination thereof, wherein the numbering of amino acid residues is according to the EU index as set forth in Edelman.

4. The antibody of claim 3, wherein the Fc region comprises an amino acid modification selected from the group consisting of S228P, F234A, L235A, L235E, G237A, E318A, and N297Q or a combination thereof.

5. The antibody of claim 4, wherein the Fc region comprises S228P and L235A amino acid modifications.

6. The antibody of any one of claims 1-5, wherein the Fc region comprises the amino acid sequence of SEQ ID NOs: 23 or 42.

7. The antibody of any one of claims 1-5, wherein the human Fc region comprises a human hinge sequence and a human Fc domain, wherein the human hinge sequence is between the VH and the human Fc domain; optionally
wherein the hinge comprises the amino acid sequence of any one of SEQ ID NOs: 76-90.

8. An isolated nucleic acid molecule encoding a light chain and a heavy chain of an antibody of any one of claims 1-7.

9. A first isolated nucleic acid molecule and a second isolated nucleic acid molecule, wherein the first isolated and second nucleic acid molecules encode, respectively a heavy chain and a light chain of an antibody of any one of claims 1-7.

10. A vector comprising the isolated nucleic acid molecule according to claim 8.

11. A first vector and a second vector, wherein the first vector comprises the first isolated nucleic acid molecule according to claim 9, and the second vector comprises the second isolated nucleic acid molecule according to claim 9.

12. A transformed cell comprising the isolated nucleic acid molecule according to claim 8, the first and second isolated nucleic acid molecules according to claim 9, the vector according to claim 10, or the first and second vectors according to claim 11.

13. A pharmaceutical composition comprising an antibody of any one of claims 1-7 and a pharmaceutically acceptable carrier.

14. An isolated antibody that binds to CD154 for use in treating an immune-related disease in a subject, wherein the antibody comprises a human or humanized variable domain, wherein the variable domain comprises a heavy chain variable region (VH) and a light chain variable region (VL), and wherein the VH is operably linked to a human Fc region derived from IgG4 and comprising S228P and L235A amino acid modifications; wherein the VH comprises
(a) a heavy chain CDR1 having the amino acid sequence of SEQ ID NO: 57,
(b) a heavy chain CDR2 having the amino acid sequence of SEQ ID NO: 58, and
(c) a heavy chain CDR3 having the amino acid sequence of SEQ ID NO: 59; and
wherein the VL comprises
(a) a light chain CDR1 having the amino acid sequence of SEQ ID NO: 60,
(b) a light chain CDR2 having the amino acid sequence of SEQ ID NO: 61, and
(c) a light chain CDR3 having the amino acid sequence of SEQ ID NO: 62.

15. The antibody for use of claim 14, wherein the immune-related disease is selected from a group consisting of: type I diabetes, juvenile diabetes, autoimmune diabetes, autoimmune hemolytic anemia, rheumatoid arthritis, systemic lupus erythematosus (SLE), psoriasis, multiple sclerosis, inflammatory bowel disease, Addison's disease, Crohn's disease, Graves' disease, Sjogren's syndrome, Hashimoto's thyroiditis, myasthenia gravis, vasculitis, pernicious anemia, celiac diseases, Guillain-Barre syndrome, ankylosing spondylitis, primary biliary cirrhosis, lupus nephritis, Goodpasture's disease, polymyositis, dermatomyositis, psoriasis, temporal arteritis, Churg-Strauss syndrome, transverse myelitis, thyroiditis, ulcerative colitis, sarcoidosis, hemolytic anemia, idiopathic thrombocytopenic purpura, neuromyelitis optica spectrum disorder, paroxysmal nocturnal hemoglobinuria, atypical hemolytic uremic syndrome, Behcet's disease, diabetic retinopathy (DR), diabetic macular edema (DME), wet age-related macular degeneration (AMD), and macular edema following retinal vein occlusion (MEfRVO).
